# EUROPEAN PATENT SPECIFICATION

(11) **EP 4 274 838 B1**
(45) Date of publication and mention of the grant of the patent: **25.09.2024**
(21) Application number: 22700425.6
(22) Date of filing: 10.01.2022
(51) Int. Cl.: C07K 7/08, C07K 14/00, A61K 47/64, A61P 35/00, A61P 37/02, A61K 38/00

(54) **BICYCLIC PEPTIDE LIGANDS SPECIFIC FOR NK CELLS**
FÜR NK-ZELLEN SPEZIFISCHE BICYCLISCHE PEPTIDLIGANDEN
LIGANDS PEPTIDIQUES BICYCLIQUES SPÉCIFIQUES DES CELLULES NK

(30) Priority: 08.01.2021 US 202163135339 P; 15.10.2021 US 202163262597 P
(43) Date of publication of application: 15.11.2023
(73) Proprietor: BicycleTx Limited, Cambridge CB21 6GS (GB)
(72) Inventor: KEEN, Nicholas, Cambridge CB21 6GS (GB); MUDD, Gemma, Cambridge CB21 6GS (GB); DUFORT, Fay, Cambridge CB21 6GS (GB); GAYNOR, Katie, Cambridge CB21 6GS (GB); CHEN, Liuhong, Cambridge CB21 6GS (GB); BRANDISH, Phil, Cambridge CB21 6GS (GB); LEITHEISER, Chris, Cambridge CB21 6GS (GB); UHLENBROICH, Sandra, Cambridge CB21 6GS (GB); REPASH, Liz, Cambridge CB21 6GS (GB); MCDONNELL, Kevin, Cambridge CB21 6GS (GB)
(74) Representative: J A Kemp LLP
(86) International application number: PCT/GB2022/050043
(87) International publication number: WO 2022/148974

(56) References cited:
- WO-A1-2016/050361
- WO-A1-2018/222987
- WO-A1-2019/025811
- WO-A1-2019/084060
- WO-A1-2019/162682
- WO-A1-2019/226617
- WO-A1-2020/128526
- DUFORT FAY ET AL: "Generation of a Bicycle NK-TICA(TM), a novel NK cell engaging molecule to enhance targeted tumor cytotoxicity", JOURNAL FOR IMMUNOTHERAPY OF CANCER, vol. 9, no. Suppl 2, 789, 10 November 2021 (2021-11-10), pages A824 - A824, XP055903491, Retrieved from the Internet <URL:https://jitc.bmj.com/content/jitc/9/Suppl_2/A824.full.pdf> DOI: 10.1136/jitc-2021-SITC2021.789

## Description

### FIELD OF THE INVENTION

The present invention relates to polypeptides which are covalently bound to molecular scaffolds such that two or more peptide loops are subtended between attachment points to the scaffold. In particular, the invention describes peptides which bind to natural killer (NK) cells. The invention also includes multimeric binding complexes comprising at least two of said bicyclic peptide ligands. The invention also includes pharmaceutical compositions comprising said peptide ligands or said multimeric binding complexes and the use of said peptide ligands, multimeric binding complexes and pharmaceutical compositions in preventing, suppressing or treating a disease or disorder mediated by natural killer (NK) cells, such as inflammatory disorders, autoimmune disease and cancer.

### BACKGROUND OF THE INVENTION

Cyclic peptides are able to bind with high affinity and specificity to protein targets and hence are an attractive molecule class for the development of therapeutics. In fact, several cyclic peptides are already successfully used in the clinic, as for example the antibacterial peptide vancomycin, the immunosuppressant drug cyclosporine or the anti-cancer drug octreotide (Driggers et al. (2008), Nat. Rev. Drug. Discov. 7(7), 608-24). Good binding properties result from a relatively large interaction surface formed between the peptide and the target as well as the reduced conformational flexibility of the cyclic structures. Typically, macrocycles bind to surfaces of several hundred square angstrom, as for example the cyclic peptide CXCR4 antagonist CVX15 (400 Å²; Wu et al. (2007), Science 330, 1066-71), a cyclic peptide with the Arg-Gly-Asp motif binding to integrin αVb3 (355 Å²) (Xiong et al. (2002), Science 296(5565), 151-5) or the cyclic peptide inhibitor upain-1 binding to urokinase-type plasminogen activator (603 Å²; Zhao et al. (2007), J. Struct. Biol. 160(1), 1-10).

Due to their cyclic configuration, peptide macrocycles are less flexible than linear peptides, leading to a smaller loss of entropy upon binding to targets and resulting in a higher binding affinity. The reduced flexibility also leads to locking target-specific conformations, increasing binding specificity compared to linear peptides. This effect has been exemplified by a potent and selective inhibitor of matrix metalloproteinase 8 (MMP-8) which lost its selectivity over other MMPs when its ring was opened (Cherney et al. (1998), J. Med. Chem. 41(11), 1749-51). The favourable binding properties achieved through macrocyclization are even more pronounced in multicyclic peptides having more than one peptide ring as for example in vancomycin, nisin and actinomycin.

Different research teams have previously tethered polypeptides with cysteine residues to a synthetic molecular structure (Kemp and McNamara (1985), J. Org. Chem; Timmerman et al. (2005), ChemBioChem). Meloen and co-workers had used tris(bromomethyl)benzene and related molecules for rapid and quantitative cyclisation of multiple peptide loops onto synthetic scaffolds for structural mimicry of protein surfaces (Timmerman et al. (2005), ChemBioChem). Methods for the generation of candidate drug compounds wherein said compounds are generated by linking cysteine containing polypeptides to a molecular scaffold as for example 1,1',1"-(1,3,5-triazinane-1,3,5-triyl)triprop-2-en-1-one (TATA) (Heinis etal.(2014) Angewandte Chemie, International Edition 53(6) 1602-1606).

Phage display-based combinatorial approaches have been developed to generate and screen large libraries of bicyclic peptides to targets of interest (Heinis et al. (2009), Nat. Chem. Biol. 5(7), 502-7 and WO 2009/098450). Briefly, combinatorial libraries of linear peptides containing three cysteine residues and two regions of six random amino acids (Cys-(Xaa)₆-Cys-(Xaa)₆-Cys) were displayed on phage and cyclised by covalently linking the cysteine side chains to a small molecule scaffold. WO 2019/226617 discloses multispecific antigen-binding constructs that are specific for a natural cytoxicity receptor present on the Nk cell surface.

### SUMMARY OF THE INVENTION

The scope of the invention is defined by the amended set of claims.

According to a first aspect of the invention, there is provided a peptide ligand specific for natural killer (NK) cells comprising a polypeptide comprising at least three reactive groups, separated by at least two loop sequences, and a molecular scaffold which forms covalent bonds with the reactive groups of the polypeptide such that at least two polypeptide loops are formed on the molecular scaffold.

According to a yet further aspect of the invention, there is provided a pharmaceutical composition comprising a peptide ligand as defined herein in combination with one or more pharmaceutically acceptable excipients.

According to a further aspect of the invention, there is provided a peptide ligand or pharmaceutical composition as defined herein for use in preventing, suppressing or treating a disease or disorder mediated by natural killer (NK) cells.

### BRIEF DESCRIPTION OF THE FIGURES

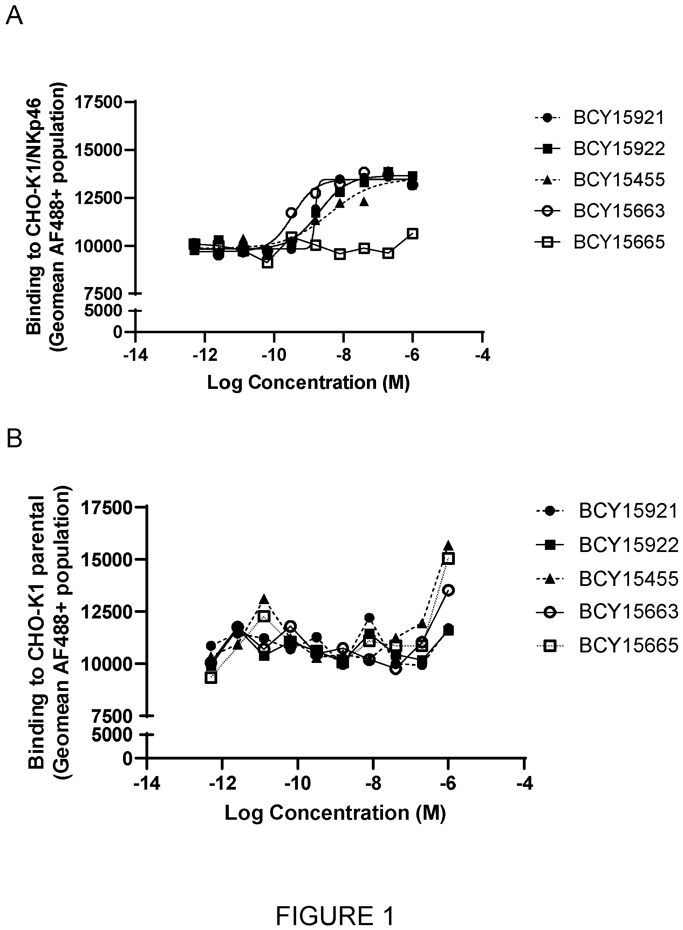

**Figure 1****:** Binding of Alexa Fluor^{®} 488 labelled NKp46 bicyclic peptide dimers to CHO-K1/NKp46 or CHO-K1 parental NKp46 null cell lines. (A) illustrates that Alexa Fluor^{®} 488-tagged NKp46 bicyclic peptide dimers bind in a dose-dependent manner to NKp46 expressing cells. No binding is observed in the CHO-K1 parental cell line (B), which is null for NKp46. Multiple AF488-NKp46 dimers were evaluated, shown in Table 4. BCY15665 is a non-binding bicyclic peptide dimer, comprised of all D-amino acids.

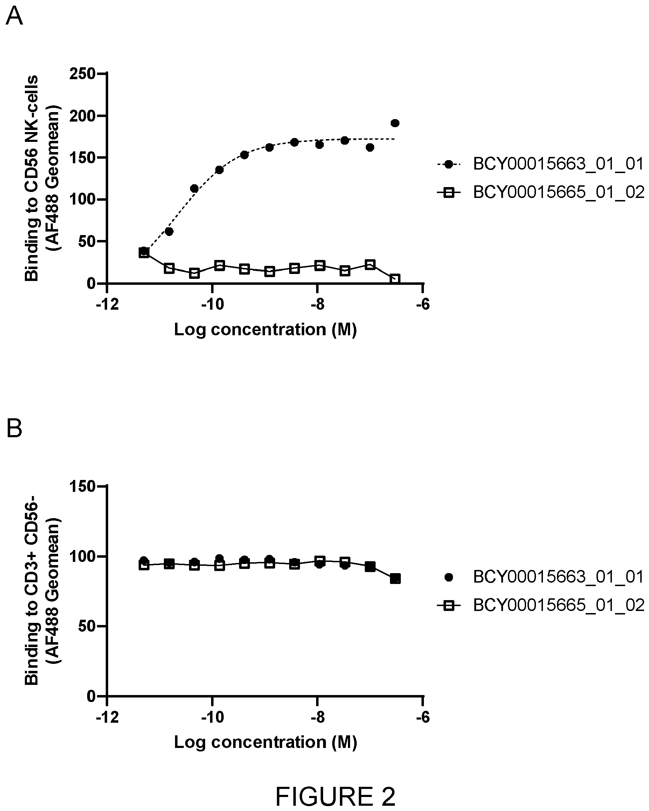

**Figure 2****:** Binding of Alexa Fluor^{®} 488 labelled NKp46 bicyclic peptide dimers to CD56+ (NK cells) or CD56- cells from human PBMCs. (A) Binding of Alexa Fluor^{®} 488 labelled NKp46 bicyclic peptide dimers to PBMC populations that express CD56 (NK cell population). (B) Cells in the PBMC population that do not express CD56 and that are CD3+ (T-cell population) elicit no binding. Data from a single representative PBMC donor (n=4). BCY15665 is a non-binding bicyclic peptide dimer, comprised of all D-amino acids.

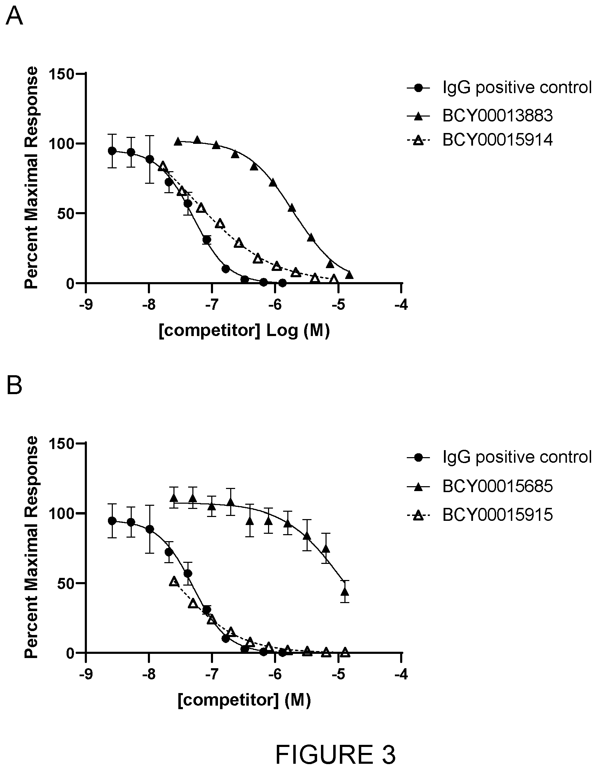

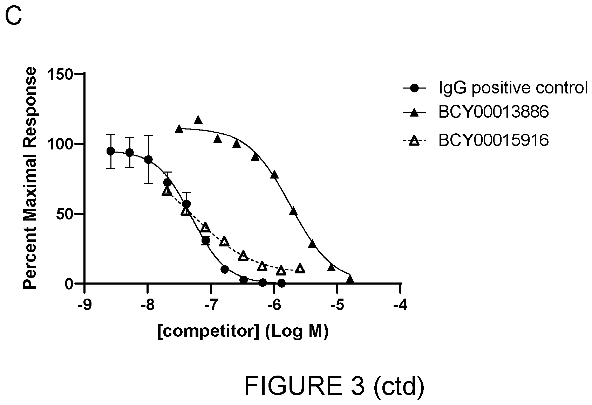

**Figure 3****:** IgG Competition Assay with C-terminally biotinylated human CD16a F176 variant (F Variant) using BCY15914 (A), BCY15915 (B) and BCY15916 (C).

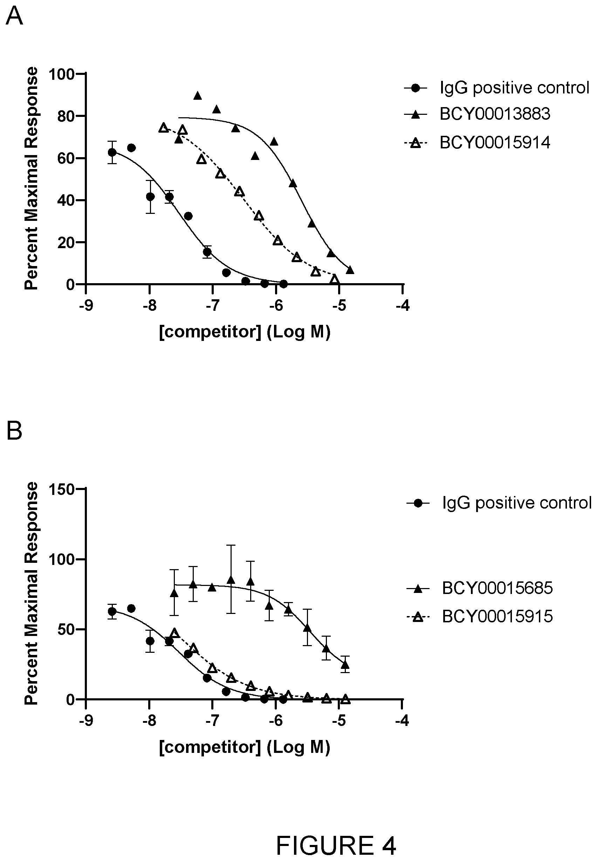

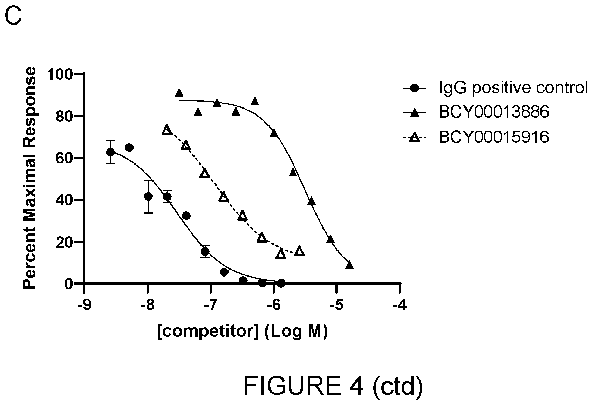

**Figure 4****:** IgG Competition Assay with N-terminally biotinylated human CD16a V176 variant (V Variant) using BCY15914 (A), BCY15915 (B) and BCY15916 (C).

### DETAILED DESCRIPTION OF THE INVENTION

According to a first aspect of the invention, there is provided a peptide ligand specific for natural killer (NK) cells comprising a polypeptide comprising at least three reactive groups, separated by at least two loop sequences, and a molecular scaffold which forms covalent bonds with the reactive groups of the polypeptide such that at least two polypeptide loops are formed on the molecular scaffold.

Natural killer (NK) cells are members of the innate immune system representing a small fraction of peripheral blood mononuclear cells. As frontline responders, these immune cells detect and eliminate unhealthy cells and bridge the innate immune response to the adaptive immune response. Due to their inherent properties, NK cells are an excellent candidate to enhance the therapeutic tools in immune oncology and autoimmunity.

NK cells are responsible for immune surveillance conducted through a variety of inhibitory and activating receptors. These activating and inhibitory receptors on the NK cellular surface are a complex means through which the activity of NK cells is kept in balance in healthy individuals. NK cells recognize the MHC class I molecules on the surface of healthy cells and are restrained through inhibitory receptors from eliminating these healthy cells. In times of stress, infection, or transformation, NK cells recognize the unhealthy cells through the loss of MHC class I on the cell surface and the induction of NK cell receptor ligands which bind to activating receptors. The recognition of non-self by the NK cells elicits a cytotoxic response, a release of cytokines and cytotoxic molecules for the elimination of the unhealthy cells.

NK cell activity is by a complex mechanism that involves both activating and inhibitory signals. Multiple reports have provided evidence for a central role of NK cell receptors in natural cytotoxicity and usefulness in the treatment of cancer. There is an unmet need for further understanding and enhancement of NK cell mediated recognition and killing of tumor cells. Reports suggest tumor cells utilize many mechanisms to reduce NK activity, and that NK cell presence and efficacy is associated with favorable prognosis in patients (Pasero et al. (2015) Oncotarget 6(16), 14360-14373, Stringaris et al. (2014) Haematologica 99(5), 836-847). It is through therapeutic intervention that one may harness the potential NK cells may play in mediating an immune response to combat cancer and autoimmune diseases.

### NKp46 Binding Bicyclic Peptides

In one embodiment, the bicyclic peptide is specific for (i.e. binds to) a natural cytotoxicity receptor present on the NK cell surface. In a further embodiment, the bicyclic peptide is specific for (i.e. binds to) a natural cytotoxicity receptor selected from NKp30, NKp44 and NKp46. In a yet further embodiment, the bicyclic peptide is specific for (i.e. binds to) NKp46.

The natural cytotoxicity receptors (NCR) are a family of stimulatory receptors expressed on the NK cell surface that elicit NK activation and cell-mediated cytotoxicity. The NCR family consists of three members, NKp30, NKp44, and NKp46. Although the cellular ligand for NKp46 is unknown, a role for NKp46 in antitumor immunity has been shown. Viral antigen-mediated NKp46 activation of NK cells results in tumor rejection (Chinnery et al. 2012). Upon interaction with its ligand, the NKp46 receptor triggers NK cells to induce directed cytotoxicity, illustrated by the use of anti-NKp46 blocking antibodies inhibiting the ability of NK cells to lyse targets (Arnon et al. 2004). The amount of NCR expression on the NK cell surface also increases NK cytotoxicity. A strong correlation between the density of NCR expression and the ability of NK cells to kill target cells, including a wide variety of tumor cells, has been identified (Moretta et al. 2006). In AML and in cervical cancer and precursor lesions, the insufficient amount of NCR or NCR ligands rendered tumor cells resistant to NK cytotoxicity (Costello et al. 2002, Garcia-Iglesias et al. 2009). In many solid tumors, NK cells are downregulated by the tumor microenvironment, among which include the tumor shedding of NCR ligands and immune editing, which prevent NK cells' ability to recognize, infiltrate, and kill the tumor cells (Nayyar 2019, Stojanovic et al. 2011, Sordo-Bahamonde et al. 2020, Watanabe et al. 2010, Izawa et al. 2011, Koo et al. 2013, Sun et al. 2015, Hasmim et al. 2015, Han et al. 2018). Stringaris et al. (2014) reported downregulation of NKp46, upregulation of NK cell inhibitory receptor NKG2A and low cytotoxic capacity of NK cells from AML patients. Furthermore, in solid cancer such as prostate cancer, there was reported a decreased expression of several activating receptors (CD16, NKp30, NKp46, NKG2D and DNAM-1), and an increase in the inhibitory receptor CD85j (Pesaro et al. 2016). In contrast, Gautheir et al. (2019) has identified NKp46 as a good candidate for the targeting of an activating receptor on NK cells in cancer, demonstrating no statistically significant downregulation of NKp46 in the periphery in SCCHN, breast, liver, lung, kidney, and metastatic melanoma cancer patients. Additionally, in multiple solid tumors sustained NKp46 expression, associated with the downregulation of other activating receptors, such as NKG2D, NKp30, and NKp44, and low CD16 expression on tumor infiltrating lymphocytes has been reported for cancers, such as acute myeloid leukemia, breast cancer, and lung carcinoma (Fauriat et al. 2007, Mamessier et al. 2011, Platonova et al. 2011, Levi et al. 2015, Kim et al. 2010, MacFarlane et al. 2017). Therefore, NKp46 has shown to be a specific NK surface marker suitable for therapeutic application to identify and targeting NK cells to tumors.

In a further embodiment, said loop sequences comprise 4, 5, 6 or 7 amino acids.

In one embodiment, said loop sequences comprise three cysteine residues separated by two loop sequences the first of which consists of 5 amino acids and the second of which consists of 4 amino acids.

In a further embodiment, said loop sequences comprise three cysteine residues separated by two loop sequences the first of which consists of 5 amino acids and the second of which consists of 4 amino acids and the bicyclic peptide ligand comprises an amino acid sequence which is selected from:
CᵢNLQKPCᵢᵢMKYPCᵢᵢᵢ (SEQ ID NO: 1); and
CᵢNLQNPCᵢᵢMKFPCᵢᵢᵢ (SEQ ID NO: 2);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three reactive groups separated by two loop sequences the first of which consists of 5 amino acids and the second of which consists of 4 amino acids, the molecular scaffold is TATA and the bicyclic peptide ligand additionally comprises N- and/or C-terminal additions and comprises an amino acid sequence which is selected from:
A-(SEQ ID NO: 1)-A-Sar₆-KFI (herein referred to as BCY14654);
A-(SEQ ID NO: 1)-A (herein referred to as BCY14456);
A-(SEQ ID NO: 2)-A-Sar₆-KFI (herein referred to as BCY15125);
A-(SEQ ID NO: 2)-A (herein referred to as BCY15102); and
A-(SEQ ID NO: 2)-A-[K(PYA)] (herein referred to as BCY18004),
wherein PYA represents pentynoic acid, Sar represents sarcosine and FI represents fluorescein.

In a still yet further embodiment, said loop sequences comprise three reactive groups separated by two loop sequences the first of which consists of 5 amino acids and the second of which consists of 4 amino acids, the molecular scaffold is TATA and the bicyclic peptide ligand additionally comprises N- and/or C-terminal additions and comprises an amino acid sequence which is selected from:
A-(SEQ ID NO: 1)-A-Sar₆-KFI (herein referred to as BCY14654); and
A-(SEQ ID NO: 2)-A-Sar₆-KFI (herein referred to as BCY15125),
wherein Sar represents sarcosine and FI represents fluorescein.

In an alternative embodiment, said loop sequences comprise three cysteine residues separated by two loop sequences the first of which consists of 5 amino acids and the second of which consists of 6 amino acids.

In a further embodiment, said loop sequences comprise three cysteine residues separated by two loop sequences the first of which consists of 5 amino acids and the second of which consists of 6 amino acids and the bicyclic peptide ligand comprises an amino acid sequence which is selected from:
CᵢNLQAPCᵢᵢMQTGKVCᵢᵢᵢ (SEQ ID NO: 3);
CᵢNLQAPCᵢᵢMLTGKVCᵢᵢᵢ (SEQ ID NO: 4; herein referred to as BCY18295 when complexed with TATA);
CᵢNLQQACᵢᵢLHTGKVCᵢᵢᵢ (SEQ ID NO: 63);
CᵢNLQAACᵢᵢMLTGKVCᵢᵢᵢ (SEQ ID NO: 64);
CᵢNLQAPCᵢᵢMATGKVCᵢᵢᵢ (SEQ ID NO: 65);
CᵢNLQAPCᵢᵢMLAGKVCᵢᵢᵢ (SEQ ID NO: 66);
CᵢNLQAPCᵢᵢMLTAKVCᵢᵢᵢ (SEQ ID NO: 67);
CᵢNLQAPCᵢᵢMLTGAVCᵢᵢᵢ (SEQ ID NO: 68);
CᵢNLQAPCᵢᵢMLTGKACᵢᵢᵢ (SEQ ID NO: 69);
CᵢN[tBuAla]QAPCᵢᵢMLTGKVCᵢᵢᵢ (SEQ ID NO: 70);
CᵢNLQA[Sar]CᵢᵢMLTGKVCᵢᵢᵢ (SEQ ID NO: 71);
CᵢNLQA[Cis-HyP]CᵢᵢMLTGKVCᵢᵢᵢ (SEQ ID NO: 72);
CᵢNLQA[HyP]CᵢᵢMLTGKVCᵢᵢᵢ (SEQ ID NO: 73);
CᵢNLQA[NMeAla]CᵢᵢMLTGKVCᵢᵢᵢ (SEQ ID NO: 74);
CᵢNLQAPCᵢᵢLLTGKVCᵢᵢᵢ (SEQ ID NO: 75);
CᵢNLQAPCᵢᵢ[Nle]LTGKVCᵢᵢᵢ (SEQ ID NO: 76);
CᵢNLQAPCᵢᵢ[Cba]LTGKVCᵢᵢᵢ (SEQ ID NO: 77);
CᵢNLQAPCᵢᵢMETGKVCᵢᵢᵢ (SEQ ID NO: 78);
CᵢNLQAPCᵢᵢMFTGKVCᵢᵢᵢ (SEQ ID NO: 79);
CᵢNLQAPCᵢᵢMYTGKVCᵢᵢᵢ (SEQ ID NO: 80);
CᵢNLQAPCᵢᵢM[Cba]TGKVCᵢᵢᵢ (SEQ ID NO: 81);
CᵢNLQAPCᵢᵢM[tBuAla]TGKVCᵢᵢᵢ (SEQ ID NO: 82);
CᵢNLQAPCᵢᵢM[Cha]TGKVCᵢᵢᵢ (SEQ ID NO: 83);
CᵢNLQAPCᵢᵢMLT[dA]KVCᵢᵢᵢ (SEQ ID NO: 84);
CᵢNLQAPCᵢᵢMLTGRVCᵢᵢᵢ (SEQ ID NO: 85);
CᵢNLQAPCᵢᵢMLTG[Agb]VCᵢᵢᵢ (SEQ ID NO: 86);
CᵢNLQAPCᵢᵢMLTGK[tBuGly]Cᵢᵢᵢ (SEQ ID NO: 87);
CᵢNLQAPCᵢᵢMLTGK[Chg]Cᵢᵢᵢ (SEQ ID NO: 88);
CᵢNLQAPCᵢᵢMLTGK[Cbg]Cᵢᵢᵢ (SEQ ID NO: 89);
CᵢNLQAPCᵢᵢMLTG[HArg]VCᵢᵢᵢ (SEQ ID NO: 90);
CᵢNLQA[Aze]CᵢᵢMLTGKVCᵢᵢᵢ (SEQ ID NO: 91);
CᵢNLQA[Pip]CᵢᵢMLTGKVCᵢᵢᵢ (SEQ ID NO: 92);
CᵢNLQAPCᵢᵢMVTGKVCᵢᵢᵢ (SEQ ID NO: 93);
CᵢNLQAPCᵢᵢM[Nle]TGKVCᵢᵢᵢ (SEQ ID NO: 94);
CᵢNLQAPCᵢᵢM[Nva]TGKVCᵢᵢᵢ (SEQ ID NO: 95);
CᵢNLQAPCᵢᵢMLTGK[Allolle]Cᵢᵢᵢ (SEQ ID NO: 96);
CᵢNLQAPCᵢᵢMLTGK[EPA]Cᵢᵢᵢ (SEQ ID NO: 97);
CᵢNLQQPCᵢᵢMLTGKVCᵢᵢᵢ (SEQ ID NO: 98);
CᵢNLQA[Nva]CᵢᵢMLTGKVCᵢᵢᵢ (SEQ ID NO: 99);
CᵢNLQAPCᵢᵢMHTGKVCᵢᵢᵢ (SEQ ID NO: 100);
CᵢNLQAPCᵢᵢLHTGKVCᵢᵢᵢ (SEQ ID NO: 101); and
CᵢNLQQACᵢᵢMQTGKVCᵢᵢᵢ (SEQ ID NO: 102);
wherein Cᵢ, Cᵢᵢ and Cᵢᵢᵢ represent first, second and third cysteine residues, respectively, and wherein Cba represents cyclobutylalanine, tBuAla represents β-t-Butyl-L-alanine, Sar represents sarcosine, Cis-HyP represents L-4-cis-hydroxyproline, HyP represents hydroxyproline, NMeAla represents N-methylalanine, Nle represents norleucine, dA represents D-alanine, Agb represents norarginine, tBuGly represents tert-butylglycine, Cha represents 3-cyclohexyl-L-alanine, Chg represents cyclohexylglycine, Cbg represents cyclobutylglycine, HArg represents homoarginie, Aze represents azetidine-2-carboxylic acid, Pip represents pipecolic acid, Nva represents norvaline, Allolle represents L-allo-isoleucine, and EPA represents diethyl-L-alanine, or a pharmaceutically acceptable salt thereof.

In a yet further embodiment, said loop sequences comprise three cysteine residues separated by two loop sequences the first of which consists of 5 amino acids and the second of which consists of 6 amino acids and the bicyclic peptide ligand comprises an amino acid sequence which is selected from:
CᵢNLQAPCᵢᵢMQTGKVCᵢᵢᵢ (SEQ ID NO: 3); and
CᵢNLQAPCᵢᵢMLTGKVCᵢᵢᵢ (SEQ ID NO: 4);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three reactive groups separated by two loop sequences the first of which consists of 5 amino acids and the second of which consists of 6 amino acids, the molecular scaffold is TATA and the bicyclic peptide ligand additionally comprises N- and/or C-terminal additions and comprises an amino acid sequence which is selected from:
A-(SEQ ID NO: 3)-A-[K(PYA)] (herein referred to as BCY15687);
A-(SEQ ID NO: 3)-A (herein referred to as BCY15098);
A-(SEQ ID NO: 4)-A-Sar₆-KFI (herein referred to as BCY15115);
A-(SEQ ID NO: 4)-A (herein referred to as BCY15099);
Ac-(SEQ ID NO: 4) (herein referred to as BCY18293);
Ac-A-(SEQ ID NO: 4)-A (herein referred to as BCY18294);
(SEQ ID NO: 4)-A (herein referred to as BCY19694);
K-(SEQ ID NO: 4)-A (herein referred to as BCY19695);
dK-(SEQ ID NO: 4)-A (herein referred to as BCY19696);
Dap-(SEQ ID NO: 4)-A (herein referred to as BCY19697);
Dab-(SEQ ID NO: 4)-A (herein referred to as BCY19698);
GABA-(SEQ ID NO: 4)-A (herein referred to as BCY19699);
A-(SEQ ID NO: 63)-A (herein referred to as BCY14454);
A-(SEQ ID NO: 64)-A (herein referred to as BCY18299);
A-(SEQ ID NO: 65)-A (herein referred to as BCY18301);
A-(SEQ ID NO: 66)-A (herein referred to as BCY18302);
A-(SEQ ID NO: 67)-A (herein referred to as BCY18303);
A-(SEQ ID NO: 68)-A (herein referred to as BCY18304);
A-(SEQ ID NO: 69)-A (herein referred to as BCY18305);
A-(SEQ ID NO: 70)-A (herein referred to as BCY18307);
A-(SEQ ID NO: 71)-A (herein referred to as BCY18309);
A-(SEQ ID NO: 72)-A (herein referred to as BCY18310);
A-(SEQ ID NO: 73)-A (herein referred to as BCY18311);
A-(SEQ ID NO: 74)-A (herein referred to as BCY18312);
A-(SEQ ID NO: 75)-A (herein referred to as BCY18313);
A-(SEQ ID NO: 76)-A (herein referred to as BCY18314);
A-(SEQ ID NO: 77)-A (herein referred to as BCY18315);
A-(SEQ ID NO: 78)-A (herein referred to as BCY18316);
A-(SEQ ID NO: 79)-A (herein referred to as BCY18317);
A-(SEQ ID NO: 80)-A (herein referred to as BCY18318);
A-(SEQ ID NO: 81)-A (herein referred to as BCY18319);
A-(SEQ ID NO: 82)-A (herein referred to as BCY18320);
A-(SEQ ID NO: 83)-A (herein referred to as BCY18321);
A-(SEQ ID NO: 84)-A (herein referred to as BCY18322);
A-(SEQ ID NO: 85)-A (herein referred to as BCY18323);
A-(SEQ ID NO: 86)-A (herein referred to as BCY18324);
A-(SEQ ID NO: 87)-A (herein referred to as BCY18325);
A-(SEQ ID NO: 88)-A (herein referred to as BCY18326);
A-(SEQ ID NO: 89)-A (herein referred to as BCY18327);
A-(SEQ ID NO: 90)-A (herein referred to as BCY18328);
A-(SEQ ID NO: 91)-A (herein referred to as BCY19700);
A-(SEQ ID NO: 92)-A (herein referred to as BCY19701);
A-(SEQ ID NO: 93)-A (herein referred to as BCY19702);
A-(SEQ ID NO: 94)-A (herein referred to as BCY19703);
A-(SEQ ID NO: 95)-A (herein referred to as BCY19704);
A-(SEQ ID NO: 96)-A (herein referred to as BCY19706);
A-(SEQ ID NO: 97)-A (herein referred to as BCY19707);
A-(SEQ ID NO: 98)-A (herein referred to as BCY19708);
A-(SEQ ID NO: 99)-A (herein referred to as BCY19710);
A-(SEQ ID NO: 100)-A (herein referred to as BCY19711);
A-(SEQ ID NO: 101)-A (herein referred to as BCY19712); and
A-(SEQ ID NO: 102)-A (herein referred to as BCY19713),
wherein PYA represents pentynoic acid, Sar represents sarcosine, FI represents fluorescein, dK represents D-lysine, Dap represents L-2,3-diaminopropionic acid, Dab represents L-2,4-diaminobutyric acid, and GABA represents γ-aminobutyric acid.

In a still yet further embodiment, said loop sequences comprise three reactive groups separated by two loop sequences the first of which consists of 5 amino acids and the second of which consists of 6 amino acids, the molecular scaffold is TATA and the bicyclic peptide ligand additionally comprises N- and/or C-terminal additions and comprises an amino acid sequence which is selected from:
A-(SEQ ID NO: 3)-A-[K(PYA)] (herein referred to as BCY15687); and
A-(SEQ ID NO: 4)-A-Sar₆-KFI (herein referred to as BCY15115),
wherein PYA represents pentynoic acid, Sar represents sarcosine and FI represents fluorescein.

In an alternative embodiment, said loop sequences comprise three cysteine residues separated by two loop sequences the first of which consists of 5 amino acids and the second of which consists of 7 amino acids.

In a further embodiment, said loop sequences comprise three cysteine residues separated by two loop sequences the first of which consists of 5 amino acids and the second of which consists of 7 amino acids and the bicyclic peptide ligand comprises an amino acid sequence which is selected from:
CᵢLLHDHCᵢᵢPNTHPKLCᵢᵢᵢ (SEQ ID NO: 5);
CᵢLLHEHCᵢᵢPNTHPKMCᵢᵢᵢ (SEQ ID NO: 6);
CᵢLLHDHCᵢᵢPNSHPEICᵢᵢᵢ (SEQ ID NO: 7);
CᵢLLHDHCᵢᵢPNTHPIICᵢᵢᵢ (SEQ ID NO: 8); and
CᵢLLHDHCᵢᵢPNTHPKMCᵢᵢᵢ (SEQ ID NO: 103);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three cysteine residues separated by two loop sequences the first of which consists of 5 amino acids and the second of which consists of 7 amino acids and the bicyclic peptide ligand comprises an amino acid sequence which is selected from:
CᵢLLHDHCᵢᵢPNTHPKLCᵢᵢᵢ (SEQ ID NO: 5);
CᵢLLHEHCᵢᵢPNTHPKMCᵢᵢᵢ (SEQ ID NO: 6);
CᵢLLHDHCᵢᵢPNSHPEICᵢᵢᵢ (SEQ ID NO: 7); and
CᵢLLHDHCᵢᵢPNTHPIICᵢᵢᵢ (SEQ ID NO: 8);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three reactive groups separated by two loop sequences the first of which consists of 5 amino acids and the second of which consists of 7 amino acids, the molecular scaffold is TATA and the bicyclic peptide ligand additionally comprises N- and/or C-terminal additions and comprises an amino acid sequence which is selected from:
A-(SEQ ID NO: 5)-A (herein referred to as BCY15103);
A-(SEQ ID NO: 5)-A-[K(PYA)] (herein referred to as BCY18005);
A-(SEQ ID NO: 5)-A-Sar₆-KFI (herein referred to as BCY15118);
A-(SEQ ID NO: 6)-A (herein referred to as BCY15104);
A-(SEQ ID NO: 6)-A-Sar₆-KFI (herein referred to as BCY15119);
A-(SEQ ID NO: 7)-A (herein referred to as BCY15105);
A-(SEQ ID NO: 7)-A-Sar₆-KFI (herein referred to as BCY15120);
A-(SEQ ID NO: 8)-A (herein referred to as BCY15106);
A-(SEQ ID NO: 8)-A-Sar₆-KFI (herein referred to as BCY15121); and
A-(SEQ ID NO: 103)-A (herein referred to as BCY14457),
wherein PYA represents pentynoic acid, Sar represents sarcosine and FI represents fluorescein.

In a still yet further embodiment, said loop sequences comprise three reactive groups separated by two loop sequences the first of which consists of 5 amino acids and the second of which consists of 7 amino acids, the molecular scaffold is TATA and the bicyclic peptide ligand additionally comprises N- and/or C-terminal additions and comprises an amino acid sequence which is selected from:
A-(SEQ ID NO: 5)-A-Sar₆-KFI (herein referred to as BCY15118);
A-(SEQ ID NO: 6)-A-Sar₆-KFI (herein referred to as BCY15119);
A-(SEQ ID NO: 7)-A-Sar₆-KFI (herein referred to as BCY15120); and
A-(SEQ ID NO: 8)-A-Sar₆-KFI (herein referred to as BCY15121),
wherein Sar represents sarcosine and FI represents fluorescein.

In an alternative embodiment, said loop sequences comprise three cysteine residues separated by two loop sequences the first of which consists of 6 amino acids and the second of which consists of 4 amino acids.

In a further embodiment, said loop sequences comprise three cysteine or penicillamine residues separated by two loop sequences the first of which consists of 6 amino acids and the second of which consists of 4 amino acids and the bicyclic peptide ligand comprises an amino acid sequence which is selected from:
CᵢYLPDWLCᵢᵢGDEYCᵢᵢᵢ (SEQ ID NO: 9);
CᵢYLPDYLCᵢᵢGDEYCᵢᵢᵢ (SEQ ID NO: 10);
CᵢDLTTHNCᵢᵢQWGICᵢᵢᵢ (SEQ ID NO: 11; herein referred to as BCY18262 when complexed with TATA);
CᵢYLPDYLCᵢᵢGDEYCᵢᵢᵢ (SEQ ID NO: 12; herein referred to as BCY15633 when complexed with TATA);
CᵢYLPDWLCᵢᵢGDEYCᵢᵢᵢ (SEQ ID NO: 13);
Cᵢ[2,6DiMeTyr]LPDYLCᵢᵢGDEYCᵢᵢᵢ (SEQ ID NO: 14);
CᵢY[tBuAla]PDYLCᵢᵢGDEYCᵢᵢᵢ (SEQ ID NO: 15);
CᵢY[Cba]PDYLCᵢᵢGDEYCᵢᵢᵢ (SEQ ID NO: 16);
CᵢYL[HyP]DYLCᵢᵢGDEYCᵢᵢᵢ (SEQ ID NO: 17);
CᵢYL[Aze]DYLCᵢᵢGDEYCᵢᵢᵢ (SEQ ID NO: 18);
CᵢYL[Aib]DYLCᵢᵢGDEYCᵢᵢᵢ (SEQ ID NO: 19);
CᵢYLPD[1NaI]LCᵢᵢGDEYCᵢᵢᵢ (SEQ ID NO: 20);
CᵢYLPD[2Nal]LCᵢᵢGDEYCᵢᵢᵢ (SEQ ID NO: 21);
CᵢYLPD[3Pal]LCᵢᵢGDEYCᵢᵢᵢ (SEQ ID NO: 22);
CᵢYLPD[2,6DiMeTyr]LCᵢᵢGDEYCᵢᵢᵢ (SEQ ID NO: 23);
CᵢYLPDW[tBuAla]CᵢᵢGDEYCᵢᵢᵢ (SEQ ID NO: 24);
CᵢYLPDW[Cba]CᵢᵢGDEYCᵢᵢᵢ (SEQ ID NO: 25);
CᵢYLPDYLCᵢᵢ[dA]DEYCᵢᵢᵢ (SEQ ID NO: 26);
CᵢYLPDYLCᵢᵢGDE[2,6DiMeTyr]Cᵢᵢᵢ (SEQ ID NO: 27);
CᵢALPDYLCᵢᵢGDEYCᵢᵢᵢ (SEQ ID NO: 28);
CᵢYAPDYLCᵢᵢGDEYCᵢᵢᵢ (SEQ ID NO: 29);
CᵢYLADYLCᵢᵢGDEYCᵢᵢᵢ (SEQ ID NO: 30);
CᵢYLPAYLCᵢᵢGDEYCᵢᵢᵢ (SEQ ID NO: 31);
CᵢYLPDALCᵢᵢGDEYCᵢᵢᵢ (SEQ ID NO: 32);
CᵢYLPDYACᵢᵢGDEYCᵢᵢᵢ (SEQ ID NO: 33);
CᵢYLPDYLCᵢᵢADEYCᵢᵢᵢ (SEQ ID NO: 34);
CᵢYLPDYLCᵢᵢGAEYCᵢᵢᵢ (SEQ ID NO: 35);
CᵢYLPDYLCᵢᵢGDAYCᵢᵢᵢ (SEQ ID NO: 36);
CᵢYLPDYLCᵢᵢGDEACᵢᵢᵢ (SEQ ID NO: 37);
CᵢY[C5A]PDYLCᵢᵢGDEYCᵢᵢᵢ (SEQ ID NO: 38);
CᵢY[Cha]PDYLCᵢᵢGDEYCᵢᵢᵢ (SEQ ID NO: 39);
CᵢY[Cba]PDYLCᵢᵢ[dA]DEYCᵢᵢᵢ (SEQ ID NO: 40);
CᵢYLPD[4FPhe]LCᵢᵢGDEYCᵢᵢᵢ (SEQ ID NO: 41);
CᵢYLPD[4MeOPhe]LCᵢᵢGDEYCᵢᵢᵢ (SEQ ID NO: 42);
CᵢYLPD[NO2Phe]LCᵢᵢGDEYCᵢᵢᵢ (SEQ ID NO: 43);
CᵢYLPD[4MePhe]LCᵢᵢGDEYCᵢᵢᵢ (SEQ ID NO: 44);
CᵢYLPD[pCoPhe]LCᵢᵢGDEYCᵢᵢᵢ (SEQ ID NO: 45);
CᵢYLPD[pCaPhe]LCᵢᵢGDEYCᵢᵢᵢ (SEQ ID NO: 46);
CᵢYLPD[4tBuPhe]LCᵢᵢGDEYCᵢᵢᵢ (SEQ ID NO: 47);
CᵢYLPD[CF3Phe]LCᵢᵢGDEYCᵢᵢᵢ (SEQ ID NO: 48);
CᵢYLPD[4CNPhe]LCᵢᵢGDEYCᵢᵢᵢ (SEQ ID NO: 49);
CᵢYLPD[4ClPhe]LCᵢᵢGDEYCᵢᵢᵢ (SEQ ID NO: 50);
CᵢYLPD[HPhe]LCᵢᵢGDEYCᵢᵢᵢ (SEQ ID NO: 51);
Cᵢ[2,6DiMeTyr]LPDWLCᵢᵢGDEYCᵢᵢᵢ (SEQ ID NO: 52);
CᵢY[Cba]PDWLCᵢᵢGDEYCᵢᵢᵢ (SEQ ID NO: 53);
CᵢYL[Aze]DWLCᵢᵢGDEYCᵢᵢᵢ (SEQ ID NO: 54);
CᵢYLPDWLCᵢᵢ[dA]DEYCᵢᵢᵢ (SEQ ID NO: 55);
CᵢSLPDYLCᵢᵢGDEYCᵢᵢᵢ (SEQ ID NO: 56);
CᵢDLTTHNCᵢᵢQWGICᵢᵢᵢ (SEQ ID NO: 57);
CᵢDLTTHNCᵢᵢLWGVCᵢᵢᵢ (SEQ ID NO: 58);
CᵢNLQLHNCᵢᵢlWGVCᵢᵢᵢ (SEQ ID NO: 59);
CᵢDLTTHNCᵢᵢlWGVCᵢᵢᵢ (SEQ ID NO: 104);
CᵢNLQLHNCᵢᵢlWGVCᵢᵢᵢ (SEQ ID NO: 105);
CᵢNLQLHNCᵢᵢQWGICᵢᵢᵢ (SEQ ID NO: 106);
Cᵢ[3tBuTyr]LPDYLCᵢᵢGDEYCᵢᵢᵢ (SEQ ID NO: 107);
CᵢYLPD[3tBuTyr]LCᵢᵢGDEYCᵢᵢᵢ (SEQ ID NO: 108);
CᵢYLPDYLCᵢᵢGDE[3tBuTyr]Cᵢᵢᵢ (SEQ ID NO: 109);
Cᵢ[K(PYA)]LPDYLCᵢᵢGDEYCᵢᵢᵢ (SEQ ID NO: 110);
CᵢYLP[K(PYA)]YLCᵢᵢGDEYCᵢᵢᵢ (SEQ ID NO: 111);
CᵢYLPDYLCᵢᵢ[K(PYA)]DEYCᵢᵢᵢ (SEQ ID NO: 112);
CᵢYLPDYLCᵢᵢGD[K(PYA)]YCᵢᵢᵢ (SEQ ID NO: 113);
CᵢYLPDYLCᵢᵢGDE[K(PYA)]Cᵢᵢᵢ (SEQ ID NO: 114);
CᵢYLPDYLCᵢᵢ[dK(PYA)]DEYCᵢᵢᵢ (SEQ ID NO: 115);
CᵢALTTHNCᵢᵢQWGICᵢᵢᵢ (SEQ ID NO: 116);
CᵢDLATHNCᵢᵢQWGICᵢᵢᵢ (SEQ ID NO: 117);
CᵢDLTAHNCᵢQWGICᵢᵢᵢ (SEQ ID NO: 118);
CᵢDLTTHNCᵢᵢAWGICᵢᵢᵢ (SEQ ID NO: 119);
CᵢDLTTHNCᵢᵢQWGACᵢᵢᵢ (SEQ ID NO: 120);
CᵢNLTTHNCᵢᵢQWGICᵢᵢᵢ (SEQ ID NO: 121);
CᵢDLQTHNCᵢᵢQWGICᵢᵢᵢ (SEQ ID NO: 122);
CᵢDLTLHNCᵢᵢQWGICᵢᵢᵢ (SEQ ID NO: 123);
CᵢDLTT[His3Me]NCᵢᵢQWGICᵢᵢᵢ (SEQ ID NO: 124);
CᵢDLTT[2Pal]NCᵢᵢQWGICᵢᵢᵢ (SEQ ID NO: 125);
CᵢDLTT[His1Me]NCᵢᵢQWGICᵢᵢᵢ (SEQ ID NO: 126);
CᵢDLTT[4ThiAz]NCᵢᵢQWGICᵢᵢᵢ (SEQ ID NO: 127);
CᵢDLTTHNCᵢᵢVWGICᵢᵢᵢ (SEQ ID NO: 128);
CᵢDLTTHNCᵢᵢ[Cba]WGICᵢᵢᵢ (SEQ ID NO: 129);
CᵢDLTTHNCᵢᵢQ[1Nal]GICᵢᵢᵢ (SEQ ID NO: 130);
CᵢDLTTHNCᵢᵢQ[Trp(Me)]GICᵢᵢᵢ (SEQ ID NO: 131);
CᵢDLTTHNCᵢᵢQWG[tBuGly]Cᵢᵢᵢ (SEQ ID NO: 132);
CᵢDLTTHNCᵢᵢQWG[tBuAla]Cᵢᵢᵢ (SEQ ID NO: 133);
CᵢDLTTHNCᵢᵢQWG[Chg]Cᵢᵢᵢ (SEQ ID NO: 134);
CᵢDLTTHNCᵢᵢQW[dA]ICᵢᵢᵢ (SEQ ID NO: 135);
CᵢS[Cba]PDYLCᵢᵢ[dA]DEYCᵢᵢᵢ (SEQ ID NO: 136)
Cᵢ[Hser][Cba]PDYLCᵢᵢ[dA]DEYCᵢᵢᵢ (SEQ ID NO: 137);
CᵢN[Cba]PDYLCᵢᵢ[dA]DEYCᵢᵢᵢ (SEQ ID NO: 138);
Cᵢ[4Pal][Cba]PDYLCᵢᵢ[dA]DEYCᵢᵢᵢ (SEQ ID NO: 139);
CᵢY[Cba][Cis-HyP]DYLCᵢᵢ[dA]DEYCᵢᵢᵢ (SEQ ID NO: 140);
CᵢY[Cba][NmeAla]DYLCᵢᵢ[dA]DEYCᵢᵢᵢ (SEQ ID NO: 141);
CᵢY[Cba]PNYLCᵢᵢ[dA]DEYCᵢᵢᵢ (SEQ ID NO: 142);
CᵢY[Cba]PQYLCᵢᵢ[dA]DEYCᵢᵢᵢ (SEQ ID NO: 143);
CᵢY[Cba]PDYLCᵢᵢ[dV]DEYCᵢᵢᵢ (SEQ ID NO: 144);
CᵢY[Cba]PDYLCᵢᵢ[dNva]DEYCᵢᵢᵢ (SEQ ID NO: 145);
CᵢY[Cba]PDYLCᵢᵢ[dF]DEYCᵢᵢᵢ (SEQ ID NO: 146);
CᵢY[Cba]PDYLCᵢᵢ[dCha]DEYCᵢᵢᵢ (SEQ ID NO: 147);
CᵢY[Cba]PDYLCᵢᵢ[dK(PYA)]DEYCᵢᵢᵢ (SEQ ID NO: 148);
CᵢY[Cba]PDYLCᵢᵢ[dNle]EYCᵢᵢᵢ (SEQ ID NO: 149);
CᵢY[Cba]PD[DOPA]LCᵢᵢ[dA]DEYCᵢᵢᵢ (SEQ ID NO: 150);
CᵢY[Cba]PD[2FTyr]LCᵢᵢ[dA]DEYCᵢᵢᵢ (SEQ ID NO: 151);
CᵢY[Cba]PDY[Nle]Cᵢᵢ[dA]DEYCᵢᵢᵢ (SEQ ID NO: 152);
[Pen]ᵢY[Cba]PDYLCᵢᵢ[dA]DEYCᵢᵢᵢ (SEQ ID NO: 153);
CᵢY[Cba]PDYL[Pen]ᵢᵢ[dA]DEYCᵢᵢᵢ (SEQ ID NO: 154);
CᵢY[Cba]PDYLCᵢᵢ[dA]DEY[Pen]ᵢᵢᵢ (SEQ ID NO: 155);
CᵢY[Cba]PDYLCᵢᵢ[dA]DEY[dCᵢᵢᵢ] (SEQ ID NO: 156);
[dCᵢ]Y[Cba]PDYLCᵢᵢ[dA]DEYCᵢᵢᵢ (SEQ ID NO: 157);
CᵢDLTTHNCᵢᵢQ[AzaTrp]GICᵢᵢᵢ (SEQ ID NO: 158);
CᵢDLTTHNCᵢᵢQ[2MeTrp]GICᵢᵢᵢ (SEQ ID NO: 159);
CᵢDLTTHNCᵢᵢQ[6MeTrp]GICᵢᵢᵢ (SEQ ID NO: 160);
CᵢDLTTHNCᵢᵢQ[7MeTrp]GICᵢᵢᵢ (SEQ ID NO: 161);
CᵢDLTTHNCᵢᵢQ[6FTrp]GICᵢᵢᵢ (SEQ ID NO: 162);
CᵢDLTTHNCᵢᵢQ[5FTrp]GICᵢᵢᵢ (SEQ ID NO: 163);
CᵢDLTTHNCᵢᵢQ[Dht]GICᵢᵢᵢ (SEQ ID NO: 164);
CᵢDLTT[2FuAla]NCᵢᵢQWGICᵢᵢᵢ (SEQ ID NO: 165);
CᵢDLTTHNCᵢᵢQWG[EPA]Cᵢᵢᵢ (SEQ ID NO: 166);
CᵢDLTTHNCᵢᵢQWG[Nle]Cᵢᵢᵢ (SEQ ID NO: 167); and
CᵢDLTTHNCᵢᵢQWG[Allolle]Cᵢᵢᵢ (SEQ ID NO: 168);
wherein Cᵢ, Penᵢ, Cᵢᵢ, Penᵢᵢ, Cᵢᵢᵢ and Penᵢᵢᵢ represent first, second and third reactive groups, respectively, wherein 2,6DiMeTyr represents 2,6-dimethyl-tyrosine, tBuAla represents t-butyl-alanine, Cba represents β-cyclobutylalanine, HyP represents hydroxyproline, Aze represents azetidine, Aib represents aminoisobutyric acid, 1Nal represents 1-naphthylalanine, 2Nal represents 2-naphthylalanine, 3Pal represents 3-pyridylalanine, C5A represents beta-cyclopentyl-L-alanine, Cha represents 3-cyclohexyl-L-alanine, 4Fphe represents 4-fluoro-L-phenylalanine, 4MeOPhe represents 4-methoxy-phenylalanine, NO2Phe represents 4-nitro-phenylalanine, 4MePhe represents 4-methyl-phenylalanine, pCoPhe represents para-carboxy-phenylalanine, pCaPhe represents p-carboxamido-phenylalanine, 4tBuPhe represents 4-tert-butyl-phenylalanine, CF3Phe represents 4-trifluoromethyl-phenylalanine, 4CNPhe represents 4-cyano-phenylalanine, 4CIPhe represents 4-chloro-phenylalanine, HPhe represents homophenylalanine, 3tBuTyr represents 3-tert-butyltyrosine, His3Me represents 3-methylhistidine, 2Pal represents 2-pyridylalanine, His1Me represents 1-methylhistidine, 4ThiAz represents beta-4-thiazol-L-ylalanine, Trp(Me) represents 1-methyltryptophan, tBuGly represents L-t-butylglycine, Chg represents cyclohexylglycine, Hser represents homoserine, NmeAla represents N-methylalanine, 4Pal represents 4-pyridylalanine, Cis-HyP represents 4-cis-hydroxyproline, dA represents D-Alanine, dV represents D-valine, dNva represents D-norvaline, dNle represents D-norleucine, dF represents D-phenylalanine, dCha represents 3-cyclohexyl-D-alanine, DOPA represents 3,4-Dihydroxyphenylalanine, 2FTyr represents 2-fluorotyrosine, Nle represents norleucine, dC represents D-cysteine, AzaTrp represents azatryptophan, 2MeTrp represents 2-methyltryptophan, 6MeTrp represents 6-methyltryptophan, 6FTrp represents 6-fluorotryptophan, 5FTrp represents 5-fluorotryptophan, Dht represents 2,3-Dihydrotryptophan, 2FuAla represents 2-furylalanine, Allolle represents L-allo-isoleucine or a pharmaceutically acceptable salt thereof.

In a yet further embodiment, said loop sequences comprise three cysteine residues separated by two loop sequences the first of which consists of 6 amino acids and the second of which consists of 4 amino acids and the bicyclic peptide ligand comprises an amino acid sequence which is selected from:
CᵢYLPDWLCᵢᵢGDEYCᵢᵢᵢ (SEQ ID NO: 9);
CᵢYLPDYLCᵢᵢGDEYCᵢᵢᵢ (SEQ ID NO: 10);
CᵢDLTTHNCᵢᵢQWGICᵢᵢᵢ (SEQ ID NO: 11);
CᵢYLPDYLCᵢᵢGDEYCᵢᵢᵢ (SEQ ID NO: 12; herein referred to as BCY15633 when complexed with TATA);
CᵢYLPDWLCᵢᵢGDEYCᵢᵢᵢ (SEQ ID NO: 13);
Cᵢ[2,6DiMeTyr]LPDYLCᵢᵢGDEYCᵢᵢᵢ (SEQ ID NO: 14);
CᵢY[tBuAla]PDYLCᵢᵢGDEYCᵢᵢᵢ (SEQ ID NO: 15);
CᵢY[Cba]PDYLCᵢᵢGDEYCᵢᵢᵢ (SEQ ID NO: 16);
CᵢYL[HyP]DYLCᵢᵢGDEYCᵢᵢᵢ (SEQ ID NO: 17);
CᵢYL[Aze]DYLCᵢᵢGDEYCᵢᵢᵢ (SEQ ID NO: 18);
CᵢYL[Aib]DYLCᵢᵢGDEYCᵢᵢᵢ (SEQ ID NO: 19);
CᵢYLPD[1Nal]LCᵢᵢGDEYCᵢᵢᵢ (SEQ ID NO: 20);
CᵢYLPD[2Nal]LCᵢᵢGDEYCᵢᵢᵢ (SEQ ID NO: 21);
CᵢYLPD[3Pal]LCᵢᵢGDEYCᵢᵢᵢ (SEQ ID NO: 22);
CᵢYLPD[2,6DiMeTyr]LCᵢᵢGDEYCᵢᵢᵢ (SEQ ID NO: 23);
CᵢYLPDW[tBuAla]CᵢᵢGDEYCᵢᵢᵢ (SEQ ID NO: 24);
CᵢYLPDW[Cba]CᵢᵢGDEYCᵢᵢᵢ (SEQ ID NO: 25);
CᵢYLPDYLCᵢᵢ[dA]DEYCᵢᵢᵢ (SEQ ID NO: 26);
CᵢYLPDYLCᵢᵢGDE[2,6DiMeTyr]Cᵢᵢᵢ (SEQ ID NO: 27);
CᵢALPDYLCᵢᵢGDEYCᵢᵢᵢ (SEQ ID NO: 28);
CᵢYAPDYLCᵢᵢGDEYCᵢᵢᵢ (SEQ ID NO: 29);
CᵢYLADYLCᵢᵢGDEYCᵢᵢᵢ (SEQ ID NO: 30);
CᵢYLPAYLCᵢᵢGDEYCᵢᵢᵢ (SEQ ID NO: 31);
CᵢYLPDALCᵢᵢGDEYCᵢᵢᵢ (SEQ ID NO: 32);
CᵢYLPDYACᵢᵢGDEYCᵢᵢᵢ (SEQ ID NO: 33);
CᵢYLPDYLCᵢᵢADEYCᵢᵢᵢ (SEQ ID NO: 34);
CᵢYLPDYLCᵢᵢGAEYCᵢᵢᵢ (SEQ ID NO: 35);
CᵢYLPDYLCᵢᵢGDAYCᵢᵢᵢ (SEQ ID NO: 36);
CᵢYLPDYLCᵢᵢGDEACᵢᵢᵢ (SEQ ID NO: 37);
CᵢY[C5A]PDYLCᵢᵢGDEYCᵢᵢᵢ (SEQ ID NO: 38);
CᵢY[Cha]PDYLCᵢᵢGDEYCᵢᵢᵢ (SEQ ID NO: 39);
CᵢY[Cba]PDYLCᵢᵢ[dA]DEYCᵢᵢᵢ (SEQ ID NO: 40);
CᵢYLPD[4Fphe]LCᵢᵢGDEYCᵢᵢᵢ (SEQ ID NO: 41);
CᵢYLPD[4MeOPhe]LCᵢᵢGDEYCᵢᵢᵢ (SEQ ID NO: 42);
CᵢYLPD[NO2Phe]LCᵢᵢGDEYCᵢᵢᵢ (SEQ ID NO: 43);
CᵢYLPD[4MePhe]LCᵢᵢGDEYCᵢᵢᵢ (SEQ ID NO: 44);
CᵢYLPD[pCoPhe]LCᵢᵢGDEYCᵢᵢᵢ (SEQ ID NO: 45);
CᵢYLPD[pCaPhe]LCᵢᵢGDEYCᵢᵢᵢ (SEQ ID NO: 46);
CᵢYLPD[4tBuPhe]LCᵢᵢGDEYCᵢᵢᵢ (SEQ ID NO: 47);
CᵢYLPD[CF3Phe]LCᵢᵢGDEYCᵢᵢᵢ (SEQ ID NO: 48);
CᵢYLPD[4CNPhe]LCᵢᵢGDEYCᵢᵢᵢ (SEQ ID NO: 49);
CᵢYLPD[4ClPhe]LCᵢᵢGDEYCᵢᵢᵢ (SEQ ID NO: 50);
CᵢYLPD[HPhe]LCᵢᵢGDEYCᵢᵢᵢ (SEQ ID NO: 51);
Cᵢ[2,6DiMeTyr]LPDWLCᵢᵢGDEYCᵢᵢᵢ (SEQ ID NO: 52);
CᵢY[Cba]PDWLCᵢᵢGDEYCᵢᵢᵢ (SEQ ID NO: 53);
CᵢYL[Aze]DWLCᵢᵢGDEYCᵢᵢᵢ (SEQ ID NO: 54);
CᵢYLPDWLCᵢᵢ[dA]DEYCᵢᵢᵢ (SEQ ID NO: 55);
CᵢSLPDYLCᵢᵢGDEYCᵢᵢᵢ (SEQ ID NO: 56);
CᵢDLTTHNCᵢᵢQWGICᵢᵢᵢ (SEQ ID NO: 57);
CᵢDLTTHNCᵢᵢLWGVCᵢᵢᵢ (SEQ ID NO: 58); and
CᵢNLQLHNCᵢᵢlWGVCᵢᵢᵢ (SEQ ID NO: 59);
wherein Cᵢ, Cᵢᵢ and Cᵢᵢᵢ represent first, second and third cysteine residues, respectively, wherein 2,6DiMeTyr represents 2,6-dimethyl-tyrosine, tBuAla represents t-butyl-alanine, Cba represents β-cyclobutylalanine, HyP represents hydroxyproline, Aze represents azetidine, Aib represents aminoisobutyric acid, 1Nal represents 1-naphthylalanine, 2Nal represents 2-naphthylalanine, 3Pal represents 3-pyridylalanine, C5A represents beta-cyclopentyl-L-alanine, Cha represents 3-cyclohexyl-L-alanine, 4Fphe represents 4-fluoro-L-phenylalanine, 4MeOPhe represents 4-methoxy-phenylalanine, NO2Phe represents 4-nitro-phenylalanine, 4MePhe represents 4-methyl-phenylalanine, pCoPhe represents para-carboxy-phenylalanine, pCaPhe represents p-carboxamido-phenylalanine, 4tBuPhe represents 4-tert-butyl-phenylalanine, CF3Phe represents 4-trifluoromethyl-phenylalanine, 4CNPhe represents 4-cyano-phenylalanine, 4CIPhe represents 4-chloro-phenylalanine, HPhe represents homophenylalanine, or a pharmaceutically aceptable salt thereof.

In a yet further embodiment, said loop sequences comprise three reactive groups separated by two loop sequences the first of which consists of 6 amino acids and the second of which consists of 4 amino acids, the molecular scaffold is TATA and the bicyclic peptide ligand additionally comprises N- and/or C-terminal additions and comprises an amino acid sequence which is selected from:
A-(SEQ ID NO: 9)-A-[dK(PYA)] (herein referred to as BCY15013);
A-(SEQ ID NO: 10)-A (herein referred to as BCY18470);
A-(SEQ ID NO: 10)-A-[dK(PYA)] (herein referred to as BCY15452);
A-(SEQ ID NO: 11)-A (herein referred to as BCY15107);Ac-(SEQ ID NO: 11) (herein referred to as BCY18260);
Ac-A-(SEQ ID NO: 11)-A (herein referred to as BCY18261);
A-(SEQ ID NO: 11)-[Aib] (herein referred to as BCY19717);
(SEQ ID NO: 11)-A (herein referred to as BCY19718);
A-(SEQ ID NO: 11)-A-[dK(PYA)] (herein referred to as BCY19719);
A-(SEQ ID NO: 11)-A-[K(PYA)] (herein referred to as BCY15686);
A-(SEQ ID NO: 12)-A (herein referred to as BCY15100);
Ac-(SEQ ID NO: 12) (herein referred to as BCY15634);
Ac-A-(SEQ ID NO: 12)-A (herein referred to as BCY15635);
dA-(SEQ ID NO: 12) (herein referred to as BCY16134;
A-(SEQ ID NO: 12)-A-Sar₆-KFI (herein referred to as BCY15116);
A-(SEQ ID NO: 13)-A (herein referred to as BCY14455);
(SEQ ID NO: 13)-Sar₆-KFI (herein referred to as BCY15023);
Ac-(SEQ ID NO: 13)-Sar₆-KFI (herein referred to as BCY15024);
A-(SEQ ID NO: 13)-A-Sar₆-KFI (herein referred to as BCY14652);
A-(SEQ ID NO: 14)-A (herein referred to as BCY15636);
A-(SEQ ID NO: 15)-A (herein referred to as BCY15638);
A-(SEQ ID NO: 16)-A (herein referred to as BCY15639);
A-(SEQ ID NO: 17)-A (herein referred to as BCY15640);
A-(SEQ ID NO: 18)-A (herein referred to as BCY15641);
A-(SEQ ID NO: 19)-A (herein referred to as BCY15642);
A-(SEQ ID NO: 20)-A (herein referred to as BCY15643);
A-(SEQ ID NO: 21)-A (herein referred to as BCY15644);
A-(SEQ ID NO: 22)-A (herein referred to as BCY15645);
A-(SEQ ID NO: 23)-A (herein referred to as BCY15646);
A-(SEQ ID NO: 24)-A (herein referred to as BCY15648);
A-(SEQ ID NO: 25)-A (herein referred to as BCY15649);
A-(SEQ ID NO: 26)-A (herein referred to as BCY15650);
A-(SEQ ID NO: 27)-A (herein referred to as BCY15651);
A-(SEQ ID NO: 28)-A (herein referred to as BCY15653);
A-(SEQ ID NO: 29)-A (herein referred to as BCY15654);
A-(SEQ ID NO: 30)-A (herein referred to as BCY15655);
A-(SEQ ID NO: 31)-A (herein referred to as BCY15656);
A-(SEQ ID NO: 32)-A (herein referred to as BCY15657);
A-(SEQ ID NO: 33)-A (herein referred to as BCY15658);
A-(SEQ ID NO: 34)-A (herein referred to as BCY15659);
A-(SEQ ID NO: 35)-A (herein referred to as BCY15660);
A-(SEQ ID NO: 36)-A (herein referred to as BCY15661);
A-(SEQ ID NO: 37)-A (herein referred to as BCY15662);
A-(SEQ ID NO: 38)-A (herein referred to as BCY16130);
A-(SEQ ID NO: 39)-A (herein referred to as BCY16131);
A-(SEQ ID NO: 40)-A (herein referred to as BCY16132);
Ac-(SEQ ID NO: 40) (herein referred to as BCY16133);
Ac-(SEQ ID NO: 40)-[K(PYA)] (herein referred to as BCY17224);
A-(SEQ ID NO: 41)-A (herein referred to as BCY16135);
A-(SEQ ID NO: 42)-A (herein referred to as BCY16136);
A-(SEQ ID NO: 43)-A (herein referred to as BCY16137);
A-(SEQ ID NO: 44)-A (herein referred to as BCY16138);
A-(SEQ ID NO: 45)-A (herein referred to as BCY16139);
A-(SEQ ID NO: 46)-A (herein referred to as BCY16140);
A-(SEQ ID NO: 47)-A (herein referred to as BCY16141);
A-(SEQ ID NO: 48)-A (herein referred to as BCY16142);
A-(SEQ ID NO: 49)-A (herein referred to as BCY16143);
A-(SEQ ID NO: 50)-A (herein referred to as BCY16144);
A-(SEQ ID NO: 51)-A (herein referred to as BCY16145);
A-(SEQ ID NO: 52)-A-Sar₆-KFI (herein referred to as BCY15025);
A-(SEQ ID NO: 53)-A-Sar₆-KFI (herein referred to as BCY15028);
A-(SEQ ID NO: 54)-A-Sar₆-KFI (herein referred to as BCY15030);
A-(SEQ ID NO: 55)-A-Sar₆-KFI (herein referred to as BCY15035);
A-(SEQ ID NO: 56)-A-Sar₆-KFI (herein referred to as BCY15117);
A-(SEQ ID NO: 57)-A-Sar₆-KFI (herein referred to as BCY15122);
A-(SEQ ID NO: 58)-A (herein referred to as BCY15108);
A-(SEQ ID NO: 58)-A-Sar₆-KFI (herein referred to as BCY15123);
A-(SEQ ID NO: 59)-A-Sar₆-KFI (herein referred to as BCY15124),
A-(SEQ ID NO: 104)-A (herein referred to as BCY14458);
A-(SEQ ID NO: 105)-A (herein referred to as BCY15109);
A-(SEQ ID NO: 106)-A (herein referred to as BCY15568);
A-(SEQ ID NO: 107)-A (herein referred to as BCY15637);
A-(SEQ ID NO: 108)-A (herein referred to as BCY15647);
A-(SEQ ID NO: 109)-A (herein referred to as BCY15652);
A-(SEQ ID NO: 110)-A (herein referred to as BCY16834);
A-(SEQ ID NO: 111)-A (herein referred to as BCY16837);
A-(SEQ ID NO: 112)-A (herein referred to as BCY16840);
A-(SEQ ID NO: 113)-A (herein referred to as BCY16842);
A-(SEQ ID NO: 114)-A (herein referred to as BCY16843);
A-(SEQ ID NO: 115)-A (herein referred to as BCY17662);
A-(SEQ ID NO: 116)-A (herein referred to as BCY18263);
A-(SEQ ID NO: 117)-A (herein referred to as BCY18265);
A-(SEQ ID NO: 118)-A (herein referred to as BCY18266);
A-(SEQ ID NO: 119)-A (herein referred to as BCY18269);
A-(SEQ ID NO: 120)-A (herein referred to as BCY18272);
A-(SEQ ID NO: 121)-A (herein referred to as BCY18273);
A-(SEQ ID NO: 122)-A (herein referred to as BCY18277);
A-(SEQ ID NO: 123)-A (herein referred to as BCY18278);
A-(SEQ ID NO: 124)-A (herein referred to as BCY18279);
A-(SEQ ID NO: 125)-A (herein referred to as BCY18280);
A-(SEQ ID NO: 126)-A (herein referred to as BCY18281);
A-(SEQ ID NO: 127)-A (herein referred to as BCY18282);
A-(SEQ ID NO: 128)-A (herein referred to as BCY18285);
A-(SEQ ID NO: 129)-A (herein referred to as BCY18286);
A-(SEQ ID NO: 130)-A (herein referred to as BCY18287);
A-(SEQ ID NO: 131)-A (herein referred to as BCY18288);
A-(SEQ ID NO: 132)-A (herein referred to as BCY18289);
A-(SEQ ID NO: 133)-A (herein referred to as BCY18290);
A-(SEQ ID NO: 134)-A (herein referred to as BCY18291);
A-(SEQ ID NO: 135)-A (herein referred to as BCY18292);
Ac-(SEQ ID NO: 136) (herein referred to as BCY18433);
Ac-(SEQ ID NO: 137) (herein referred to as BCY18434);
Ac-(SEQ ID NO: 138) (herein referred to as BCY18435);
Ac-(SEQ ID NO: 139) (herein referred to as BCY18437);
Ac-(SEQ ID NO: 140) (herein referred to as BCY18439);
Ac-(SEQ ID NO: 141) (herein referred to as BCY18440);
Ac-(SEQ ID NO: 142) (herein referred to as BCY18441);
Ac-(SEQ ID NO: 143) (herein referred to as BCY18442);
Ac-(SEQ ID NO: 144) (herein referred to as BCY18443);
Ac-(SEQ ID NO: 145) (herein referred to as BCY18444);
Ac-(SEQ ID NO: 146) (herein referred to as BCY19682);
Ac-(SEQ ID NO: 147) (herein referred to as BCY19683);
Ac-(SEQ ID NO: 148) (herein referred to as BCY19684);
Ac-(SEQ ID NO: 149) (herein referred to as BCY19685);
Ac-(SEQ ID NO: 150) (herein referred to as BCY19686);
Ac-(SEQ ID NO: 151) (herein referred to as BCY19687);
Ac-(SEQ ID NO: 152) (herein referred to as BCY19688);
Ac-(SEQ ID NO: 153) (herein referred to as BCY19689);
Ac-(SEQ ID NO: 154) (herein referred to as BCY19690);
Ac-(SEQ ID NO: 155) (herein referred to as BCY19691);
Ac-(SEQ ID NO: 156) (herein referred to as BCY19692);
Ac-(SEQ ID NO: 157) (herein referred to as BCY19693);
A-(SEQ ID NO: 158)-A (herein referred to as BCY19720);
A-(SEQ ID NO: 159)-A (herein referred to as BCY19721);
A-(SEQ ID NO: 160)-A (herein referred to as BCY19722);
A-(SEQ ID NO: 161)-A (herein referred to as BCY19723);
A-(SEQ ID NO: 162)-A (herein referred to as BCY19724);
A-(SEQ ID NO: 163)-A (herein referred to as BCY19725);
A-(SEQ ID NO: 164)-A (herein referred to as BCY19730);
A-(SEQ ID NO: 165)-A (herein referred to as BCY19732);
A-(SEQ ID NO: 166)-A (herein referred to as BCY19733);
A-(SEQ ID NO: 167)-A (herein referred to as BCY19734); and
A-(SEQ ID NO: 168)-A (herein referred to as BCY19735);
wherein Aib represents aminoisobutyric acid, PYA represents pentynoic acid, Sar represents sarcosine and FI represents fluorescein.

In a still yet further embodiment, said loop sequences comprise three reactive groups separated by two loop sequences the first of which consists of 6 amino acids and the second of which consists of 4 amino acids, the molecular scaffold is TATA and the bicyclic peptide ligand additionally comprises N- and/or C-terminal additions and comprises an amino acid sequence which is selected from:
A-(SEQ ID NO: 9)-A-[dK(PYA)] (herein referred to as BCY15013);
A-(SEQ ID NO: 10)-A-[dK(PYA)] (herein referred to as BCY15452);
A-(SEQ ID NO: 11)-A-[K(PYA)] (herein referred to as BCY15686);
A-(SEQ ID NO: 12)-A (herein referred to as BCY15100);
(SEQ ID NO: 12) (herein referred to as BCY15633);
Ac-(SEQ ID NO: 12) (herein referred to as BCY15634);
Ac-A-(SEQ ID NO: 12)-A (herein referred to as BCY15635);
dA-(SEQ ID NO: 12) (herein referred to as BCY16134;
A-(SEQ ID NO: 12)-A-Sar₆-KFI (herein referred to as BCY15116);
A-(SEQ ID NO: 13)-A (herein referred to as BCY14455);
(SEQ ID NO: 13)-Sar₆-KFI (herein referred to as BCY15023);
Ac-(SEQ ID NO: 13)-Sar₆-KFI (herein referred to as BCY15024);
A-(SEQ ID NO: 13)-A-Sar₆-KFI (herein referred to as BCY14652);
A-(SEQ ID NO: 14)-A (herein referred to as BCY15636);
A-(SEQ ID NO: 15)-A (herein referred to as BCY15638);
A-(SEQ ID NO: 16)-A (herein referred to as BCY15639);
A-(SEQ ID NO: 17)-A (herein referred to as BCY15640);
A-(SEQ ID NO: 18)-A (herein referred to as BCY15641);
A-(SEQ ID NO: 19)-A (herein referred to as BCY15642);
A-(SEQ ID NO: 20)-A (herein referred to as BCY15643);
A-(SEQ ID NO: 21)-A (herein referred to as BCY15644);
A-(SEQ ID NO: 22)-A (herein referred to as BCY15645);
A-(SEQ ID NO: 23)-A (herein referred to as BCY15646);
A-(SEQ ID NO: 24)-A (herein referred to as BCY15648);
A-(SEQ ID NO: 25)-A (herein referred to as BCY15649);
A-(SEQ ID NO: 26)-A (herein referred to as BCY15650);
A-(SEQ ID NO: 27)-A (herein referred to as BCY15651);
A-(SEQ ID NO: 28)-A (herein referred to as BCY15653);
A-(SEQ ID NO: 29)-A (herein referred to as BCY15654);
A-(SEQ ID NO: 30)-A (herein referred to as BCY15655);
A-(SEQ ID NO: 31)-A (herein referred to as BCY15656);
A-(SEQ ID NO: 32)-A (herein referred to as BCY15657);
A-(SEQ ID NO: 33)-A (herein referred to as BCY15658);
A-(SEQ ID NO: 34)-A (herein referred to as BCY15659);
A-(SEQ ID NO: 35)-A (herein referred to as BCY15660);
A-(SEQ ID NO: 36)-A (herein referred to as BCY15661);
A-(SEQ ID NO: 37)-A (herein referred to as BCY15662);
A-(SEQ ID NO: 38)-A (herein referred to as BCY16130);
A-(SEQ ID NO: 39)-A (herein referred to as BCY16131);
A-(SEQ ID NO: 40)-A (herein referred to as BCY16132);
Ac-(SEQ ID NO: 40) (herein referred to as BCY16133);
Ac-(SEQ ID NO: 40)-[K(PYA)] (herein referred to as BCY17224);
A-(SEQ ID NO: 41)-A (herein referred to as BCY16135);
A-(SEQ ID NO: 42)-A (herein referred to as BCY16136);
A-(SEQ ID NO: 43)-A (herein referred to as BCY16137);
A-(SEQ ID NO: 44)-A (herein referred to as BCY16138);
A-(SEQ ID NO: 45)-A (herein referred to as BCY16139);
A-(SEQ ID NO: 46)-A (herein referred to as BCY16140);
A-(SEQ ID NO: 47)-A (herein referred to as BCY16141);
A-(SEQ ID NO: 48)-A (herein referred to as BCY16142);
A-(SEQ ID NO: 49)-A (herein referred to as BCY16143);
A-(SEQ ID NO: 50)-A (herein referred to as BCY16144);
A-(SEQ ID NO: 51)-A (herein referred to as BCY16145);
A-(SEQ ID NO: 52)-A-Sar₆-KFI (herein referred to as BCY15025);
A-(SEQ ID NO: 53)-A-Sar₆-KFI (herein referred to as BCY15028);
A-(SEQ ID NO: 54)-A-Sar₆-KFI (herein referred to as BCY15030);
A-(SEQ ID NO: 55)-A-Sar₆-KFI (herein referred to as BCY15035);
A-(SEQ ID NO: 56)-A-Sar₆-KFI (herein referred to as BCY15117);
A-(SEQ ID NO: 57)-A-Sar₆-KFI (herein referred to as BCY15122);
A-(SEQ ID NO: 58)-A-Sar₆-KFI (herein referred to as BCY15123); and
A-(SEQ ID NO: 59)-A-Sar₆-KFI (herein referred to as BCY15124),
wherein PYA represents pentynoic acid, Sar represents sarcosine and FI represents fluorescein.

### CD16a Binding Bicyclic Peptides

In one embodiment, the bicyclic peptide is specific for (i.e. binds to) an Fc receptor present on the NK cell surface. In a further embodiment, the bicyclic peptide is specific for (i.e. binds to) a low-affinity Fc gamma receptor (FcγR) selected from FcγRIIA, FcγRIIB, FcγRIIC, FcγRIIIA, and FcγRIIIB. In a yet further embodiment, the bicyclic peptide is specific for (i.e. binds to) FcγRIIIA (also known as CD16a).

Fc receptors are expressed on the surface of many leukocytes. In humans, five classic low-affinity Fc gamma receptors (FcγRs) (FcγRIIA, FcyRIIB, FcyRIIC, FcγRIIIA, and FcγRIIIB) bind to the Fc portion of immunoglobulin G (IgG) and are mediators of inflammation via immune cell activation as well as inhibition (Muta et al. 1994, Ravetch et al. 2001). The FcγRIIIA (CD16a) is activated by engagement with the Fc portion of the IgG molecule and is critical for the antibody-dependent cell cytotoxicity (ADCC) process. ADCC is a mechanism in which antigen-specific antibodies direct NK cells to kill the antigen-expressing cancer cells (Arnould et al. 2006). Playing a vital role in the anti-tumour effects of IgG1 mAbs, several studies have shown that part of the anti-tumor effect of trastuzumab, a human IgG1 anti-human epidermal growth factor receptor 2 (HER-2) antibody, as well as the EGFR-antibody cetuximab in metastatic colorectal patients, is through ADCC (Zhang et al. 2007, 2020, Wu et al. 2003). Similar results can be described for rituximab, a chimeric IgG1 mAb for B-cell differentiation antigen CD20 (Manches et al. 2003, Clynes et al. 2000). The usefulness of CD16 expression in directing immune cells to promote tumour cell killing has been illustrated in overexpression studies. Through retroviral transduction of Ig-Fc, the expression of IgFc on the surface of B16 melanoma cells lead to tumor killing *in vivo* (Riddle et al. 2005). The role of FcyR engagement for directing NK cell to tumors has also been illustrated in studies whereby chimeric antigen receptor T cells express CD16 scFv and are directed to antibody coated tumor cells. The introduction of CD16-CarT in in vivo models to EGFR or CD20 tumor-bearing mice treated with cetuximab or rituximab enhanced immune cell targeting and ADCC killing thereby eradicating evasive tumors (Rataj et al. 2019, Caratelli et al. 2017).

The contributions of FcyR genes to autoimmune diseases have attracted substantial attention, and functional FcyR polymorphisms have been reported to play important roles in the pathogenesis of autoimmune diseases (Salmon et al. 2001, Morgan et al. 2003, Wu et al. 1997). FcγR-knockout mouse models indicate that both activating and inhibitory FcyRs influence the development of autoimmune diseases (Nabbe et al. 2003, Kleinau et al. 2000, Bolland et al. 2000). The variations in FcyR expression significantly affect IgG immune complex-mediated signal thresholds. Notably, proinflammatory and anti-inflammatory cytokines could modulate the expression levels of activating and inhibitory FcyRs that affect the threshold immune cell response to IgG immune complexes (Pricop et al. 2001, Boruchov et al. 2005). The FcγRIIIA and FcγRIIIB copy number variations have been associated with systemic lupus erythematosus (SLE) and rheumatoid arthritis (RA) in Taiwanese patients (Chen et al. 2014). A high FcγRIIIA copy number was demonstrated to be a risk factor for SLE. Higher frequencies of cytokine-producing FcγRIIIA-positive dendritic cells (DCs) were observed in SLE patients, particularly in those with active disease, suggesting that FcγRIIIA-mediated inflammatory cytokine production in DCs might contribute to disease pathogenesis (Henriques et al. 2012). It is thought that the higher density of activating FcyRIIIA on the surface of immune cells (NK cells, monocytes, DCs, macrophages, and subsets of T cells) could tip the delicate balance of immune responses toward intense inflammation, which may result in the development of SLE (Chen et al. 2014). A correlation has been demonstrated between a low FcγRIIIA copy number and low CD16A expression on NK cells, suggesting that FcγRIIIA copy number has physiologic implications in NK cell functions. Most importantly, FcγRIIIA deficiency is associated with 2 distinct autoimmune diseases (SLE and RA), suggesting that defective FcγRIIIA functions may represent a common risk factor for various autoimmune diseases. The disease associations suggest that modulation of FcγRIIIA function may be an important therapeutic target for lupus nephritis (Chen et al. 2014).

Therapeutic innovations have provided some hope as to a means to block inflammation in autoimmune disease through FcyR interactions. Previous studies support the potential of FcyR inhibition as a therapeutic strategy to inhibit immune complex-mediated events in autoimmune diseases (Clarkson et al. 1986, Flaherty et al. 2012). The IgG Fc fragment by itself has demonstrated good efficacy in animal models of RA and in humans with thrombocytopenia (ITP) or Kawasaki disease (Anthony et al. 2008, Debré M et al. 1993, Hsu et al. 1993). Multivalent Fc constructs have been shown to inhibit immune complex processes in murine models of thrombocytopenia and arthritis (Ortiz et al. 2016). Furthermore, the infusion of soluble FcyR3a and 2a can inhibit immune complex triggered inflammation in the murine lupus model (Li et al. 2014). The blockade of immune complex formation on NK cells is an avenue to explore for the decreased activation of inflammation and thus autoimmune diseases.

In a further embodiment, said loop sequences comprise 3, 4, 5, 6, 7 or 8 amino acids. In a yet further embodiment, said loop sequences comprise 3, 4, 5, 7 or 8 amino acids.

In one embodiment, said loop sequences comprise three cysteine residues separated by two loop sequences the first of which consists of 4 amino acids and the second of which consists of 8 amino acids.

In a further embodiment, said loop sequences comprise three cysteine residues separated by two loop sequences the first of which consists of 4 amino acids and the second of which consists of 8 amino acids and the bicyclic peptide ligand comprises an amino acid sequence which is:
CᵢTWPYCᵢᵢKSWGDVQDCᵢᵢᵢ (SEQ ID NO: 265);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three reactive groups separated by two loop sequences the first of which consists of 4 amino acids and the second of which consists of 8 amino acids, the molecular scaffold is TATB and the bicyclic peptide ligand additionally comprises N- and/or C-terminal additions and comprises an amino acid sequence which is selected from:
A-(SEQ ID NO: 265)-A (herein referred to as BCY15044); and
A-(SEQ ID NO: 265)-AGAAAE (herein referred to as BCY19416).

In an alternative embodiment, said loop sequences comprise three cysteine residues separated by two loop sequences the first of which consists of 5 amino acids and the second of which consists of 4 amino acids.

In a further embodiment, said loop sequences comprise three cysteine residues separated by two loop sequences the first of which consists of 5 amino acids and the second of which consists of 4 amino acids and the bicyclic peptide ligand comprises an amino acid sequence which is selected from:
CᵢPPTVYCᵢᵢHPLDCᵢᵢᵢ (SEQ ID NO: 60);
CᵢPPAVYCᵢᵢHPLDCᵢᵢᵢ (SEQ ID NO: 169);
CᵢPPEVFCᵢᵢHPLDCᵢᵢᵢ (SEQ ID NO: 170);
CᵢPPEVYCᵢᵢHPLDCᵢᵢᵢ (SEQ ID NO: 171);
CᵢPPEVWCᵢᵢHPLDCᵢᵢᵢ (SEQ ID NO: 172);
CᵢPPQVYCᵢᵢHPLDCᵢᵢᵢ (SEQ ID NO: 173); and
CᵢPPEVWCᵢᵢHPLDCᵢᵢᵢ (SEQ ID NO: 174);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three cysteine residues separated by two loop sequences the first of which consists of 5 amino acids and the second of which consists of 4 amino acids and the bicyclic peptide ligand comprises an amino acid sequence which is:
CᵢPPTVYCᵢᵢHPLDCᵢᵢᵢ (SEQ ID NO: 60);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three reactive groups separated by two loop sequences the first of which consists of 5 amino acids and the second of which consists of 4 amino acids, the molecular scaffold is TATA and the bicyclic peptide ligand additionally comprises N- and/or C-terminal additions and comprises an amino acid sequence which is selected from:
A-(SEQ ID NO: 60)-A (herein referred to as BCY11808);
A-(SEQ ID NO: 60)-A-[K(PYA)] (herein referred to as BCY15685);
A-(SEQ ID NO: 60)-A-[Sar6]-[KBiot] (herein referred to as BCY12621);
A-(SEQ ID NO: 169)-A (herein referred to as BCY11673);
A-(SEQ ID NO: 170)-A (herein referred to as BCY11809);
A-(SEQ ID NO: 171)-A (herein referred to as BCY11810);
A-(SEQ ID NO: 172)-A (herein referred to as BCY11811)
A-(SEQ ID NO: 173)-A (herein referred to as BCY11812); and
A-(SEQ ID NO: 174)-A-[Sar6]-[KBiot] (herein referred to as BCY12622);
wherein Sar represents sarcosine and Biot represents biotin.

In a still yet further embodiment, said loop sequences comprise three reactive groups separated by two loop sequences the first of which consists of 5 amino acids and the second of which consists of 4 amino acids, the molecular scaffold is TATA and the bicyclic peptide ligand additionally comprises N- and/or C-terminal additions and comprises an amino acid sequence which is:
A-(SEQ ID NO: 60)-A-[K(PYA)] (herein referred to as BCY15685).

In an alternative embodiment, said loop sequences comprise three cysteine residues separated by two loop sequences the first of which consists of 6 amino acids and the second of which consists of 3 amino acids.

In a further embodiment, said loop sequences comprise three cysteine residues separated by two loop sequences the first of which consists of 6 amino acids and the second of which consists of 3 amino acids and the bicyclic peptide ligand comprises an amino acid sequence which is:
CᵢDWWDPGCᵢᵢTYFCᵢᵢᵢ (SEQ ID NO: 266);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three reactive groups separated by two loop sequences the first of which consists of 6 amino acids and the second of which consists of 3 amino acids, the molecular scaffold is TATB and the bicyclic peptide ligand additionally comprises N- and/or C-terminal additions and comprises an amino acid sequence which is selected from:
A-(SEQ ID NO: 266)-A (herein referred to as BCY15045); and
A-(SEQ ID NO: 266)-AGAAAE (herein referred to as BCY19417).

In an alternative embodiment, said loop sequences comprise three cysteine residues separated by two loop sequences the first of which consists of 7 amino acids and the second of which consists of 3 amino acids.

In a further embodiment, said loop sequences comprise three cysteine residues separated by two loop sequences the first of which consists of 7 amino acids and the second of which consists of 3 amino acids and the bicyclic peptide ligand comprises an amino acid sequence which is selected from:
CᵢRWSVEDPCᵢᵢGAWCᵢᵢᵢ (SEQ ID NO: 61);
CᵢRWS[tBuGly]EDPCᵢᵢGAWCᵢᵢᵢ (SEQ ID NO: 175);
CᵢRWSVED[HyP]CᵢᵢGAWCᵢᵢᵢ (SEQ ID NO: 176);
CᵢRWSVED[Aze]CᵢᵢGAWCᵢᵢᵢ (SEQ ID NO: 177);
CᵢRWSVEDPCᵢᵢ[dA]AWCᵢᵢᵢ (SEQ ID NO: 178);
CᵢRWSVE[Gla]PCᵢᵢGAWCᵢᵢᵢ (SEQ ID NO: 179);
CᵢRWSVED[Aze]CᵢᵢGAWCᵢᵢᵢ (SEQ ID NO: 180);
CᵢMWIRSDPCᵢᵢGAWCᵢᵢᵢ (SEQ ID NO: 181);
CᵢRWYPDDPCᵢᵢGAWCᵢᵢᵢ (SEQ ID NO: 182);
CᵢEWHISEPCᵢᵢGAWCᵢᵢᵢ (SEQ ID NO: 183);
CᵢNWHVYEPCᵢᵢGAWCᵢᵢᵢ (SEQ ID NO: 184);
CᵢRWHFSEPCᵢᵢGAWCᵢᵢᵢ (SEQ ID NO: 185);
CᵢRWNLDDPCᵢᵢGAWCᵢᵢᵢ (SEQ ID NO: 186);
CᵢMWIRSDPCᵢᵢGAWCᵢᵢᵢ (SEQ ID NO: 187);
CᵢRWYPDDPCᵢᵢGAWCᵢᵢᵢ (SEQ ID NO: 188);
CᵢEWHISEPCᵢᵢGAWCᵢᵢᵢ (SEQ ID NO: 189);
CᵢNWHVYEPCᵢᵢGAWCᵢᵢᵢ (SEQ ID NO: 190);
CᵢRWHFSEPCᵢᵢGAWCᵢᵢᵢ (SEQ ID NO: 191);
CᵢRWNLDDPCᵢᵢGAWCᵢᵢᵢ (SEQ ID NO: 192);
CᵢR[2MeTrp]SVEDPCᵢᵢGAWCᵢᵢᵢ (SEQ ID NO: 193);
CᵢR[6MeTrp]SVEDPCᵢᵢGAWCᵢᵢᵢ (SEQ ID NO: 194);
CᵢR[6FTrp]SVEDPCᵢᵢGAWCᵢᵢᵢ (SEQ ID NO: 195);
CᵢR[5FTrp]SVEDPCᵢᵢGAWCᵢᵢᵢ (SEQ ID NO: 196);
CᵢR[6CITrp]SVEDPCᵢᵢGAWCᵢᵢᵢ (SEQ ID NO: 197);
CᵢR[Trp(S)]SVEDPCᵢᵢGAWCᵢᵢᵢ (SEQ ID NO: 198);
CᵢR[AzaTrp]SVEDPCᵢᵢGAWCᵢᵢᵢ (SEQ ID NO: 199);
CᵢRWSVEDPCᵢᵢGA[2MeTrp]Cᵢᵢᵢ (SEQ ID NO: 200);
CᵢRWSVEDPCᵢᵢGA[6FTrp]Cᵢᵢᵢ (SEQ ID NO: 201);
CᵢRWSVEDPCᵢᵢGA[5FTrp]Cᵢᵢᵢ (SEQ ID NO: 202);
CᵢRWSVEDPCᵢᵢGA[4MeoTrp]Cᵢᵢᵢ (SEQ ID NO: 203);
CᵢRWSVEDPCᵢᵢGA[5MeoTrp]Cᵢᵢᵢ (SEQ ID NO: 204);
CᵢRWSVEDPCᵢᵢGA[Trp(S)]Cᵢᵢᵢ (SEQ ID NO: 205);
CᵢRWSVEDPCᵢᵢGA[DOPA]Cᵢᵢᵢ (SEQ ID NO: 206);
CᵢRWYPDDPCᵢᵢGAWCᵢᵢᵢ (SEQ ID NO: 207);
CᵢRWHFSEPCᵢᵢGAWCᵢᵢᵢ (SEQ ID NO: 208);
CᵢR[2MeTrp]HFSEPCᵢᵢGAWCᵢᵢᵢ (SEQ ID NO: 209);
CᵢRWYFSEPCᵢᵢGAWCᵢᵢᵢ (SEQ ID NO: 210);
CᵢRWSFSEPCᵢᵢGAWCᵢᵢᵢ (SEQ ID NO: 211);
CᵢRWHPSEPCᵢᵢGAWCᵢᵢᵢ (SEQ ID NO: 212);
CᵢRWYPSEPCᵢᵢGAWCiii (SEQ ID NO: 213);
CᵢRWHPDEPCᵢᵢGAWCᵢᵢᵢ (SEQ ID NO: 214);
CᵢRWHPEEPCᵢᵢGAWCᵢᵢᵢ (SEQ ID NO: 215);
CᵢRWHFDEPCᵢᵢGAWCᵢᵢᵢ (SEQ ID NO: 216);
CᵢRWHFDDPCᵢᵢGAWCᵢᵢᵢ (SEQ ID NO: 217);
CᵢRWYFDEPCᵢᵢGAWCᵢᵢᵢ (SEQ ID NO: 218);
CᵢRW[His1Me]FSEPCᵢᵢGAWCᵢᵢᵢ (SEQ ID NO: 219);
CᵢRW[His3Me]FSEPCᵢᵢGAWCᵢᵢᵢ (SEQ ID NO: 220);
CᵢRW[4MeoPhe]FSEPCᵢᵢGAWCᵢᵢᵢ (SEQ ID NO: 221);
CᵢRW[DOPA]FSEPCᵢᵢGAWCᵢᵢᵢ (SEQ ID NO: 222);
CᵢRW[26DiMeTyr]FSEPCᵢᵢGAWCᵢᵢᵢ (SEQ ID NO: 223);
CᵢRW[3tBuTyr]FSEPCᵢᵢGAWCᵢᵢᵢ (SEQ ID NO: 224);
CᵢRW[4FPhe]FSEPCᵢᵢGAWCᵢᵢᵢ (SEQ ID NO: 225);
CᵢRWH[4MePhe]SEPCᵢᵢGAWCᵢᵢᵢ (SEQ ID NO: 226);
CᵢRWH[Aze]SEPCᵢᵢGAWCᵢᵢᵢ (SEQ ID NO: 227);
CᵢRWH[HyP]SEPCᵢᵢGAWCᵢᵢᵢ (SEQ ID NO: 228);
CᵢRWH[4tBuPhe]SEPCᵢᵢGAWCᵢᵢᵢ (SEQ ID NO: 229);
CᵢRWH[HPhe]SEPCᵢᵢGAWCᵢᵢᵢ (SEQ ID NO: 230, herein referred to as BCY21693 when complexed with TBMT);
CᵢRWH[4PhePro]SEPCᵢᵢGAWCᵢᵢᵢ (SEQ ID NO: 231);
CᵢRWHF[HSer]EPCᵢᵢGAWCᵢᵢᵢ (SEQ ID NO: 232);
CᵢRWHFS[Gla]PCᵢᵢGAWCᵢᵢᵢ (SEQ ID NO: 233);
CᵢRWHFS[HGlu]PCᵢᵢGAWCᵢᵢᵢ (SEQ ID NO: 234);
CᵢRWHFSEPCᵢᵢ[dA]AWCᵢᵢᵢ (SEQ ID NO: 235);
CᵢRWYFSE[Aze]Cᵢᵢ[dA]AWCᵢᵢᵢ (SEQ ID NO: 236);
CᵢRWHFDE[Aze]Cᵢᵢ[dA]AWCᵢᵢᵢ (SEQ ID NO: 237);
CᵢRWYPSE[Aze]Cᵢᵢ[dA]AWCᵢᵢᵢ (SEQ ID NO: 238);
Cᵢ[HArg]WHFSEPCᵢᵢGAWCᵢᵢᵢ (SEQ ID NO: 239);
CᵢRWH[Cis-HyP]SEPCᵢᵢGAWCᵢᵢᵢ (SEQ ID NO: 240);
CᵢRWHFSEPCᵢᵢGAWCᵢᵢᵢ (SEQ ID NO: 241);
CᵢRWHFSEPCᵢᵢGAWCᵢᵢᵢ (SEQ ID NO: 242);
CᵢRWHFSEPCᵢᵢGAWCᵢᵢᵢ (SEQ ID NO: 243);
CᵢRWH[4BnPro]SEPCᵢᵢGAWCᵢᵢᵢ (SEQ ID NO: 267); and
CᵢRWH[HPhe]SE[Aze]CᵢᵢGAWCᵢᵢᵢ (SEQ ID NO: 268);
wherein Cᵢ, Cᵢᵢ and Cᵢᵢᵢ represent first, second and third cysteine residues, respectively, wherein Gla represents γ-carboxyglutamic acid, Aze represents azetidine-2-carboxylic acid, 2MeTrp represents 2-methyltryptophan, 6MeTrp represents 6-methyltryptophan, 6FTrp represents 6-fluorotryptophan, 5FTrp represents 5-fluorotryptophan, 6CITrp represents 6-Chloro-L-tryptophan, 5MeoTrp represents 5-Methoxy-L-tryptophan, Trp(S) represents 3-(3-Benzothienyl)-L-alanine, AzaTrp represents azatryptophan, 4MeoTrp represents 4-Methoxy-L-tryptophan, DOPA represents 3,4-Dihydroxyphenylalanine, His1Me represents 1-methylhistidine, His3Me represents 3-methylhistidine, 4MeoPhe represents 4-Methoxyphenylalanine, 26DiMeTyr represents 2,6-Dimethyl-L-tyrosine, 3tBuTyr represents 3-tert-butyltyrosine, 4FPhe represents 4-fluoro-L-phenylalanine, 4MePhe represents 4-methyl-phenylalanine, HyP represents hydroxyproline, 4tBuPhe represents 4-t-butyl-phenylalanine, HPhe represents homophenylalanine, 4PhePro represents (2S,4S)-4-PhenylProline, HSer represents homoserine, HGIu represents L-2-aminoadipic acid, dA represents D-Alanine, HArg represents homoarginine, tBuGly represents tert-butylglycine, Cis-HyP represents 4-cis-hydroxyproline, and 4BnPro represents (2S,4R)-4-benzyl-pyrrolidine-2-carboxylic acid or a pharmaceutically acceptable salt thereof.

In a yet further embodiment, said loop sequences comprise three cysteine residues separated by two loop sequences the first of which consists of 7 amino acids and the second of which consists of 3 amino acids and the bicyclic peptide ligand comprises an amino acid sequence which is:
CᵢRWSVEDPCᵢᵢGAWCᵢᵢᵢ (SEQ ID NO: 61);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three reactive groups separated by two loop sequences the first of which consists of 7 amino acids and the second of which consists of 3 amino acids, the molecular scaffold is TBMT and the bicyclic peptide ligand additionally comprises N- and/or C-terminal additions and comprises an amino acid sequence which is selected from:
A-(SEQ ID NO: 61)-A (herein referred to as BCY14950);
Ac-A-(SEQ ID NO: 61)-A (herein referred to as BCY16599);
A-(SEQ ID NO: 61)-AGAAAE (herein referred to as BCY19040);
A-(SEQ ID NO: 61)-A-[K(PYA)] (herein referred to as BCY13883);
Ac-A-(SEQ ID NO: 61)-A-[K(PYA)] (herein referred to as BCY19596);
A-(SEQ ID NO: 175)-A (herein referred to as BCY14953);
A-(SEQ ID NO: 176)-A (herein referred to as BCY14954);
[PYA]-A-(SEQ ID NO: 177) (herein referred to as BCY14955);
A-(SEQ ID NO: 178)-A (herein referred to as BCY14956);
A-(SEQ ID NO: 179)-A (herein referred to as BCY16608);
A-(SEQ ID NO: 180)-A (herein referred to as BCY16610);
A-(SEQ ID NO: 181)-A (herein referred to as BCY19032);
A-(SEQ ID NO: 182)-A (herein referred to as BCY19033);
A-(SEQ ID NO: 183)-A (herein referred to as BCY19034);
A-(SEQ ID NO: 184)-A (herein referred to as BCY19035);
A-(SEQ ID NO: 185)-A (herein referred to as BCY19036);
A-(SEQ ID NO: 186)-A (herein referred to as BCY19037);
A-(SEQ ID NO: 187)-AGAAAE (herein referred to as BCY19038);
A-(SEQ ID NO: 188)-AGAAAE (herein referred to as BCY19039);
A-(SEQ ID NO: 189)-AGAAAE (herein referred to as BCY19041);
A-(SEQ ID NO: 190)-AGAAAE (herein referred to as BCY19042);
A-(SEQ ID NO: 191)-AGAAAE (herein referred to as BCY19043);
A-(SEQ ID NO: 192)-AGAAAE (herein referred to as BCY19044);
A-(SEQ ID NO: 193)-A (herein referred to as BCY19045);
A-(SEQ ID NO: 194)-A (herein referred to as BCY19046);
A-(SEQ ID NO: 195)-A (herein referred to as BCY19048);
A-(SEQ ID NO: 196)-A (herein referred to as BCY19049);
A-(SEQ ID NO: 197)-A (herein referred to as BCY19050);
A-(SEQ ID NO: 198)-A (herein referred to as BCY19053);
A-(SEQ ID NO: 199)-A (herein referred to as BCY19055);
A-(SEQ ID NO: 200)-A (herein referred to as BCY19057);
A-(SEQ ID NO: 201)-A (herein referred to as BCY19060);
A-(SEQ ID NO: 202)-A (herein referred to as BCY19061);
A-(SEQ ID NO: 203)-A (herein referred to as BCY19063);
A-(SEQ ID NO: 204)-A (herein referred to as BCY19064);
A-(SEQ ID NO: 205)-A (herein referred to as BCY19065);
A-(SEQ ID NO: 206)-A (herein referred to as BCY19068);
A-(SEQ ID NO: 207)-A-[K(PYA)] (herein referred to as BCY20360);
A-(SEQ ID NO: 208)-A-[K(PYA)] (herein referred to as BCY20361);
A-(SEQ ID NO: 209)-A (herein referred to as BCY20363);
A-(SEQ ID NO: 210)-A (herein referred to as BCY20364);
A-(SEQ ID NO: 211)-A (herein referred to as BCY20365);
A-(SEQ ID NO: 212)-A (herein referred to as BCY20366);
A-(SEQ ID NO: 213)-A (herein referred to as BCY20367);
A-(SEQ ID NO: 214)-A (herein referred to as BCY20368);
A-(SEQ ID NO: 215)-A (herein referred to as BCY20369);
A-(SEQ ID NO: 216)-A (herein referred to as BCY20370);
A-(SEQ ID NO: 217)-A (herein referred to as BCY20371);
A-(SEQ ID NO: 218)-A (herein referred to as BCY20372);
A-(SEQ ID NO: 219)-A (herein referred to as BCY20373);
A-(SEQ ID NO: 220)-A (herein referred to as BCY20374);
A-(SEQ ID NO: 221)-A (herein referred to as BCY20375);
A-(SEQ ID NO: 222)-A (herein referred to as BCY20376);
A-(SEQ ID NO: 223)-A (herein referred to as BCY20377);
A-(SEQ ID NO: 224)-A (herein referred to as BCY20378);
A-(SEQ ID NO: 225)-A (herein referred to as BCY20379);
A-(SEQ ID NO: 226)-A (herein referred to as BCY20380);
A-(SEQ ID NO: 227)-A (herein referred to as BCY20382);
A-(SEQ ID NO: 228)-A (herein referred to as BCY20383);
A-(SEQ ID NO: 229)-A (herein referred to as BCY20384);
A-(SEQ ID NO: 230)-A (herein referred to as BCY20385);
(SEQ ID NO: 230)-A (herein referred to as BCY21690);
Ac-(SEQ ID NO: 230)-A (herein referred to as BCY21691);
A-(SEQ ID NO: 230) (herein referred to as BCY21692);
Ac-(SEQ ID NO: 230) (herein referred to as BCY21694);
[Nle]-(SEQ ID NO: 230)-A (herein referred to as BCY21695);
Ac-[Nle]-(SEQ ID NO: 230)-A (herein referred to as BCY21696);
Ac-A-(SEQ ID NO: 230)-A (herein referred to as BCY21697);
A-(SEQ ID NO: 231)-A (herein referred to as BCY20386);
A-(SEQ ID NO: 232)-A (herein referred to as BCY20388);
A-(SEQ ID NO: 233)-A (herein referred to as BCY20389);
A-(SEQ ID NO: 234)-A (herein referred to as BCY20390);
A-(SEQ ID NO: 235)-A (herein referred to as BCY20392);
A-(SEQ ID NO: 236)-A (herein referred to as BCY20394);
A-(SEQ ID NO: 237)-A (herein referred to as BCY20395);
A-(SEQ ID NO: 238)-A (herein referred to as BCY20396);
A-(SEQ ID NO: 239)-A (herein referred to as BCY20397);
A-(SEQ ID NO: 240)-A (herein referred to as BCY20526);
Ac-A-(SEQ ID NO: 241)-A (herein referred to as BCY20527);
[Aib]-(SEQ ID NO: 242)-A (herein referred to as BCY20528);
A-(SEQ ID NO: 243)-[Aib] (herein referred to as BCY20529),
A-(SEQ ID NO: 267)-A (herein referred to as BCY20387); and
A-(SEQ ID NO: 268)-A (herein referred to as BCY21689);
wherein PYA represents pentynoic acid and Aib represents aminoisobutryic acid.

In a still yet further embodiment, said loop sequences comprise three reactive groups separated by two loop sequences the first of which consists of 7 amino acids and the second of which consists of 3 amino acids, the molecular scaffold is TBMT and the bicyclic peptide ligand additionally comprises N- and/or C-terminal additions and comprises an amino acid sequence which is:
A-(SEQ ID NO: 61)-A-[K(PYA)] (herein referred to as BCY13883),
wherein PYA represents pentynoic acid.

In an alternative embodiment, said loop sequences comprise three cysteine residues separated by two loop sequences the first of which consists of 8 amino acids and the second of which consists of 3 amino acids.

In a further embodiment, said loop sequences comprise three cysteine residues separated by two loop sequences the first of which consists of 8 amino acids and the second of which consists of 3 amino acids and the bicyclic peptide ligand comprises an amino acid sequence which is selected from:
CᵢVGLEELGPCᵢᵢSDLCᵢᵢᵢ (SEQ ID NO: 62; herein referred to as BCY16494 when complexed with TBMT);
CᵢVGLLELGPCᵢᵢSDLCᵢᵢᵢ (SEQ ID NO: 244);
CᵢPGLEELGPCᵢᵢSDLCᵢᵢᵢ (SEQ ID NO: 245);
CᵢV[dA]LEELGPCᵢᵢSDLCᵢᵢᵢ (SEQ ID NO: 246);
CᵢVG[tBuAla]EELGPCᵢᵢSDLCᵢᵢᵢ (SEQ ID NO: 247);
CᵢVG[Cba]EELGPCᵢᵢSDLCᵢᵢᵢ (SEQ ID NO: 248);
CᵢVGFEELGPCᵢᵢSDLCᵢᵢᵢ (SEQ ID NO: 249);
CᵢVG[1Nal]EELGPCᵢᵢSDLCᵢᵢᵢ (SEQ ID NO: 250);
CᵢVG[2Nal]EELGPCᵢᵢSDLCᵢᵢᵢ (SEQ ID NO: 251);
CᵢVGLPELGPCᵢᵢSDLCᵢᵢᵢ (SEQ ID NO: 252);
CᵢVGL[tBuAla]ELGPCᵢᵢSDLCᵢᵢᵢ (SEQ ID NO: 253);
CᵢVGL[Cba]ELGPCᵢᵢSDLCᵢᵢᵢ (SEQ ID NO: 254);
CᵢVGL[Cha]ELGPCᵢᵢSDLCᵢᵢᵢ (SEQ ID NO: 255);
CᵢVGL[Nle]ELGPCᵢᵢSDLCᵢᵢᵢ (SEQ ID NO: 256);
CᵢVGLE[Gla]LGPCᵢᵢSDLCᵢᵢᵢ (SEQ ID NO: 257);
CᵢVGLEELG[Aze]CᵢᵢSDLCᵢᵢᵢ (SEQ ID NO: 258);
CᵢVGLEELGPCᵢᵢSD[tBuAla]Cᵢᵢᵢ (SEQ ID NO: 259);
CᵢVGLEELGPCᵢᵢSD[Cba]Cᵢᵢᵢ (SEQ ID NO: 260);
CᵢVGLEELGPCᵢᵢSD[Cha]Cᵢᵢᵢ (SEQ ID NO: 261);
CᵢVG[HLeu]EELGPCᵢᵢSDLCᵢᵢᵢ (SEQ ID NO: 262);
CᵢVGL[HLeu]ELGPCᵢᵢSDLCᵢᵢᵢ (SEQ ID NO: 263); and
CᵢVGLE[HGlu]LGPCᵢᵢSDLCᵢᵢᵢ (SEQ ID NO: 264);
wherein Cᵢ, Cᵢᵢ and Cᵢᵢᵢ represent first, second and third cysteine residues, respectively, and wherein dA represents D-Alanine, tBuAla represents β-t-Butyl-L-alanine, Cba represents cyclobutylalanine, 1Nal represents 1-napthylalanine, 2Nal represents 2-naphthylalanine, Cha represents 3-cyclohexyl-D-alanine, Nle represents norleucine, Gla represents γ-carboxyglutamic acid, Aze represents azetidine-2-carboxylic acid, HLeu represents homoleucine, HGIu represents L-2-aminoadipic acid, or a pharmaceutically acceptable salt thereof.

In a yet further embodiment, said loop sequences comprise three cysteine residues separated by two loop sequences the first of which consists of 8 amino acids and the second of which consists of 3 amino acids and the bicyclic peptide ligand comprises an amino acid sequence which is:
CᵢVGLEELGPCᵢᵢSDLCᵢᵢᵢ (SEQ ID NO: 62);
wherein Cᵢ, Cᵢᵢ and Cᵢᵢᵢ represent first, second and third cysteine residues, respectively, or a pharmaceutically acceptable salt thereof.

In a yet further embodiment, said loop sequences comprise three reactive groups separated by two loop sequences the first of which consists of 8 amino acids and the second of which consists of 3 amino acids, the molecular scaffold is TBMT and the bicyclic peptide ligand additionally comprises N- and/or C-terminal additions and comprises an amino acid sequence which is selected from:
A-(SEQ ID NO: 62)-A (herein referred to as BCY16493);
Ac-(SEQ ID NO: 62) (herein referred to as BCY16495);
A-(SEQ ID NO: 62)-A-[K(PYA)] (herein referred to as BCY13886);
A-(SEQ ID NO: 244)-A-[K(PYA)] (herein referred to as BCY16165);
A-(SEQ ID NO: 245)-A (herein referred to as BCY16498);
A-(SEQ ID NO: 246)-A (herein referred to as BCY16499);
A-(SEQ ID NO: 247)-A (herein referred to as BCY16501);
A-(SEQ ID NO: 248)-A (herein referred to as BCY16502);
A-(SEQ ID NO: 249)-A (herein referred to as BCY16504);
A-(SEQ ID NO: 250)-A (herein referred to as BCY16505);
A-(SEQ ID NO: 251)-A (herein referred to as BCY16506);
A-(SEQ ID NO: 252)-A (herein referred to as BCY16507);
A-(SEQ ID NO: 253)-A (herein referred to as BCY16508);
A-(SEQ ID NO: 254)-A (herein referred to as BCY16509);
A-(SEQ ID NO: 255)-A (herein referred to as BCY16510);
A-(SEQ ID NO: 256)-A (herein referred to as BCY16511);
A-(SEQ ID NO: 257)-A (herein referred to as BCY16512);
A-(SEQ ID NO: 258)-A (herein referred to as BCY16520);
A-(SEQ ID NO: 259)-A (herein referred to as BCY16522);
A-(SEQ ID NO: 260)-A (herein referred to as BCY16523);
A-(SEQ ID NO: 261)-A (herein referred to as BCY16524);
A-(SEQ ID NO: 262)-A (herein referred to as BCY16527);
A-(SEQ ID NO: 263)-A (herein referred to as BCY16528); and
A-(SEQ ID NO: 264)-A (herein referred to as BCY16529),
wherein PYA represents pentynoic acid.

In a still yet further embodiment, said loop sequences comprise three reactive groups separated by two loop sequences the first of which consists of 8 amino acids and the second of which consists of 3 amino acids, the molecular scaffold is TBMT and the bicyclic peptide ligand additionally comprises N- and/or C-terminal additions and comprises an amino acid sequence which is:
A-(SEQ ID NO: 62)-A-[K(PYA)] (herein referred to as BCY13886);
wherein PYA represents pentynoic acid.

In a further embodiment, the pharmaceutically acceptable salt is selected from the free acid or the sodium, potassium, calcium or ammonium salt.

Unless defined otherwise, all technical and scientific terms used herein have the same meaning as commonly understood by those of ordinary skill in the art, such as in the arts of peptide chemistry, cell culture and phage display, nucleic acid chemistry and biochemistry. Standard techniques are used for molecular biology, genetic and biochemical methods (see Sambrook et al., Molecular Cloning: A Laboratory Manual, 3rd ed., 2001, Cold Spring Harbor Laboratory Press, Cold Spring Harbor, NY; Ausubel et al., Short Protocols in Molecular Biology (1999) 4th ed., John Wiley & Sons, Inc.), which are incorporated herein by reference.

### Numbering

When referring to amino acid residue positions within the peptides of the invention, reactive groups, i.e. cysteine or penicillamine residues (Cᵢ, Penᵢ, Cᵢᵢ, Penᵢᵢ and Cᵢᵢᵢ and Penᵢᵢᵢ) are omitted from the numbering as they are invariant, therefore, the numbering of amino acid residues within the peptides of the invention is referred to as below:
-Cᵢ-N₁-L₂-Q₃-A₄-P₅-Cᵢᵢ-M₆-Q₇-T₈-G₉-K₁₀-V₁₁-Cᵢᵢᵢ- (SEQ ID NO: 1).

For the purpose of this description, all bicyclic peptides are assumed to be cyclised with TATA, TATB or TBMT and yielding a tri-substituted structure. Cyclisation with TATA, TATB or TBMT occurs on the first, second and third reactive groups (i.e. Cᵢ, Cᵢᵢ, Cᵢᵢᵢ).

### Molecular Format

N- or C-terminal extensions to the bicycle core sequence are added to the left or right side of the sequence, separated by a hyphen. For example, an N-terminal biotin-G-Sar₅ tail would be denoted as:
[Biot]-G-[Sars]-A-(SEQ ID NO: X).

### Inversed Peptide Sequences

In light of the disclosure in Nair et al. (2003) J. Immunol. 170(3), 1362-1373, it is envisaged that the peptide sequences disclosed herein would also find utility in their retro-inverso form. For example, the sequence is reversed (i.e. N-terminus become C-terminus and *vice versa*) and their stereochemistry is likewise also reversed (i.e. D-amino acids become L-amino acids and *vice versa*).

### Peptide Ligand Definition

A peptide ligand, as referred to herein, refers to a peptide, peptidic or peptidomimetic covalently bound to a molecular scaffold. Typically, such peptides, peptidics or peptidomimetics comprise a peptide having natural or non-natural amino acids, two or more reactive groups (i.e. cysteine or penicillamine residues) which are capable of forming covalent bonds to the scaffold, and a sequence subtended between said reactive groups which is referred to as the loop sequence, since it forms a loop when the peptide, peptidic or peptidomimetic is bound to the scaffold. In the present case, the peptides, peptidics or peptidomimetics comprise at least three cysteine or penicillamine residues (referred to herein as Cᵢ, Penᵢ, Cᵢᵢ, Penᵢᵢ and Cᵢᵢᵢ and Penᵢᵢᵢ), and form at least two loops on the scaffold.

### Advantages of the Peptide Ligands

Certain bicyclic peptides of the present invention have a number of advantageous properties which enable them to be considered as suitable drug-like molecules for injection, inhalation, nasal, ocular, oral or topical administration. Such advantageous properties include:
- Species cross-reactivity. This is a typical requirement for preclinical pharmacodynamics and pharmacokinetic evaluation;
- Protease stability. Bicyclic peptide ligands should in most circumstances demonstrate stability to plasma proteases, epithelial ("membrane-anchored") proteases, gastric and intestinal proteases, lung surface proteases, intracellular proteases and the like. Protease stability should be maintained between different species such that a bicyclic peptide lead candidate can be developed in animal models as well as administered with confidence to humans;
- Desirable solubility profile. This is a function of the proportion of charged and hydrophilic versus hydrophobic residues and intra/inter-molecular H-bonding, which is important for formulation and absorption purposes; and
- An optimal plasma half-life in the circulation. Depending upon the clinical indication and treatment regimen, it may be required to develop a bicyclic peptide with short or prolonged *in vivo* exposure times for the management of either chronic or acute disease states. The optimal exposure time will be governed by the requirement for sustained exposure (for maximal therapeutic efficiency) versus the requirement for short exposure times to minimise toxicological effects arising from sustained exposure to the agent.

### Pharmaceutically Acceptable Salts

It will be appreciated that salt forms are within the scope of this invention, and references to peptide ligands include the salt forms of said ligands.

The salts of the present invention can be synthesized from the parent compound that contains a basic or acidic moiety by conventional chemical methods such as methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free acid or base forms of these compounds with the appropriate base or acid in water or in an organic solvent, or in a mixture of the two.

Acid addition salts (mono- or di-salts) may be formed with a wide variety of acids, both inorganic and organic. Examples of acid addition salts include mono- or di-salts formed with an acid selected from the group consisting of acetic, 2,2-dichloroacetic, adipic, alginic, ascorbic (e.g. L-ascorbic), L-aspartic, benzenesulfonic, benzoic, 4-acetamidobenzoic, butanoic, (+) camphoric, camphor-sulfonic, (+)-(1*S*)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, D-gluconic, glucuronic (e.g. D-glucuronic), glutamic (e.g. L-glutamic), α-oxoglutaric, glycolic, hippuric, hydrohalic acids (e.g. hydrobromic, hydrochloric, hydriodic), isethionic, lactic (e.g. (+)-L-lactic, (±)-DL-lactic), lactobionic, maleic, malic, (-)-L-malic, malonic, (±)-DL-mandelic, methanesulfonic, naphthalene-2-sulfonic, naphthalene-1,5-disulfonic, 1-hydroxy-2-naphthoic, nicotinic, nitric, oleic, orotic, oxalic, palmitic, pamoic, phosphoric, propionic, pyruvic, L-pyroglutamic, salicylic, 4-amino-salicylic, sebacic, stearic, succinic, sulfuric, tannic, (+)-L-tartaric, thiocyanic, p-toluenesulfonic, undecylenic and valeric acids, as well as acylated amino acids and cation exchange resins.

One particular group of salts consists of salts formed from acetic, hydrochloric, hydriodic, phosphoric, nitric, sulfuric, citric, lactic, succinic, maleic, malic, isethionic, fumaric, benzenesulfonic, toluenesulfonic, sulfuric, methanesulfonic (mesylate), ethanesulfonic, naphthalenesulfonic, valeric, propanoic, butanoic, malonic, glucuronic and lactobionic acids. One particular salt is the hydrochloride salt. Another particular salt is the acetate salt.

If the compound is anionic, or has a functional group which may be anionic (e.g. -COOH may be -COO⁻), then a salt may be formed with an organic or inorganic base, generating a suitable cation. Examples of suitable inorganic cations include, but are not limited to, alkali metal ions such as Li⁺, Na⁺ and K⁺, alkaline earth metal cations such as Ca²⁺ and Mg²⁺, and other cations such as Al³⁺ or Zn⁺. Examples of suitable organic cations include, but are not limited to, ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: methylamine, ethylamine, diethylamine, propylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

Where the peptides of the invention contain an amine function, these may form quaternary ammonium salts, for example by reaction with an alkylating agent according to methods well known to the skilled person. Such quaternary ammonium compounds are within the scope of the peptides of the invention.

### Multimeric Binding Complexes

According to a further aspect of the invention, there is provided a multimeric binding complex which comprises at least two bicyclic peptide ligands as defined herein, wherein said peptide ligands may be the same or different.

In one embodiment, the multimeric binding complexes comprise more than one bicyclic peptide which are the same (i.e. homomultimers). In an alternative embodiment, the multimeric binding complexes comprise bicyclic peptides which are different (i.e. heteromultimers).

In one embodiment, the multimeric binding complex additionally comprises a fluorophore.

In one embodiment, the multimeric binding complex comprises two bicyclic peptides which are the same (i.e. homodimers). Examples of suitable dimeric NKp46 homodimers are shown in Table A below:

**Table A: Dimeric NKp46 Multimeric Binding Complexes of the Invention**

| ***Complex No.*** | ***NKp46 BCY No.*** | ***Attachment Point*** | ***Linker*** | ***Fluorophore*** |
|---|---|---|---|---|
| BCY15663 | BCY15452 | C-term D-Lys(PYA) | N-(amine-PEG₂)-N-bis(PEG₃-azide) | AF488 |
| BCY15921 | BCY15686 | C-term Lys(PYA) | N-(amine-PEG₂)-N-bis(PEG₃-azide) | AF488 |
| BCY15922 | BCY15687 | C-term Lys(PYA) | N-(amine-PEG₂)-N-bis(PEG₃-azide) | AF488 |
| BCY15455 | BCY15013 | C-term D-Lys(PYA) | N-(amine-PEG₂)-N-bis(PEG₃-azide) | AF488 |
| BCY18043 | BCY18004 | C-term Lys(PYA) | N-(amine-PEG₂)-N-bis(PEG₃-azide) | AF488 |

Examples of suitable dimeric CD16a homodimers are shown in Table B below:

**Table B: Dimeric CD16a Multimeric Binding Complexes of the Invention**

| ***Complex No.*** | ***CD16a BCY No.*** | ***Attachment Point*** | ***Linker*** | ***Fluorophore*** |
|---|---|---|---|---|
| BCY15914 | BCY13883 | C-term Lys(PYA) | N-(amine-PEG₂)-N-bis(PEG₃-azide) | AF488 |
| BCY15915 | BCY15685 | C-term Lys(PYA) | N-(amine-PEG₂)-N-bis(PEG₃-azide) | AF488 |
| BCY15916 | BCY13886 | C-term Lys(PYA) | N-(amine-PEG₂)-N-bis(PEG₃-azide) | AF488 |

### Modified Derivatives

It will be appreciated that modified derivatives of the peptide ligands as defined herein are within the scope of the present invention. Examples of such suitable modified derivatives include one or more modifications selected from: N-terminal and/or C-terminal modifications; replacement of one or more amino acid residues with one or more non-natural amino acid residues (such as replacement of one or more polar amino acid residues with one or more isosteric or isoelectronic amino acids; replacement of one or more non-polar amino acid residues with other non-natural isosteric or isoelectronic amino acids); addition of a spacer group; replacement of one or more oxidation sensitive amino acid residues with one or more oxidation resistant amino acid residues; replacement of one or more amino acid residues with one or more replacement amino acids, such as an alanine, replacement of one or more L-amino acid residues with one or more D-amino acid residues; N-alkylation of one or more amide bonds within the bicyclic peptide ligand; replacement of one or more peptide bonds with a surrogate bond; peptide backbone length modification; substitution of the hydrogen on the alpha-carbon of one or more amino acid residues with another chemical group; modification of amino acids such as cysteine, lysine, glutamate/aspartate and tyrosine with suitable amine, thiol, carboxylic acid and phenol-reactive reagents so as to functionalise said amino acids; and introduction or replacement of amino acids that introduce orthogonal reactivities that are suitable for functionalisation, for example azide or alkyne-group bearing amino acids that allow functionalisation with alkyne or azide-bearing moieties, respectively.

In one embodiment, the modified derivative comprises an N-terminal and/or C-terminal modification. In a further embodiment, wherein the modified derivative comprises an N-terminal modification using suitable amino-reactive chemistry, and/or C-terminal modification using suitable carboxy-reactive chemistry. In a further embodiment, said N-terminal or C-terminal modification comprises addition of an effector group, including but not limited to a cytotoxic agent, a radiochelator or a chromophore.

In a further embodiment, the modified derivative comprises an N-terminal modification. In a further embodiment, the N-terminal modification comprises an N-terminal acetyl group. In this embodiment, the N-terminal residue is capped with acetic anhydride or other appropriate reagents during peptide synthesis leading to a molecule which is N-terminally acetylated. This embodiment provides the advantage of removing a potential recognition point for aminopeptidases and avoids the potential for degradation of the bicyclic peptide.

In an alternative embodiment, the N-terminal modification comprises the addition of a molecular spacer group which facilitates the conjugation of effector groups and retention of potency of the bicyclic peptide to its target.

In a further embodiment, the modified derivative comprises a C-terminal modification. In a further embodiment, the C-terminal modification comprises an amide group. In this embodiment, the C-terminal residue is synthesized as an amide during peptide synthesis leading to a molecule which is C-terminally amidated. This embodiment provides the advantage of removing a potential recognition point for carboxypeptidase and reduces the potential for proteolytic degradation of the bicyclic peptide.

In one embodiment, the modified derivative comprises replacement of one or more amino acid residues with one or more non-natural amino acid residues. In this embodiment, non-natural amino acids may be selected having isosteric/isoelectronic side chains which are neither recognised by degradative proteases nor have any adverse effect upon target potency.

Alternatively, non-natural amino acids may be used having constrained amino acid side chains, such that proteolytic hydrolysis of the nearby peptide bond is conformationally and sterically impeded. In particular, these concern proline analogues, bulky sidechains, Cα-disubstituted derivatives (for example, aminoisobutyric acid, Aib), and cyclo amino acids, a simple derivative being amino-cyclopropylcarboxylic acid.

In one embodiment, the modified derivative comprises the addition of a spacer group. In a further embodiment, the modified derivative comprises the addition of a spacer group to the N-terminal cysteine or penicillamine (Cᵢ or Penᵢ) and/or the C-terminal cysteine or penicillamine (Cᵢᵢᵢ or Penᵢᵢᵢ).

In one embodiment, the modified derivative comprises replacement of one or more oxidation sensitive amino acid residues with one or more oxidation resistant amino acid residues. In a further embodiment, the modified derivative comprises replacement of a tryptophan residue with a naphthylalanine or alanine residue. This embodiment provides the advantage of improving the pharmaceutical stability profile of the resultant bicyclic peptide ligand.

In one embodiment, the modified derivative comprises replacement of one or more charged amino acid residues with one or more hydrophobic amino acid residues. In an alternative embodiment, the modified derivative comprises replacement of one or more hydrophobic amino acid residues with one or more charged amino acid residues. The correct balance of charged versus hydrophobic amino acid residues is an important characteristic of the bicyclic peptide ligands. For example, hydrophobic amino acid residues influence the degree of plasma protein binding and thus the concentration of the free available fraction in plasma, while charged amino acid residues (in particular arginine) may influence the interaction of the peptide with the phospholipid membranes on cell surfaces. The two in combination may influence half-life, volume of distribution and exposure of the peptide drug, and can be tailored according to the clinical endpoint. In addition, the correct combination and number of charged versus hydrophobic amino acid residues may reduce irritation at the injection site (if the peptide drug has been administered subcutaneously).

In one embodiment, the modified derivative comprises replacement of one or more L-amino acid residues with one or more D-amino acid residues. This embodiment is believed to increase proteolytic stability by steric hindrance and by a propensity of D-amino acids to stabilise β-turn conformations (Tugyi et al. (2005) PNAS, 102(2), 413-418).

In one embodiment, the modified derivative comprises removal of any amino acid residues and substitution with alanines, such as D-alanines. This embodiment provides the advantage of identifying key binding residues and removing potential proteolytic attack site(s).

It should be noted that each of the above mentioned modifications serve to deliberately improve the potency or stability of the peptide. Further potency improvements based on modifications may be achieved through the following mechanisms:
- Incorporating hydrophobic moieties that exploit the hydrophobic effect and lead to lower off rates, such that higher affinities are achieved;
- Incorporating charged groups that exploit long-range ionic interactions, leading to faster on rates and to higher affinities (see for example Schreiber et al., Rapid, electrostatically assisted association of proteins (1996), Nature Struct. Biol. 3, 427-31); and
- Incorporating additional constraint into the peptide, by for example constraining side chains of amino acids correctly such that loss in entropy is minimal upon target binding, constraining the torsional angles of the backbone such that loss in entropy is minimal upon target binding and introducing additional cyclisations in the molecule for identical reasons.
(for reviews see Gentilucci et al., Curr. Pharmaceutical Design, (2010), 16, 3185-203, and Nestor et al., Curr. Medicinal Chem (2009), 16, 4399-418).

### Isotopic Variations

The present invention includes all pharmaceutically acceptable (radio)isotope-labelled peptide ligands of the invention, wherein one or more atoms are replaced by atoms having the same atomic number, but an atomic mass or mass number different from the atomic mass or mass number usually found in nature, and peptide ligands of the invention, wherein metal chelating groups are attached (termed "effector") that are capable of holding relevant (radio)isotopes, and peptide ligands of the invention, wherein certain functional groups are covalently replaced with relevant (radio)isotopes or isotopically labelled functional groups.

Examples of isotopes suitable for inclusion in the peptide ligands of the invention comprise isotopes of hydrogen, such as ²H (D) and ³H (T), carbon, such as ¹¹C, ¹³C and ¹⁴C, chlorine, such as ³⁶Cl, fluorine, such as ¹⁸F, iodine, such as ¹²³I, ¹²⁵I and ¹³¹I, nitrogen, such as ¹³N and ¹⁵N, oxygen, such as ¹⁵O,¹⁷O and ¹⁸O, phosphorus, such as ³²P, sulphur, such as ³⁵S, copper, such as ⁶⁴Cu, gallium, such as ⁶⁷Ga or ⁶⁸Ga, yttrium, such as ⁹⁰Y and lutetium, such as ¹⁷⁷Lu, and Bismuth, such as ²¹³Bi.

Certain isotopically-labelled peptide ligands of the invention, for example, those incorporating a radioactive isotope, are useful in drug and/or substrate tissue distribution studies, and to clinically assess the presence and/or absence of the target on diseased tissues. The peptide ligands of the invention can further have valuable diagnostic properties in that they can be used for detecting or identifying the formation of a complex between a labelled compound and other molecules, peptides, proteins, enzymes or receptors. The detecting or identifying methods can use compounds that are labelled with labelling agents such as radioisotopes, enzymes, fluorescent substances, luminous substances (for example, luminol, luminol derivatives, luciferin, aequorin and luciferase), etc. The radioactive isotopes tritium, *i.e.* ³H (T), and carbon-14, i.e. ¹⁴C, are particularly useful for this purpose in view of their ease of incorporation and ready means of detection.

Substitution with heavier isotopes such as deuterium, i.e. ²H (D), may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining target occupancy.

Isotopically-labelled compounds of peptide ligands of the invention can generally be prepared by conventional techniques known to those skilled in the art or by processes analogous to those described in the accompanying Examples using an appropriate isotopically-labelled reagent in place of the non-labelled reagent previously employed.

### Molecular Scaffold

In one embodiment, the molecular scaffold comprises a non-aromatic molecular scaffold. References herein to "non-aromatic molecular scaffold" refers to any molecular scaffold as defined herein which does not contain an aromatic (i.e. unsaturated) carbocyclic or heterocyclic ring system.

Suitable examples of non-aromatic molecular scaffolds are described in Heinis et al. (2014) Angewandte Chemie, International Edition 53(6) 1602-1606.

As noted in the foregoing documents, the molecular scaffold may be a small molecule, such as a small organic molecule.

In one embodiment the molecular scaffold may be a macromolecule. In one embodiment the molecular scaffold is a macromolecule composed of amino acids, nucleotides or carbohydrates.

In one embodiment the molecular scaffold comprises reactive groups that are capable of reacting with functional group(s) of the polypeptide to form covalent bonds.

The molecular scaffold may comprise chemical groups which form the linkage with a peptide, such as amines, thiols, alcohols, ketones, aldehydes, nitriles, carboxylic acids, esters, alkenes, alkynes, azides, anhydrides, succinimides, maleimides, alkyl halides and acyl halides.

In one embodiment, the molecular scaffold is 1,1',1"-(1,3,5-triazinane-1,3,5-triyl)triprop-2-en-1-one (also known as triacryloylhexahydro-s-triazine (TATA):

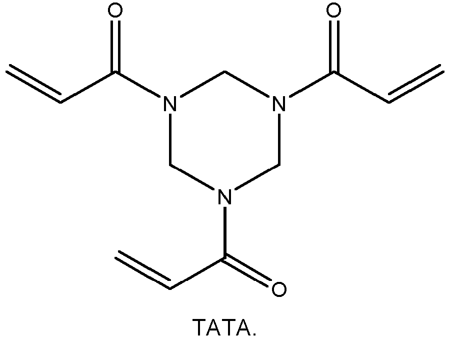

Thus, following cyclisation with the bicyclic peptides of the invention on the Cᵢ, Penᵢ, Cᵢᵢ, Penᵢᵢ, Cᵢᵢᵢ and Penᵢᵢᵢ cysteine or penicillamine residues, the molecular scaffold forms a tri-substituted 1,1',1"-(1,3,5-triazinane-1,3,5-triyl)tripropan-1-one derivative of TATA having the following structure: 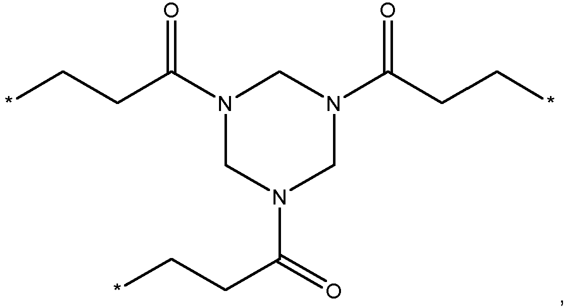 wherein * denotes the point of attachment of the three cysteine residues.

In an alternative embodiment, the molecular scaffold is 2,4,6-tris(bromomethyl)-s-triazine (TBMT):

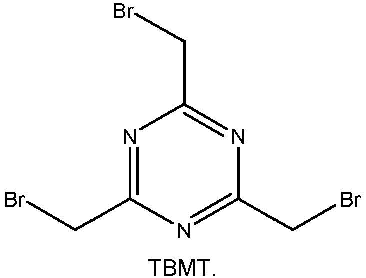

Thus, following cyclisation with the bicyclic peptides of the invention on the Cᵢ, Penᵢ, Cᵢᵢ, Penᵢᵢ, Cᵢᵢᵢ and Penᵢᵢᵢ cysteine or penicillamine residues, the molecular scaffold forms a tri-substituted derivative of TBMT having the following structure: 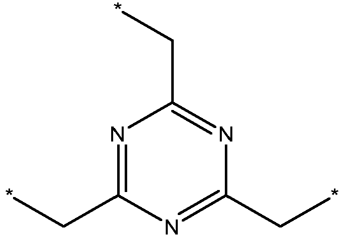 wherein * denotes the point of attachment of the three cysteine residues.

In an alternative embodiment, the molecular scaffold is 1,3,5-tris(bromoacetyl) hexahydro-1, 3,5-triazine (TATB):

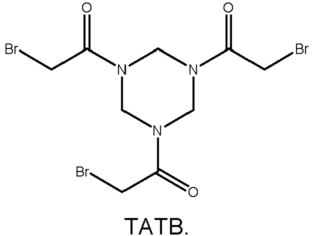

Thus, following cyclisation with the bicyclic peptides of the invention on the Cᵢ, Penᵢ, Cᵢᵢ, Penᵢᵢ, Cᵢᵢᵢ and Penᵢᵢᵢ cysteine or penicillamine residues, the molecular scaffold forms a tri-substituted 1,3,5-tris(bromoacetyl) hexahydro-1,3,5-triazine derivative of TATB having the following structure: 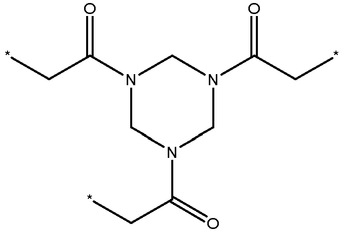 wherein * denotes the point of attachment of the three cysteine residues.

### Synthesis

The peptides of the present invention may be manufactured synthetically by standard techniques followed by reaction with a molecular scaffold *in vitro.* When this is performed, standard chemistry may be used. This enables the rapid large scale preparation of soluble material for further downstream experiments or validation. Such methods could be accomplished using conventional chemistry such as that disclosed in Timmerman *et al.* (*supra*).

Thus, the invention also relates to the manufacture of polypeptides or conjugates selected as set out herein, wherein the manufacture comprises optional further steps as explained below. In one embodiment, these steps are carried out on the end product polypeptide/conjugate made by chemical synthesis.

Optionally amino acid residues in the polypeptide of interest may be substituted when manufacturing a conjugate or complex.

Peptides can also be extended, to incorporate for example another loop and therefore introduce multiple specificities.

To extend the peptide, it may simply be extended chemically at its N-terminus or C-terminus or within the loops using orthogonally protected lysines (and analogues) using standard solid phase or solution phase chemistry. Standard (bio)conjugation techniques may be used to introduce an activated or activatable N- or C-terminus. Alternatively additions may be made by fragment condensation or native chemical ligation e.g. as described in (Dawson et al. 1994. Synthesis of Proteins by Native Chemical Ligation. Science 266:776-779), or by enzymes, for example using subtiligase as described in (Chang et al. Proc Natl Acad Sci USA. 1994 Dec 20; 91(26): 12544-8 or in Hikari et al. Bioorganic & Medicinal Chemistry Letters Volume 18, Issue 22, 15 November 2008, Pages 6000-6003).

Alternatively, the peptides may be extended or modified by further conjugation through disulphide bonds. This has the additional advantage of allowing the first and second peptide to dissociate from each other once within the reducing environment of the cell. In this case, the molecular scaffold (e.g. TATA, TATB or TBMT) could be added during the chemical synthesis of the first peptide so as to react with the three cysteine groups; a further cysteine or thiol could then be appended to the N- or C-terminus of the first peptide, so that this cysteine or thiol only reacted with a free cysteine or thiol of the second peptide, forming a disulphide-linked bicyclic peptide-peptide conjugate.

Furthermore, addition of other functional groups or effector groups may be accomplished in the same manner, using appropriate chemistry, coupling at the N- or C-termini or via side chains. In one embodiment, the coupling is conducted in such a manner that it does not block the activity of either entity.

### Pharmaceutical Compositions

According to a further aspect of the invention, there is provided a pharmaceutical composition comprising a peptide ligand as defined herein in combination with one or more pharmaceutically acceptable excipients.

Generally, the present peptide ligands will be utilised in purified form together with pharmacologically appropriate excipients or carriers. Typically, these excipients or carriers include aqueous or alcoholic/aqueous solutions, emulsions or suspensions, including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The peptide ligands of the present invention may be used as separately administered compositions or in conjunction with other agents. These can include antibodies, antibody fragments and various immunotherapeutic drugs, such as cyclosporine, methotrexate, adriamycin or cisplatinum and immunotoxins. Further examples of other agents which may be administered separately or in conjunction with the peptide ligands of the invention include cytokines, lymphokines, other hematopoietic factors, thrombolytic and anti-thrombotic factors. Pharmaceutical compositions can include "cocktails" of various cytotoxic or other agents in conjunction with the protein ligands of the present invention, or even combinations of selected polypeptides according to the present invention having different specificities, such as polypeptides selected using different target ligands, whether or not they are pooled prior to administration.

The route of administration of pharmaceutical compositions according to the invention may be any of those commonly known to those of ordinary skill in the art. For therapy, the peptide ligands of the invention can be administered to any patient in accordance with standard techniques. The administration can be by any appropriate mode, including parenterally, intravenously, intramuscularly, intraperitoneally, transdermally, via the pulmonary route, or also, appropriately, by direct infusion with a catheter. Preferably, the pharmaceutical compositions according to the invention will be administered intravenously. The dosage and frequency of administration will depend on the age, sex and condition of the patient, concurrent administration of other drugs, counterindications and other parameters to be taken into account by the clinician.

The peptide ligands of this invention can be lyophilised for storage and reconstituted in a suitable carrier prior to use. This technique has been shown to be effective and art-known lyophilisation and reconstitution techniques can be employed. It will be appreciated by those skilled in the art that lyophilisation and reconstitution can lead to varying degrees of activity loss and that levels may have to be adjusted upward to compensate.

The compositions containing the present peptide ligands or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease, but generally range from 0.005 to 5.0 mg of selected peptide ligand per kilogram of body weight, with doses of 0.05 to 2.0 mg/kg/dose being more commonly used. For prophylactic applications, compositions containing the present peptide ligands or cocktails thereof may also be administered in similar or slightly lower dosages.

A composition containing a peptide ligand according to the present invention may be utilised in prophylactic and therapeutic settings to aid in the alteration, inactivation, killing or removal of a select target cell population in a mammal. In addition, the peptide ligands described herein may be used extracorporeally or *in vitro* selectively to kill, deplete or otherwise effectively remove a target cell population from a heterogeneous collection of cells. Blood from a mammal may be combined extracorporeally with the selected peptide ligands whereby the undesired cells are killed or otherwise removed from the blood for return to the mammal in accordance with standard techniques.

### Therapeutic Uses

The references to methods of treatment in the subsequent paragraphs of this description are to be interpreted as references to the compounds, pharmaceutical compositions and medicaments of the present invention for use in a method for treatment of the human (or animal) body by therapy (or for *in vivo* diagnosis).

Polypeptide ligands selected according to the method of the present invention may be employed in *in vivo* therapeutic and prophylactic applications, *in vitro* and *in vivo* diagnostic applications, *in vitro* assay and reagent applications, and the like. Ligands having selected levels of specificity are useful in applications which involve testing in non-human animals, where cross-reactivity is desirable, or in diagnostic applications, where cross-reactivity with homologues or paralogues needs to be carefully controlled. In some applications, such as vaccine applications, the ability to elicit an immune response to predetermined ranges of antigens can be exploited to tailor a vaccine to specific diseases and pathogens.

Substantially pure peptide ligands of at least 90 to 95% homogeneity are preferred for administration to a mammal, and 98 to 99% or more homogeneity is most preferred for pharmaceutical uses, especially when the mammal is a human. Once purified, partially or to homogeneity as desired, the selected polypeptides may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY).

According to a further aspect of the invention, there is provided a peptide ligand or a drug conjugate as defined herein, for use in preventing, suppressing or treating a disease or disorder mediated by NK cells.

According to a further aspect of the invention, there is provided a method of preventing, suppressing or treating a disease or disorder mediated by NK cells, which comprises administering to a patient in need thereof the peptide ligand as defined herein.

In one embodiment, the disease or disorder mediated by NK cells is selected from inflammatory disorders, autoimmune disease and cancer. In a further embodiment, the disease or disorder mediated by NK cells is selected from: rheumatoid arthritis (RA), bone erosion, intraperitoneal abscesses, inflammatory bowel disease, allograft rejection, psoriasis, angiogenesis, atherosclerosis, asthma, multiple sclerosis, systemic lupus erythematosus (SLE), ocular surface disorders (such as dry eye), ankylosing spondylitis, psoriatic arthritis, cancer (such as multiple myeloma and breast cancer).

In a further embodiment, the disease or disorder mediated by NK cells is selected from cancer.

Examples of cancers (and their benign counterparts) which may be treated (or inhibited) include, but are not limited to tumours of epithelial origin (adenomas and carcinomas of various types including adenocarcinomas, squamous carcinomas, transitional cell carcinomas and other carcinomas) such as carcinomas of the bladder and urinary tract, breast, gastrointestinal tract (including the esophagus, stomach (gastric), small intestine, colon, rectum and anus), liver (hepatocellular carcinoma), gall bladder and biliary system, exocrine pancreas, kidney,lung (for example adenocarcinomas, small cell lung carcinomas, non-small cell lung carcinomas, bronchioalveolar carcinomas and mesotheliomas), head and neck (for example cancers of the tongue, buccal cavity, larynx, pharynx, nasopharynx, tonsil, salivary glands, nasal cavity and paranasal sinuses), ovary, fallopian tubes, peritoneum, vagina, vulva, penis, cervix, myometrium, endometrium, thyroid (for example thyroid follicular carcinoma), adrenal, prostate, skin and adnexae (for example melanoma, basal cell carcinoma, squamous cell carcinoma, keratoacanthoma, dysplastic naevus); haematological malignancies (i.e. leukemias, lymphomas) and premalignant haematological disorders and disorders of borderline malignancy including haematological malignancies and related conditions of lymphoid lineage (for example acute lymphocytic leukemia [ALL], chronic lymphocytic leukemia [CLL], B-cell lymphomas such as diffuse large B-cell lymphoma [DLBCL], follicular lymphoma, Burkitt's lymphoma, mantle cell lymphoma, T-cell lymphomas and leukaemias, natural killer [NK] cell lymphomas, Hodgkin's lymphomas, hairy cell leukaemia, monoclonal gammopathy of uncertain significance, plasmacytoma, multiple myeloma, and post-transplant lymphoproliferative disorders), and haematological malignancies and related conditions of myeloid lineage (for example acute myelogenousleukemia [AML], chronic myelogenousleukemia [CML], chronic myelomonocyticleukemia [CMML], hypereosinophilic syndrome, myeloproliferative disorders such as polycythaemia vera, essential thrombocythaemia and primary myelofibrosis, myeloproliferative syndrome, myelodysplastic syndrome, and promyelocyticleukemia); tumours of mesenchymal origin, for example sarcomas of soft tissue, bone or cartilage such as osteosarcomas, fibrosarcomas, chondrosarcomas, rhabdomyosarcomas,leiomyosarcomas, liposarcomas, angiosarcomas, Kaposi's sarcoma, Ewing's sarcoma, synovial sarcomas, epithelioid sarcomas, gastrointestinal stromal tumours, benign and malignant histiocytomas, and dermatofibrosarcomaprotuberans; tumours of the central or peripheral nervous system (for example astrocytomas, gliomas and glioblastomas, meningiomas, ependymomas, pineal tumours and schwannomas); endocrine tumours (for example pituitary tumours, adrenal tumours, islet cell tumours, parathyroid tumours, carcinoid tumours and medullary carcinoma of the thyroid); ocular and adnexal tumours (for example retinoblastoma); germ cell and trophoblastic tumours (for example teratomas, seminomas, dysgerminomas, hydatidiform moles and choriocarcinomas); and paediatric and embryonal tumours (for example medulloblastoma, neuroblastoma, Wilms tumour, and primitive neuroectodermal tumours); or syndromes, congenital or otherwise, which leave the patient susceptible to malignancy (for example Xeroderma Pigmentosum).

References herein to the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the peptide ligands in protecting against or treating the disease are available. The use of animal model systems is facilitated by the present invention, which allows the development of polypeptide ligands which can cross react with human and animal targets, to allow the use of animal models.

The invention is further described below with reference to the following examples.

### EXAMPLES

### Materials and Methods

### Preparation of Bicyclic Peptide Ligands (General Method)

Bicyclic peptides were synthesized on Rink amide resin using standard Fmoc (9-fluorenylmethyloxycarbonyl) solid-phase peptide synthesis, either by manual coupling (for large scale) or using a Biotage Syroll automated peptide synthesizer (for small scale). Following TFA-based cleavage from the resin, peptides were precipitated with diethyl ether and dissolved in 50:50 acetonitrile/water. The crude peptides (at ~1 mM concentration) were then cyclized with 1.3 equiv. of the scaffold, using ammonium bicarbonate (100 mM) as a base. Completion of cyclization was determined by matrix-assisted laser desorption ionization time-of-flight (MALDI-TOF) or LC-MS. Once complete, the cyclization reaction was quenched using N-acetyl cysteine (10 equiv. with respect to the peptide), and the solutions were lyophilized. The residue was dissolved in an appropriate solvent and purified by RP-HPLC. Peptide fractions of sufficient purity and the correct molecular weight (verified by either MALDI-TOF and HPLC or LC-MS) were pooled and lyophilized. Concentrations were determined by UV absorption using the extinction coefficient at 280 nm, which was based on Trp/Tyr content.

All amino acids, unless noted otherwise, were used in the L-configurations.

### EXAMPLES

In general, some of the fluorescent dimeric bicyclic peptide complexes of the invention may be prepared in accordance with the following general method:

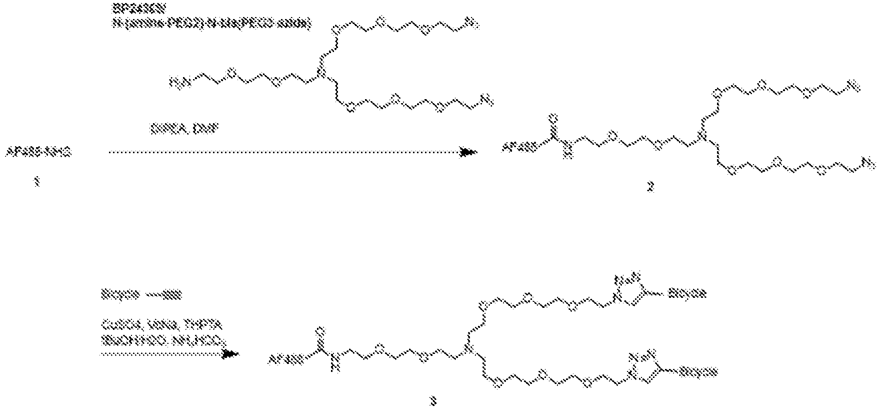

A mixture of BP24369 (1.0 eq) and AF488-NHS (1, 1.5 eq.) is dissolved in DMF. The pH of this solution is adjusted to 8 by dropwise addition of DIEA (3.0 eq). The reaction mixture is stirred at 25 °C for 1 hr, then a sample taken for analysis. If the reaction is incomplete, additional AF488-NHS is added and the mixture left to stir for a further 1 hr. Once complete, the reaction mixture is filtered to remove undissolved residue and the crude product purified by preparative HPLC to give intermediate **2.**

A mixture of intermediate **2** (1.0 eq), **Bicycle** (2.1 eq) and THPTA (2.2 eq) are dissolved in degassed t-BuOH/H₂O (1:1), then aqueous CuSO₄ (0.4M, 2.2 eq) and VcNa (4.0 eq) is added. Finally, 0.2 M NH₄HCO₃ is added to adjust pH to 8. The reaction mixture is stirred for up to 16 hr under N₂ atmosphere. The reaction mixture is directly purified by prep-HPLC.

Fluorescently labelled dimeric bicyclic peptide complexes which were prepared using this method are listed below:

| **NKp46 fluorescent dimers** | **CD16a fluorescent dimers** |
|---|---|
| BCY15663 | BCY15914 |
| BCY15921 | BCY15915 |
| BCY15922 | BCY15916 |
| BCY15455 | |
| BCY18043 | |

More detailed experimental for selected bicyclic peptide multimeric complexes of the invention are provided herein below:

### Synthesis of intermediate AF488-BP24369

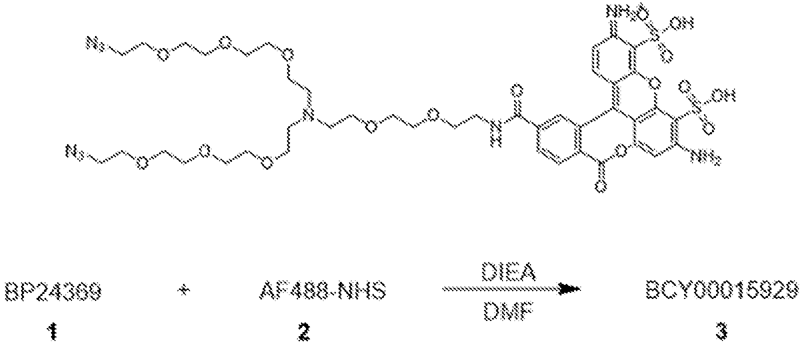

A mixture of compound 1 (10.0 mg, 18.2 µmol, 1.0 eq.), compound 2 (17.0 mg, 27.3 µmol, 1.5 eq.) was dissolved in DMF (0.4 mL). The pH of this solution was adjusted to 8 by dropwise addition of DIEA (15.8 µL, 181.60 µmol, 3.0 eq.). The reaction mixture was stirred at 25 °C for 1 hr. LC-MS showed **BP24369** was not completely consumed, so additional compound 2 (17.0 mg, 27.24 µmol, 1.5 eq.) was added to the reaction mixture, which was stirred at 25 °C for 1 hr. LC-MS showed one main peak with desired m/z. The reaction mixture was filtered to remove undissolved residue. The crude product was purified by preparative HPLC, and **AF488-BP24369** (10.0 mg, 8.90 µmol, 49.02% yield, 95.0% purity) was obtained as a red solid. Calculated MW: 1067.1, observed *m*/*z:* 1068.3 ([M+H]⁺), 534.7 ([M+2H]²⁺).

### Synthesis of BCY15663

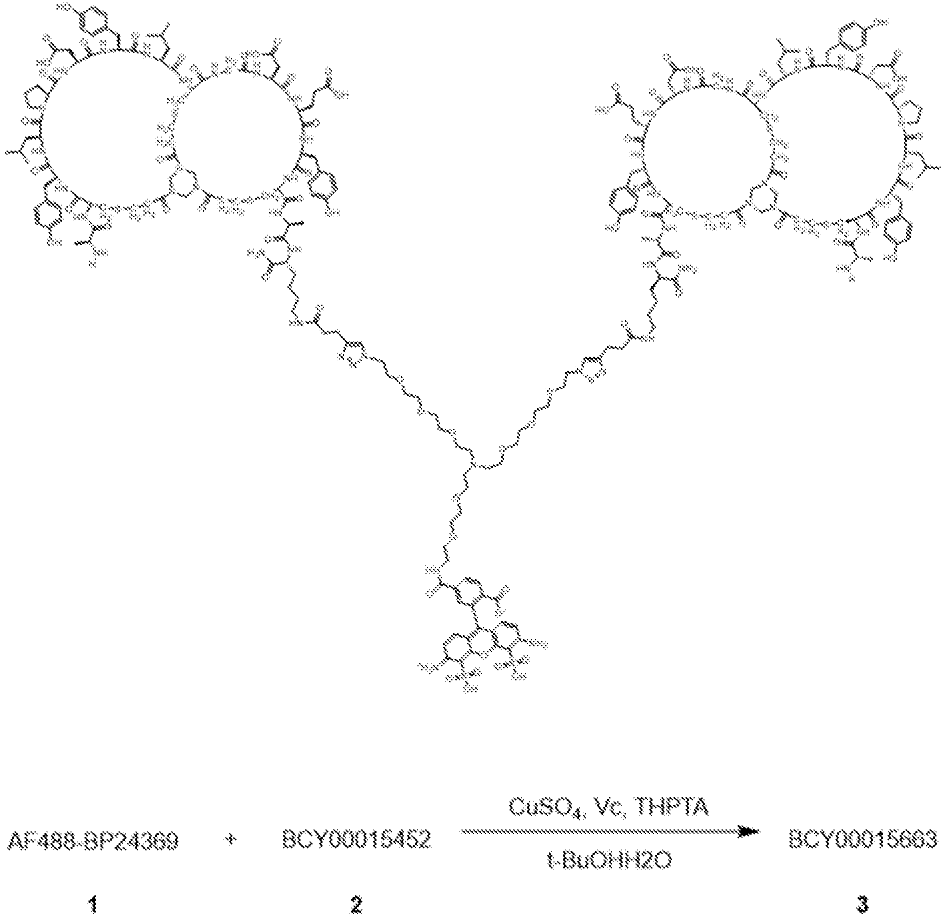

A mixture of compound 1 (3.0 mg, 2.81 µmol, 1.0 eq.), compound 2 (14.0 mg, 6.47 µmol, 2.3 eq.), and THPTA (1.2 mg, 2.81 µmol, 1.0 eq.) was dissolved in t-BuOH/H₂O (1:1, 0.5 mL), degassed and purged with N₂, and then an aqueous solution of CuSO₄ (0.4 M, 7.0 µL, 1.0 eq.) and Vc (2.0 mg, 11.25 µmol, 4.0 eq.) were added under N₂. The pH of this solution was adjusted to 8, and the solution turned light yellow. The reaction mixture was stirred at 25 °C for 1 hr under N₂ atmosphere. LC-MS showed **AF488-BP24369** was consumed completely, some **BCY15452** remained and one main peak with desired m/z was detected. The reaction mixture was filtered to remove undissolved residue. The crude product was purified by preparative HPLC, and **BCY15663** (9.4 mg, 1.66 µmol, 59.06% yield, 96.4% purity) was obtained as a white solid. Calculated MW: 5377.9, observed m/z: 1345.5 ([M+4H]⁴⁺).

### BCY15921

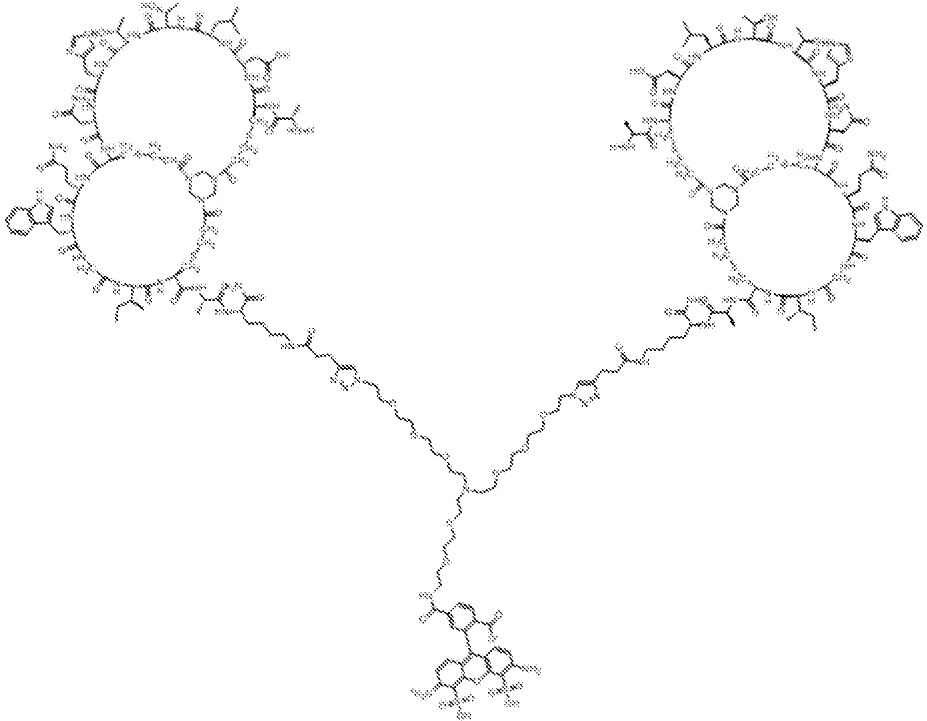

### BCY15922

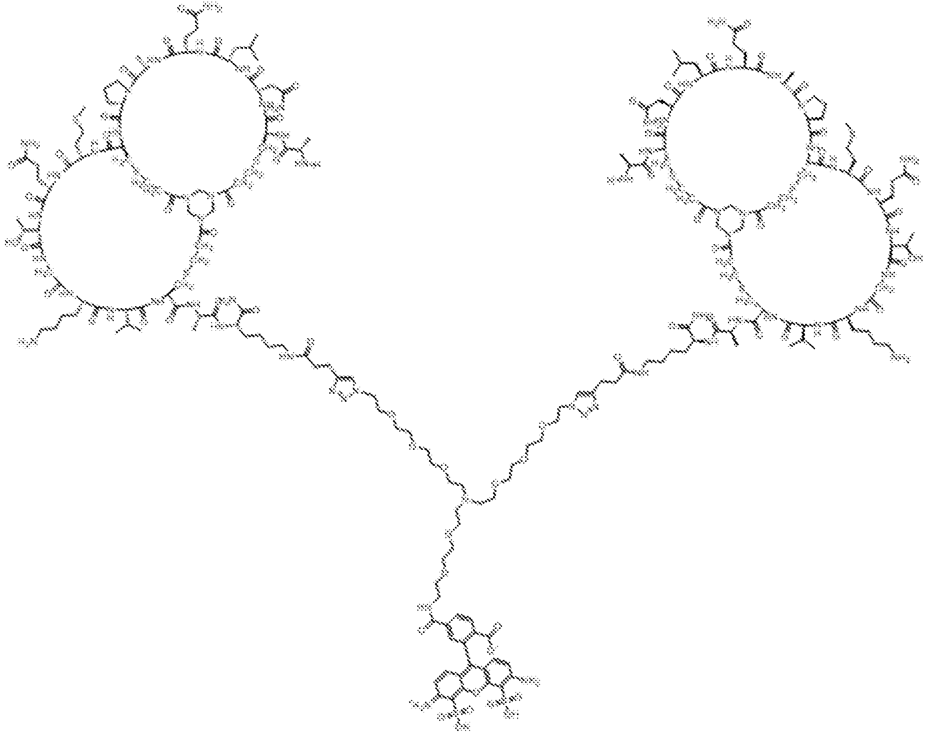

### BCY15455

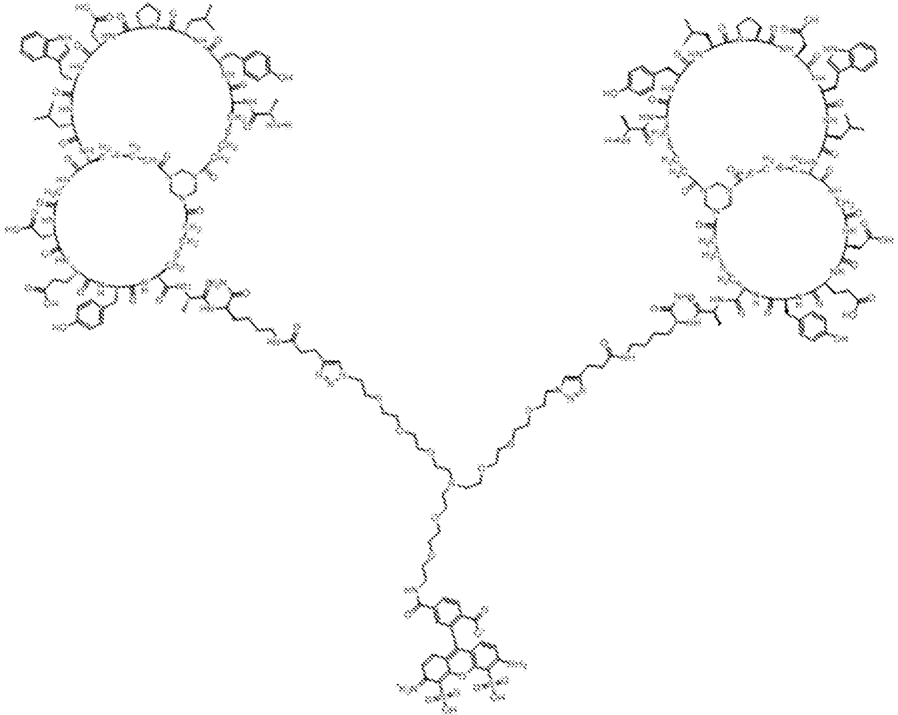

### BCY18043

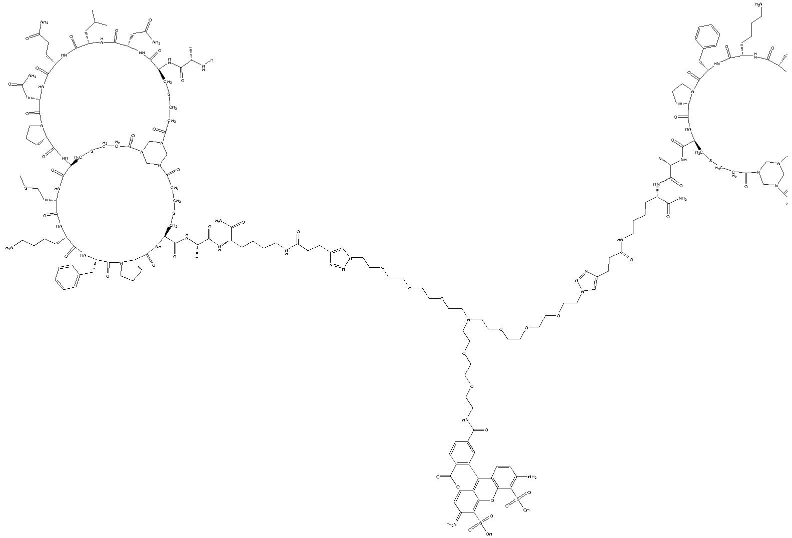

### Synthesis of BCY15916

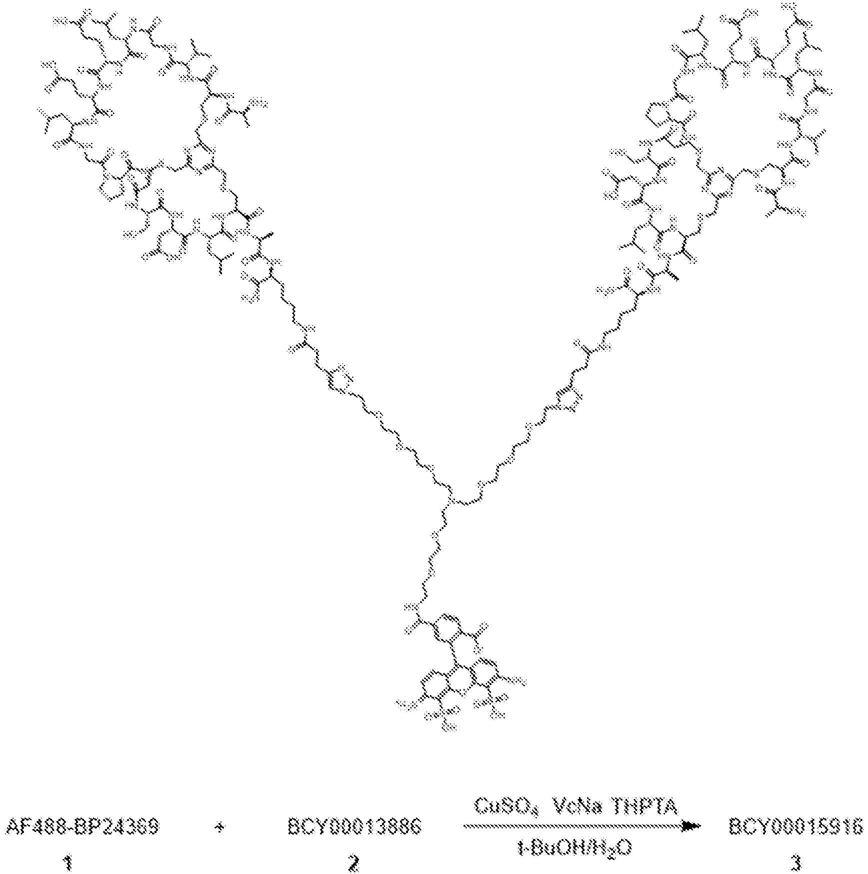

A mixture of compound 1 (2.0 mg, 1.87 µmol, 1.0 eq.), compound 2 (7.5 mg, 3.94 µmol, 2.1 eq.), and THPTA (1.8 mg, 4.12 µmol, 2.2 eq.) was dissolved in t-BuOH/H₂O (1:1, 0.5 mL, degassed and purged with N₂), and then an aqueous solution of CuSO₄ (0.4 M, 10.3 µL, 2.2 eq.) and VcNa (1.5 mg, 7.5 µmol, 4.0 eq.) were added under N₂. The pH of this solution was adjusted to 8 by dropwise addition of 0.2 M NH₄HCO₃ (in 1:1 t-BuOH/H₂O), and the solution turned light yellow. The reaction mixture was stirred at 25-30 °C for 1 hr under an N₂ atmosphere. LC-MS showed compound 1 was consumed completely, and one main peak with desired m/z was detected. The reaction mixture was filtered to remove undissolved residue. The crude product was purified by preparative HPLC, and **BCY15916** (1.6 mg, 0.30 µmol, 16.3% yield, 93.3% purity) was obtained as a white solid. Calculated MW: 4875.5, observed m/z: 1219.6 ([M+4H]⁴⁺).

### BCY15914

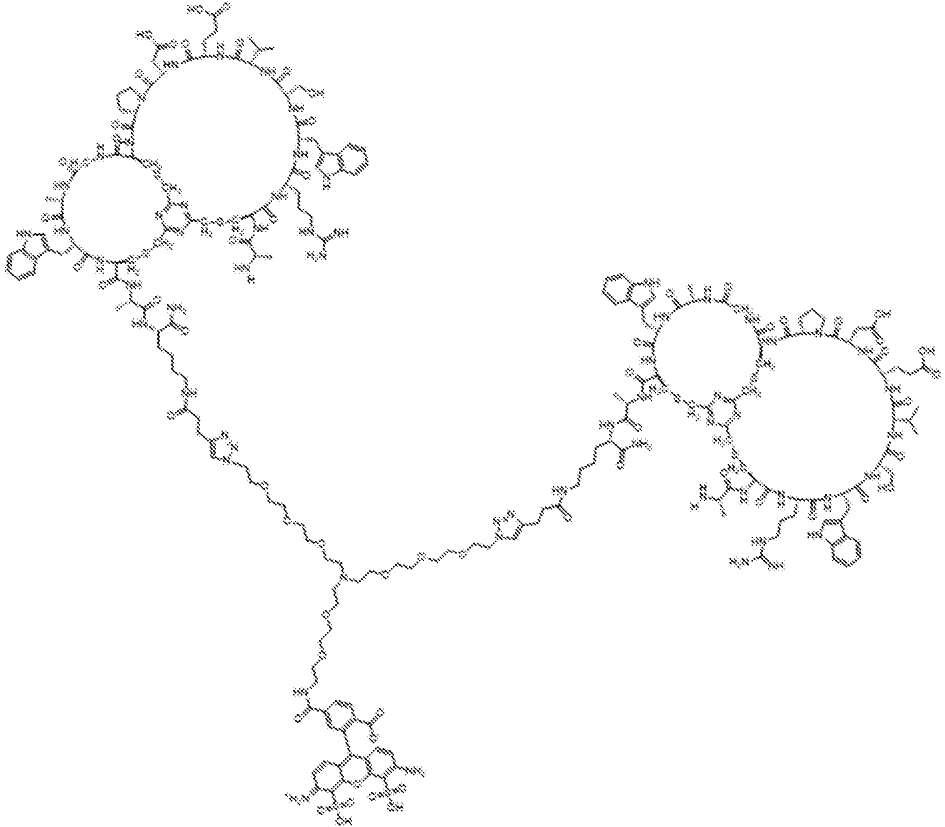

### BCY15915

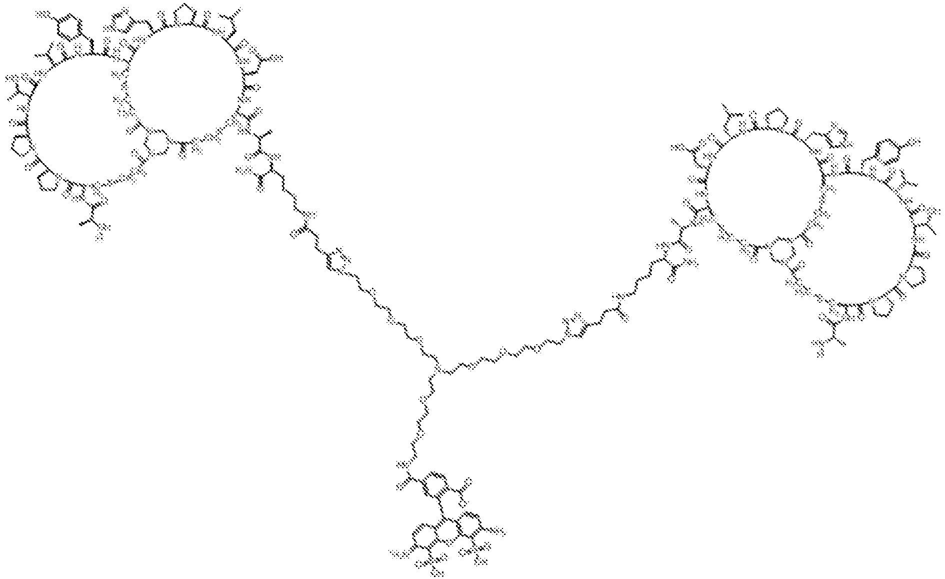

### BIOLOGICAL DATA

### 1. NKp46 Fluorescence Polarization (FP) Direct Binding Assay

Affinity of the peptides of the invention for human NKp46 (Acro Biosystems, NC1-H5257 or NC1-H52H4) was determined using a fluorescence polarisation assay. Peptides of the invention were labelled with a fluorescent tag (fluorescein) and diluted to 2.5nM in 25mM HEPES with 100mM NaCl and 0.005% P20, pH 7.4. NKp46 protein was titrated starting at 0.2-5µM in the same assay buffer as the peptide to assay 1nM peptide in a total volume of 25µL in black walled and bottomed low bind low volume 384 well plates. The assay was typically set up by adding 5µL assay buffer, 10µL NKp46 protein then 10µL fluorescent peptide. The concentrations of NKp46 protein were either 2-fold or 3-fold dilutions to give 12 different concentrations starting at 0.2-5µM. Measurements were conducted on a BMG PHERAstar FS equipped with an FP 485 520 520 optic module at 25°C with 200 flashes per well and a positioning delay of 0.1 second. Each well was measured every 5 minutes for 60 minutes. The gain used for analysis was determined for each tracer at the end of the 60 minutes where there was no protein in the well. The mP were fit to a standard 1:1 binding model with a quadratic equation to generate a K_{D} value. Selected peptides of the invention were tested in the above-mentioned assay and the results are shown in Table 1.

FP direct binding assay materials:
Human NKp46 Fc tag (Aero Biosystems, NC1-H5257)
Human NKp46 His tag (Aero Biosystems, NC1-H52H4)

**Table 1: Fluorescence Polarisation Direct Binding Assay Data for Selected Bicyclic Peptides of the Invention**

| ***Bicyclic Peptide*** | ***Kd (nM)*** |
|---|---|
| BCY15023 | 1405 |
| BCY15024 | 1042 |
| BCY15025 | 58 |
| BCY15028 | 12 |
| BCY15030 | 106 |
| BCY15035 | 12 |
| BCY15116 | 38 |
| BCY15115 | 440 |
| BCY14652 | 65 |
| BCY15117 | 35 |
| BCY14654 | 1026 |
| BCY15125 | 602 |
| BCY15118 | 647 |
| BCY15119 | 1254 |
| BCY15120 | 1746 |
| BCY15121 | 1741 |
| BCY15122 | 290 |
| BCY15123 | 416 |

### 2. NKp46 Fluorescence Polarization (FP) Competition Binding Assay

Affinities of peptides of the invention were determined in a fluorescence polarization (FP) competition assay using the fluorescent labelled peptide BCY15035 as the tracer that is competed off by untagged peptides.

The untagged competitor peptides were diluted in assay buffer 25mM HEPES, 100mM NaCl, 0.01 % P20, pH 7.4. to a top concentration of 5000 nM and diluted 2-fold in 11 steps. The human NKp46-his tagged protein (AcroBiosystems) was diluted to 40 nM final concentration in the assay. Finally, the fluorescent tracer peptide BCY15035 was added at 1 nM. The assay was typically set up by adding 5µL competitor, 10µL NKp46 protein then 10µL fluorescent peptide. The total volume of 25µL was prepared in black walled and flat-bottomed low binding low volume 384 well plates. Measurements were performed using Envision (PerkinElmer) equipped with the excitation filter FITC FP 480, emission filter FITC FP P-pol 535 and 2^{nd} emission filter FITC FP S-pol 535 and set to 30 flashes. The gain was set in a well containing tracer alone without target protein. The mP-values in the final read at 60 minutes were transformed into percentage inhibition and used to calculate the Ki-values (Kcomp) in the software Sigmaplot (Systat Software Inc) using the equation below: f= ymax+(ymin-ymax)/Lig*((Lig*((2*((Lig+Kcomp+Lig+Comp-Prot*c)"2-3*(Kcomp*(Lig- Prot*c)+Klig*(Comp-Prot*c)+Klig*Kcomp))^0.5*COS(ARCCOS((-2*(Klig+Kcomp+Lig+Comp- Prot*c)^3+9*(Klig+Kcomp+Lig+Comp-Prot*c)*(Kcomp*(Lig-Prot*c)+Klig*(Comp- Prot*c)+Klig*Kcomp)-27*(-1*Klig*Kcomp*Prot*c))/(2*((((Klig+Kcomp+Lig+Comp-Prot*c)^2- 3*(Kcomp*(Lig-Prot*c)+Klig*(Comp-Prot*c)+Klig*Kcomp))^3)^0.5)))/3))- (Klig+Kcomp+Lig+Comp-Prot*c)))/((3*Klig)+((2*((Klig+Kcomp+Lig+Comp-Prot*c)^2- 3*(Kcomp*(Lig-Prot*c)+Klig*(Comp-Prot*c)+Klig*Kcomp))^0.5*COS(ARCCOS((- 2*(Klig+Kcomp+Lig+Comp-Prot*c)^3+9*(Klig+Kcomp+Lig+Comp-Prot*c)*(Kcomp*(Lig- Prot*c)+Klig*(Comp-Prot*c)+Klig*Kcomp)-27*(- 1*Klig*Kcomp*Prot*c))/(2*((((Klig+Kcomp+Lig+Comp-Prot*c)^2-3*(Kcomp*(Lig- Prot*c)+Klig*(Comp-Prot*c)+Klig*Kcomp))^3)^0.5)))/3))-(Klig+Kcomp+Lig+Comp-Prot*c))))

Selected peptides of the invention were tested in the above-mentioned assay and the results are shown in Table 2.

**Table 2: Competition Fluorescence Polarisation Assay Data for Selected Bicyclic Peptides of the Invention**

| ***Bicyclic Peptide*** | ***Ki (nM)*** |
|---|---|
| BCY15100 | 49 |
| BCY14455 | 282 |
| BCY15633 | 86 |
| BCY15634 | 98 |
| BCY15635 | 61 |
| BCY15636 | 143 |
| BCY15638 | 255 |
| BCY15639 | 17 |
| BCY15640 | 156 |
| BCY15641 | 129 |
| BCY15642 | 910 |
| BCY15643 | 755 |
| BCY15644 | 159 |
| BCY15645 | >5000 |
| BCY15646 | 107 |
| BCY15648 | 1619 |
| BCY15649 | 391 |
| BCY15650 | 16 |
| BCY15651 | 204 |
| BCY15653 | 411 |
| BCY15654 | >5000 |
| BCY15655 | 324 |
| BCY15656 | 39 |
| BCY15657 | >5000 |
| BCY15658 | >5000 |
| BCY15659 | 226 |
| BCY15660 | >5000 |
| BCY15661 | 1779 |
| BCY15662 | 672 |
| BCY16130 | 321 |
| BCY16131 | >5000 |
| BCY16132 | 8 |
| BCY16133 | 10 |
| BCY16134 | 77 |
| BCY16135 | 639 |
| BCY16136 | 269 |
| BCY16137 | >5000 |
| BCY16138 | >5000 |
| BCY16139 | >5000 |
| BCY16140 | 122 |
| BCY16141 | >5000 |
| BCY16142 | 317 |
| BCY16143 | 464 |
| BCY16144 | 718 |
| BCY16145 | >5000 |

### 3a. NKp46 SPR Assay (Method 1)

SPR experiments were performed to determine kₐ (M⁻¹s⁻¹), k_{d}(s⁻¹), K_{D} (nM) values (where measurable) of various bicyclic peptides to immobilised NKp46.

For analysis of peptide binding to NKp46, a Biacore 3000 instrument was used with a Cytiva Streptavidin chip. Capture of the protein (recombinant human NKp46-Fc fusion biotinylated via Avi-tag) was carried out at 25°C with HBS-N (10 mM HEPES, 0.15 M NaCl, pH 7.4) as the running buffer and biotinylated NKp46 was captured to a level of 400-800 RU using a dilution of protein to 0.2µM in buffer. Buffer was then changed to PBS/0.05% Tween 20 and a dilution series of the peptides was prepared in this buffer with a final DMSO concentration of 0.5% with a top peptide concentration of 100nM-5000nM and 6 further 2 or 3-fold dilutions. The SPR analysis was run at 25°C and a flow rate of 50µl/min with 60 seconds association and 200-400 seconds dissociation time. Data were corrected for DMSO excluded volume effects. All data were double-referenced for blank injections and reference surface using standard processing procedures and data processing and kinetic fitting were performed using Scrubber software, version 2.0c (BioLogic Software). Data were fitted using simple 1:1 binding model allowing for mass transport effects where appropriate.

Selected peptides of the invention were tested in the above-mentioned assay and the results are shown in Table 3A.

**Table 3A: SPR Assay Data for Selected Bicyclic Peptides of the Invention**

| ***Bicyclic Peptide*** | ***Kd (nM)*** |
|---|---|
| BCY15013 | 138 |
| BCY15452 | 27 |
| BCY15686 | 276 |
| BCY15687 | 960 |

### 3b. NKp46 SPR Assay (Method 2)

Peptides were tested against Fc-tagged human NKp46 (ACROBiosystems, cat no. NC1-H5257).

For analysis of peptide binding, a Biacore 8k+ instrument was used with a CM5 chip (Cytiva). All experiments were carried out at 25°C. Anti-human IgG (Fc) antibody (Cytiva) was immobilized on all flow cells by standard amine coupling chemistry. The running buffer was HBS-N (Cytiva, 10 mM HEPES, 0.15 M NaCl, pH 7.4) and the flow rate was 10 µL/min. The carboxymethyl dextran surface was activated with a 1:1 ratio of 0.4 M 1-ethyl-3-(3-dimethylaminopropyl) carbodiimide hydrochloride (EDC)/0.1 M N-hydroxy succinimide (NHS) (Cytiva) for 420s. Anti-human IgG (Fc) antibody was diluted to 12.5 µg/mL in 10 mM sodium acetate pH 5.0 and injected onto the chip surface for 360 s. Residual activated groups were blocked with a 420 s injection of 1 M ethanolamine (pH 8.5). The final surface density was 3000 - 5000 RU. Fc-tagged human NKp46 was diluted to 0.1 µM in HBS-N and was captured on flow cell 2 only of the sensor chip at a flow rate of 10 µL/min to 500 - 1000 RU. The buffer was then changed to PBS-P+ (Cytiva) supplemented with 2 % DMSO. Peptide dilutions were prepared in this buffer to give a final concentration of 2 % DMSO. The flow rate was 50 µL/min with 60 s association and 400 s dissociation. Data were corrected for DMSO volume exclusion effects. All data were double-referenced against blank injections and reference surface response using standard processing procedures. Data processing and kinetic fitting was performed using Biacore Insight Evaluation Software with Extended Screening and Characterization Extension (Cytiva). Data were fitted using steady state affinity 1:1 binding model to determine K_{D}. Data were fitted using kinetic 1:1 binding model to determine kinetic constants where appropriate.

Selected peptides of the invention were tested in Method 1 and/or Method 2 and the results are shown in Table 3B.

**Table 3B: SPR Assay Data for Selected Bicyclic Peptides of the Invention**

| ***Bicyclic Peptide*** | ***Kd (nM)*** |
|---|---|
| BCY15686 | 250±24 (n=3) |
| BCY15452 | 13±3 (n=2) |
| BCY14455 | 230 (n=1) |
| BCY15100 | 36±14 (n=4) |
| BCY16133 | 4.9 (n=1) |
| BCY17224 | 3.5±1 (n=6) |
| BCY14454 | 2760 (n=1) |
| BCY14456 | 703 (n=1) |
| BCY14457 | 2270 (n=1) |
| BCY14458 | 1260 (n=1) |
| BCY15013 | 59 (n=1) |
| BCY15098 | 1070 (n=2) |
| BCY15099 | 1117±681 (n=7) |
| BCY15102 | 1090±99 (n=2) |
| BCY15103 | 1665±544 (n=2) |
| BCY15104 | 2880 (n=1) |
| BCY15105 | 3730 (n=1) |
| BCY15106 | 2670 (n=1) |
| BCY15107 | 396±114 (n=9) |
| BCY15108 | 642 (n=1) |
| BCY15109 | 274 (n=1) |
| BCY15568 | 69 (n=1) |
| BCY15637 | 131 (n=1) |
| BCY15639 | 11±8 (n=2) |
| BCY15647 | 80 (n=1) |
| BCY15650 | 10±3 (n=2) |
| BCY15652 | 44 (n=1) |
| BCY15656 | 34±3 (n=2) |
| BCY15687 | 783±167 (n=3) |
| BCY16132 | 3.9 (n=1) |
| BCY16834 | 83 (n=1) |
| BCY16837 | 42 (n=1) |
| BCY16840 | 60 (n=1) |
| BCY16842 | 2470 (n=1) |
| BCY16843 | 3010 (n=1) |
| BCY17662 | 8.1 (n=1) |
| BCY18004 | 414 (n=1) |
| BCY18005 | 729 (n=1) |
| BCY18260 | 1240 (n=1) |
| BCY18261 | 543 (n=1) |
| BCY18262 | 3180 (n=1) |
| BCY18263 | 1740 (n=1) |
| BCY18265 | 1480 (n=1) |
| BCY18266 | >10000 (n=1) |
| BCY18269 | 1640 (n=1) |
| BCY18272 | 3190 (n=1) |
| BCY18273 | 326 (n=1) |
| BCY18277 | 1520 (n=1) |
| BCY18278 | 335 (n=1) |
| BCY18279 | 599 (n=1) |
| BCY18280 | 1270 (n=1) |
| BCY18281 | 424 (n=1) |
| BCY18282 | 661 (n=1) |
| BCY18285 | 3760 (n=1) |
| BCY18286 | 1230 (n=1) |
| BCY18287 | 637 (n=1) |
| BCY18288 | 354 (n=1) |
| BCY18289 | 572 (n=1) |
| BCY18290 | 4890 (n=1) |
| BCY18291 | 2850 (n=1) |
| BCY18292 | 1460 (n=1) |
| BCY18293 | 3380 (n=1) |
| BCY18294 | 3260 (n=1) |
| BCY18295 | 6410 (n=1) |
| BCY18299 | 1800 (n=1) |
| BCY18301 | 4030 (n=1) |
| BCY18302 | 5380 (n=1) |
| BCY18303 | 2750 (n=1) |
| BCY18304 | 1530 (n=1) |
| BCY18305 | 907 (n=1) |
| BCY18307 | 2110 (n=1) |
| BCY18309 | 2570 (n=1) |
| BCY18310 | 802 (n=1) |
| BCY18311 | 795 (n=1) |
| BCY18312 | 1810 (n=1) |
| BCY18313 | 2750 (n=1) |
| BCY18314 | 3150 (n=1) |
| BCY18315 | 878 (n=1) |
| BCY18316 | 1510 (n=1) |
| BCY18317 | 2080 (n=1) |
| BCY18318 | 1920 (n=1) |
| BCY18319 | 991 (n=1) |
| BCY18320 | 622 (n=1) |
| BCY18321 | 548 (n=1) |
| BCY18322 | 466 (n=1) |
| BCY18323 | 2340 (n=1) |
| BCY18324 | 1500 (n=1) |
| BCY18325 | 854 (n=1) |
| BCY18326 | 2780 (n=1) |
| BCY18327 | 1670 (n=1) |
| BCY18328 | 887 (n=1) |
| BCY18433 | 36 (n=1) |
| BCY18434 | 44 (n=1) |
| BCY18435 | 91 (n=1) |
| BCY18437 | 47 (n=1) |
| BCY18439 | 36 (n=1) |
| BCY18440 | 149 (n=1) |
| BCY18441 | 7.7 (n=1) |
| BCY18442 | 7.1 (n=1) |
| BCY18443 | 6.3 (n=1) |
| BCY18444 | 3.9 (n=1) |
| BCY18470 | 22 (n=1) |
| BCY19682 | 9 (n=1) |
| BCY19683 | 10 (n=1) |
| BCY19684 | 9.4 (n=1) |
| BCY19685 | 10 (n=1) |
| BCY19686 | 44 (n=1) |
| BCY19687 | 28 (n=1) |
| BCY19688 | >500 (n=1) |
| BCY19689 | 16 (n=1) |
| BCY19690 | 19 (n=1) |
| BCY19691 | >500 (n=1) |
| BCY19692 | >500 (n=1) |
| BCY19693 | 17 (n=1) |
| BCY19694 | 2510 (n=1) |
| BCY19695 | 2380 (n=1) |
| BCY19696 | >10000 (n=1) |
| BCY19697 | 3730 (n=1) |
| BCY19698 | >10000 (n=1) |
| BCY19699 | 2610 (n=1) |
| BCY19700 | >10000 (n=1) |
| BCY19701 | 4030 (n=1) |
| BCY19702 | 2680 (n=1) |
| BCY19703 | 1690 (n=1) |
| BCY19704 | 1620 (n=1) |
| BCY19706 | 1200 (n=1) |
| BCY19707 | 1050 (n=1) |
| BCY19708 | 1740 (n=1) |
| BCY19710 | 2360 (n=1) |
| BCY19711 | 2750 (n=1) |
| BCY19712 | 3140 (n=1) |
| BCY19713 | 1830 (n=1) |
| BCY19717 | 598 (n=1) |
| BCY19718 | 1110 (n=1) |
| BCY19719 | 323 (n=1) |
| BCY19720 | 3750 (n=1) |
| BCY19721 | 374 (n=1) |
| BCY19722 | 1460 (n=1) |
| BCY19723 | 2270 (n=1) |
| BCY19724 | 1390 (n=1) |
| BCY19725 | 457 (n=1) |
| BCY19730 | >10000 (n=1) |
| BCY19732 | 964 (n=1) |
| BCY19733 | 354 (n=1) |
| BCY19734 | 611 (n=1) |
| BCY19735 | 460 (n=1) |

### 4. Binding to CHO-K1/NKp46 cell line

The affinity of NKp46 dimeric bicyclic peptides conjugated to Alexa Fluor^{®} 488 to commercially available NKp46 overexpressing CHO-K1 cells was determined using a flow cytometry assay.

Commercially available CHO-K1 cells stably transfected with human activating natural cell receptor 46 (NKp46, also known as natural cytotoxicity triggering receptor 1 (NCR1) or CD355), herein referred to as CHO-K1/NKp46 cells, were procured from Abeomics. CHO-K1/NKp46 cells were grown in DMEM medium (Corning^{®} 15-017 CV) supplemented with 10% heat-inactivated fetal bovine serum (FBS; Corning^{®} 35-01 1-CV), 10 mM HEPES (Gibco^{™} 15-630-080), 1% Penicillin Streptomycin (Corning^{™} 30-002-CI), and 10 µg/mL of Blasticidin (Gibco^{™} A1113902). Culture maintenance and passage conditions were followed according to manufacturer recommendations.

On the day of the experiment, CHO-K1/NKp46 were detached from the culture vessel with Trypsin/EDTA, washed once at 500 rpm for 5 minutes in 15 mL of prewarmed working medium defined as: DMEM medium (Corning^{®} 15-017 CV) supplemented with 10% heat-inactivated fetal bovine serum (FBS; Corning^{®} 35-011-CV), 10 mM HEPES (Gibco^{™} 15-630-080), and 1% Penicillin Streptomycin (Corning^{™} 30-002-CI). Cell pellet was then resuspended in working medium at a concentration of 3×10⁵ cells/mL. Subsequently, 100 µL of cell suspension was plated in a 96-well V-bottom polypropylene plate (Greiner Bio-One 651201).

NKp46 dimeric bicyclic peptides conjugated to Alexa Fluor^{®} 488 were diluted in working medium and were added to the plate at a starting concentration of 1 µM and titrated in a 1:4 dilution series to perform an 11-point serial dilution. Plates were then incubated for 1 hour at room temperature in the dark. Post-incubation, the plates were centrifuged at 500 rpm for 5 minutes and supernatant discarded. Samples were then washed twice in 200 µL of 1X phosphate buffer saline (PBS; Gibco^{™} 10-010-023) at 500 rpm for 5 minutes. Cells were resuspended in 100 µL of PBS and transferred to a new 96-well V-bottom polypropylene plate (Greiner Bio-One 651201). Plates were centrifuged at 500 rpm for 5 minutes and supernatant discarded.

Preparation of samples for flow cytometry: Zombie Violet^{™} Fixable Viability Dye (BioLegend^{®} 423113) was prepared as a 1:1000 dilution in PBS and 100 µL of viability dye was added to each well and incubated in the dark at 4°C for 30 minutes. To ensure NKp46 expression, untreated CHO-K1/NKp46 cells were stained with either anti-human NKp46 or an isotype control. Antibody cocktails were prepared by diluting 1.5 µL of either, PE anti-human NKp46 (BioLegend^{®} 331908; clone 9E2) or PE isotype control IgG1, *κ* (BioLegend^{®} 400112), per 100 µL of stain buffer (1X PBS supplemented with 2% FBS).Cells were resuspended in master mix cocktail (100 µL) and incubated at 4°C for 30 minutes in the dark. Subsequently, cells were washed 3 times in 100 µL of stain buffer for 5 minutes at 500 rpm and supernatant was discarded. Cells resuspended in 200 µL of stain buffer were kept at 4°C and in the dark until read by BD FACSCelesta^{™} flow cytometer.

Flow cytometry results were analyzed in FlowJo^{™} software with a gating strategy to exclude debris, gate on single and live cells only, and determining the geometric mean of the Alexa Fluor^{®} 488 +/- populations. The binding affinity of the bicyclic peptides were calculated using a four-parameter logistic regression using Prism GraphPad software.

The data shown in Figure 1 and Table 4 illustrates that Alexa Fluor^{®} 488-tagged NKp46 bicyclic peptide dimers bind in a dose-dependent manner to NKp46 expressing cells.

**Table 4: Binding affinities (K_{d,app}) for the Alexa Fluor^{®} 488 NKp46 dimer bicyclic peptides binding to CHO-K1/NKp46 cells**

| **Alexa Fluor**^{®} **488 NKp46 Dimer Bicyclic Peptide** | **Binding Affinity to CHO-K1/NKp46 population K_{d,app} (nM)** |
|---|---|
| BCY15921 | 1.48 |
| BCY15922 | 1.48 |
| BCY15455 | 2.25 |
| BCY15663 | 0.30 |

### 5. Binding to PBMCs

The affinity of NKp46 dimeric bicyclic peptides conjugated to Alexa Fluor^{®} 488 to primary healthy peripheral blood mononuclear cells (PBMCs) was evaluated using a flow cytometry assay.

On the day of the experiment, medium was prepared by supplementing RPMI-1640 (Gibco^{™} 11875-093; with L-glutamine) with 10% heat-inactivated fetal bovine serum (FBS; Corning^{®} 35-011-CV), 10 mM HEPES (Gibco^{™} 15-630-080), and 1% Penicillin Streptomycin (Corning^{™} 30-002-CI), herein referred to as working medium. Previously isolated human peripheral blood mononuclear cells (PBMCs), from whole blood, were quick thawed in a water bath and washed once at 500 rpm for 5 minutes in 10 mL of prewarmed working medium. PBMC pellet was then resuspended to concentration of 5×10⁶ cells/mL in working medium and rested overnight (12-18 hours) horizontally in a tissue-culture coated flask (T-183; CELLTREAT Scientific 229351).

Post-overnight incubation, PBMCs were removed from the flask and washed once at 500 rpm for 5 minutes in 10 mL of prewarmed working medium. PBMC pellet was then resuspended in working medium at a concentration of 5×10⁵ cells/mL. Subsequently, 100 µL of cell suspension was plated in a 96-well V-bottom polypropylene plate (Greiner Bio-One 651201). Bicyclic peptides were diluted in working medium and added to the corresponding cell plate at a starting concentration of 300 nM titrated in a 1:4 dilution series to perform a 12-point serial dilution. Plates were then incubated 1 hour at 37°C, 5% CO₂. Post-incubation, plate was centrifuged at 500 rpm for 5 minutes and supernatant discarded. Samples were then washed once in 200 µL of 1X phosphate buffer saline (PBS; Gibco^{™} 10-010-023) at 500 rpm for 5 minutes and supernatant was discarded.

Preparation of samples for flow cytometry: Zombie Aqua^{™} Fixable Viability Dye (BioLegend^{®} 423102) was prepared as a 1:1000 dilution in PBS and 100 µL of viability dye was added to each well and incubated in the dark at 4°C for 30 minutes. Subsequently, wells were washed with 100 µL of PBS for 5 minutes at 500 rpm and supernatant was discarded. Next, human TruStain FcX^{™} block (BioLegend^{®} 422302) was prepared by diluting 1.5 µL of FcX in 25 µL of stain buffer (1X PBS supplemented with 2% FBS). Fc block solution (25 µL/well) was incubated at room temperature (RT) for 10 minutes in the dark. Antibody master mix cocktail was prepared by diluting 1.5 µL the following antibodies per 100 µL of stain buffer: Brilliant Violet 605^{™} anti-human CD3 (BioLegend^{®} 344836; clone SK7), and PE/Cyanine7 anti-human CD56 (BioLegend^{®} 362510; clone 5.1H11). Cells were resuspended in master mix cocktail (100 µL) and incubated at 4°C for 30 minutes in the dark. Subsequently, cells were washed 3 times in 100 µL of stain buffer for 5 minutes at 500 rpm and supernatant was discarded. Cells were resuspended in 200 µL of stain buffer and kept at 4°C in the dark until read by BD FACSCelesta^{™} flow cytometer.

Flow cytometry results were analyzed in FlowJo^{™} software with a gating strategy to evaluate the lymphocyte population then exclude debris, gate on single and live cells only, and determining the geometric mean of the Alexa Fluor^{®} 488 in the CD3+ and CD3-/CD56+ populations. The binding affinities were calculated using a four-parameter logistic regression using Prism GraphPad^{™} 8.0.2 software.

**Table 5: Binding affinities (K_{d,app}) for the Alexa Fluor^{®} 488 NKp46 dimers binding to sub-populations in PBMC cells**

| **Alexa Fluor**^{®} **488 NKp46 Dimer Bicyclic Peptide** | **Binding Affinity to Total CD56 population K_{d},ₐₚₚ (nM)** |
|---|---|
| BCY15663 | 0.028 ± 0.006 |

The data shown in Table 5 and Figure 2 demonstrates that Alexa Fluor^{®} 488-tagged NKp46 BCY15663 elicits a dose-dependent binding to NK cells only (CD56+ populations). Comparatively, non-binding Alexa Fluor^{®} 488-tagged NKp46 BCY15665 elicits no binding in any PBMC population.

### 6a. FcγRIII (CD16a) SPR experimental description (Method A)

SPR experiments were performed to determine kₐ (M⁻¹s⁻¹), k_{d} (s⁻¹), K_{D} (nM) values (where measurable) of various bicyclic peptides to immobilized human FcγRIII proteins.

For analysis of peptide binding, a Biacore 3000 or T200 instrument was used with a CM5 chip (GE Healthcare). Streptavidin was immobilized on the chip using standard amine-coupling chemistry at 25°C with HBS-N (10 mM HEPES, 0.15 M NaCl, pH 7.4) as the running buffer. Briefly, the carboxymethyl dextran surface was activated with a 7 min injection of a 1:1 ratio of 0.4 M 1-ethyl-3-(3-dimethylaminopropyl) carbodiimide hydrochloride (EDC) / 0.1 M *N-*hydroxy succinimide (NHS) at a flow rate of 10 µl/min. For capture of streptavidin, the protein was diluted to 0.2 mg/ml in 10 mM sodium acetate (pH 4.5) and captured by injecting 120µl of onto the activated chip surface. Residual activated groups were blocked with a 7 min injection of 1 M ethanolamine (pH 8.5) and biotinylated FcγRIII captured to a level of 400-1,400 RU. Buffer was changed to PBS/0.05% Tween 20 and a dilution series of the peptides was prepared in this buffer with a final DMSO concentration of 0.5%. The top peptide concentration ranged from 200nM - 20µM (depending on expected affinity) with 6 further 2-or 3-fold dilutions. The SPR analysis was run at 25°C at a flow rate of 50µl/min with 60 seconds association and dissociation between 60 and 200 seconds. Data were corrected for DMSO excluded volume effects. All data were double-referenced for blank injections and reference surface using standard processing procedures and data processing and kinetic fitting were performed using Scrubber software, version 2.0c (BioLogic Software). Data were fitted using simple 1:1 binding model allowing for mass transport effects where appropriate.

Selected peptides of the invention were tested in the above-mentioned assay and the results are shown in Table 6A:

**Table 6A: SPR Assay Data for Selected Bicyclic Peptides of the Invention**

| ***Bicyclic Peptide*** | ***Kd (nM)*** |
|---|---|
| BCY13886 | 2200 |
| BCY13883 | 1800 |

### 6b. FcγRIII (CD16a) SPR experimental description (Method B)

For analysis of peptide binding, a Biacore 8k+ instrument was used with a CM5 chip (Cytiva). All experiments were carried out at 25°C. Streptavidin was immobilized on all flow cells by standard amine coupling chemistry. The running buffer was HBS-N (Cytiva, 10 mM HEPES, 0.15 M NaCl, pH 7.4) and the flow rate was 10 µl/min. The carboxymethyl dextran surface was activated with a 1:1 ratio of 0.4 M 1-ethyl-3-(3-dimethylaminopropyl) carbodiimide hydrochloride (EDC)/0.1 M N-hydroxy succinimide (NHS) (Cytiva) for 420s. Streptavidin (ProSpec, cat no. PRO-791) was diluted to 0.2 mg/ml in 10 mM sodium acetate pH 4.5 and was injected onto the chip surface for 600 s. Residual activated groups were blocked with a 420 s injection of 1 M ethanolamine (pH 8.5). The final surface density was 2000 - 3000 RU. Biotinylated CD16a was diluted to 5 µg/ml in HBS-N and was captured on flow cell 2 only of the streptavidin chip at a flow rate of 10 µl/min to 1500 - 2500 RU. The buffer was then changed to PBS-P+ (Cytiva) supplemented with 0.5-2 % DMSO. Peptide dilutions were prepared in this buffer to give a final concentration of 0.5-2 % DMSO. The flow rate was 30 µl/min with 100 s association and 400 s dissociation. Data were corrected for DMSO volume exclusion effects. All data were double-referenced against blank injections and reference surface response using standard processing procedures. Data processing and kinetic fitting was performed using Biacore Insight Evaluation Software with Extended Screening and Characterization Extension (Cytiva). Data were fitted using steady state affinity 1:1 binding model to determine K_{D}. Data were fitted using kinetic 1:1 binding model to determine kinetic constants where appropriate.

Selected peptides of the invention were tested in Method A and/or Method B and the results are shown in Table 6B:

**Table 6B: SPR Assay Data for Selected Bicyclic Peptides of the Invention**

| ***Bicyclic Peptide*** | ***Kd (nM) (F Variant)*** | ***Kd (nM) (V Variant)*** |
|---|---|---|
| BCY11673 | 17130 (n=1) | 3306 (n=1) |
| BCY11808 | 6800 (n=1) | 3710 (n=1) |
| BCY11809 | 28560 (n=1) | 4326 (n=1) |
| BCY11810 | 3611 (n=1) | 831.5 (n=1) |
| BCY11811 | 8237 (n=1) | 1226 (n=1) |
| BCY11812 | 11800 (n=1) | 2404 (n=1) |
| BCY12621 | 3600 (n=1) | 1900 (n=1) |
| BCY12622 | 1000 (n=1) | 4000 (n=1) |
| BCY13883 | 1759±496 (n=7) | 1518±677 (n=7) |
| BCY13886 | 8358±5291 (n=4) | 4285±1743 (n=4) |
| BCY14950 | 1992±534 (n=5) | 1840±1197 (n=5) |
| BCY14953 | 2980 (n=1) | 2440 (n=1) |
| BCY14954 | 6160 (n=1) | 4350 (n=1) |
| BCY14955 | 1520 (n=1) | 440 (n=1) |
| BCY14956 | 2660 (n=1) | 2150 (n=1) |
| BCY15044 | 15460 (n=1) | 39620 (n=1) |
| BCY15045 | 21940 (n=1) | 39650 (n=1) |
| BCY15685 | 7373±3880 (n=7) | 1846±377 (n=6) |
| BCY16165 | 3592±944 (n=5) | 3230 (n=1) |
| BCY16493 | 4400 (n=1) | 7330 (n=1) |
| BCY16494 | 2870 (n=1) | 2820 (n=1) |
| BCY16495 | 3900 (n=1) | 5150 (n=1) |
| BCY16498 | 2360 (n=1) | 2810 (n=1) |
| BCY16499 | 8750 (n=1) | 9590 (n=1) |
| BCY16501 | 7960±948 (n=2) | 3225±2510 (n=2) |
| BCY16502 | 3090 (n=1) | 4910 (n=1) |
| BCY16504 | >5000 (n=1) | >5000 (n=1) |
| BCY16505 | >5000 (n=1) | >5000 (n=1) |
| BCY16506 | >5000 (n=1) | >5000 (n=1) |
| BCY16507 | >5000 (n=1) | >5000 (n=1) |
| BCY16508 | 2980 (n=1) | 4590 (n=1) |
| BCY16509 | 2820 (n=1) | 4990 (n=1) |
| BCY16510 | 3180 (n=1) | >5000 (n=1) |
| BCY16511 | 4020 (n=1) | 5000 (n=1) |
| BCY16512 | 4200 (n=1) | 4390 (n=1) |
| BCY16520 | >5000 (n=1) | >5000 (n=1) |
| BCY16522 | 2560 (n=1) | 4400 (n=1) |
| BCY16523 | 2600 (n=1) | 4870 (n=1) |
| BCY16524 | 3890 (n=1) | >5000 (n=1) |
| BCY16527 | 3970 (n=1) | >5000 (n=1) |
| BCY16528 | 3060 (n=1) | >5000 (n=1) |
| BCY16529 | >5000 (n=1) | >5000 (n=1) |
| BCY16599 | 1880 (n=1) | 386 (n=1) |
| BCY16608 | 2890 (n=1) | 516 (n=1) |
| BCY16610 | 896±631 (n=2) | 614±395 (n=2) |
| BCY19032 | 10500 (n=1) | 4640 (n=1) |
| BCY19033 | 1017±487 (n=2) | 421±133 (n=2) |
| BCY19034 | 1810 (n=1) | 1080 (n=1) |
| BCY19035 | 4000 (n=1) | 1110 (n=1) |
| BCY19036 | 618±249 (n=4) | 382±117 (n=4) |
| BCY19037 | 1070 (n=1) | 737 (n=1) |
| BCY19038 | >10000 (n=1) | 6850 (n=1) |
| BCY19039 | 772 (n=1) | 281 (n=1) |
| BCY19040 | 1600 (n=1) | 1240 (n=1) |
| BCY19041 | 1820 (n=1) | 1310 (n=1) |
| BCY19042 | 4270 (n=1) | 1370 (n=1) |
| BCY19043 | 797 (n=1) | 731 (n=1) |
| BCY19044 | 1670 (n=1) | 858 (n=1) |
| BCY19045 | 1260 (n=1) | 744 (n=1) |
| BCY19046 | 1600 (n=1) | 902 (n=1) |
| BCY19048 | 3290 (n=1) | 5690 (n=1) |
| BCY19049 | 1300 (n=1) | 1140 (n=1) |
| BCY19050 | 2150 (n=1) | 2850 (n=1) |
| BCY19053 | 2720 (n=1) | 2620 (n=1) |
| BCY19055 | 5880 (n=1) | 6960 (n=1) |
| BCY19057 | 7650 (n=1) | 6110 (n=1) |
| BCY19060 | 5880 (n=1) | 3090 (n=1) |
| BCY19061 | 3730 (n=1) | 6320 (n=1) |
| BCY19063 | >10000 (n=1) | 25400 (n=1) |
| BCY19064 | 2440 (n=1) | 2960 (n=1) |
| BCY19065 | 2900 (n=1) | 1320 (n=1) |
| BCY19068 | > 10000 (n=1) | >10000 (n=1) |
| BCY19416 | >10000 (n=1) | >10000 (n=1) |
| BCY19417 | >10000 (n=1) | >10000 (n=1) |
| BCY19596 | 1710 (n=1) | 837 (n=1) |
| BCY20360 | 998 (n=1) | 496 (n=1) |
| BCY20361 | 553 (n=1) | 401 (n=1) |
| BCY20363 | > 10000 (n=1) | >10000 (n=1) |
| BCY20364 | 4151 (n=1) | 1371 (n=1) |
| BCY20365 | 6790 (n=1) | 3486 (n=1) |
| BCY20366 | 706 (n=1) | 244 (n=1) |
| BCY20367 | 1673 (n=1) | 473 (n=1) |
| BCY20368 | 1428 (n=1) | 531 (n=1) |
| BCY20369 | 910 (n=1) | 415 (n=1) |
| BCY20370 | 1267 (n=1) | 639 (n=1) |
| BCY20371 | 2151 (n=1) | 1795 (n=1) |
| BCY20372 | 2414 (n=1) | 869 (n=1) |
| BCY20373 | 2696 (n=1) | 1017 (n=1) |
| BCY20374 | 958 (n=1) | 799 (n=1) |
| BCY20375 | 4040 (n=1) | 2156 (n=1) |
| BCY20376 | 2875 (n=1) | 1542 (n=1) |
| BCY20377 | 5171 (n=1) | 3566 (n=1) |
| BCY20378 | 5206 (n=1) | 1978 (n=1) |
| BCY20379 | 4319 (n=1) | 1430 (n=1) |
| BCY20380 | 426 (n=1) | 152 (n=1) |
| BCY20382 | 385 (n=1) | 158 (n=1) |
| BCY20383 | 470 (n=1) | 202 (n=1) |
| BCY20384 | 263 (n=1) | 129 (n=1) |
| BCY20385 | 103 (n=1) | 36 (n=1) |
| BCY20386 | 352 (n=1) | 132 (n=1) |
| BCY20388 | 2983 (n=1) | 1075 (n=1) |
| BCY20389 | 459 (n=1) | 250 (n=1) |
| BCY20390 | >10000 (n=1) | 6652 (n=1) |
| BCY20392 | 825 (n=1) | 385 (n=1) |
| BCY20394 | 2376 (n=1) | 1001 (n=1) |
| BCY20395 | 3397 (n=1) | 2136 (n=1) |
| BCY20396 | 1659 (n=1) | 537 (n=1) |
| BCY20397 | 753 (n=1) | 430 (n=1) |
| BCY20526 | 371 (n=1) | 157 (n=1) |
| BCY20527 | 348 (n=1) | 147 (n=1) |
| BCY20528 | 395 (n=1) | 176 (n=1) |
| BCY20529 | 558 (n=1) | 249 (n=1) |

### 7. IgG Competition Assay

Bicyclic peptides were evaluated with a CD16a-IgG competition assay using Perkin Elmer AlphaScreen^{™} technology. Acceptor beads (Perkin Elmer 6762003) were chemically conjugated via a reductive amination reaction with a mouse IgG2a mAb to link via the free amines, based the manufacturer's protocol. Briefly, beads were centrifuged and resuspended in a 130mM sodium phosphate buffered solution pH 8.0 containing 0.2mg mAb, 20mM NaBH₃CN (Sigma 296945) and 0.06% P20 surfactant (Cytiva Life Sciences, BR-1000-54) and incubated on a rotary shaker at 44°C for 22 hours. Unbound sites were then blocked by adding a final concentration of 3mg/ml carboxymethoxylamine (CMO) (Sigma C13408), before continuing shaking incubation for a further 1 hour. Beads were washed by centrifugation and resuspension in 100mM TRIS-HCl pH 8.0 (Sigma T2694), before a final resuspension in PBS (GIBCO 14190) + 0.05% Proclin-300 (Sigma 48912-U), at 5mg/ml and storage at 4°C until use.

The assay was performed in white 384 well plates (PerkinElmer, 6007290) using assay buffer 25 mM HEPES (Sigma-Aldrich, H0887), 100 mM NaCl (Sigma-Aldrich, S5150), 0.5 % BSA (Sigma-Aldrich, A3294), 0.05 % P20 (Cytiva Life Sciences, BR-1000-54), pH7.4 for all dilutions. Bicyclic peptides were titrated and incubated with 10nM final concentration of C-terminally biotinylated human CD16a F176 (ACRO Biosystems CDA-H82E8) or N-terminally biotinylated V176 variant (R&D Systems 4325-Fc, biotinylated in-house) and incubated at room temperature for 30 minutes. Mouse IgG2a-conjugated acceptor beads described above were added at a final concentration of 20ug/ml and incubated at room temperature for 30 minutes under subdued lighting. Streptavidin coated donor beads (PerkinElmer, 6760002) were added a final concentration of 20ug/ml and incubated at room temperature for 60 minutes under subdued lighting. Luminescence upon excitation at 570nM was read using a BMG Labtech Pherastar FS plate-reader. Data was normalised to a high control containing all assay components except Bicyclic peptide and fit to a four-parameter nonlinear regression in GraphPad Prism 8.0.2 to generate an IC50 value.

Selected peptides of the invention were tested in the above-mentioned assay and the results are shown in Table 7 and Figures 3 and 4. The data in Table 7 and Figure 3 and 4 show that the peptides of the invention bind to a biologically relevant epitope on FcγRIIIA (F and V-variants) and are able to compete with IgG for binding to this epitope.

**Table 7: IgG Competition Assay Data for Selected Bicyclic Peptides of the Invention**

| ***Bicyclic Peptide*/*Dimer*** | ***IC50 (nM) (F Variant)*** | ***IC50 (nM) (V Variant)*** |
|---|---|---|
| BCY15685 | 11000 | 3480 |
| BCY13886 | 1830 | 3190 |
| BCY13883 | 1950 | 2540 |
| BCY15914 | 51.1 | 308 |
| BCY15915 | 27.8 | 32.5 |
| BCY15916 | 52.6 | 113 |

### 8. CD16a/FCgRIIIa HTRF Assay

Binding to cellular CD16a/FCgRllla (F158 variant) was determined through competition with IgG for binding to CD16a receptors expressed on HEK293 cells. This assay was based on fluorescence resonance energy transfer (FRET) whereby a flash of light will induce FRET between the CD16a Terbium donor dye from the IgG-d2 acceptor when the donor-acceptor pair is in close proximity, which resulting in a fluorescent signal. Unlabeled antibody or Bicycle peptides competing with the IgG-d2 acceptor reduce the overall signal in a dose-dependent manner which enables IC50-values to be determined.

The experimental procedure followed the manufacturer's instructions detailed in the product information for the CD16 (FCyRllla) kit (Cisbio-6FR3APAG). Two human IgG antibodies were included as controls (Cisbio- 6FR3APAG top concentration 267nM and R&D systems-1-001-A top concentration 3000 nM). The Tag-lite buffer was prepared to generate the IgG-d2 acceptor solution. Bicycle peptides were dispensed using Echo compound handler at a top concentration of 20 µM. The competing unlabeled antibodies and bicyclic peptides were added in a three-fold dilution series in Tag-lite buffer into PerkinElmer-ProxiPlate-384 Plus.

The HEK293 cell expressing Terbium-labelled CD16a were thawed and washed in PBS and added to the wells of PerkinElmer-ProxiPlate-384 Plus containing the competitor compounds. Finally, the acceptor IgG-d2 reagent was added and the plate was incubated for 2h at RT. The FRET signal was read on the Envision plate reader using Ex 340nM and Em 655/620nM settings. The data was plotted in Graph pad prism and IC50-values were determined to indicate unlabeled antibody or bicyclic peptide competition for cellular binding to the CD16a receptor.

Selected peptides of the invention were tested in the above-mentioned assay and the results are shown in Table 8:

**Table 8: IgG Competition Assay Data for Selected Bicyclic Peptides of the Invention (HTRF)**

| ***Bicyclic Peptide*** | **IC50 (nM)** |
|---|---|
| BCY11808 | 9445 |
| BCY14950 | 2035±635 (n=5) |
| BCY14953 | 1904 |
| BCY14955 | 1407±494 (n=2) |
| BCY14956 | 1405±204 (n=2) |
| BCY15044 | 7462 |
| BCY15045 | 4022 |
| BCY15685 | 7556 |
| BCY16493 | 4794 |
| BCY16599 | 1529 |
| BCY16608 | 1802 |
| BCY19033 | 1423 |
| BCY19034 | 1608 |
| BCY19035 | 2006 |
| BCY19036 | 953±12 |
| BCY19037 | 2347 |
| BCY19596 | 1619±107 (n=2) |
| BCY20366 | 685 |
| BCY20380 | 898 |
| BCY20382 | 749 |
| BCY20383 | 1107 |
| BCY20384 | 526 |
| BCY20385 | 229±132 (n=2) |
| BCY20386 | 1060 |
| BCY20387 | 278 |
| BCY20389 | 851 |
| BCY20526 | 590 |
| BCY20527 | 651 |
| BCY20528 | 617 |
| BCY21689 | 1241 |
| BCY21690 | 1088 |
| BCY21691 | 153 |
| BCY21692 | 88 |
| BCY21693 | 377 |
| BCY21694 | 166 |
| BCY21695 | 100 |
| BCY21696 | 102 |
| BCY21697 | 120 |

### LIST OF REFERENCES

Muta T et al, Nature. 1994 Mar 3;368(6466):70-3. doi: 10.1038/368070a0. Erratum in: Nature. 1994 May 26;369(6478):340. PMID: 8107887.
Ravetch JV, Bolland S. IgG Fc receptors. Annu Rev Immunol. 2001;19:275-90. doi: 10.1146/annurev.immunol.19.1.275. PMID: 11244038.
Arnould L et al, Br J Cancer. 2006 Jan 30;94(2):259-67. doi: 10.1038/sj.bjc.6602930. PMID: 16404427; PMCID: PMC2361112.
Zhang Wet al, J Clin Oncol. 2007 Aug 20;25(24):3712-8. doi: 10.1200/JCO.2006.08.8021. PMID: 17704420.,
Zhang X etal, Mol Immunol. 2020 Mar;119:48-58. doi: 10.1016/j.molimm.2020.01.009. Epub 2020 Jan 21. PMID: 31978707.,
Wu SY etal, Mol Cancer. 2020 Aug 6;19(1):120. doi: 10.1186/s12943-020-01238-x. PMID: 32762681; PMCID: PMC7409673.
Manches O et al, Blood. 2003 Feb 1;101(3):949-54. doi: 10.1182/blood-2002-02-0469. Epub 2002 Oct 3. PMID: 12393572.
Clynes RA etal, Nat Med. 2000 Apr;6(4):443-6. doi: 10.1038/74704. PMID: 10742152. Riddle DS etal, Hum Gene Ther. 2005 Jul;16(7):830-44. doi: 10.1089/hum.2005.16.830. PMID: 16000065.
Rataj F et al, Br J Cancer. 2019 Jan;120(1):79-87. doi: 10.1038/s41416-018-0341-1. Epub 2018 Nov 15. PMID: 30429531; PMCID: PMC6325122.
Caratelli S et al, Front Immunol. 2017 Apr 27;8:457. doi: 10.3389/fimmu.2017.00457. PMID: 28496440; PMCID: PMC5406408.
Salmon JE et al, Arthritis Rheum. 2001 Apr;44(4):739-50. doi: 10.1002/1529-0131(200104)44:4<739::AID-ANR129>3.0.CO;2-O. PMID: 11315912.
Morgan AW et al, Rheumatology (Oxford). 2003 Apr;42(4):528-33. doi: 10.1093/rheumatology/keg169. PMID: 12649399.
Wu J etal, J Clin Invest. 1997 Sep 1;100(5):1059-70. doi: 10.1172/JCI119616. PMID: 9276722; PMCID: PMC508280.
Nabbe KC et al, Arthritis Rheum. 2003 Jan;48(1):255-65. doi: 10.1002/art. 10721. PMID: 12528127.
Kleinau S et al, J Exp Med. 2000 May 1;191(9):1611-6. doi: 10.1084/jem.191.9.1611. PMID: 10790435; PMCID: PMC2213438.
Bolland S etal, Immunity. 2000 Aug;13(2):277-85. doi: 10.1016/s1074-7613(00)00027-3. PMID: 10981970.
Salmon JE et al, Arthritis Rheum. 2001 Apr;44(4):739-50. doi: 10.1002/1529-0131(200104)44:4<739::AID-ANR129>3.0.CO;2-O. PMID: 11315912.
Boruchov AM et al, J Clin Invest. 2005 Oct;115(10):2914-23. doi: 10.1172/JCI24772. Epub 2005 Sep 15. PMID: 16167082; PMCID: PMC1201664.
Chen JY etal, Arthritis Rheumatol. 2014 Nov;66(11):3113-21. doi: 10.1002/art.38813. PMID: 25154742; PMCID: PMC4232894.
Henriques A et al, Rheumatol Int. 2012 Apr;32(4):863-9. doi: 10.1007/s00296-010-1709-6. Epub 2011 Jan 8. PMID: 21221593.
Chen JY et al, Arthritis Rheumatol. 2014 Nov;66(11):3113-21. doi: 10.1002/art.38813. PMID: 25154742; PMCID: PMC4232894.
Clarkson SB et al, N Engl J Med. 1986 May 8;314(19):1236-9. doi: 10.1056/NEJM198605083141907. PMID: 2939345.
Flaherty MM etal, Toxicol Sci. 2012 Jan;125(1):299-309. doi: 10.1093/toxsci/kfr278. Epub 2011 Oct 24. PMID: 22025730.
Anthony RM et al, Science. 2008 Apr 18;320(5874):373-6. doi: 10.1126/science.1154315. PMID: 18420934; PMCID: PMC2409116.
Debré M et al, Lancet. 1993 Oct 16;342(8877):945-9. doi: 10.1016/0140-6736(93)92000-j. PMID: 8105212.,
Hsu CH etal, Pediatr Infect Dis J. 1993 Jun;12(6):509-12. doi: 10.1097/00006454-199306000-00010. PMID: 8345983.
Ortiz DF et al, Sci Transl Med. 2016 Nov 16;8(365):365ra158. doi: 10.1126/scitranslmed.aaf9418. PMID: 27856797.
Li X et al, Expert Opin Ther Targets. 2014 Mar;18(3):335-50. doi: 10.1517/14728222.2014.877891. PMID: 24521454; PMCID: PMC4019044.
Pasero C et al, Oncotarget. 2015 Jun 10;6(16):14360-73. doi: 10.18632/oncotarget.3965. PMID: 25961317; PMCID: PMC4546472.
Stringaris K et al, Haematologica. 2014 May;99(5):836-47. doi: 10.3324/haematol.2013.087536. Epub 2014 Jan 31. PMID: 24488563; PMCID: PMC4008119.
Chinnery F etal, Oncoimmunology. 2012 Sep 1;1(6):874-883. doi: 10.4161/onci.20636. PMID: 23162755; PMCID: PMC3489743.
Arnon TI et al, Blood. 2004 Jan 15;103(2):664-72. doi: 10.1182/blood-2003-05-1716. Epub 2003 Sep 22. PMID: 14504081.
Moretta Let al, Semin Immunol. 2006 Jun;18(3):151-8. doi: 10.1016/j.smim.2006.03.002. Epub 2006 May 26. PMID: 16730454.
Costello RT et al, Blood. 2002 May 15;99(10):3661-7. doi: 10.1182/blood.v99.10.3661. PMID: 11986221.
Garcia-Iglesias T et al, BMC Cancer. 2009 Jun 16;9:186. doi: 10.1186/1471-2407-9-186. PMID: 19531227; PMCID: PMC2704222.
Nayyar G et al, Front Oncol. 2019 Feb 11;9:51. doi: 10.3389/fonc.2019.00051. PMID: 30805309; PMCID: PMC6378304.
Stojanovic A et al, J Innate Immun. 2011;3(4):355-64. doi: 10.1159/000325465. Epub 2011 Apr 18. PMID: 21502747.
Sordo-Bahamonde C et al, Cancers (Basel). 2020 Apr 7;12(4):893. doi: 10.3390/cancers12040893. PMID: 32272610; PMCID: PMC7226138.
Watanabe M et al, Dis Esophagus. 2010 Nov;23(8):675-81. doi: 10.1111/j. 1442-2050.2010.01073.x. PMID: 20545975.
Izawa S et al, Cancer Immunol Immunother. 2011 Dec;60(12):1801-10. doi: 10.1007/s00262-011-1082-7. Epub 2011 Aug 3. PMID: 21811786.
Koo KC etal, PLoS One. 2013 Nov 4;8(11):e78049. doi: 10.1371/journal.pone.0078049. PMID: 24223759; PMCID: PMC3817174.
Sun C etal, Cell Mol Immunol. 2015 May;12(3):292-302. doi: 10.1038/cmi.2014.91. Epub 2014 Oct 13. PMID: 25308752; PMCID: PMC4654321.
Hasmim M et al, Front Immunol. 2015 Sep 23;6:482. doi: 10.3389/fimmu.2015.00482. PMID: 26441986; PMCID: PMC4585210.
Han B et al, J Immunol Res. 2018 Sep 3;2018:6248590. doi: 10.1155/2018/6248590. PMID: 30255106; PMCID: PMC6140275.
Stringaris K et al, Haematologica. 2014 May;99(5):836-47. doi: 10.3324/haematol.2013.087536. Epub 2014 Jan 31. PMID: 24488563; PMCID: PMC4008119.
Rocca YS et al, Front Immunol. 2016 Oct 10;7:413. doi: 10.3389/fimmu.2016.00413. PMID: 27777574; PMCID: PMC5056190.
Gauthier L et al, Cell. 2019 Jun 13;177(7):1701-1713.e16. doi: 10.1016/j.cell.2019.04.041. Epub 2019 May 30. PMID: 31155232.
Fauriat C et al, Blood. 2007 Jan 1;109(1):323-30. doi: 10.1182/blood-2005-08-027979. Epub 2006 Aug 29. PMID: 16940427.
Mamessier E etal, Cancer Res. 2011 Nov 1;71(21):6621-32. doi: 10.1158/0008-5472.CAN-11-0792. Epub 2011 Sep 21. PMID: 21937679.
Platonova S et al, Cancer Res. 2011 Aug 15;71(16):5412-22. doi: 10.1158/0008-5472.CAN-10-4179. Epub 2011 Jun 27. PMID: 21708957.
Levi I etal, Oncotarget. 2015 May 30;6(15):13835-43. doi: 10.18632/oncotarget.3453. PMID: 26079948; PMCID: PMC4537053.
Kim HS et al, Immunity. 2010 Feb 26;32(2):175-86. doi: 10.1016/j.immuni.2010.02.004. PMID: 20189481; PMCID: PMC2843589.
MacFarlane AW4th et al, Oncoimmunology. 2017 May 19;6(7):e1330235. doi: 10.1080/2162402X.2017.1330235. PMID: 28811973; PMCID: PMC5543845.
Pasero C et al, Oncotarget. 2015 Jun 10;6(16):14360-73. doi: 10.18632/oncotarget.3965. PMID: 25961317; PMCID: PMC4546472.
Stringaris K et al, Haematologica. 2014 May;99(5):836-47. doi: 10.3324/haematol.2013.087536. Epub 2014 Jan 31. PMID: 24488563; PMCID: PMC4008119.

## Claims

1. A peptide ligand specific for natural killer (NK) cells, or a pharmaceutically acceptable salt thereof, comprising a polypeptide comprising at least three reactive groups, separated by at least two loop sequences, and a molecular scaffold which forms covalent bonds with the reactive groups of the polypeptide such that at least two polypeptide loops are formed on the molecular scaffold, wherein the bicyclic peptide ligand comprises an amino acid sequence which is selected from:
CᵢNLQKPCᵢᵢMKYPCᵢᵢᵢ (SEQ ID NO: 1);
CᵢNLQNPCᵢᵢMKFPCᵢᵢᵢ (SEQ ID NO: 2);
CᵢNLQAPCᵢᵢMQTGKVCᵢᵢᵢ (SEQ ID NO: 3);
CᵢNLQAPCᵢᵢMLTGKVCᵢᵢᵢ (SEQ ID NO: 4; herein referred to as BCY18295 when complexed with TATA);
CᵢLLHDHCᵢᵢPNTHPKLCᵢᵢᵢ (SEQ ID NO: 5);
CᵢLLHEHCᵢᵢPNTHPKMCᵢᵢᵢ (SEQ ID NO: 6);
CᵢLLHDHCᵢᵢPNSHPEICᵢᵢᵢ (SEQ ID NO: 7);
CᵢLLHDHCᵢᵢPNTHPIICᵢᵢᵢ (SEQ ID NO: 8);
CᵢYLPDWLCᵢᵢGDEYCᵢᵢᵢ (SEQ ID NO: 9);
CᵢYLPDYLCᵢᵢGDEYCᵢᵢᵢ (SEQ ID NO: 10);
CᵢDLTTHNCᵢᵢQWGICᵢᵢᵢ (SEQ ID NO: 11; herein referred to as BCY18262 when complexed with TATA);
CᵢYLPDYLCᵢᵢGDEYCᵢᵢᵢ (SEQ ID NO: 12; herein referred to as BCY15633 when complexed with TATA);
CᵢYLPDWLCᵢᵢGDEYCᵢᵢᵢ (SEQ ID NO: 13);
Cᵢ[2,6DiMeTyr]LPDYLCᵢᵢGDEYCᵢᵢᵢ (SEQ ID NO: 14);
CᵢY[tBuAla]PDYLCᵢᵢGDEYCᵢᵢᵢ (SEQ ID NO: 15);
CᵢY[Cba]PDYLCᵢᵢGDEYCᵢᵢᵢ (SEQ ID NO: 16);
CᵢYL[HyP]DYLCᵢᵢGDEYCᵢᵢᵢ (SEQ ID NO: 17);
CᵢYL[Aze]DYLCᵢᵢGDEYCᵢᵢᵢ (SEQ ID NO: 18);
CᵢYL[Aib]DYLCᵢᵢGDEYCᵢᵢᵢ (SEQ ID NO: 19);
CᵢYLPD[1Nal]LCᵢᵢGDEYCᵢᵢᵢ (SEQ ID NO: 20);
CᵢYLPD[2Nal]LCᵢᵢGDEYCᵢᵢᵢ (SEQ ID NO: 21);
CᵢYLPD[3Pal]LCᵢᵢGDEYCᵢᵢᵢ (SEQ ID NO: 22);
CᵢYLPD[2,6DiMeTyr]LCᵢᵢGDEYCᵢᵢᵢ (SEQ ID NO: 23);
CᵢYLPDW[tBuAla]CᵢᵢGDEYCᵢᵢᵢ (SEQ ID NO: 24);
CᵢYLPDW[Cba]CᵢᵢGDEYCᵢᵢᵢ (SEQ ID NO: 25);
CᵢYLPDYLCᵢᵢ[dA]DEYCᵢᵢᵢ (SEQ ID NO: 26);
CᵢYLPDYLCᵢᵢGDE[2,6DiMeTyr]Cᵢᵢᵢ (SEQ ID NO: 27);
CᵢALPDYLCᵢᵢGDEYCᵢᵢᵢ (SEQ ID NO: 28);
CᵢYAPDYLCᵢᵢGDEYCᵢᵢᵢ (SEQ ID NO: 29);
CᵢYLADYLCᵢᵢGDEYCᵢᵢᵢ (SEQ ID NO: 30);
CᵢYLPAYLCᵢᵢGDEYCᵢᵢᵢ (SEQ ID NO: 31);
CᵢYLPDALCᵢᵢGDEYCᵢᵢᵢ (SEQ ID NO: 32);
CᵢYLPDYACᵢᵢGDEYCᵢᵢᵢ (SEQ ID NO: 33);
CᵢYLPDYLCᵢᵢADEYCᵢᵢᵢ (SEQ ID NO: 34);
CᵢYLPDYLCᵢᵢGAEYCᵢᵢᵢ (SEQ ID NO: 35);
CᵢYLPDYLCᵢᵢGDAYCᵢᵢᵢ (SEQ ID NO: 36);
CᵢYLPDYLCᵢᵢGDEACᵢᵢᵢ (SEQ ID NO: 37);
CᵢY[CSA]PDYLCᵢᵢGDEYCᵢᵢᵢ (SEQ ID NO: 38);
CᵢY[Cha]PDYLCᵢᵢGDEYCᵢᵢᵢ (SEQ ID NO: 39);
CᵢY[Cba]PDYLCᵢᵢ[dA]DEYCᵢᵢᵢ (SEQ ID NO: 40);
CᵢYLPD[4FPhe]LCᵢᵢGDEYCᵢᵢᵢ (SEQ ID NO: 41);
CᵢYLPD[4MeOPhe]LCᵢᵢGDEYCᵢᵢᵢ (SEQ ID NO: 42);
CᵢYLPD[NO2Phe]LCᵢᵢGDEYCᵢᵢᵢ (SEQ ID NO: 43);
CᵢYLPD[4MePhe]LCᵢᵢGDEYCᵢᵢᵢ (SEQ ID NO: 44);
CᵢYLPD[pCoPhe]LCᵢᵢGDEYCᵢᵢᵢ (SEQ ID NO: 45);
CᵢYLPD[pCaPhe]LCᵢᵢGDEYCᵢᵢᵢ (SEQ ID NO: 46);
CᵢYLPD[4tBuPhe]LCᵢᵢGDEYCᵢᵢᵢ (SEQ ID NO: 47);
CᵢYLPD[CF3Phe]LCᵢᵢGDEYCᵢᵢᵢ (SEQ ID NO: 48);
CᵢYLPD[4CNPhe]LCᵢᵢGDEYCᵢᵢᵢ (SEQ ID NO: 49);
CᵢYLPD[4ClPhe]LCᵢᵢGDEYCᵢᵢᵢ (SEQ ID NO: 50);
CᵢYLPD[HPhe]LCᵢᵢGDEYCᵢᵢᵢ (SEQ ID NO: 51);
Cᵢ[2,6DiMeTyr]LPDWLCᵢᵢGDEYCᵢᵢᵢ (SEQ ID NO: 52);
CᵢY[Cba]PDWLCᵢᵢGDEYCᵢᵢᵢ (SEQ ID NO: 53);
CᵢYL[Aze]DWLCᵢᵢGDEYCᵢᵢᵢ (SEQ ID NO: 54);
CᵢYLPDWLCᵢᵢ[dA]DEYCᵢᵢᵢ (SEQ ID NO: 55);
CᵢSLPDYLCᵢᵢGDEYCᵢᵢᵢ (SEQ ID NO: 56);
CᵢDLTTHNCᵢᵢQWGICᵢᵢᵢ (SEQ ID NO: 57);
CᵢDLTTHNCᵢᵢLWGVCᵢᵢᵢ (SEQ ID NO: 58);
CᵢNLQLHNCᵢᵢIWGVCᵢᵢᵢ (SEQ ID NO: 59);
CᵢPPTVYCᵢᵢHPLDCᵢᵢᵢ (SEQ ID NO: 60);
CᵢRWSVEDPCᵢᵢGAWCᵢᵢᵢ (SEQ ID NO: 61);
CᵢVGLEELGPCᵢᵢSDLCᵢᵢᵢ (SEQ ID NO: 62; herein referred to as BCY16494 when complexed with TBMT);
CᵢNLQQACᵢᵢLHTGKVCᵢᵢᵢ (SEQ ID NO: 63);
CᵢNLQAACᵢᵢMLTGKVCᵢᵢᵢ (SEQ ID NO: 64);
CᵢNLQAPCᵢᵢMATGKVCᵢᵢᵢ (SEQ ID NO: 65);
CᵢNLQAPCᵢᵢMLAGKVCᵢᵢᵢ (SEQ ID NO: 66);
CᵢNLQAPCᵢᵢMLTAKVCᵢᵢᵢ (SEQ ID NO: 67);
CᵢNLQAPCᵢᵢMLTGAVCᵢᵢᵢ (SEQ ID NO: 68);
CᵢNLQAPCᵢᵢMLTGKACᵢᵢᵢ (SEQ ID NO: 69);
CᵢN[tBuAla]QAPCᵢᵢMLTGKVCᵢᵢᵢ (SEQ ID NO: 70);
CᵢNLQA[Sar]CᵢᵢMLTGKVCᵢᵢᵢ (SEQ ID NO: 71);
CᵢNLQA[Cis-HyP]CᵢᵢMLTGKVCᵢᵢᵢ (SEQ ID NO: 72);
CᵢNLQA[HyP]CᵢᵢMLTGKVCᵢᵢᵢ (SEQ ID NO: 73);
CᵢNLQA[NMeAla]CᵢᵢMLTGKVCᵢᵢᵢ (SEQ ID NO: 74);
CᵢNLQAPCᵢᵢLLTGKVCᵢᵢᵢ (SEQ ID NO: 75);
CᵢNLQAPCᵢᵢ[Nle]LTGKVCᵢᵢᵢ (SEQ ID NO: 76);
CᵢNLQAPCᵢᵢ[Cba]LTGKVCᵢᵢᵢ (SEQ ID NO: 77);
CᵢNLQAPCᵢᵢMETGKVCᵢᵢᵢ (SEQ ID NO: 78);
CᵢNLQAPCᵢᵢMFTGKVCᵢᵢᵢ (SEQ ID NO: 79);
CᵢNLQAPCᵢᵢMYTGKVCᵢᵢᵢ (SEQ ID NO: 80);
CᵢNLQAPCᵢᵢM[Cba]TGKVCᵢᵢᵢ (SEQ ID NO: 81);
CᵢNLQAPCᵢᵢM[tBuAla]TGKVCᵢᵢᵢ (SEQ ID NO: 82);
CᵢNLQAPCᵢᵢM[Cha]TGKVCᵢᵢᵢ (SEQ ID NO: 83);
CᵢNLQAPCᵢᵢMLT[dA]KVCᵢᵢᵢ (SEQ ID NO: 84);
CᵢNLQAPCᵢᵢMLTGRVCᵢᵢᵢ (SEQ ID NO: 85);
CᵢNLQAPCᵢᵢMLTG[Agb]VCᵢᵢᵢ (SEQ ID NO: 86);
CᵢNLQAPCᵢᵢMLTGK[tBuGly]Cᵢᵢᵢ (SEQ ID NO: 87);
CᵢNLQAPCᵢᵢMLTGK[Chg]Cᵢᵢᵢ (SEQ ID NO: 88);
CᵢNLQAPCᵢᵢMLTGK[Cbg]Cᵢᵢᵢ (SEQ ID NO: 89);
CᵢNLQAPCᵢᵢMLTG[HArg]VCᵢᵢᵢ (SEQ ID NO: 90);
CᵢNLQA[Aze]CᵢᵢMLTGKVCᵢᵢᵢ (SEQ ID NO: 91);
CᵢNLQA[Pip]CᵢᵢMLTGKVCᵢᵢᵢ (SEQ ID NO: 92);
CᵢNLQAPCᵢᵢMVTGKVCᵢᵢᵢ (SEQ ID NO: 93);
CᵢNLQAPCᵢᵢM[Nle]TGKVCᵢᵢᵢ (SEQ ID NO: 94);
CᵢNLQAPCᵢᵢM[Nva]TGKVCᵢᵢᵢ (SEQ ID NO: 95);
CᵢNLQAPCᵢᵢMLTGK[AlloIle]Cᵢᵢᵢ (SEQ ID NO: 96);
CᵢNLQAPCᵢᵢMLTGK[EPA]Cᵢᵢᵢ (SEQ ID NO: 97);
CᵢNLQQPCᵢᵢMLTGKVCᵢᵢᵢ (SEQ ID NO: 98);
CᵢNLQA[Nva]CᵢᵢMLTGKVCᵢᵢᵢ (SEQ ID NO: 99);
CᵢNLQAPCᵢᵢMHTGKVCᵢᵢᵢ (SEQ ID NO: 100);
CᵢNLQAPCᵢᵢLHTGKVCᵢᵢᵢ (SEQ ID NO: 101);
CᵢNLQQACᵢᵢMQTGKVCᵢᵢᵢ (SEQ ID NO: 102);
CᵢLLHDHCᵢᵢPNTHPKMCᵢᵢᵢ (SEQ ID NO: 103);
CᵢDLTTHNᵢᵢIWGVCᵢᵢᵢ (SEQ ID NO: 104);
CᵢNLQLHNCᵢᵢIWGVCᵢᵢᵢ (SEQ ID NO: 105);
CᵢNLQLHNCᵢᵢQWGICᵢᵢᵢ (SEQ ID NO: 106);
Cᵢ[3tBuTyr]LPDYLCᵢᵢGDEYCᵢᵢᵢ (SEQ ID NO: 107);
CᵢYLPD[3tBuTyr]LCᵢᵢGDEYCᵢᵢᵢ (SEQ ID NO: 108);
CᵢYLPDYLCᵢᵢGDE[3tBuTyr]Cᵢᵢᵢ (SEQ ID NO: 109);
Cᵢ[K(PYA)]LPDYLCᵢᵢGDEYCᵢᵢᵢ (SEQ ID NO: 110);
CᵢYLP[K(PYA)]YLCᵢᵢGDEYCᵢᵢᵢ (SEQ ID NO: 111);
CᵢYLPDYLCᵢᵢ[K(PYA)]DEYCᵢᵢᵢ (SEQ ID NO: 112);
CᵢYLPDYLCᵢᵢGD[K(PYA)]YCᵢᵢᵢ (SEQ ID NO: 113);
CᵢYLPDYLCᵢᵢGDE[K(PYA)]Cᵢᵢᵢ (SEQ ID NO: 114);
CᵢYLPDYLCᵢᵢ[dK(PYA)]DEYCᵢᵢᵢ (SEQ ID NO: 115);
CᵢALTTHNCᵢᵢQWGICᵢᵢᵢ (SEQ ID NO: 116);
CᵢDLATHNCᵢᵢQWGICᵢᵢᵢ (SEQ ID NO: 117);
CᵢDLTAHNCᵢᵢQWGICᵢᵢᵢ (SEQ ID NO: 118);
CᵢDLTTHNCᵢᵢAWGICᵢᵢᵢ (SEQ ID NO: 119);
CᵢDLTTHNCᵢᵢQWGACᵢᵢᵢ (SEQ ID NO: 120);
CᵢLTTHNCᵢᵢQWGICᵢᵢᵢ (SEQ ID NO: 121);
CᵢDLQTHNCᵢᵢQWGICᵢᵢᵢ (SEQ ID NO: 122);
CᵢDLTLHNCᵢᵢQWGICᵢᵢᵢ (SEQ ID NO: 123);
CᵢDLTT[His3Me]NCᵢᵢQWGICᵢᵢᵢ (SEQ ID NO: 124);
CᵢDLTT[2Pal]NCᵢᵢQWGICᵢᵢᵢ (SEQ ID NO: 125);
CᵢDLTT[His1Me]NCᵢᵢQWGICᵢᵢᵢ (SEQ ID NO: 126);
CᵢDLTT[4ThiAz]NCᵢᵢQWGICᵢᵢᵢ (SEQ ID NO: 127);
CᵢDLTTHNCᵢᵢVWGICᵢᵢᵢ (SEQ ID NO: 128);
CᵢDLTTHNCᵢᵢ[Cba]WGICᵢᵢᵢ (SEQ ID NO: 129);
CᵢDLTTHNCᵢᵢQ[1Nal]GICᵢᵢᵢ (SEQ ID NO: 130);
CᵢDLTTHNCᵢᵢQ[Trp(Me)]GICᵢᵢᵢ (SEQ ID NO: 131);
CᵢDLTTHNCᵢᵢQWG[tBuGly]Cᵢᵢᵢ (SEQ ID NO: 132);
CᵢDLTTHNCᵢᵢQWG[tBuAla]Cᵢᵢᵢ (SEQ ID NO: 133);
CᵢDLTTHNCᵢᵢQWG[Chg]Cᵢᵢᵢ (SEQ ID NO: 134);
CᵢDLTTHNCᵢᵢQW[dA]ICᵢᵢᵢ (SEQ ID NO: 135);
CᵢS[Cba]PDYLCᵢᵢ[dA]DEYCᵢᵢᵢ (SEQ ID NO: 136)
Cᵢ[HSer][Cba]PDYLCᵢᵢ[dA]DEYCᵢᵢᵢ (SEQ ID NO: 137);
CᵢN[Cba]PDYLCᵢᵢ[dA]DEYCᵢᵢᵢ (SEQ ID NO: 138);
Cᵢ[4Pal][Cba]PDYLCᵢᵢ[dA]DEYCᵢᵢᵢ (SEQ ID NO: 139);
CᵢY[Cba][Cis-HyP]DYLCᵢᵢ[dA]DEYCᵢᵢᵢ (SEQ ID NO: 140);
CᵢY[Cba][NMeAla]DYLCᵢᵢ[dA]DEYCᵢᵢᵢ (SEQ ID NO: 141);
CᵢY[Cba]PNYLCᵢᵢ[dA]DEYCᵢᵢᵢ (SEQ ID NO: 142);
CᵢY[Cba]PQYLCᵢᵢ[dA]DEYCᵢᵢᵢ (SEQ ID NO: 143);
CᵢY[Cba]PDYLCᵢᵢ[dV]DEYCᵢᵢᵢ (SEQ ID NO: 144);
CᵢY[Cba]PDYLCᵢᵢ[dNva]DEYCᵢᵢᵢ (SEQ ID NO: 145);
CᵢY[Cba]PDYLCᵢᵢ[dF]DEYCᵢᵢᵢ (SEQ ID NO: 146);
CᵢY[Cba]PDYLCᵢᵢ[dCha]DEYCᵢᵢᵢ (SEQ ID NO: 147);
CᵢY[Cba]PDYLCᵢᵢ[dK(PYA)]DEYCᵢᵢᵢ (SEQ ID NO: 148);
CᵢY[Cba]PDYLCᵢᵢ[dNIe]DEYCᵢᵢᵢ (SEQ ID NO: 149);
CᵢY[Cba]PD[DOPA]LCᵢᵢ[dA]DEYCᵢᵢᵢ (SEQ ID NO: 150);
CᵢY[Cba]PD[2FTyr]LCᵢᵢ[dA]DEYCᵢᵢᵢ (SEQ ID NO: 151);
CᵢY[Cba]PDY[Nle]Cᵢᵢ[dA]DEYCᵢᵢᵢ (SEQ ID NO: 152);
[Pen]ᵢY[Cba]PDYLCᵢᵢ[dA]DEYCᵢᵢᵢ (SEQ ID NO: 153);
CᵢY[Cba]PDYL[Pen]ᵢᵢ[dA]DEYCᵢᵢᵢ (SEQ ID NO: 154);
CᵢY[Cba]PDYLCᵢᵢ[dA]DEY[Pen]ᵢᵢᵢ (SEQ ID NO: 155);
CᵢY[Cba]PDYLCᵢᵢ[dA]DEY[dCᵢᵢᵢ] (SEQ ID NO: 156);
[dCᵢ]Y[Cba]PDYLCᵢᵢ[dA]DEYCᵢᵢᵢ (SEQ ID NO: 157);
CᵢDLTTHNCᵢᵢQ[AzaTrp]GICᵢᵢᵢ (SEQ ID NO: 158);
CᵢDLTTHNCᵢᵢQ[2MeTrp]GICᵢᵢᵢ (SEQ ID NO: 159);
CᵢDLTTHNCᵢᵢQ[eMeTrp]GICᵢᵢᵢ (SEQ ID NO: 160);
CᵢDLTTHNCᵢᵢQ[7MeTrp]GICᵢᵢᵢ (SEQ ID NO: 161);
CᵢDLTTHNCᵢᵢQ[eFTrp]GICᵢᵢᵢ (SEQ ID NO: 162);
CᵢDLTTHNCᵢᵢQ[5FTrp]GICᵢᵢᵢ (SEQ ID NO: 163);
CᵢDLTTHNCᵢᵢQ[Dht]GICᵢᵢᵢ (SEQ ID NO: 164);
CᵢDLTT[2FuAla]NCᵢᵢQWGICᵢᵢᵢ (SEQ ID NO: 165);
CᵢDLTTHNCᵢᵢQWG[EPA]Cᵢᵢᵢ (SEQ ID NO: 166);
CᵢDLTTHNCᵢᵢQWG[Nle]Cᵢᵢᵢ (SEQ ID NO: 167);
CᵢDLTTHNCᵢᵢQWG[Allolle]Cᵢᵢᵢ (SEQ ID NO: 168);
CᵢPPAVYCᵢᵢHPLDCᵢᵢᵢ (SEQ ID NO: 169);
CᵢPPEVFCᵢᵢHPLDCᵢᵢᵢ (SEQ ID NO: 170);
CᵢPPEVYCᵢᵢHPLDCᵢᵢᵢ (SEQ ID NO: 171);
CᵢPPEVWCᵢᵢHPLDCᵢᵢᵢ (SEQ ID NO: 172);
CᵢPPQVYCᵢᵢHPLDCᵢᵢᵢ (SEQ ID NO: 173);
CᵢPPEVWCᵢᵢHPLDCᵢᵢᵢ (SEQ ID NO: 174);
CᵢRWS[tBuGly]EDPCᵢᵢGAWCᵢᵢᵢ (SEQ ID NO: 175);
CᵢRWSVED[HyP]CᵢᵢGAWCᵢᵢᵢ (SEQ ID NO: 176);
CᵢRWSVED[Aze]CᵢᵢGAWCᵢᵢᵢ (SEQ ID NO: 177);
CᵢRWSVEDPCᵢᵢ[dA]AWCᵢᵢᵢ (SEQ ID NO: 178);
CᵢRWSVE[Gla]PCᵢᵢGAWCᵢᵢᵢ (SEQ ID NO: 179);
CᵢRWSVED[Aze]CᵢᵢGAWCᵢᵢᵢ (SEQ ID NO: 180);
CᵢMWIRSDPCᵢᵢGAWCᵢᵢᵢ (SEQ ID NO: 181);
CᵢRWYPDDPCᵢᵢGAWCᵢᵢᵢ (SEQ ID NO: 182);
CᵢEWHISEPCᵢᵢGAWCᵢᵢᵢ (SEQ ID NO: 183);
CᵢNWHVYEPCᵢᵢGAWCᵢᵢᵢ (SEQ ID NO: 184);
CᵢRWHFSEPCᵢᵢGAWCᵢᵢᵢ (SEQ ID NO: 185);
CᵢRWNLDDPCᵢᵢGAWCᵢᵢᵢ (SEQ ID NO: 186);
CᵢMWIRSDPCᵢᵢGAWCᵢᵢᵢ (SEQ ID NO: 187);
CᵢRWYPDDPCᵢᵢGAWCᵢᵢᵢ (SEQ ID NO: 188);
CᵢEWHISEPCᵢᵢGAWCᵢᵢᵢ (SEQ ID NO: 189);
CᵢNWHVYEPCᵢᵢGAWCᵢᵢᵢ (SEQ ID NO: 190);
CᵢRWHFSEPCᵢᵢGAWCᵢᵢᵢ (SEQ ID NO: 191);
CᵢRWNLDDPCᵢᵢGAWCᵢᵢᵢ (SEQ ID NO: 192);
CᵢR[2MeTrp]SVEDPCᵢᵢGAWCᵢᵢᵢ (SEQ ID NO: 193);
CᵢR[6MeTrp]SVEDPCᵢᵢGAWCᵢᵢᵢ (SEQ ID NO: 194);
CᵢR[6FTrp]SVEDPCᵢᵢGAWCᵢᵢᵢ (SEQ ID NO: 195);
CᵢR[5FTrp]SVEDPCᵢᵢGAWCᵢᵢᵢ (SEQ ID NO: 196);
CᵢR[6ClTrp]SVEDPCᵢᵢGAWCᵢᵢᵢ (SEQ ID NO: 197);
CᵢR[Trp(S)]SVEDPCᵢᵢGAWCᵢᵢᵢ (SEQ ID NO: 198);
CᵢR[AzaTrp]SVEDPCᵢᵢGAWCᵢᵢᵢ (SEQ ID NO: 199);
CᵢRWSVEDPCᵢᵢGA[2MeTrp]Cᵢᵢᵢ (SEQ ID NO: 200);
CᵢRWSVEDPCᵢᵢGA[6FTrp]Cᵢᵢᵢ (SEQ ID NO: 201);
CᵢRWSVEDPCᵢᵢGA[5FTrp]Cᵢᵢᵢ (SEQ ID NO: 202);
CᵢRWSVEDPCᵢᵢGA[4MeoTrp]Cᵢᵢᵢ (SEQ ID NO: 203);
CᵢRWSVEDPCᵢᵢGA[5MeoTrp]Cᵢᵢᵢ (SEQ ID NO: 204);
CᵢRWSVEDPCᵢᵢGA[Trp(S)]Cᵢᵢᵢ (SEQ ID NO: 205);
CᵢRWSVEDPCᵢᵢGA[DOPA]Cᵢᵢᵢ (SEQ ID NO: 206);
CᵢRWYPDDPCᵢᵢGAWCᵢᵢᵢ (SEQ ID NO: 207);
CᵢRWHFSEPCᵢᵢGAWCᵢᵢᵢ (SEQ ID NO: 208);
CᵢR[2MeTrp]HFSEPCᵢᵢGAWCᵢᵢᵢ (SEQ ID NO: 209);
CᵢRWYFSEPCᵢᵢGAWCᵢᵢᵢ (SEQ ID NO: 210);
CᵢRWSFSEPCᵢᵢGAWCᵢᵢᵢ (SEQ ID NO: 211);
CᵢRWHPSEPCᵢᵢGAWCᵢᵢᵢ (SEQ ID NO: 212);
CᵢRWYPSEPCᵢᵢGAWCᵢᵢᵢ (SEQ ID NO: 213);
CᵢRWHPDEPCᵢᵢGAWCᵢᵢᵢ (SEQ ID NO: 214);
CᵢRWHPEEPCᵢᵢGAWCᵢᵢᵢ (SEQ ID NO: 215);
CᵢRWHPDEPCᵢᵢGAWCᵢᵢᵢ (SEQ ID NO: 216);
CᵢRWHFDDPCᵢᵢGAWCᵢᵢᵢ (SEQ ID NO: 217);
CᵢRWYFDEPCᵢᵢGAWCᵢᵢᵢ (SEQ ID NO: 218);
CᵢRW[His1Me]FSEPCᵢᵢGAWCᵢᵢᵢ (SEQ ID NO: 219);
CᵢRW[His3Me]FSEPCᵢᵢGAWCᵢᵢᵢ (SEQ ID NO: 220);
CᵢRW[4MeoPhe]FSEPCᵢᵢGAWCᵢᵢᵢ (SEQ ID NO: 221);
CᵢRW[DOPA]FSEPCᵢᵢGAWCᵢᵢᵢ (SEQ ID NO: 222);
CᵢRW[26DiMeTyr]FSEPCᵢᵢGAWCᵢᵢᵢ (SEQ ID NO: 223);
CᵢRW[3tBuTyr]FSEPCᵢᵢGAWCᵢᵢᵢ (SEQ ID NO: 224);
CᵢRW[4FPhe]FSEPCᵢᵢGAWCᵢᵢᵢ (SEQ ID NO: 225);
CᵢRWH[4MePhe]SEPCᵢᵢGAWCᵢᵢᵢ (SEQ ID NO: 226);
CᵢRWH[Aze]SEPCᵢᵢGAWCᵢᵢᵢ (SEQ ID NO: 227);
CᵢRWH[HyP]SEPCᵢᵢGAWCᵢᵢᵢ (SEQ ID NO: 228);
CᵢRWH[4tBuPhe]SEPCᵢᵢGAWCᵢᵢᵢ (SEQ ID NO: 229);
CᵢRWH[HPhe]SEPCᵢᵢGAWCᵢᵢᵢ (SEQ ID NO: 230; herein referred to as BCY21693 when complexed with TBMT);
CᵢRWH[4PhePro]SEPCᵢᵢGAWCᵢᵢᵢ (SEQ ID NO: 231);
CᵢRWHF[HSer]EPCᵢᵢGAWCᵢᵢᵢ (SEQ ID NO: 232);
CᵢRWHFS[Gla]PCᵢᵢGAWCᵢᵢᵢ (SEQ ID NO: 233);
CᵢRWHFS[HGlu]PCᵢᵢGAWCᵢᵢᵢ (SEQ ID NO: 234);
CᵢRWHFSEPCᵢᵢ[dA]AWCᵢᵢᵢ (SEQ ID NO: 235);
CᵢRWYFSE[Aze]Cᵢᵢ[dA]AWCᵢᵢᵢ (SEQ ID NO: 236);
CᵢRWHFDE[Aze]Cᵢᵢ[dA]AWCᵢᵢᵢ (SEQ ID NO: 237);
CᵢRWYPSE[Aze]Cᵢᵢ[dA]AWCᵢᵢᵢ (SEQ ID NO: 238);
Cᵢ[HArg]WHFSEPCᵢᵢGAWCᵢᵢᵢ (SEQ ID NO: 239);
CᵢRWH[Cis-HyP]SEPCᵢᵢGAWCᵢᵢᵢ (SEQ ID NO: 240);
CᵢRWHFSEPCᵢᵢGAWCᵢᵢᵢ (SEQ ID NO: 241);
CᵢRWHFSEPCᵢᵢGAWCᵢᵢᵢ (SEQ ID NO: 242);
CᵢRWHFSEPCᵢᵢGAWCᵢᵢᵢ (SEQ ID NO: 243);
CᵢVGLLELGPCᵢᵢSDLCᵢᵢᵢ (SEQ ID NO: 244);
CᵢPGLEELGPCᵢᵢSDLCᵢᵢᵢ (SEQ ID NO: 245);
CᵢV[dA]LEELGPCᵢᵢSDLCᵢᵢᵢ (SEQ ID NO: 246);
CᵢVG[tBuAla]EELGPCᵢᵢSDLCᵢᵢᵢ (SEQ ID NO: 247);
CᵢVG[Cba]EELGPCᵢᵢSDLCᵢᵢᵢ (SEQ ID NO: 248);
CᵢVGFEELGPCᵢᵢSDLCᵢᵢᵢ (SEQ ID NO: 249);
CᵢVG[1Nal]EELGPCᵢᵢSDLCᵢᵢᵢ (SEQ ID NO: 250);
CᵢVG[2Nal]EELGPCᵢᵢSDLCᵢᵢᵢ (SEQ ID NO: 251);
CᵢVGLPELGPCᵢᵢSDLCᵢᵢᵢ (SEQ ID NO: 252);
CᵢVGL[tBuAla]ELGPCᵢᵢSDLCᵢᵢᵢ (SEQ ID NO: 253);
CᵢVGL[Cba]ELGPCᵢᵢSDLCᵢᵢᵢ (SEQ ID NO: 254);
CᵢVGL[Cha]ELGPCᵢᵢSDLCᵢᵢᵢ (SEQ ID NO: 255);
CᵢVGL[Nle]ELGPCᵢᵢSDLCᵢᵢᵢ (SEQ ID NO: 256);
CᵢVGLE[Gla]LGPCᵢᵢSDLCᵢᵢᵢ (SEQ ID NO: 257);
CᵢVGLEELG[Aze]CᵢᵢSDLCᵢᵢᵢ (SEQ ID NO: 258);
CᵢVGLEELGPCᵢᵢSD[tBuAla]Cᵢᵢᵢ (SEQ ID NO: 259);
CᵢVGLEELGPCᵢᵢSD[Cba]Cᵢᵢᵢ (SEQ ID NO: 260);
CᵢVGLEELGPCᵢᵢSD[Cha]Cᵢᵢᵢ (SEQ ID NO: 261);
CᵢVG[HLeu]EELGPCᵢᵢSDLCᵢᵢᵢ (SEQ ID NO: 262);
CᵢVGL[HLeu]ELGPCᵢᵢSDLCᵢᵢᵢ (SEQ ID NO: 263);
CᵢVGLE[HGlu]LGPCᵢᵢSDLCᵢᵢᵢ (SEQ ID NO: 264);
CᵢTWPYCᵢᵢKSWGDVQDCᵢᵢᵢ (SEQ ID NO: 265);
CᵢDWWDPGCᵢᵢTYFCᵢᵢᵢ (SEQ ID NO: 266);
CᵢRWH[4BnPro]SEPCᵢᵢGAWCᵢᵢᵢ (SEQ ID NO: 267); and
CᵢRWH[HPhe]SE[Aze]CᵢᵢGAWCᵢᵢᵢ (SEQ ID NO: 268);
wherein Cᵢ, Penᵢ, Cᵢᵢ, Penᵢᵢ, Cᵢᵢᵢ and Penᵢᵢᵢ represent first, second and third reactive groups, respectively, and wherein 2,6DiMeTyr represents 2,6-dimethyl-tyrosine, tBuAla represents β-t-Butyl-L-alanine, Cba represents β-cyclobutylalanine, HyP represents hydroxyproline, Aze represents azetidine-2-carboxylic acid, Aib represents aminoisobutyric acid, 1Nal represents 1-naphthylalanine, 2Nal represents 2-naphthylalanine, 3Pal represents 3-pyridylalanine, C5A represents beta-cyclopentyl-L-alanine, Cha represents 3-cyclohexyl-L-alanine, 4FPhe represents 4-fluoro-L-phenylalanine, 4MeOPhe represents 4-methoxy-phenylalanine, NO2Phe represents 4-nitro-phenylalanine, 4MePhe represents 4-methyl-phenylalanine, pCoPhe represents para-carboxy-phenylalanine, pCaPhe represents p-carboxamido-phenylalanine, 4tBuPhe represents 4-tert-butyl-phenylalanine, CF3Phe represents 4-trifluoromethyl-phenylalanine, 4CNPhe represents 4-cyano-phenylalanine, 4CIPhe represents 4-chloro-phenylalanine, HPhe represents homophenylalanine, 3tBuTyr represents 3-tert-butyltyrosine, His3Me represents 3-methylhistidine, 2Pal represents 2-pyridylalanine, His1Me represents 1-methylhistidine, 4ThiAz represents beta-4-thiazol-L-ylalanine, Trp(Me) represents 1-methyltryptophan, tBuGly represents tert-butylglycine, Chg represents cyclohexylglycine, HSer represents homoserine, NMeAla represents N-methylalanine, 4Pal represents 4-pyridylalanine, Cis-HyP represents L-4-cis-hydroxyproline, dA represents D-Alanine, dV represents D-valine, Nva represents norvaline, dNva represents D-norvaline, Nle represents norleucine, dNle represents D-norleucine, dF represents D-phenylalanine, dCha represents 3-cyclohexyl-D-alanine, DOPA represents 3,4-Dihydroxyphenylalanine, 2FTyr represents 2-fluorotyrosine, dC represents D-cysteine, AzaTrp represents azatryptophan, 2MeTrp represents 2-methyltryptophan, 6MeTrp represents 6-methyltryptophan, 6FTrp represents 6-fluorotryptophan, 5FTrp represents 5-fluorotryptophan, Dht represents 2,3-Dihydrotryptophan, 2FuAla represents 2-furylalanine, Allolle represents L-allo-isoleucine, Sar represents sarcosine, Agb represents norarginine, Cbg represents cyclobutylglycine, HArg represents homoarginine, Pep represents pipecolic acid, EPA represents diethyl-L-alanine, Gla represents γ-carboxyglutamic acid, 6CITrp represents 6-Chloro-L-tryptophan, 4MeoTrp represents 4-Methoxy-L-tryptophan, 5MeoTrp represents 5-Methoxy-L-tryptophan, Trp(S) represents 3-(3-Benzothienyl)-L-alanine, 26DiMeTyr represents 2,6-Dimethyl-L-tyrosine, 4PhePro represents (2S,4S)-4-PhenylProline, HGIu represents L-2-aminoadipic acid, 4BnPro represents (2S,4R)-4-benzyl-pyrrolidine-2-carboxylic acid and HLeu represents homoleucine.

2. The peptide ligand as defined in claim 1, wherein said bicyclic peptide is specific for NKp46 and wherein the bicyclic peptide ligand comprises an amino acid sequence which is selected from:
CᵢNLQKPCᵢᵢMKYPCᵢᵢᵢ (SEQ ID NO: 1); and
CᵢNLQNPCᵢᵢMKFPCᵢᵢᵢ (SEQ ID NO: 2);
CᵢNLQAPCᵢᵢMQTGKVCᵢᵢᵢ (SEQ ID NO: 3);
CᵢNLQAPCᵢᵢMLTGKVCᵢᵢᵢ (SEQ ID NO: 4; herein referred to as BCY18295 when complexed with TATA);
CᵢLLHDHCᵢᵢPNTHPKLCᵢᵢᵢ (SEQ ID NO: 5);
CᵢLLHEHCᵢᵢPNTHPKMCᵢᵢᵢ (SEQ ID NO: 6);
CᵢLLHDHCᵢᵢPNSHPEICᵢᵢᵢ (SEQ ID NO: 7);
CᵢLLHDHCᵢᵢPNTHPIICᵢᵢᵢ (SEQ ID NO: 8);
CᵢYLPDWLCᵢᵢGDEYCᵢᵢᵢ (SEQ ID NO: 9);
CᵢYLPDYLCᵢᵢGDEYCᵢᵢᵢ (SEQ ID NO: 10);
CᵢDLTTHNCᵢᵢQWGICᵢᵢᵢ (SEQ ID NO: 11; herein referred to as BCY18262 when complexed with TATA);
CᵢYLPDYLCᵢᵢGDEYCᵢᵢᵢ (SEQ ID NO: 12; herein referred to as BCY15633 when complexed with TATA);
CᵢYLPDWLCᵢᵢGDEYCᵢᵢᵢ (SEQ ID NO: 13);
Cᵢ[2,6DiMeTyr]LPDYLCᵢᵢGDEYCᵢᵢᵢ (SEQ ID NO: 14);
CᵢY[tBuAla]PDYLCᵢᵢGDEYCᵢᵢᵢ (SEQ ID NO: 15);
CᵢY[Cba]PDYLCᵢᵢGDEYCᵢᵢᵢ (SEQ ID NO: 16);
CᵢYL[HyP]DYLCᵢᵢGDEYCᵢᵢᵢ (SEQ ID NO: 17);
CᵢYL[Aze]DYLCᵢᵢGDEYCᵢᵢᵢ (SEQ ID NO: 18);
CᵢYL[Aib]DYLCᵢᵢGDEYCᵢᵢᵢ (SEQ ID NO: 19);
CᵢYLPD[1Nal]LCᵢᵢGDEYCᵢᵢᵢ (SEQ ID NO: 20);
CᵢYLPD[2Nal]LCᵢᵢGDEYCᵢᵢᵢ (SEQ ID NO: 21);
CᵢYLPD[3Pal]LCᵢᵢGDEYCᵢᵢᵢ (SEQ ID NO: 22);
CᵢYLPD[2,6DiMeTyr]LCᵢᵢGDEYCᵢᵢᵢ (SEQ ID NO: 23);
CᵢYLPDW[tBuAla]CᵢᵢGDEYCᵢᵢᵢ (SEQ ID NO: 24);
CᵢYLPDW[Cba]CᵢᵢGDEYCᵢᵢᵢ (SEQ ID NO: 25);
CᵢYLPDYLCᵢᵢ[dA]DEYCᵢᵢᵢ (SEQ ID NO: 26);
CᵢYLPDYLCᵢᵢGDE[2,6DiMeTyr]Cᵢᵢᵢ (SEQ ID NO: 27);
CᵢALPDYLCᵢᵢGDEYCᵢᵢᵢ (SEQ ID NO: 28);
CᵢYAPDYLCᵢᵢGDEYCᵢᵢᵢ (SEQ ID NO: 29);
CᵢYLADYLCᵢᵢGDEYCᵢᵢᵢ (SEQ ID NO: 30);
CᵢYLPAYLCᵢᵢGDEYCᵢᵢᵢ (SEQ ID NO: 31);
CᵢYLPDALCᵢᵢGDEYCᵢᵢᵢ (SEQ ID NO: 32);
CᵢYLPDYACᵢᵢGDEYCᵢᵢᵢ (SEQ ID NO: 33);
CᵢYLPDYLCᵢᵢADEYCᵢᵢᵢ (SEQ ID NO: 34);
CᵢYLPDYLCᵢᵢGAEYCᵢᵢᵢ (SEQ ID NO: 35);
CᵢYLPDYLCᵢᵢGDAYCᵢᵢᵢ (SEQ ID NO: 36);
CᵢYLPDYLCᵢᵢGDEACᵢᵢᵢ (SEQ ID NO: 37);
CᵢY[C5A]PDYLCᵢᵢGDEYCᵢᵢᵢ (SEQ ID NO: 38);
CᵢY[Cha]PDYLCᵢᵢGDEYCᵢᵢᵢ (SEQ ID NO: 39);
CᵢY[Cba]PDYLCᵢᵢ[dA]DEYCᵢᵢᵢ (SEQ ID NO: 40);
CᵢYLPD[4FPhe]LCᵢᵢGDEYCᵢᵢᵢ (SEQ ID NO: 41);
CᵢYLPD[4MeOPhe]LCᵢᵢGDEYCᵢᵢᵢ (SEQ ID NO: 42);
CᵢYLPD[NO2Phe]LCᵢᵢGDEYCᵢᵢᵢ (SEQ ID NO: 43);
CᵢYLPD[4MePhe]LCᵢᵢGDEYCᵢᵢᵢ (SEQ ID NO: 44);
CᵢYLPD[pCoPhe]LCᵢᵢGDEYCᵢᵢᵢ (SEQ ID NO: 45);
CᵢYLPD[pCaPhe]LCᵢᵢGDEYCᵢᵢᵢ (SEQ ID NO: 46);
CᵢYLPD[4tBuPhe]LCᵢᵢGDEYCᵢᵢᵢ (SEQ ID NO: 47);
CᵢYLPD[CF3Phe]LCᵢᵢGDEYCᵢᵢᵢ (SEQ ID NO: 48);
CᵢYLPD[4CNPhe]LCᵢᵢGDEYCᵢᵢᵢ (SEQ ID NO: 49);
CᵢYLPD[4ClPhe]LCᵢᵢGDEYCᵢᵢᵢ (SEQ ID NO: 50);
CᵢYLPD[HPhe]LCᵢᵢGDEYCᵢᵢᵢ (SEQ ID NO: 51);
Cᵢ[2,6DiMeTyr]LPDWLCᵢᵢGDEYCᵢᵢᵢ (SEQ ID NO: 52);
CᵢY[Cba]PDWLCᵢᵢGDEYCᵢᵢᵢ (SEQ ID NO: 53);
CᵢYL[Aze]DWLCᵢᵢGDEYCᵢᵢᵢ (SEQ ID NO: 54);
CᵢYLPDWLCᵢᵢ[dA]DEYCᵢᵢᵢ (SEQ ID NO: 55);
CᵢSLPDYLCᵢᵢGDEYCᵢᵢᵢ (SEQ ID NO: 56);
CᵢDLTTHNCᵢᵢQWGICᵢᵢᵢ (SEQ ID NO: 57);
CᵢDLTTHNCᵢᵢLWGVCᵢᵢᵢ (SEQ ID NO: 58);
CᵢNLQLHNCᵢᵢIWGVCᵢᵢᵢ (SEQ ID NO: 59);
CᵢNLQQACᵢᵢLHTGKVCᵢᵢᵢ (SEQ ID NO: 63);
CᵢNLQAACᵢᵢMLTGKVCᵢᵢᵢ (SEQ ID NO: 64);
CᵢNLQAPCᵢᵢMATGKVCᵢᵢᵢ (SEQ ID NO: 65);
CᵢNLQAPCᵢᵢMLAGKVCᵢᵢᵢ (SEQ ID NO: 66);
CᵢNLQAPCᵢᵢMLTAKVCᵢᵢᵢ (SEQ ID NO: 67);
CᵢNLQAPCᵢᵢMLTGAVCᵢᵢᵢ (SEQ ID NO: 68);
CᵢNLQAPCᵢᵢMLTGKACᵢᵢᵢ (SEQ ID NO: 69);
CᵢN[tBuAla]QAPCᵢᵢMLTGKVCᵢᵢᵢ (SEQ ID NO: 70);
CᵢNLQA[Sar]CᵢᵢMLTGKVCᵢᵢᵢ (SEQ ID NO: 71);
CᵢNLQA[Cis-HyP]CᵢᵢMLTGKVCᵢᵢᵢ (SEQ ID NO: 72);
CᵢNLQA[HyP]CᵢᵢMLTGKVCᵢᵢᵢ (SEQ ID NO: 73);
CᵢNLQA[NMeAla]CᵢᵢMLTGKVCᵢᵢᵢ (SEQ ID NO: 74);
CᵢNLQAPCᵢᵢLLTGKVCᵢᵢᵢ (SEQ ID NO: 75);
CᵢNLQAPCᵢᵢ[Nle]LTGKVCᵢᵢᵢ (SEQ ID NO: 76);
CᵢNLQAPCᵢᵢ[Cba]LTGKVCᵢᵢᵢ (SEQ ID NO: 77);
CᵢNLQAPCᵢᵢMETGKVCᵢᵢᵢ (SEQ ID NO: 78);
CᵢNLQAPCᵢᵢMFTGKVCᵢᵢᵢ (SEQ ID NO: 79);
CᵢNLQAPCᵢᵢMYTGKVCᵢᵢᵢ (SEQ ID NO: 80);
CᵢNLQAPCᵢᵢM[Cba]TGKVCᵢᵢᵢ (SEQ ID NO: 81);
CᵢNLQAPCᵢᵢM[tBuAla]TGKVCᵢᵢᵢ (SEQ ID NO: 82);
CᵢNLQAPCᵢᵢM[Cha]TGKVCᵢᵢᵢ (SEQ ID NO: 83);
CᵢNLQAPCᵢᵢMLT[dA]KVCᵢᵢᵢ (SEQ ID NO: 84);
CᵢNLQAPCᵢᵢMLTGRVCᵢᵢᵢ (SEQ ID NO: 85);
CᵢNLQAPCᵢᵢMLTG[Agb]VCᵢᵢᵢ (SEQ ID NO: 86);
CᵢNLQAPCᵢᵢMLTGK[tBuGly]Cᵢᵢᵢ (SEQ ID NO: 87);
CᵢNLQAPCᵢᵢMLTGK[Chg]Cᵢᵢᵢ (SEQ ID NO: 88);
CᵢNLQAPCᵢᵢMLTGK[Cbg]Cᵢᵢᵢ (SEQ ID NO: 89);
CᵢNLQAPCᵢᵢMLTG[HArg]VCᵢᵢᵢ (SEQ ID NO: 90);
CᵢNLQA[Aze]CᵢᵢMLTGKVCᵢᵢᵢ (SEQ ID NO: 91);
CᵢNLQA[Pip]CᵢᵢMLTGKVCᵢᵢᵢ (SEQ ID NO: 92);
CᵢNLQAPCᵢᵢMVTGKVCᵢᵢᵢ (SEQ ID NO: 93);
CᵢNLQAPCᵢᵢM[Nle]TGKVCᵢᵢᵢ (SEQ ID NO: 94);
CᵢNLQAPCᵢᵢM[Nva]TGKVCᵢᵢᵢ (SEQ ID NO: 95);
CᵢNLQAPCᵢᵢMLTGK[AlloIle]Cᵢᵢᵢ (SEQ ID NO: 96);
CᵢNLQAPCᵢᵢMLTGK[EPA]Cᵢᵢᵢ (SEQ ID NO: 97);
CᵢNLQQPCᵢᵢMLTGKVCᵢᵢᵢ (SEQ ID NO: 98);
CᵢNLQA[Nva]CᵢᵢMLTGKVCᵢᵢᵢ (SEQ ID NO: 99);
CᵢNLQAPCᵢᵢMHTGKVCᵢᵢᵢ (SEQ ID NO: 100);
CᵢNLQAPCᵢᵢLHTGKVCᵢᵢᵢ (SEQ ID NO: 101);
CᵢNLQQACᵢᵢMQTGKVCᵢᵢᵢ (SEQ ID NO: 102);
CᵢLLHDHCᵢᵢPNTHPKMCᵢᵢᵢ (SEQ ID NO: 103);
CᵢDLTTHNCᵢᵢIWGVCᵢᵢᵢ (SEQ ID NO: 104);
CᵢNLQLHNCᵢᵢIWGVCᵢᵢᵢ (SEQ ID NO: 105);
CᵢNLQLHNCᵢᵢQWGICᵢᵢᵢ (SEQ ID NO: 106);
Cᵢ[3tBuTyr]LPDYLCᵢᵢGDEYCᵢᵢᵢ (SEQ ID NO: 107);
CᵢYLPD[3tBuTyr]LCᵢᵢGDEYCᵢᵢᵢ (SEQ ID NO: 108);
CᵢYLPDYLCᵢᵢGDE[3tBuTyr]Cᵢᵢᵢ (SEQ ID NO: 109);
Cᵢ[K(PYA)]LPDYLCᵢᵢGDEYCᵢᵢᵢ (SEQ ID NO: 110);
CᵢYLP[K(PYA)]YLCᵢᵢGDEYCᵢᵢᵢ (SEQ ID NO: 111);
CᵢYLPDYLCᵢᵢ[K(PYA)]DEYCᵢᵢᵢ (SEQ ID NO: 112);
CᵢYLPDYLCᵢᵢGD[K(PYA)]YCᵢᵢᵢ (SEQ ID NO: 113);
CᵢYLPDYLCᵢᵢGDE[K(PYA)]Cᵢᵢᵢ (SEQ ID NO: 114);
CᵢYLPDYLCᵢᵢ[dK(PYA)]DEYCᵢᵢᵢ (SEQ ID NO: 115);
CᵢALTTHNCᵢᵢQWGICᵢᵢᵢ (SEQ ID NO: 116);
CᵢDLATHNCᵢᵢQWGICᵢᵢᵢ (SEQ ID NO: 117);
CᵢDLTAHNCᵢᵢQWGICᵢᵢᵢ (SEQ ID NO: 118);
CᵢDLTTHNCᵢᵢAWGICᵢᵢᵢ (SEQ ID NO: 119);
CᵢDLTTHNCᵢᵢQWGACᵢᵢᵢ (SEQ ID NO: 120);
CᵢNLTTHNCᵢᵢQWGICᵢᵢᵢ (SEQ ID NO: 121);
CᵢDLQTHNCᵢᵢQWGICᵢᵢᵢ (SEQ ID NO: 122);
CᵢDLTLHNCᵢᵢQWGICᵢᵢᵢ (SEQ ID NO: 123);
CᵢDLTT[His3Me]NCᵢᵢQWGICᵢᵢᵢ (SEQ ID NO: 124);
CᵢDLTT[2Pal]NCᵢᵢQWGICᵢᵢᵢ (SEQ ID NO: 125);
CᵢDLTT[His1Me]NCᵢᵢQWGICᵢᵢᵢ (SEQ ID NO: 126);
CᵢDLTT[4ThiAz]NCᵢᵢQWGICᵢᵢᵢ (SEQ ID NO: 127);
CᵢDLTTHNCᵢᵢVWGICᵢᵢᵢ (SEQ ID NO: 128);
CᵢDLTTHNCᵢᵢ[Cba]WGICᵢᵢᵢ (SEQ ID NO: 129);
CᵢDLTTHNCᵢᵢQ[1Nal]GICᵢᵢᵢ (SEQ ID NO: 130);
CᵢDLTTHNCᵢᵢQ[Trp(Me)]GICᵢᵢᵢ (SEQ ID NO: 131);
CᵢDLTTHNCᵢᵢQWG[tBuGly]Cᵢᵢᵢ (SEQ ID NO: 132);
CᵢDLTTHNCᵢᵢQWG[tBuAla]Cᵢᵢᵢ (SEQ ID NO: 133);
CᵢDLTTHNCᵢᵢQWG[Chg]Cᵢᵢᵢ (SEQ ID NO: 134);
CᵢDLTTHNCᵢᵢQW[dA]ICᵢᵢᵢ (SEQ ID NO: 135);
CᵢS[Cba]PDYLCᵢᵢ[dA]DEYCᵢᵢᵢ (SEQ ID NO: 136)
Cᵢ[Hser][Cba]PDYLCᵢᵢ[dA]DEYCᵢᵢᵢ (SEQ ID NO: 137);
CᵢN[Cba]PDYLCᵢᵢ[dA]DEYCᵢᵢᵢ (SEQ ID NO: 138);
Cᵢ[4Pal][Cba]PDYLCᵢᵢ[dA]DEYCᵢᵢᵢ (SEQ ID NO: 139);
CᵢY[Cba][Cis-HyP]DYLCᵢᵢ[dA]DEYCᵢᵢᵢ (SEQ ID NO: 140);
CᵢY[Cba][NmeAla]DYLCᵢᵢ[dA]DEYCᵢᵢᵢ (SEQ ID NO: 141);
CᵢY[Cba]PNYLCᵢᵢ[dA]DEYCᵢᵢᵢ (SEQ ID NO: 142);
CᵢY[Cba]PQYLCᵢᵢ[dA]DEYCᵢᵢᵢ (SEQ ID NO: 143);
CᵢY[Cba]PDYLCᵢᵢ[dV]DEYCᵢᵢᵢ (SEQ ID NO: 144);
CᵢY[Cba]PDYLCᵢᵢ[dNva]DEYCᵢᵢᵢ (SEQ ID NO: 145);
CᵢY[Cba]PDYLCᵢᵢ[dF]DEYCᵢᵢᵢ (SEQ ID NO: 146);
CᵢY[Cba]PDYLCᵢᵢ[dCha]DEYCᵢᵢᵢ (SEQ ID NO: 147);
CᵢY[Cba]PDYLCᵢᵢ[dK(PYA)]DEYCᵢᵢᵢ (SEQ ID NO: 148);
CᵢY[Cba]PDYLCᵢᵢ[dNle]DEYCᵢᵢᵢ (SEQ ID NO: 149);
CᵢY[Cba]PD[DOPA]LCᵢᵢ[dA]DEYCᵢᵢᵢ (SEQ ID NO: 150);
CᵢY[Cba]PD[2FTyr]LCᵢᵢ[dA]DEYCᵢᵢᵢ (SEQ ID NO: 151);
CᵢY[Cba]PDY[Nle]Cᵢᵢ[dA]DEYCᵢᵢᵢ (SEQ ID NO: 152);
[Pen]ᵢY[Cba]PDYLCᵢᵢ[dA]DEYCᵢᵢᵢ (SEQ ID NO: 153);
CᵢY[Cba]PDYL[Pen]ᵢᵢ[dA]DEYCᵢᵢᵢ (SEQ ID NO: 154);
CᵢY[Cba]PDYLCᵢᵢ[dA]DEY[Pen]ᵢᵢᵢ (SEQ ID NO: 155);
CᵢY[Cba]PDYLCᵢᵢ[dA]DEY[dCᵢᵢᵢ] (SEQ ID NO: 156);
[dCᵢ]Y[Cba]PDYLCᵢᵢ[dA]DEYCᵢᵢᵢ (SEQ ID NO: 157);
CᵢDLTTHNCᵢᵢQ[AzaTrp]GICᵢᵢᵢ (SEQ ID NO: 158);
CᵢDLTTHNCᵢᵢQ[2MeTrp]GICᵢᵢᵢ (SEQ ID NO: 159);
CᵢDLTTHNCᵢᵢQ[eMeTrp]GICᵢᵢᵢ (SEQ ID NO: 160);
CᵢDLTTHNCᵢᵢQ[7MeTrp]GICᵢᵢᵢ (SEQ ID NO: 161);
CᵢDLTTHNCᵢᵢQ[eFTrp]GICᵢᵢᵢ (SEQ ID NO: 162);
CᵢDLTTHNCᵢᵢQ[SFTrp]GICᵢᵢᵢ (SEQ ID NO: 163);
CᵢDLTTHNCᵢᵢQ[Dht]GICᵢᵢᵢ (SEQ ID NO: 164);
CᵢDLTT[2FuAla]NCᵢᵢQWGICᵢᵢᵢ (SEQ ID NO: 165);
CᵢDLTTHNCᵢᵢQWG[EPA]Cᵢᵢᵢ (SEQ ID NO: 166);
CᵢDLTTHNCᵢᵢQWG[Nle]Cᵢᵢᵢ (SEQ ID NO: 167); and
CᵢDLTTHNCᵢᵢQWG[AlloIle]Cᵢᵢᵢ (SEQ ID NO: 168).

3. The peptide ligand as defined in claim 2, wherein the molecular scaffold is TATA and the bicyclic peptide ligand additionally comprises N- and/or C-terminal additions and comprises an amino acid sequence which is selected from:
A-(SEQ ID NO: 1)-A-Sar₆-KFI (herein referred to as BCY14654);
A-(SEQ ID NO: 1)-A (herein referred to as BCY14456);
A-(SEQ ID NO: 2)-A-Sar₆-KFI (herein referred to as BCY15125);
A-(SEQ ID NO: 2)-A (herein referred to as BCY15102);
A-(SEQ ID NO: 2)-A-[K(PYA)] (herein referred to as BCY18004);
A-(SEQ ID NO: 3)-A-[K(PYA)] (herein referred to as BCY15687);
A-(SEQ ID NO: 3)-A (herein referred to as BCY15098);
A-(SEQ ID NO: 4)-A-Sar₆-KFI (herein referred to as BCY15115);
A-(SEQ ID NO: 4)-A (herein referred to as BCY15099);
Ac-(SEQ ID NO: 4) (herein referred to as BCY18293);
Ac-A-(SEQ ID NO: 4)-A (herein referred to as BCY18294);
(SEQ ID NO: 4)-A (herein referred to as BCY19694);
K-(SEQ ID NO: 4)-A (herein referred to as BCY19695);
dK-(SEQ ID NO: 4)-A (herein referred to as BCY19696);
Dap-(SEQ ID NO: 4)-A (herein referred to as BCY19697);
Dab-(SEQ ID NO: 4)-A (herein referred to as BCY19698);
GABA-(SEQ ID NO: 4)-A (herein referred to as BCY19699);
A-(SEQ ID NO: 5)-A (herein referred to as BCY15103);
A-(SEQ ID NO: 5)-A-[K(PYA)] (herein referred to as BCY18005);
A-(SEQ ID NO: 5)-A-Sar₆-KFI (herein referred to as BCY15118);
A-(SEQ ID NO: 6)-A (herein referred to as BCY15104);
A-(SEQ ID NO: 6)-A-Sar₆-KFI (herein referred to as BCY15119);
A-(SEQ ID NO: 7)-A (herein referred to as BCY15105);
A-(SEQ ID NO: 7)-A-Sar₆-KFI (herein referred to as BCY15120);
A-(SEQ ID NO: 8)-A (herein referred to as BCY15106);
A-(SEQ ID NO: 8)-A-Sar₆-KFI (herein referred to as BCY15121);
A-(SEQ ID NO: 9)-A-[dK(PYA)] (herein referred to as BCY15013);
A-(SEQ ID NO: 10)-A (herein referred to as BCY18470);
A-(SEQ ID NO: 10)-A-[dK(PYA)] (herein referred to as BCY15452);
A-(SEQ ID NO: 11)-A (herein referred to as BCY15107);
Ac-(SEQ ID NO: 11) (herein referred to as BCY18260);
Ac-A-(SEQ ID NO: 11)-A (herein referred to as BCY18261);
A-(SEQ ID NO: 11)-[Aib] (herein referred to as BCY19717);
(SEQ ID NO: 11)-A (herein referred to as BCY19718);
A-(SEQ ID NO: 11)-A-[dK(PYA)] (herein referred to as BCY19719);
A-(SEQ ID NO: 11)-A-[K(PYA)] (herein referred to as BCY15686);
A-(SEQ ID NO: 12)-A (herein referred to as BCY15100);
Ac-(SEQ ID NO: 12) (herein referred to as BCY15634);
Ac-A-(SEQ ID NO: 12)-A (herein referred to as BCY15635);
dA-(SEQ ID NO: 12) (herein referred to as BCY16134;
A-(SEQ ID NO: 12)-A-Sar₆-KFI (herein referred to as BCY15116);
A-(SEQ ID NO: 13)-A (herein referred to as BCY14455);
(SEQ ID NO: 13)-Sar₆-KFI (herein referred to as BCY15023);
Ac-(SEQ ID NO: 13)-Sar₆-KFI (herein referred to as BCY15024);
A-(SEQ ID NO: 13)-A-Sar₆-KFI (herein referred to as BCY14652);
A-(SEQ ID NO: 14)-A (herein referred to as BCY15636);
A-(SEQ ID NO: 15)-A (herein referred to as BCY15638);
A-(SEQ ID NO: 16)-A (herein referred to as BCY15639);
A-(SEQ ID NO: 17)-A (herein referred to as BCY15640);
A-(SEQ ID NO: 18)-A (herein referred to as BCY15641);
A-(SEQ ID NO: 19)-A (herein referred to as BCY15642);
A-(SEQ ID NO: 20)-A (herein referred to as BCY15643);
A-(SEQ ID NO: 21)-A (herein referred to as BCY15644);
A-(SEQ ID NO: 22)-A (herein referred to as BCY15645);
A-(SEQ ID NO: 23)-A (herein referred to as BCY15646);
A-(SEQ ID NO: 24)-A (herein referred to as BCY15648);
A-(SEQ ID NO: 25)-A (herein referred to as BCY15649);
A-(SEQ ID NO: 26)-A (herein referred to as BCY15650);
A-(SEQ ID NO: 27)-A (herein referred to as BCY15651);
A-(SEQ ID NO: 28)-A (herein referred to as BCY15653);
A-(SEQ ID NO: 29)-A (herein referred to as BCY15654);
A-(SEQ ID NO: 30)-A (herein referred to as BCY15655);
A-(SEQ ID NO: 31)-A (herein referred to as BCY15656);
A-(SEQ ID NO: 32)-A (herein referred to as BCY15657);
A-(SEQ ID NO: 33)-A (herein referred to as BCY15658);
A-(SEQ ID NO: 34)-A (herein referred to as BCY15659);
A-(SEQ ID NO: 35)-A (herein referred to as BCY15660);
A-(SEQ ID NO: 36)-A (herein referred to as BCY15661);
A-(SEQ ID NO: 37)-A (herein referred to as BCY15662);
A-(SEQ ID NO: 38)-A (herein referred to as BCY16130);
A-(SEQ ID NO: 39)-A (herein referred to as BCY16131);
A-(SEQ ID NO: 40)-A (herein referred to as BCY16132);
Ac-(SEQ ID NO: 40) (herein referred to as BCY16133);
Ac-(SEQ ID NO: 40)-[K(PYA)] (herein referred to as BCY17224);
A-(SEQ ID NO: 41)-A (herein referred to as BCY16135);
A-(SEQ ID NO: 42)-A (herein referred to as BCY16136);
A-(SEQ ID NO: 43)-A (herein referred to as BCY16137);
A-(SEQ ID NO: 44)-A (herein referred to as BCY16138);
A-(SEQ ID NO: 45)-A (herein referred to as BCY16139);
A-(SEQ ID NO: 46)-A (herein referred to as BCY16140);
A-(SEQ ID NO: 47)-A (herein referred to as BCY16141);
A-(SEQ ID NO: 48)-A (herein referred to as BCY16142);
A-(SEQ ID NO: 49)-A (herein referred to as BCY16143);
A-(SEQ ID NO: 50)-A (herein referred to as BCY16144);
A-(SEQ ID NO: 51)-A (herein referred to as BCY16145);
A-(SEQ ID NO: 52)-A-Sar₆-KFI (herein referred to as BCY15025);
A-(SEQ ID NO: 53)-A-Sar₆-KFI (herein referred to as BCY15028);
A-(SEQ ID NO: 54)-A-Sar₆-KFI (herein referred to as BCY15030);
A-(SEQ ID NO: 55)-A-Sar₆-KFI (herein referred to as BCY15035);
A-(SEQ ID NO: 56)-A-Sar₆-KFI (herein referred to as BCY15117);
A-(SEQ ID NO: 57)-A-Sar₆-KFI (herein referred to as BCY15122);
A-(SEQ ID NO: 58)-A (herein referred to as BCY15108);
A-(SEQ ID NO: 58)-A-Sar₆-KFI (herein referred to as BCY15123);
A-(SEQ ID NO: 59)-A-Sar₆-KFI (herein referred to as BCY15124);
A-(SEQ ID NO: 63)-A (herein referred to as BCY14454);
A-(SEQ ID NO: 64)-A (herein referred to as BCY18299);
A-(SEQ ID NO: 65)-A (herein referred to as BCY18301);
A-(SEQ ID NO: 66)-A (herein referred to as BCY18302);
A-(SEQ ID NO: 67)-A (herein referred to as BCY18303);
A-(SEQ ID NO: 68)-A (herein referred to as BCY18304);
A-(SEQ ID NO: 69)-A (herein referred to as BCY18305);
A-(SEQ ID NO: 70)-A (herein referred to as BCY18307);
A-(SEQ ID NO: 71)-A (herein referred to as BCY18309);
A-(SEQ ID NO: 72)-A (herein referred to as BCY18310);
A-(SEQ ID NO: 73)-A (herein referred to as BCY18311);
A-(SEQ ID NO: 74)-A (herein referred to as BCY18312);
A-(SEQ ID NO: 75)-A (herein referred to as BCY18313);
A-(SEQ ID NO: 76)-A (herein referred to as BCY18314);
A-(SEQ ID NO: 77)-A (herein referred to as BCY18315);
A-(SEQ ID NO: 78)-A (herein referred to as BCY18316);
A-(SEQ ID NO: 79)-A (herein referred to as BCY18317);
A-(SEQ ID NO: 80)-A (herein referred to as BCY18318);
A-(SEQ ID NO: 81)-A (herein referred to as BCY18319);
A-(SEQ ID NO: 82)-A (herein referred to as BCY18320);
A-(SEQ ID NO: 83)-A (herein referred to as BCY18321);
A-(SEQ ID NO: 84)-A (herein referred to as BCY18322);
A-(SEQ ID NO: 85)-A (herein referred to as BCY18323);
A-(SEQ ID NO: 86)-A (herein referred to as BCY18324);
A-(SEQ ID NO: 87)-A (herein referred to as BCY18325);
A-(SEQ ID NO: 88)-A (herein referred to as BCY18326);
A-(SEQ ID NO: 89)-A (herein referred to as BCY18327);
A-(SEQ ID NO: 90)-A (herein referred to as BCY18328);
A-(SEQ ID NO: 91)-A (herein referred to as BCY19700);
A-(SEQ ID NO: 92)-A (herein referred to as BCY19701);
A-(SEQ ID NO: 93)-A (herein referred to as BCY19702);
A-(SEQ ID NO: 94)-A (herein referred to as BCY19703);
A-(SEQ ID NO: 95)-A (herein referred to as BCY19704);
A-(SEQ ID NO: 96)-A (herein referred to as BCY19706);
A-(SEQ ID NO: 97)-A (herein referred to as BCY19707);
A-(SEQ ID NO: 98)-A (herein referred to as BCY19708);
A-(SEQ ID NO: 99)-A (herein referred to as BCY19710);
A-(SEQ ID NO: 100)-A (herein referred to as BCY19711);
A-(SEQ ID NO: 101)-A (herein referred to as BCY19712);
A-(SEQ ID NO: 102)-A (herein referred to as BCY19713);
A-(SEQ ID NO: 103)-A (herein referred to as BCY14457);
A-(SEQ ID NO: 104)-A (herein referred to as BCY14458);
A-(SEQ ID NO: 105)-A (herein referred to as BCY15109);
A-(SEQ ID NO: 106)-A (herein referred to as BCY15568);
A-(SEQ ID NO: 107)-A (herein referred to as BCY15637);
A-(SEQ ID NO: 108)-A (herein referred to as BCY15647);
A-(SEQ ID NO: 109)-A (herein referred to as BCY15652);
A-(SEQ ID NO: 110)-A (herein referred to as BCY16834);
A-(SEQ ID NO: 111)-A (herein referred to as BCY16837);
A-(SEQ ID NO: 112)-A (herein referred to as BCY16840);
A-(SEQ ID NO: 113)-A (herein referred to as BCY16842);
A-(SEQ ID NO: 114)-A (herein referred to as BCY16843);
A-(SEQ ID NO: 115)-A (herein referred to as BCY17662);
A-(SEQ ID NO: 116)-A (herein referred to as BCY18263);
A-(SEQ ID NO: 117)-A (herein referred to as BCY18265);
A-(SEQ ID NO: 118)-A (herein referred to as BCY18266);
A-(SEQ ID NO: 119)-A (herein referred to as BCY18269);
A-(SEQ ID NO: 120)-A (herein referred to as BCY18272);
A-(SEQ ID NO: 121)-A (herein referred to as BCY18273);
A-(SEQ ID NO: 122)-A (herein referred to as BCY18277);
A-(SEQ ID NO: 123)-A (herein referred to as BCY18278);
A-(SEQ ID NO: 124)-A (herein referred to as BCY18279);
A-(SEQ ID NO: 125)-A (herein referred to as BCY18280);
A-(SEQ ID NO: 126)-A (herein referred to as BCY18281);
A-(SEQ ID NO: 127)-A (herein referred to as BCY18282);
A-(SEQ ID NO: 128)-A (herein referred to as BCY18285);
A-(SEQ ID NO: 129)-A (herein referred to as BCY18286);
A-(SEQ ID NO: 130)-A (herein referred to as BCY18287);
A-(SEQ ID NO: 131)-A (herein referred to as BCY18288);
A-(SEQ ID NO: 132)-A (herein referred to as BCY18289);
A-(SEQ ID NO: 133)-A (herein referred to as BCY18290);
A-(SEQ ID NO: 134)-A (herein referred to as BCY18291);
A-(SEQ ID NO: 135)-A (herein referred to as BCY18292);
Ac-(SEQ ID NO: 136) (herein referred to as BCY18433);
Ac-(SEQ ID NO: 137) (herein referred to as BCY18434);
Ac-(SEQ ID NO: 138) (herein referred to as BCY18435);
Ac-(SEQ ID NO: 139) (herein referred to as BCY18437);
Ac-(SEQ ID NO: 140) (herein referred to as BCY18439);
Ac-(SEQ ID NO: 141) (herein referred to as BCY18440);
Ac-(SEQ ID NO: 142) (herein referred to as BCY18441);
Ac-(SEQ ID NO: 143) (herein referred to as BCY18442);
Ac-(SEQ ID NO: 144) (herein referred to as BCY18443);
Ac-(SEQ ID NO: 145) (herein referred to as BCY18444);
Ac-(SEQ ID NO: 146) (herein referred to as BCY19682);
Ac-(SEQ ID NO: 147) (herein referred to as BCY19683);
Ac-(SEQ ID NO: 148) (herein referred to as BCY19684);
Ac-(SEQ ID NO: 149) (herein referred to as BCY19685);
Ac-(SEQ ID NO: 150) (herein referred to as BCY19686);
Ac-(SEQ ID NO: 151) (herein referred to as BCY19687);
Ac-(SEQ ID NO: 152) (herein referred to as BCY19688);
Ac-(SEQ ID NO: 153) (herein referred to as BCY19689);
Ac-(SEQ ID NO: 154) (herein referred to as BCY19690);
Ac-(SEQ ID NO: 155) (herein referred to as BCY19691);
Ac-(SEQ ID NO: 156) (herein referred to as BCY19692);
Ac-(SEQ ID NO: 157) (herein referred to as BCY19693);
A-(SEQ ID NO: 158)-A (herein referred to as BCY19720);
A-(SEQ ID NO: 159)-A (herein referred to as BCY19721);
A-(SEQ ID NO: 160)-A (herein referred to as BCY19722);
A-(SEQ ID NO: 161)-A (herein referred to as BCY19723);
A-(SEQ ID NO: 162)-A (herein referred to as BCY19724);
A-(SEQ ID NO: 163)-A (herein referred to as BCY19725);
A-(SEQ ID NO: 164)-A (herein referred to as BCY19730);
A-(SEQ ID NO: 165)-A (herein referred to as BCY19732);
A-(SEQ ID NO: 166)-A (herein referred to as BCY19733);
A-(SEQ ID NO: 167)-A (herein referred to as BCY19734); and
A-(SEQ ID NO: 168)-A (herein referred to as BCY19735);
wherein PYA represents pentynoic acid, Sar represents sarcosine and FI represents fluorescein Aib represents aminoisobutyric acid, dK represents D-lysine, Dap represents L-2,3-diaminopropionic acid, Dab represents L-2,4-diaminobutyric acid, and GABA represents γ-aminobutyric acid.

4. The peptide ligand as defined in claim 1, wherein said bicyclic peptide is specific for FcyRIIIA (also known as CD16a) and wherein the bicyclic peptide ligand comprises an amino acid sequence which is:
CᵢPPTVYCᵢᵢHPLDCᵢᵢᵢ (SEQ ID NO: 60);
CᵢRWSVEDPCᵢᵢGAWCᵢᵢᵢ (SEQ ID NO: 61);
CᵢVGLEELGPCᵢᵢSDLCᵢᵢᵢ (SEQ ID NO: 62; herein referred to as BCY16494 when complexed with TBMT);
CᵢPPAVYCᵢᵢHPLDCᵢᵢᵢ (SEQ ID NO: 169);
CᵢPPEVFCᵢᵢHPLDCᵢᵢᵢ (SEQ ID NO: 170);
CᵢPPEVYCᵢᵢHPLDCᵢᵢᵢ (SEQ ID NO: 171);
CᵢPPEVWCᵢᵢHPLDCᵢᵢᵢ (SEQ ID NO: 172);
CᵢPPQVYCᵢᵢHPLDCᵢᵢᵢ (SEQ ID NO: 173);
CᵢPPEVWCᵢᵢHPLDCᵢᵢᵢ (SEQ ID NO: 174);
CᵢRWS[tBuGly]EDPCᵢᵢGAWCᵢᵢᵢ (SEQ ID NO: 175);
CᵢRWSVED[HyP]CᵢᵢGAWCᵢᵢᵢ (SEQ ID NO: 176);
CᵢRWSVED[Aze]CᵢᵢGAWCᵢᵢᵢ (SEQ ID NO: 177);
CᵢRWSVEDPCᵢᵢ[dA]AWCᵢᵢᵢ (SEQ ID NO: 178);
CᵢRWSVE[Gla]PCᵢᵢGAWCᵢᵢᵢ (SEQ ID NO: 179);
CᵢRWSVED[Aze]CᵢᵢGAWCᵢᵢᵢ (SEQ ID NO: 180);
CᵢMWIRSDPCᵢᵢGAWCᵢᵢᵢ (SEQ ID NO: 181);
CᵢRWYPDDPCᵢᵢGAWCᵢᵢᵢ (SEQ ID NO: 182);
CᵢEWHISEPCᵢᵢGAWCᵢᵢᵢ (SEQ ID NO: 183);
CᵢNWHVYEPCᵢᵢGAWCᵢᵢᵢ (SEQ ID NO: 184);
CᵢRWHFSEPCᵢᵢGAWCᵢᵢᵢ (SEQ ID NO: 185);
CᵢRWNLDDPCᵢᵢGAWCᵢᵢᵢ (SEQ ID NO: 186);
CᵢMWIRSDPCᵢᵢGAWCᵢᵢᵢ (SEQ ID NO: 187);
CᵢRWYPDDPCᵢᵢGAWCᵢᵢᵢ (SEQ ID NO: 188);
CᵢEWHISEPCᵢᵢGAWCᵢᵢᵢ (SEQ ID NO: 189);
CᵢNWHVYEPCᵢᵢGAWCᵢᵢᵢ (SEQ ID NO: 190);
CᵢRWHFSEPCᵢᵢGAWCᵢᵢᵢ (SEQ ID NO: 191);
CᵢRWNLDDPCᵢᵢGAWCᵢᵢᵢ (SEQ ID NO: 192);
CᵢR[2MeTrp]SVEDPCᵢᵢGAWCᵢᵢᵢ (SEQ ID NO: 193);
CᵢR[6MeTrp]SVEDPCᵢᵢGAWCᵢᵢᵢ (SEQ ID NO: 194);
CᵢR[6FTrp]SVEDPCᵢᵢGAWCᵢᵢᵢ (SEQ ID NO: 195);
CᵢR[5FTrp]SVEDPCᵢᵢGAWCᵢᵢᵢ (SEQ ID NO: 196);
CᵢR[6CITrp]SVEDPCᵢᵢGAWCᵢᵢᵢ (SEQ ID NO: 197);
CᵢR[Trp(S)]SVEDPCᵢᵢGAWCᵢᵢᵢ (SEQ ID NO: 198);
CᵢR[AzaTrp]SVEDPCᵢᵢGAWCᵢᵢᵢ (SEQ ID NO: 199);
CᵢRWSVEDPCᵢᵢGA[2MeTrp]Cᵢᵢᵢ (SEQ ID NO: 200);
CᵢRWSVEDPCᵢᵢGA[6FTrp]Cᵢᵢᵢ (SEQ ID NO: 201);
CᵢRWSVEDPCᵢᵢGA[5FTrp]Cᵢᵢᵢ (SEQ ID NO: 202);
CᵢRWSVEDPCᵢᵢGA[4MeoTrp]Cᵢᵢᵢ (SEQ ID NO: 203);
CᵢRWSVEDPCᵢᵢGA[5MeoTrp]Cᵢᵢᵢ (SEQ ID NO: 204);
CᵢRWSVEDPCᵢᵢGA[Trp(S)]Cᵢᵢᵢ (SEQ ID NO: 205);
CᵢRWSVEDPCᵢᵢGA[DOPA]Cᵢᵢᵢ (SEQ ID NO: 206);
CᵢRWYPDDPCᵢᵢGAWCᵢᵢᵢ (SEQ ID NO: 207);
CᵢRWHFSEPCᵢᵢGAWCᵢᵢᵢ (SEQ ID NO: 208);
CᵢR[2MeTrp]HFSEPCᵢᵢGAWCᵢᵢᵢ (SEQ ID NO: 209);
CᵢRWYFSEPCᵢᵢGAWCᵢᵢᵢ (SEQ ID NO: 210);
CᵢRWSFSEPCᵢᵢGAWCᵢᵢᵢ (SEQ ID NO: 211);
CᵢRWHPSEPCᵢᵢGAWCᵢᵢᵢ (SEQ ID NO: 212);
CᵢRWYPSEPCᵢᵢGAWCᵢᵢᵢ (SEQ ID NO: 213);
CᵢRWHPDEPCᵢᵢGAWCᵢᵢᵢ (SEQ ID NO: 214);
CᵢRWHPEEPCᵢᵢGAWCᵢᵢᵢ (SEQ ID NO: 215);
CᵢRWHFDEPCᵢᵢGAWCᵢᵢᵢ (SEQ ID NO: 216);
CᵢRWHFDDPCᵢᵢGAWCᵢᵢᵢ (SEQ ID NO: 217);
CᵢRWYFDEPCᵢᵢGAWCᵢᵢᵢ (SEQ ID NO: 218);
CᵢRW[His1Me]FSEPCᵢᵢGAWCᵢᵢᵢ (SEQ ID NO: 219);
CᵢRW[His3Me]FSEPCᵢᵢGAWCᵢᵢᵢ (SEQ ID NO: 220);
CᵢRW[4MeoPhe]FSEPCᵢᵢGAWCᵢᵢᵢ (SEQ ID NO: 221);
CᵢRW[DOPA]FSEPCᵢᵢGAWCᵢᵢᵢ (SEQ ID NO: 222);
CᵢRW[26DiMeTyr]FSEPCᵢᵢGAWCᵢᵢᵢ (SEQ ID NO: 223);
CᵢRW[3tBuTyr]FSEPCᵢᵢGAWCᵢᵢᵢ (SEQ ID NO: 224);
CᵢRW[4FPhe]FSEPCᵢᵢGAWCᵢᵢᵢ (SEQ ID NO: 225);
CᵢRWH[4MePhe]SEPCᵢᵢGAWCᵢᵢᵢ (SEQ ID NO: 226);
CᵢRWH[Aze]SEPCᵢᵢGAWCᵢᵢᵢ (SEQ ID NO: 227);
CᵢRWH[HyP]SEPCᵢᵢGAWCᵢᵢᵢ (SEQ ID NO: 228);
CᵢRWH[4tBuPhe]SEPCᵢᵢGAWCᵢᵢᵢ (SEQ ID NO: 229);
CᵢRWH[HPhe]SEPCᵢᵢGAWCᵢᵢᵢ (SEQ ID NO: 230, herein referred to as BCY21693 when complexed with TBMT);
CᵢRWH[4PhePro]SEPCᵢᵢGAWCᵢᵢᵢ (SEQ ID NO: 231);
CᵢRWHF[HSer]EPCᵢᵢGAWCᵢᵢᵢ (SEQ ID NO: 232);
CᵢRWHFS[Gla]PCᵢᵢGAWCᵢᵢᵢ (SEQ ID NO: 233);
CᵢRWHFS[HGlu]PCᵢᵢGAWCᵢᵢᵢ (SEQ ID NO: 234);
CᵢRWHFSEPCᵢᵢ[dA]AWCᵢᵢᵢ (SEQ ID NO: 235);
CᵢRWYFSE[Aze]Cᵢᵢ[dA]AWCᵢᵢᵢ (SEQ ID NO: 236);
CᵢRWHFDE[Aze]Cᵢᵢ[dA]AWCᵢᵢᵢ (SEQ ID NO: 237);
CᵢRWYPSE[Aze]Cᵢᵢ[dA]AWCᵢᵢᵢ (SEQ ID NO: 238);
Cᵢ[HArg]WHFSEPCᵢᵢGAWCᵢᵢᵢ (SEQ ID NO: 239);
CᵢRWH[Cis-HyP]SEPCᵢᵢGAWCᵢᵢᵢ (SEQ ID NO: 240);
CᵢRWHFSEPCᵢᵢGAWCᵢᵢᵢ (SEQ ID NO: 241);
CᵢRWHFSEPCᵢᵢGAWCᵢᵢᵢ (SEQ ID NO: 242);
CᵢRWHFSEPCᵢᵢGAWCᵢᵢᵢ (SEQ ID NO: 243);
CᵢVGLLELGPCᵢᵢSDLCᵢᵢᵢ (SEQ ID NO: 244);
CᵢPGLEELGPCᵢᵢSDLCᵢᵢᵢ (SEQ ID NO: 245);
CᵢV[dA]LEELGPCᵢᵢSDLCᵢᵢᵢ (SEQ ID NO: 246);
CᵢVG[tBuAla]EELGPCᵢᵢSDLCᵢᵢᵢ (SEQ ID NO: 247);
CᵢVG[Cba]EELGPCᵢᵢSDLCᵢᵢᵢ (SEQ ID NO: 248);
CᵢVGFEELGPCᵢᵢSDLCᵢᵢᵢ (SEQ ID NO: 249);
CᵢVG[1Nal]EELGPCᵢᵢSDLCᵢᵢᵢ (SEQ ID NO: 250);
CᵢVG[2Nal]EELGPCᵢᵢSDLCᵢᵢᵢ (SEQ ID NO: 251);
CᵢVGLPELGPCᵢᵢSDLCᵢᵢᵢ (SEQ ID NO: 252);
CᵢVGL[tBuAla]ELGPCᵢᵢSDLCᵢᵢᵢ (SEQ ID NO: 253);
CᵢVGL[Cba]ELGPCᵢᵢSDLCᵢᵢᵢ (SEQ ID NO: 254);
CᵢVGL[Cha]ELGPCᵢᵢSDLCᵢᵢᵢ (SEQ ID NO: 255);
CᵢVGL[Nle]ELGPCᵢᵢSDLCᵢᵢᵢ (SEQ ID NO: 256);
CᵢVGLE[Gla]LGPCᵢᵢSDLCᵢᵢᵢ (SEQ ID NO: 257);
CᵢVGLEELG[Aze]CᵢᵢSDLCᵢᵢᵢ (SEQ ID NO: 258);
CᵢVGLEELGPCᵢᵢSD[tBuAla]Cᵢᵢᵢ (SEQ ID NO: 259);
CᵢVGLEELGPCᵢᵢSD[Cba]Cᵢᵢᵢ (SEQ ID NO: 260);
CᵢVGLEELGPCᵢᵢSD[Cha]Cᵢᵢᵢ (SEQ ID NO: 261);
CᵢVG[HLeu]EELGPCᵢᵢSDLCᵢᵢᵢ (SEQ ID NO: 262);
CᵢVGL[HLeu]ELGPCᵢᵢSDLCᵢᵢᵢ (SEQ ID NO: 263);
CᵢVGLE[HGlu]LGPCᵢᵢSDLCᵢᵢᵢ (SEQ ID NO: 264);
CᵢTWPYCᵢᵢKSWGDVQDCᵢᵢᵢ (SEQ ID NO: 265);
CᵢDWWDPGCᵢᵢTYFCᵢᵢᵢ (SEQ ID NO: 266);
CᵢRWH[4BnPro]SEPCᵢᵢGAWCᵢᵢᵢ (SEQ ID NO: 267); and
CᵢRWH[HPhe]SE[Aze]CᵢᵢGAWCᵢᵢᵢ (SEQ ID NO: 268).

5. The peptide ligand as defined in claim 4, wherein the molecular scaffold is TATB and the bicyclic peptide ligand additionally comprises N- and/or C-terminal additions and comprises an amino acid sequence which is selected from:
A-(SEQ ID NO: 265)-A (herein referred to as BCY15044);
A-(SEQ ID NO: 265)-AGAAAE (herein referred to as BCY19416).
A-(SEQ ID NO: 266)-A (herein referred to as BCY15045); and
A-(SEQ ID NO: 266)-AGAAAE (herein referred to as BCY19417).

6. The peptide ligand as defined in claim 4, wherein the molecular scaffold is TATA and the bicyclic peptide ligand additionally comprises N- and/or C-terminal additions and comprises an amino acid sequence which is selected from:
A-(SEQ ID NO: 60)-A (herein referred to as BCY11808);
A-(SEQ ID NO: 60)-A-[K(PYA)] (herein referred to as BCY15685);
A-(SEQ ID NO: 60)-A-[Sar6]-[KBiot] (herein referred to as BCY12621);
A-(SEQ ID NO: 169)-A (herein referred to as BCY11673);
A-(SEQ ID NO: 170)-A (herein referred to as BCY11809);
A-(SEQ ID NO: 171)-A (herein referred to as BCY11810);
A-(SEQ ID NO: 172)-A (herein referred to as BCY11811)
A-(SEQ ID NO: 173)-A (herein referred to as BCY11812); and
A-(SEQ ID NO: 174)-A-[Sar6]-[KBiot] (herein referred to as BCY12622);
wherein Sar represents sarcosine and Biot represents biotin.

7. The peptide ligand as defined in claim 4, wherein the molecular scaffold is TBMT and the bicyclic peptide ligand additionally comprises N- and/or C-terminal additions and comprises an amino acid sequence which is selected from:
A-(SEQ ID NO: 61)-A (herein referred to as BCY14950);
Ac-A-(SEQ ID NO: 61)-A (herein referred to as BCY16599);
A-(SEQ ID NO: 61)-AGAAAE (herein referred to as BCY19040);
A-(SEQ ID NO: 61)-A-[K(PYA)] (herein referred to as BCY13883);
Ac-A-(SEQ ID NO: 61)-A-[K(PYA)] (herein referred to as BCY19596);
A-(SEQ ID NO: 62)-A (herein referred to as BCY16493);
Ac-(SEQ ID NO: 62) (herein referred to as BCY16495);
A-(SEQ ID NO: 62)-A-[K(PYA)] (herein referred to as BCY13886);
A-(SEQ ID NO: 175)-A (herein referred to as BCY14953);
A-(SEQ ID NO: 176)-A (herein referred to as BCY14954);
[PYA]-A-(SEQ ID NO: 177) (herein referred to as BCY14955);
A-(SEQ ID NO: 178)-A (herein referred to as BCY14956);
A-(SEQ ID NO: 179)-A (herein referred to as BCY16608);
A-(SEQ ID NO: 180)-A (herein referred to as BCY16610);
A-(SEQ ID NO: 181)-A (herein referred to as BCY19032);
A-(SEQ ID NO: 182)-A (herein referred to as BCY19033);
A-(SEQ ID NO: 183)-A (herein referred to as BCY19034);
A-(SEQ ID NO: 184)-A (herein referred to as BCY19035);
A-(SEQ ID NO: 185)-A (herein referred to as BCY19036);
A-(SEQ ID NO: 186)-A (herein referred to as BCY19037);
A-(SEQ ID NO: 187)-AGAAAE (herein referred to as BCY19038);
A-(SEQ ID NO: 188)-AGAAAE (herein referred to as BCY19039);
A-(SEQ ID NO: 189)-AGAAAE (herein referred to as BCY19041);
A-(SEQ ID NO: 190)-AGAAAE (herein referred to as BCY19042);
A-(SEQ ID NO: 191)-AGAAAE (herein referred to as BCY19043);
A-(SEQ ID NO: 192)-AGAAAE (herein referred to as BCY19044);
A-(SEQ ID NO: 193)-A (herein referred to as BCY19045);
A-(SEQ ID NO: 194)-A (herein referred to as BCY19046);
A-(SEQ ID NO: 195)-A (herein referred to as BCY19048);
A-(SEQ ID NO: 196)-A (herein referred to as BCY19049);
A-(SEQ ID NO: 197)-A (herein referred to as BCY19050);
A-(SEQ ID NO: 198)-A (herein referred to as BCY19053);
A-(SEQ ID NO: 199)-A (herein referred to as BCY19055);
A-(SEQ ID NO: 200)-A (herein referred to as BCY19057);
A-(SEQ ID NO: 201)-A (herein referred to as BCY19060);
A-(SEQ ID NO: 202)-A (herein referred to as BCY19061);
A-(SEQ ID NO: 203)-A (herein referred to as BCY19063);
A-(SEQ ID NO: 204)-A (herein referred to as BCY19064);
A-(SEQ ID NO: 205)-A (herein referred to as BCY19065);
A-(SEQ ID NO: 206)-A (herein referred to as BCY19068);
A-(SEQ ID NO: 207)-A-[K(PYA)] (herein referred to as BCY20360);
A-(SEQ ID NO: 208)-A-[K(PYA)] (herein referred to as BCY20361);
A-(SEQ ID NO: 209)-A (herein referred to as BCY20363);
A-(SEQ ID NO: 210)-A (herein referred to as BCY20364);
A-(SEQ ID NO: 211)-A (herein referred to as BCY20365);
A-(SEQ ID NO: 212)-A (herein referred to as BCY20366);
A-(SEQ ID NO: 213)-A (herein referred to as BCY20367);
A-(SEQ ID NO: 214)-A (herein referred to as BCY20368);
A-(SEQ ID NO: 215)-A (herein referred to as BCY20369);
A-(SEQ ID NO: 216)-A (herein referred to as BCY20370);
A-(SEQ ID NO: 217)-A (herein referred to as BCY20371);
A-(SEQ ID NO: 218)-A (herein referred to as BCY20372);
A-(SEQ ID NO: 219)-A (herein referred to as BCY20373);
A-(SEQ ID NO: 220)-A (herein referred to as BCY20374);
A-(SEQ ID NO: 221)-A (herein referred to as BCY20375);
A-(SEQ ID NO: 222)-A (herein referred to as BCY20376);
A-(SEQ ID NO: 223)-A (herein referred to as BCY20377);
A-(SEQ ID NO: 224)-A (herein referred to as BCY20378);
A-(SEQ ID NO: 225)-A (herein referred to as BCY20379);
A-(SEQ ID NO: 226)-A (herein referred to as BCY20380);
A-(SEQ ID NO: 227)-A (herein referred to as BCY20382);
A-(SEQ ID NO: 228)-A (herein referred to as BCY20383);
A-(SEQ ID NO: 229)-A (herein referred to as BCY20384);
A-(SEQ ID NO: 230)-A (herein referred to as BCY20385);
(SEQ ID NO: 230)-A (herein referred to as BCY21690);
Ac-(SEQ ID NO: 230)-A (herein referred to as BCY21691);
A-(SEQ ID NO: 230) (herein referred to as BCY21692);
Ac-(SEQ ID NO: 230) (herein referred to as BCY21694);
[Nle]-(SEQ ID NO: 230)-A (herein referred to as BCY21695);
Ac-[Nle]-(SEQ ID NO: 230)-A (herein referred to as BCY21696);
Ac-A-(SEQ ID NO: 230)-A (herein referred to as BCY21697);
A-(SEQ ID NO: 231)-A (herein referred to as BCY20386);
A-(SEQ ID NO: 232)-A (herein referred to as BCY20388);
A-(SEQ ID NO: 233)-A (herein referred to as BCY20389);
A-(SEQ ID NO: 234)-A (herein referred to as BCY20390);
A-(SEQ ID NO: 235)-A (herein referred to as BCY20392);
A-(SEQ ID NO: 236)-A (herein referred to as BCY20394);
A-(SEQ ID NO: 237)-A (herein referred to as BCY20395);
A-(SEQ ID NO: 238)-A (herein referred to as BCY20396);
A-(SEQ ID NO: 239)-A (herein referred to as BCY20397);
A-(SEQ ID NO: 240)-A (herein referred to as BCY20526);
Ac-A-(SEQ ID NO: 241)-A (herein referred to as BCY20527);
[Aib]-(SEQ ID NO: 242)-A (herein referred to as BCY20528);
A-(SEQ ID NO: 243)-[Aib] (herein referred to as BCY20529);
A-(SEQ ID NO: 244)-A-[K(PYA)] (herein referred to as BCY16165);
A-(SEQ ID NO: 245)-A (herein referred to as BCY16498);
A-(SEQ ID NO: 246)-A (herein referred to as BCY16499);
A-(SEQ ID NO: 247)-A (herein referred to as BCY16501);
A-(SEQ ID NO: 248)-A (herein referred to as BCY16502);
A-(SEQ ID NO: 249)-A (herein referred to as BCY16504);
A-(SEQ ID NO: 250)-A (herein referred to as BCY16505);
A-(SEQ ID NO: 251)-A (herein referred to as BCY16506);
A-(SEQ ID NO: 252)-A (herein referred to as BCY16507);
A-(SEQ ID NO: 253)-A (herein referred to as BCY16508);
A-(SEQ ID NO: 254)-A (herein referred to as BCY16509);
A-(SEQ ID NO: 255)-A (herein referred to as BCY16510);
A-(SEQ ID NO: 256)-A (herein referred to as BCY16511);
A-(SEQ ID NO: 257)-A (herein referred to as BCY16512);
A-(SEQ ID NO: 258)-A (herein referred to as BCY16520);
A-(SEQ ID NO: 259)-A (herein referred to as BCY16522);
A-(SEQ ID NO: 260)-A (herein referred to as BCY16523);
A-(SEQ ID NO: 261)-A (herein referred to as BCY16524);
A-(SEQ ID NO: 262)-A (herein referred to as BCY16527);
A-(SEQ ID NO: 263)-A (herein referred to as BCY16528);
A-(SEQ ID NO: 264)-A (herein referred to as BCY16529),
A-(SEQ ID NO: 267)-A (herein referred to as BCY20387); and
A-(SEQ ID NO: 268)-A (herein referred to as BCY21689),
wherein PYA represents pentynoic acid and Aib represents aminoisobutryic acid.

8. The peptide ligand as defined in any one of claims 1 to 7, wherein the pharmaceutically acceptable salt is selected from the free acid or the sodium, potassium, calcium or ammonium salt.

9. A multimeric binding complex which comprises at least two bicyclic peptide ligands as defined in any one of claims 1 to 8, wherein said peptide ligands may be the same or different.

10. The multimeric binding complex as defined in claim 9, which comprises an NKp46 homodimer selected from BCY15663, BCY15921, BCY15922, BCY15455, and BCY18043.

11. The multimeric binding complex as defined in claim 9, which comprises a CD16a homodimer selected from BCY15914, BCY15915, and BCY15916.

12. A pharmaceutical composition which comprises the peptide ligand as defined in any one of claims 1 to 8 or the multimeric binding complex as defined in any one of claims 9 to 11, in combination with one or more pharmaceutically acceptable excipients.

13. The peptide ligand as defined in any one of claims 1 to 8, or the multimeric binding complex as defined in any one of claims 9 to 11, or the pharmaceutical composition of claim 12, for use in therapy.

14. The peptide ligand as defined in any one of claims 1 to 8, or the multimeric binding complex as defined in any one of claims 9 to 11, or the pharmaceutical composition of claim 12, for use in preventing, suppressing or treating a disease or disorder mediated by natural killer (NK) cells, such as inflammatory disorders, autoimmune disease and cancer.

## Patentansprüche

1. Peptidligand, der spezifisch für natürliche Killerzellen (NK-Zellen) ist, oder ein pharmazeutisch annehmbares Salz davon, umfassend ein Polypeptid, das mindestens drei reaktive Gruppen umfasst, die durch mindestens zwei Schleifensequenzen getrennt sind, und ein molekulares Gerüst, das kovalente Bindungen mit den reaktiven Gruppen des Polypeptids bildet, so dass zumindest zwei Polypeptidschleifen auf dem molekularen Gerüst gebildet werden, wobei der bicyclische Peptidligand eine Aminosäuresequenz umfasst, die ausgewählt ist aus:
CᵢNLQKPCᵢᵢMKYPCᵢᵢᵢ (SEQ ID NO: 1);
CᵢNLQNPCᵢᵢMKFPCᵢᵢᵢ (SEQ ID NO: 2);
CᵢNLQAPCᵢᵢMOTGKVCᵢᵢᵢ (SEQ ID NO: 3);
CᵢNLQAPCᵢᵢMLTGKVCᵢᵢᵢ (SEQ ID NO: 4, hierin bezeichnet als BCY18295, wenn komplexiert mit TATA);
CᵢLLHDHCᵢᵢPNTHPKLCᵢᵢᵢ (SEQ ID NO: 5);
CᵢLLHEHCᵢᵢPNTHPKMCᵢᵢᵢ (SEQ ID NO: 6);
CᵢLLHDHCᵢᵢPNSHPEICᵢᵢᵢ (SEQ ID NO: 7);
CᵢLLHDHCᵢᵢPNTHPIICᵢᵢᵢ (SEQ ID NO: 8);
CᵢYLPDWLCᵢᵢGDEYCᵢᵢᵢ (SEQ ID NO: 9);
CᵢYLPDYLCᵢᵢGDEYCᵢᵢᵢ (SEQ ID NO: 10);
CᵢDLTTHNCᵢᵢQWGICᵢᵢᵢ (SEQ ID NO: 11, hierin bezeichnet als BCY18262, wenn komplexiert mit TATA);
CᵢYLPDYLCᵢᵢGDEYCᵢᵢᵢ (SEQ ID NO: 12; hierin bezeichnet als BCY15633, wenn komplexiert mit TATA);
CᵢYLPDWLCᵢᵢGDEYCᵢᵢᵢ (SEQ ID NO: 13);
Cᵢ[2,6DiMeTyr]LPDYLCᵢᵢGDEYCᵢᵢᵢ (SEQ ID NO: 14);
CᵢY[tBuAla]PDYLCᵢᵢGDEYCiii (SEQ ID NO: 15);
CᵢY[Cba]PDYLCᵢᵢGDEYCᵢᵢᵢ (SEQ ID NO: 16);
CᵢYL[HyP]DYLCᵢᵢGDEYCᵢᵢᵢ (SEQ ID NO: 17);
CᵢYL[Aze]DYLCᵢᵢGDEYCᵢᵢᵢ (SEQ ID NO: 18);
CᵢYL[Aib]DYLCᵢᵢGDEYCᵢᵢᵢ (SEQ ID NO: 19);
CᵢYLPD[1Nal]LCᵢᵢGDEYCᵢᵢᵢ (SEQ ID NO: 20);
CᵢYLPD[2Nal]LCᵢᵢGDEYCᵢᵢᵢ (SEQ ID NO: 21);
CᵢYLPD[3Pal]LCᵢᵢGDEYCᵢᵢᵢ (SEQ ID NO: 22);
CᵢYLPD[2,6DiMeTyr]LCᵢᵢGDEYCᵢᵢᵢ (SEQ ID NO: 23);
CᵢYLPOW[tBuAla]CᵢᵢGDEYCᵢᵢᵢ (SEQ ID NO: 24);
CᵢYLPOW[Cba]CᵢᵢGDEYCᵢᵢᵢ (SEQ ID NO: 25);
CᵢYLPDYLCᵢᵢ[dA]DEYCᵢᵢᵢ (SEQ ID NO: 26);
CᵢYLPDYLCᵢᵢGDE[2,6DiMeTyr]Cᵢᵢᵢ (SEQ ID NO: 27);
CᵢALPDYLCᵢᵢGDEYCᵢᵢᵢ (SEQ ID NO: 28);
CᵢYAPDYLCᵢᵢGDEYCᵢᵢᵢ (SEQ ID NO: 29);
CᵢYLADYLCᵢᵢGDEYCᵢᵢᵢ (SEQ ID NO: 30);
CᵢYLPAYLCᵢᵢGDEYCᵢᵢᵢ (SEQ ID NO: 31);
CᵢYLPDALCᵢᵢGDEYCᵢᵢᵢ (SEQ ID NO: 32);
CᵢYLPDYACᵢᵢGDEYCᵢᵢᵢ (SEQ ID NO: 33);
CᵢYLPDYLCᵢᵢADEYCᵢᵢᵢ (SEQ ID NO: 34);
CᵢYLPDYLCᵢᵢGAEYCᵢᵢᵢ (SEQ ID NO: 35);
CᵢYLPDYLCᵢᵢGDAYCᵢᵢᵢ (SEQ ID NO: 36);
CᵢYLPDYLCᵢᵢGDEACᵢᵢᵢ (SEQ ID NO: 37);
CᵢY[C5A]PDYLCᵢᵢGDEYCᵢᵢᵢ (SEQ ID NO: 38);
CᵢY[Cha]PDYLCᵢᵢGDEYCᵢᵢᵢ (SEQ ID NO: 39);
CᵢY[Cba]PDYLCᵢᵢ[dA]DEYCᵢᵢᵢ (SEQ ID NO: 40);
CᵢYLPD[4FPhe]LCᵢᵢGDEYCᵢᵢᵢ (SEQ ID NO: 41);
CᵢYLPD[4MeOPhe]LCᵢᵢGDEYCᵢᵢᵢ (SEQ ID NO: 42);
CᵢYLPD[NO2Phe]LCᵢᵢGDEYCᵢᵢᵢ (SEQ ID NO: 43);
CᵢYLPD[4MePhe]LCᵢᵢGDEYCᵢᵢᵢ (SEQ ID NO: 44);
CᵢYLPD[pCoPhe]LCᵢᵢGDEYCᵢᵢᵢ (SEQ ID NO: 45);
CᵢYLPD[pCaPhe]LCᵢᵢGDEYCᵢᵢᵢ (SEQ ID NO: 46);
CᵢYLPD[4tBuPhe]LCᵢᵢGDEYCᵢᵢᵢ (SEQ ID NO: 47);
CᵢYLPD[CF3Phe]LCᵢᵢGDEYCᵢᵢᵢ (SEQ ID NO: 48);
CᵢYLPD[4CNPhe]LCᵢᵢGDEYCᵢᵢᵢ (SEQ ID NO: 49);
CᵢYLPD[4ClPhe]LCᵢᵢGDEYCᵢᵢᵢ (SEQ ID NO: 50);
CᵢYLPD[HPhe]LCᵢᵢGDEYCᵢᵢᵢ (SEQ ID NO: 51);
Cᵢ[2,6DiMeTyr]LPDWLCᵢᵢGDEYCᵢᵢᵢ (SEQ ID NO: 52);
CᵢY[Cba]PDWLCᵢᵢGDEYCᵢᵢᵢ (SEQ ID NO: 53);
CᵢYL[Aze]DWLCᵢᵢGDEYCᵢᵢᵢ (SEQ ID NO: 54);
CᵢYLPDWLCᵢᵢ[dA]DEYCᵢᵢᵢ (SEQ ID NO: 55);
CᵢSLPDYLCᵢᵢGDEYCᵢᵢᵢ (SEQ ID NO: 56);
CᵢDLTTHNCᵢᵢQWGICᵢᵢᵢ (SEQ ID NO: 57);
CᵢDLTTHNCᵢᵢLWGVCᵢᵢᵢ (SEQ ID NO: 58);
CᵢNLQLHNCᵢᵢIWGVCᵢᵢᵢ (SEQ ID NO: 59);
CᵢPPTVYCᵢᵢHPLOCᵢᵢᵢ (SEQ ID NO: 60);
CᵢRWSVEDPCᵢᵢGAWCᵢᵢᵢ (SEQ ID NO: 61);
CᵢVGLEELGPCᵢᵢSDLCᵢᵢᵢ (SEQ ID NO: 62; hierin bezeichnet als BCY16494, wenn komplexiert mit TBMT);
CᵢNLQQACᵢᵢLHTGKVCᵢᵢᵢ (SEQ ID NO: 63);
CᵢNLQAACᵢᵢMLTGKVCᵢᵢᵢ (SEQ ID NO: 64);
CᵢNLQAPCᵢᵢMATGKVCᵢᵢᵢ (SEQ ID NO: 65);
CᵢNLQAPCᵢᵢMLAGKVCᵢᵢᵢ (SEQ ID NO: 66);
CᵢNLQAPCᵢᵢMLTAKVCᵢᵢᵢ (SEQ ID NO: 67);
CᵢNLQAPCᵢᵢMLTGAVCᵢᵢᵢ (SEQ ID NO: 68);
CᵢNLQAPCᵢᵢMLTGKACᵢᵢᵢ (SEQ ID NO: 69);
CᵢN[tBuAla]OAPCᵢᵢMLTGKVCᵢᵢᵢ (SEQ ID NO: 70);
CᵢNLQA[Sar]CᵢᵢMLTGKVCᵢᵢᵢ (SEQ ID NO: 71);
CᵢNLQA[Cis-HyP]CᵢᵢMLTGKVCᵢᵢᵢ (SEQ ID NO: 72);
CᵢNLQA[HyP]CᵢᵢMLTGKVCᵢᵢᵢ (SEQ ID NO: 73);
CᵢNLQA[NMeAla]CᵢᵢMLTGKVCᵢᵢᵢ (SEQ ID NO: 74);
CᵢNLQAPCᵢᵢLLTGKVCᵢᵢᵢ (SEQ ID NO: 75);
CᵢNLQAPCᵢᵢ[Nle]LTGKVCᵢᵢᵢ (SEQ ID NO: 76);
CᵢNLQAPCᵢᵢ[Cba]LTGKVCᵢᵢᵢ (SEQ ID NO: 77);
CᵢNLQAPCᵢᵢMETGKVCᵢᵢᵢ (SEQ ID NO: 78);
CᵢNLQAPCᵢᵢMFTGKVCᵢᵢᵢ (SEQ ID NO: 79);
CᵢNLQAPCᵢᵢMYTGKVCᵢᵢᵢ (SEQ ID NO: 80);
CᵢNLQAPCᵢᵢM[Cba]TGKVCᵢᵢᵢ (SEQ ID NO: 81);
CᵢNLQAPCᵢᵢM[tBuAla]TGKVCᵢᵢᵢ (SEQ ID NO: 82);
CᵢNLQAPCᵢᵢM[Cha]TGKVCᵢᵢᵢ (SEQ ID NO: 83);
CᵢNLQAPCᵢᵢMLT[dA]KVCᵢᵢᵢ (SEQ ID NO: 84);
CᵢNLQAPCᵢᵢMLTGRVCᵢᵢᵢ (SEQ ID NO: 85);
CᵢNLQAPCᵢᵢMLTG[Agb]VCᵢᵢᵢ (SEQ ID NO: 86);
CᵢNLQAPCᵢᵢMLTGK[tBuGly]Cᵢᵢᵢ (SEQ ID NO: 87);
CᵢNLQAPCᵢᵢMLTGK[Chg]Cᵢᵢᵢ (SEQ ID NO: 88);
CᵢNLQAPCᵢᵢMLTGK[Cbg]Cᵢᵢᵢ (SEQ ID NO: 89);
CᵢNLQAPCᵢᵢMLTG[HArg]VCᵢᵢᵢ (SEQ ID NO: 90);
CᵢNLQA[Aze]CᵢᵢMLTGKVCᵢᵢᵢ (SEQ ID NO: 91);
CᵢNLQA[Pip]CᵢᵢMLTGKVCᵢᵢᵢ (SEQ ID NO: 92);
CᵢNLQAPCᵢᵢMVTGKVCᵢᵢᵢ (SEQ ID NO: 93);
CᵢNLQAPCᵢᵢM[Nle]TGKVCᵢᵢᵢ (SEQ ID NO: 94);
CᵢNLQAPCᵢᵢM[Nva]TGKVCᵢᵢᵢ (SEQ ID NO: 95);
CᵢNLQAPCᵢᵢMLTGK[Allolle]Cᵢᵢᵢ (SEQ ID NO: 96);
CᵢLCQAPCᵢᵢMLTGK[EPA]Cᵢᵢᵢ (SEQ ID NO: 97);
CᵢNLQQPCᵢᵢMLTGKVCᵢᵢᵢ (SEQ ID NO: 98);
CᵢNLQA[Nva]CᵢᵢMLTGKVCᵢᵢᵢ (SEQ ID NO: 99);
CᵢNLQAPCᵢᵢMHTGKVCᵢᵢᵢ (SEQ ID NO: 100);
CᵢNLQAPCᵢᵢLHTGKVCᵢᵢᵢ (SEQ ID NO: 101);
CᵢNLQQACᵢᵢMQTGKVCᵢᵢᵢ (SEQ ID NO: 102);
CᵢLLHDHCᵢᵢPNTHPKMCᵢᵢᵢ (SEQ ID NO: 103);
CᵢDLTTHNCᵢᵢIWGVCᵢᵢᵢ (SEQ ID NO: 104);
CᵢNLQLHNCᵢᵢIWGVCᵢᵢᵢ (SEQ ID NO: 105);
CᵢNLQLHNCᵢᵢQWGICᵢᵢᵢ (SEQ ID NO: 106);
Cᵢ[3tBuTyr]LPDYLCᵢᵢGDEYCᵢᵢᵢ (SEQ ID NO: 107);
CᵢYLPD[3tBuTyr]LCᵢᵢGDEYCᵢᵢᵢ (SEQ ID NO: 108);
CᵢYLPDYLCᵢᵢGDE[3tBuTyr]Cᵢᵢᵢ (SEQ ID NO: 109);
Cᵢ[K(PYA)]LPOYLCᵢᵢGDEYCᵢᵢᵢ (SEQ ID NO: 110);
CᵢYLP[K(PYA)]YLCᵢᵢGDEYCᵢᵢᵢ (SEQ ID NO: 111);
CᵢYLPDYLCᵢᵢ[K(PYA)]DEYCᵢᵢᵢ (SEQ ID NO: 112);
CᵢYLPDYLCᵢᵢGD[K(PYA)]YCᵢᵢᵢ (SEQ ID NO: 113);
CᵢYLPDYLCᵢᵢGDE[K(PYA)]Cᵢᵢᵢ (SEQ ID NO: 114);
CᵢYLPDYLCᵢᵢ[dK(PYA)DEYCᵢᵢᵢ (SEQ ID NO: 115);
CᵢALTTHNCᵢᵢQWGICᵢᵢᵢ (SEQ ID NO: 116);
CᵢDLATHNCᵢᵢQWGICᵢᵢᵢ (SEQ ID NO: 117);
CᵢDLTAHNCᵢᵢQWGICᵢᵢᵢ (SEQ ID NO: 118);
CᵢDLTTHNCᵢᵢAWGICᵢᵢᵢ (SEQ ID NO: 119);
CᵢDLTTHNCᵢᵢQWGACᵢᵢᵢ (SEQ ID NO: 120);
CᵢNLTTHNCᵢᵢQWGICᵢᵢᵢ (SEQ ID NO: 121);
CᵢDLQTHNCᵢᵢQWGICᵢᵢᵢ (SEQ ID NO: 122);
CᵢDLTLHNCᵢᵢQWGICᵢᵢᵢ (SEQ ID NO: 123);
CᵢDLTT[His3Me]NCᵢᵢQWGICᵢᵢᵢ (SEQ ID NO: 124);
CᵢDLTT[2Pal]NCᵢᵢQWGICᵢᵢᵢ (SEQ ID NO: 125);
CᵢDLTT[His1Me]NCᵢᵢQWGICᵢᵢᵢ (SEQ ID NO: 126);
CᵢDLTT[4ThiAz]NCᵢᵢQWGICᵢᵢᵢ (SEQ ID NO: 127);
CᵢDLTTHNCᵢᵢVWGICᵢᵢᵢ (SEQ ID NO: 128);
CᵢDLTTHNCᵢᵢ[Cba]WGICᵢᵢᵢ (SEQ ID NO: 129);
CᵢDLTTHNCᵢᵢQ[1NaI]GICᵢᵢᵢ (SEQ ID NO: 130);
GᵢDLTTHNCᵢᵢQ[Trp(Me)]GICᵢᵢᵢ (SEQ ID NO: 131);
CᵢDLTTHNCᵢᵢQWG[tBuGly]ᵢᵢᵢ (SEQ ID NO: 132);
CᵢDLTTHNCᵢᵢQWG[tBuAla]Cᵢᵢᵢ (SEQ ID NO: 133);
CᵢDLTTHNCᵢᵢQWG[Chg]Cᵢᵢᵢ (SEQ ID NO: 134);
CᵢDLTTHNCᵢᵢQW[dA]ICᵢᵢᵢ (SEQ ID NO: 135);
CᵢS[Cba]PDYLCᵢᵢ[dA]DEYCᵢᵢᵢ (SEQ ID NO: 136)
Cᵢ[HSer][Cba]PDYLCᵢᵢ[dA]DEYCᵢᵢᵢ (SEQ ID NO: 137);
CᵢN[Cba]PDYLCᵢᵢ[dA]DEYCᵢᵢᵢ (SEQ ID NO: 138);
Cᵢ[4Pal][Cba]PDYLCᵢᵢ[dA]DEYCᵢᵢᵢ (SEQ ID NO: 139);
CᵢY[Cba][Cis-HyP]DYLCᵢᵢ[dA]DEYCᵢᵢᵢ (SEQ ID NO: 140);
CᵢY[Cba][NMeAla]DYLCᵢᵢ[dA]DEYCᵢᵢᵢ (SEQ ID NO: 141);
CᵢY[Cba]PNYLCᵢᵢ[dA]DEYCᵢᵢᵢ (SEQ ID NO: 142);
CᵢY[Cba]PQYLCᵢᵢ[dA]DEYCᵢᵢᵢ (SEQ ID NO: 143);
CᵢY[Cba]PDYLCᵢᵢ[dV]DEYCᵢᵢᵢ (SEQ ID NO: 144);
CᵢY[Cba]PDYLCᵢᵢ[dNva]DEYCᵢᵢᵢ (SEQ ID NO: 145);
CᵢY[Cba]PDYLCᵢᵢ[dF]DEYCᵢᵢᵢ (SEQ ID NO: 146);
CᵢY[Cba]PDYLCᵢᵢ[dCha]DEYCᵢᵢᵢ (SEQ ID NO: 147);
CᵢY[Cba]PDYLCᵢᵢ[dK(PYA)]DEYCᵢᵢᵢ (SEQ ID NO: 148);
CᵢY[Cba]PDYLCᵢᵢ[dNle]DEYCᵢᵢᵢ (SEQ ID NO: 149);
CᵢY[Cba]PD[DOPA]LCᵢᵢ[dA]DEYCᵢᵢᵢ (SEQ ID NO: 150);
CᵢY[Cba]PD[2FTyr]LCᵢᵢ[dA]DEYCᵢᵢᵢ (SEQ ID NO: 151);
CᵢY[Cba]PDY[Nle]Cᵢᵢ[dA]DEYCᵢᵢᵢ (SEQ ID NO: 152);
[Pen]ᵢY[Cba]PDYLCᵢᵢ[dA]DEYCᵢᵢᵢ (SEQ ID NO: 153);
CᵢY[Cba]PDYL[Pen]ᵢᵢ[dA]DEYCᵢᵢᵢ (SEQ ID NO: 154);
CᵢY[Cba]PDYLCᵢᵢ[dA]DEY[Pen]ᵢᵢᵢ (SEQ ID NO: 155);
CᵢY[Cba]PDYLCᵢᵢ[dA]DEY[dCᵢᵢᵢ] (SEQ ID NO: 156);
[dCᵢ]Y[Cba]PDYLCᵢᵢ[dA]DEYCᵢᵢᵢ (SEQ ID NO: 157);
CᵢDLTTHNCᵢᵢQ[AzaTrp]GICᵢᵢᵢ (SEQ ID NO: 158);
CᵢDLTTHNCᵢᵢQ[2MeTrp]GICᵢᵢᵢ (SEQ ID NO: 159);
CᵢDLTTHCᵢᵢQ[6MeTrp]GICᵢᵢᵢ (SEQ ID NO: 160);
CᵢDLTTHNCᵢᵢQ[7MeTrp]GICᵢᵢᵢ (SEQ ID NO: 161);
CᵢDLTTHNCᵢᵢQ[6FTrp]GICᵢᵢᵢ (SEQ ID NO: 162);
CᵢDLTTHNCᵢᵢQ[5FTrp]GICᵢᵢᵢ (SEQ ID NO: 163);
CᵢDLTTHNCᵢᵢQ[Dht]GICᵢᵢᵢ (SEQ ID NO: 164);
CᵢDLTT[2FuAla]NCᵢᵢQWGICᵢᵢᵢ (SEQ ID NO: 165);
CᵢDLTTHNCᵢᵢQWG[EPA]Cᵢᵢᵢ (SEQ ID NO: 166);
CᵢDLTTHNCᵢᵢQWG[Nle]Cᵢᵢᵢ (SEQ ID NO: 167);
CᵢDLTTHNCᵢᵢQWG[Allolle]Cᵢᵢᵢ (SEQ ID NO: 168);
CᵢPPAVYCᵢᵢHPLDCᵢᵢᵢ (SEQ ID NO: 169);
CᵢPPEVFCᵢᵢHPLDCᵢᵢᵢ (SEQ ID NO: 170);
CᵢPPEVYCᵢᵢHPLDCᵢᵢᵢ (SEQ ID NO: 171);
CᵢPPEVWCᵢᵢHPLDCᵢᵢᵢ (SEQ ID NO: 172);
CᵢPPQVYCᵢᵢHPLOCᵢᵢᵢ (SEQ ID NO: 173);
CᵢPPEVWCᵢᵢHPLDCᵢᵢᵢ (SEQ ID NO: 174);
CᵢRWS[tBuGly]EDPCᵢᵢGAWCᵢᵢᵢ (SEQ ID NO: 175);
CᵢRWSVED[HyP]CᵢᵢGAWCᵢᵢᵢ (SEQ ID NO: 176);
CᵢRWSVED[Aze]CᵢᵢGAWCᵢᵢᵢ (SEQ ID NO: 177);
CᵢRWSVEDPCᵢᵢ[dA]AWCᵢᵢᵢ (SEQ ID NO: 178);
CᵢRWSVE[Gla]PCᵢᵢGAWCᵢᵢᵢ (SEQ ID NO: 179);
CᵢRWSVED[Aze]CᵢᵢGAWCᵢᵢᵢ (SEQ ID NO: 180);
CᵢMWIRSDPCᵢᵢGAWCᵢᵢᵢ (SEQ ID NO: 181);
CᵢRWYPDDPCᵢᵢGAWCᵢᵢᵢ (SEQ ID NO: 182);
CᵢEWHISEPCᵢᵢGAWCᵢᵢᵢ (SEQ ID NO: 183);
CᵢNWHVYEPCᵢᵢGAWCᵢᵢᵢ (SEQ ID NO: 184);
CiRWHFSEPCiiGAWCiii (SEQ ID NO: 185);
CᵢRWNLDDPCᵢᵢGAWCᵢᵢᵢ (SEQ ID NO: 186);
CᵢMWIRSDPCᵢᵢGAWCᵢᵢᵢ (SEQ ID NO: 187);
CᵢRWYPDDPCᵢᵢGAWCᵢᵢᵢ (SEQ ID NO: 188);
CᵢEWHISEPCᵢᵢGAWCᵢᵢᵢ (SEQ ID NO: 189);
CᵢNWHVYEPCᵢᵢGAWCᵢᵢᵢ (SEQ ID NO: 190);
CᵢRWHFSEPCᵢᵢGAWCᵢᵢᵢ (SEQ ID NO: 191);
CᵢRWNLDDPCᵢᵢGAWCᵢᵢᵢ (SEQ ID NO: 192);
CᵢR[2MeTrp]SVEDPCᵢᵢGAVVCᵢᵢᵢ (SEQ ID NO: 193);
CᵢR[6MeTrp]SVEDPCᵢᵢGAWCᵢᵢᵢ (SEQ ID NO: 194);
CᵢR[6FTrp]SVEDPCᵢᵢGAWCᵢᵢᵢ (SEQ ID NO: 195);
CᵢR[5FTrp]SVEDPCᵢᵢGAWCᵢᵢᵢ (SEQ ID NO: 196);
CᵢR[6ClTrp]SVEDPCᵢᵢGAWCᵢᵢᵢ (SEQ ID NO: 197);
CᵢR[Trp(S)]SVEDPCᵢᵢGAWCᵢᵢᵢ (SEQ ID NO: 198);
CᵢR[AzaTrp]SVEDPCᵢᵢGAWCᵢᵢᵢ (SEQ ID NO: 199);
CᵢRWSVEDPCᵢᵢGA[2MeTrp]Cᵢᵢᵢ (SEQ ID NO: 200);
CᵢRWSVEOPCᵢᵢGA[6FTrp]Cᵢᵢᵢ (SEQ ID NO: 201);
CᵢRWSVEDPCᵢᵢGA[5FTrp]Cᵢᵢᵢ (SEQ ID NO: 202);
CᵢRWSVEDPCᵢᵢGA[4MeoTrp]Cᵢᵢᵢ (SEQ ID NO: 203);
CᵢRWSVEDPCᵢᵢGA[5MeoTrp]Cᵢᵢᵢ (SEQ ID NO: 204);
CᵢRWSVEDPCᵢᵢGA[Trp(S)]Cᵢᵢᵢ (SEQ ID NO: 205);
CᵢRWSVEDPCᵢᵢGA[DOPA]Cᵢᵢᵢ (SEQ ID NO: 206);
CᵢRWYPDDPCᵢᵢGAWCᵢᵢᵢ (SEQ ID NO: 207);
CᵢRWHFSEPCᵢᵢGAWCᵢᵢᵢ (SEQ ID NO: 208);
CᵢR[2MeTrp]HFSEPCᵢᵢGAWCᵢᵢᵢ (SEQ ID NO: 209);
CᵢRWYFSEPCᵢᵢGAWCᵢᵢᵢ (SEQ ID NO: 210);
CᵢRWSFSEPCᵢᵢGAWCᵢᵢᵢ (SEQ ID NO: 211);
CᵢRWHPSEPCᵢᵢGAWCᵢᵢᵢ (SEQ ID NO: 212);
CᵢRWYPSEPCᵢᵢGAWCᵢᵢᵢ (SEQ ID NO: 213);
CᵢRWHPDEPCᵢᵢGAWCᵢᵢᵢ (SEQ ID NO: 214);
CᵢRWHPEEPCᵢᵢGAWCᵢᵢ (SEQ ID NO: 215);
CᵢRWHFDEPCᵢᵢGAWCᵢᵢᵢ (SEQ ID NO: 216);
CᵢRWHFDDPCᵢᵢGAWCᵢᵢᵢ (SEQ ID NO: 217);
CiRWYFDEPCiiGAWCiii (SEQ ID NO: 218);
CᵢRW[His1Me]FSEPCᵢᵢGAWCᵢᵢᵢ (SEQ ID NO: 219);
CᵢRW[His3Me]FSEPCᵢᵢGAWCᵢᵢᵢ (SEQ ID NO: 220);
CᵢRW[4MeoPhe]FSEPCᵢᵢGAWCᵢᵢᵢ (SEQ ID NO: 221);
CᵢRW[DOPA]FSEPCᵢᵢGAWCᵢᵢᵢ (SEQ ID NO: 222);
CᵢRW[26DiMeTyr]FSEPCᵢᵢGAWCᵢᵢᵢ (SEQ ID NO: 223);
CᵢRW[3tBuTyr]FSEPCᵢᵢGAWCᵢᵢᵢ (SEQ ID NO: 224);
CiRW[4FPhe]FSEPCiiGAWCiii (SEQ ID NO: 225);
CᵢRWH[4MePhe]SEPCᵢᵢGAWCᵢᵢᵢ (SEQ ID NO: 226);
CᵢRWH[Aze]SEPCᵢᵢGAWCᵢᵢᵢ (SEQ ID NO: 227);
CᵢRWH[HyP]SEPCᵢᵢGAWCᵢᵢᵢ (SEQ ID NO: 228);
CᵢRWH[4tBuPhe]SEPCᵢᵢGAWCᵢᵢᵢ (SEQ ID NO: 229);
CᵢRWH[HPhe]SEPCᵢᵢGAWCᵢᵢᵢ (SEQ ID NO: 230; hierin bezeichnet als BCY21693, wenn komplexiert mit TBMT);
CᵢRWH[4PhePro]SEPCᵢᵢGAWCᵢᵢᵢ (SEQ ID NO: 231);
CᵢRWHF[HSer]EPCᵢᵢGAWCᵢᵢᵢ (SEQ ID NO: 232);
CᵢRWHFS[Gla]PCᵢᵢGAWCᵢᵢᵢ (SEQ ID NO: 233);
CᵢRWHFS[HGlu]PCᵢᵢGAWCᵢᵢᵢ (SEQ ID NO: 234);
CᵢRWHFSEPCᵢᵢ[dA]AWCᵢᵢᵢ (SEQ ID NO: 235);
CᵢRWfFSE[Aze]Cᵢᵢ[dA]AWCᵢᵢᵢ (SEQ ID NO: 236);
CᵢRMFDE[Aze]Cᵢᵢ[dA]AWCᵢᵢᵢ (SEQ ID NO: 237);
CᵢRWYPSE[Aze]Cᵢᵢ[dA]AWCᵢᵢᵢ (SEQ ID NO: 238);
Cᵢ[HArg]WHFSEPCᵢᵢGAWCᵢᵢᵢ (SEQ ID NO: 239);
CᵢRWH[Cis-HyP]SEPCᵢᵢGAWCᵢᵢᵢ (SEQ ID NO: 240);
CᵢRWHFSEPCᵢᵢGAWCᵢᵢᵢ (SEQ ID NO: 241);
CᵢRWHFSEPCᵢᵢGAWCᵢᵢᵢ (SEQ ID NO: 242);
CᵢRWHFSEPCᵢᵢGAWCᵢᵢᵢ (SEQ ID NO: 243);
CᵢVGLLELGPCᵢᵢSDLCᵢᵢᵢ (SEQ ID NO: 244);
CᵢPGLEELGPCᵢᵢSDLCᵢᵢᵢ (SEQ ID NO: 245);
CᵢV[dA]LEELGPCᵢᵢSDLCᵢᵢᵢ (SEQ ID NO: 246);
CᵢVG[tBuAla]EELGPCᵢᵢSDLCᵢᵢᵢ (SEQ ID NO: 247);
CᵢVG[Cba]EELGPCᵢᵢSDLCᵢᵢᵢ (SEQ ID NO: 248);
CᵢVGFEELGPCᵢᵢSDLCᵢᵢᵢ (SEQ ID NO: 249);
CᵢVG[1Nal]EELGPCᵢᵢSDLCᵢᵢᵢ (SEQ ID NO: 250);
CᵢVG[2Nal]EELGPCᵢᵢSDLCᵢᵢᵢ (SEQ ID NO: 251);
CᵢVGLPELGPCᵢᵢSDLCᵢᵢᵢ (SEQ ID NO: 252);
CᵢVGL[tBuAla]ELGPCᵢᵢSDLCᵢᵢᵢ (SEQ ID NO: 253);
CᵢVGL[Cba]ELGPCᵢᵢSDLCᵢᵢᵢ (SEQ ID NO: 254);
CᵢVGL[Cha]ELGPCᵢᵢSDLCᵢᵢᵢ (SEQ ID NO: 255);
CᵢVGL[Nle]ELGPCᵢᵢSDLCᵢᵢᵢ (SEQ ID NO: 256);
CᵢVGLE[Gla]LGPCᵢᵢSDLCᵢᵢᵢ (SEQ ID NO: 257);
CᵢVGLEELG[Aze]CᵢᵢSDLCᵢᵢᵢ (SEQ ID NO: 258);
CᵢVGLEELGPCᵢᵢSD[tBuAla]Cᵢᵢᵢ (SEQ ID NO: 259);
CᵢVGLEELGPCᵢᵢSD[Cba]Cᵢᵢᵢ (SEQ ID NO: 260);
CᵢVGLEELGPCᵢᵢSD[Cha]Cᵢᵢᵢ (SEQ ID NO: 261);
CᵢVG[HLeu]EELGPCᵢᵢSDLCᵢᵢᵢ (SEQ ID NO: 262);
CᵢVGL[HLeu]ELGPCᵢᵢSDLCᵢᵢᵢ (SEQ ID NO: 263);
CᵢVGLE[HGlu]LGPCᵢᵢSDLCᵢᵢᵢ (SEQ ID NO: 264);
CᵢTWPYCᵢᵢKSWGDVQDCᵢᵢᵢ (SEQ ID NO: 265);
CᵢDWWDPGCᵢᵢTYFCᵢᵢᵢ (SEQ ID NO: 266);
CᵢRWH[4BnPro]SEPCᵢᵢGAWCᵢᵢᵢ (SEQ ID NO: 267); und
CᵢRWH[HPhe]SE[Aze]CᵢᵢGAWCᵢᵢᵢ (SEQ ID NO: 268);
wobei Cᵢ, Penᵢ, Cᵢᵢ, Penᵢᵢ, Cᵢᵢᵢ und Penᵢᵢᵢ jeweils die erste, zweite und dritte reaktive Gruppe darstellen und wobei 2,6DiMeTyr 2,6-Dimethyl-tyrosin darstellt, tBuAla β-t-Butyl-L-alanin darstellt, Cba β-Cyclobutylalanin darstellt, HyP Hydroxyprolin darstellt, Aze Azetidin-2-carbonsäure darstellt, Aib Aminoisobuttersäure darstellt, 1Nal 1-Naphthylalanin darstellt, 2Nal 2-Naphthylalanin darstellt, 3Pal 3-Pyridylalanin darstellt, C5A beta-Cyclopentyl-L-alanin darstellt, Cha 3-Cyclohexyl-L-alanin darstellt, 4FPhe 4-Fluor-L-phenylalanin darstellt, 4MeOPhe 4-Methoxy-phenylalanin darstellt, NO2Phe 4-Nitrophenylalanin darstellt, 4MePhe 4-Methyl-phenylalanin darstellt, pCoPhe para-Carboxy-phenylalanin darstellt, pCaPhe p-Carboxamido-phenylalanin darstellt, 4tBuPhe 4-tert-Butyl-phenylalanin darstellt, CF3Phe 4-Trifluormethyl-phenylalanin darstellt, 4CNPhe 4-Cyano-phenylalanin darstellt, 4CIPhe 4-Chlorphenylalanin darstellt, HPhe Homophenylalanin darstellt, 3tBuTyr 3 -tert-Butyltyrosin darstellt, His3Me 3-Methylhistidin darstellt, 2Pal 2-Pyridylalanin darstellt, HislMe 1-Methylhistidin darstellt, 4ThiAz beta-4-Thiazol-L-ylalanin darstellt, Trp(Me) 1-Methyltryptophan darstellt, tBuGly tert-Butylglycin darstellt, Chg Cyclohexylglycin darstellt, HSer Homoserin darstellt, NMeAla N-Methylalanin darstellt, 4Pal 4-Pyridylalanin darstellt, Cis-HyP L-4-cis-Hydroxyprolin darstellt, dA D-Alanin darstellt, dV D-Valin darstellt, Nva Norvalin darstellt, dNva D-Norvalin darstellt, Nle Norleucin darstellt, dNle D-Norleucin darstellt, dF D-Phenylalanin darstellt, dCha 3-Cyclohexyl-D-alanin darstellt, DOPA 3,4-Dihydroxyphenylalanin darstellt, 2FTyr 2-Fluortyrosin darstellt, dC D-Cystein darstellt, AzaTrp Azatryptophan darstellt, 2MeTrp 2-Methyltryptophan darstellt, 6MeTrp 6-Methyltryptophan darstellt, 6FTrp 6-Fluortryptophan darstellt, 5FTrp 5-Fluortryptophan darstellt, Dht 2,3-Dihydrotryptophan darstellt, 2FuAla 2-Furylalanin darstellt, Allolle L-allo-Isoleucine darstellt, Sar Sarcosin darstellt, Agb Norarginin darstellt, Cbg Cyclobutylglycin darstellt, HArg Homoarginin darstellt, Pep Pipecolinsäure darstellt, EPA Diethyl-L-alanin darstellt, Gla y-Carboxyglutaminsäure darstellt, 6CITrp 6-Chlor-L-tryptophan darstellt, 4MeoTrp 4-Methoxy-L-tryptophan darstellt, 5MeoTrp 5-Methoxy-L-tryptophan darstellt, Trp(S) 3-(3-Benzothienyl)-L-alanin darstellt, 26DiMeTyr 2,6 -Dimethyl-L-tyrosin darstellt, 4PhePro (2S,4S)-4-Phenylprolin darstellt, HGlu L-2-Aminoadipinsäure darstellt, 4BnPro (2S,4R)-4-Benzylpyrrolidin-2-carbonsäure darstellt und HLeu Homoleucin darstellt.

2. Peptidligand nach Anspruch 1, wobei das bicyclische Peptid spezifisch für NKp46 ist und wobei der bicyclische Peptidligand eine Aminosäuresequenz umfasst, die ausgewählt ist aus:
CᵢNLQKPCᵢᵢMKYPCᵢᵢᵢ (SEQ ID NO: 1); und
CᵢNLQNPCᵢᵢMKFPCᵢᵢᵢ (SEQ ID NO: 2);
CᵢNLQAPCᵢᵢMQTGKVCᵢᵢᵢ (SEQ ID NO: 3);
CᵢNLQAPCᵢᵢMLTGKVCᵢᵢᵢ (SEQ ID NO: 4, hierin bezeichnet als BCY18295, wenn komplexiert mit TATA);
CᵢLLHDHCᵢᵢPNTHPKLCᵢᵢᵢ (SEQ ID NO: 5);
CᵢLLHEHCᵢᵢPNTHPKMCᵢᵢᵢ (SEQ ID NO: 6);
CᵢLLHDHCᵢᵢPNSHPEICᵢᵢᵢ (SEQ ID NO: 7);
CᵢLLHDHCᵢᵢPNTHPIICᵢᵢᵢ (SEQ ID NO: 8);
CᵢYLPDWLCᵢᵢGDEYCᵢᵢᵢ (SEQ ID NO: 9);
CᵢYLPDYLCᵢᵢGDEYCᵢᵢᵢ (SEQ ID NO: 10);
CᵢDLTTHNCᵢᵢQWGICᵢᵢᵢ (SEQ ID NO: 11, hierin bezeichnet als BCY18262, wenn komplexiert mit TATA);
CᵢYLPDYLCᵢᵢGDEYCᵢᵢᵢ (SEQ ID NO: 12; hierin bezeichnet als BCY15633, wenn komplexiert mit TATA);
CᵢYLPDWLCᵢᵢGDEYCᵢᵢᵢ (SEQ ID NO: 13);
Cᵢ[2,6DiMeTyr]LPDYLCᵢᵢGDEYCᵢᵢᵢ (SEQ ID NO: 14);
CᵢY[tBuAla]PDYLCᵢᵢGDEYCᵢᵢᵢ (SEQ ID NO: 15);
CᵢY[Cba]PDYLCᵢᵢGDEYCᵢᵢᵢ (SEQ ID NO: 16);
CᵢYL[HyP]DYLCᵢᵢGDEYCᵢᵢᵢ (SEQ ID NO: 17);
CᵢYL[Aze]DYLCᵢᵢGDEYCᵢᵢᵢ (SEQ ID NO: 18);
CᵢYL[Aib]DYLCᵢᵢGDEYCᵢᵢᵢ (SEQ ID NO: 19);
CᵢYLPD[1Nal]LCᵢᵢGDEYCᵢᵢᵢ (SEQ ID NO: 20);
CᵢYLPO[2Nal]LCᵢᵢGDEYCᵢᵢᵢ (SEQ ID NO: 21);
CᵢYLPD[3Pal]LCᵢᵢGDEYCᵢᵢᵢ (SEQ ID NO: 22);
CᵢYLPD[2,6DiMeTyr]LCᵢᵢGDEYCᵢᵢᵢ (SEQ ID NO: 23);
CᵢYLPDW[tBuAla]CᵢᵢGDEYCᵢᵢᵢ (SEQ ID NO: 24);
CᵢYLPDW[Cba]CᵢᵢGDEYCᵢᵢᵢ (SEQ ID NO: 25);
CᵢYLPDYLCᵢᵢ[dA]DEYCᵢᵢᵢ (SEQ ID NO: 26);
CᵢYLPDYLCᵢᵢGDE[2,6DiMeTyr]Cᵢᵢᵢ (SEQ ID NO: 27);
CᵢALPDYLCᵢᵢGDEYCᵢᵢᵢ (SEQ ID NO: 28);
CᵢYAPDYLCᵢᵢGDEYCᵢᵢᵢ (SEQ ID NO: 29);
CᵢYLADYLCᵢᵢGDEYCᵢᵢᵢ (SEQ ID NO: 30);
CᵢYLPAYLCᵢᵢGDEYCᵢᵢᵢ (SEQ ID NO: 31);
CᵢYLPDALCᵢᵢGDEYCᵢᵢᵢ (SEQ ID NO: 32);
CᵢYLPDYACᵢᵢGDEYCᵢᵢᵢ (SEQ ID NO: 33);
CᵢYLPDYLCᵢᵢADEYCᵢᵢᵢ (SEQ ID NO: 34);
CᵢYLPDYLCᵢᵢGAEYCᵢᵢ (SEQ ID NO: 35);
CᵢYLPDYLCᵢᵢGDAYCᵢᵢᵢ (SEQ ID NO: 36);
CᵢYLPDYLCᵢᵢGDEACᵢᵢᵢ (SEQ ID NO: 37);
CᵢY[C5A]PDYLCᵢᵢGDEYCᵢᵢᵢ (SEQ ID NO: 38);
CᵢY[Cha]PDYLCᵢᵢGDEYCᵢᵢᵢ (SEQ ID NO: 39);
CᵢY[Cba]PDYLCᵢᵢ[dA]DEYCᵢᵢᵢ (SEQ ID NO: 40);
CᵢYLPD[4FPhe]LCᵢᵢGDEYCᵢᵢᵢ (SEQ ID NO: 41);
CᵢYLPD[4MeOPhe]LCᵢᵢGDEYCᵢᵢᵢ (SEQ ID NO: 42);
CᵢYLPD[NO2Phe]LCᵢᵢGDEYCᵢᵢᵢ (SEQ ID NO: 43);
CᵢYLPD[4MePhe]LCᵢᵢGDEYCᵢᵢᵢ (SEQ ID NO: 44);
CᵢYLPD[pCoPhe]LCᵢᵢGDEYCᵢᵢᵢ (SEQ ID NO: 45);
CᵢYLPD[pCaPhe]LCᵢᵢGDEYCᵢᵢᵢ (SEQ ID NO: 46);
CᵢYLPO[4tBuPhe]LCᵢᵢGDEYCᵢᵢᵢ (SEQ ID NO: 47);
CᵢYLPD[CF3Phe]LCᵢᵢGDEYCᵢᵢᵢ (SEQ ID NO: 48);
CᵢYLPD[4CNPhe]LCᵢᵢGDEYCᵢᵢᵢ (SEQ ID NO: 49);
CᵢYLPD[4ClPhe]LCᵢᵢGDEYCᵢᵢᵢ (SEQ ID NO: 50);
CᵢYLPD[HPhe]LCᵢᵢGDEYCᵢᵢᵢ (SEQ ID NO: 51);
Cᵢ[2,6DiMeTyr]LPDWLCᵢᵢGDEYCᵢᵢᵢ (SEQ ID NO: 52);
CᵢY[Cba]PDWLCᵢᵢGDEYCᵢᵢᵢ (SEQ ID NO: 53);
CᵢYL[Aze]DWLCᵢᵢGDEYCᵢᵢᵢ (SEQ ID NO: 54);
CᵢYLPDWLCᵢᵢ[dA]DEYCᵢᵢᵢ (SEQ ID NO: 55);
CᵢSLPDYLCᵢᵢGDEYCᵢᵢᵢ (SEQ ID NO: 56);
CᵢDLTTHNCᵢᵢQWGICᵢᵢᵢ (SEQ ID NO: 57);
CᵢDLTTHNCᵢᵢLWGVCᵢᵢᵢ (SEQ ID NO: 58);
CᵢNLQLHNCᵢᵢIWGVCᵢᵢᵢ (SEQ ID NO: 59);
CᵢNLQQACᵢᵢLHTGKVCᵢᵢᵢ (SEQ ID NO: 63);
CᵢNLQAACᵢᵢMLTGKVCᵢᵢᵢ (SEQ ID NO: 64);
CᵢNLQAPCᵢᵢMATGKVCᵢᵢᵢ (SEQ ID NO: 65);
CᵢNLQAPCᵢᵢMLAGKVCᵢᵢᵢ (SEQ ID NO: 66);
CᵢNLQAPCᵢᵢMLTAKVCᵢᵢᵢ (SEQ ID NO: 67);
CᵢNLQAPCᵢᵢMLTGAVCᵢᵢᵢ (SEQ ID NO: 68);
CᵢNLQAPCᵢᵢMLTGKACᵢᵢᵢ (SEQ ID NO: 69);
CᵢN[tBuAla]QAPCᵢᵢMLTGKVCᵢᵢᵢ (SEQ ID NO: 70);
CᵢNLQA[Sar]CᵢᵢMLTGKVCᵢᵢᵢ (SEQ ID NO: 71);
CᵢNLQA[Cis-HyP]CᵢᵢMLTGKVCᵢᵢᵢ (SEQ ID NO: 72);
CᵢNLQA[HyP]CᵢᵢMLTGKVCᵢᵢᵢ (SEQ ID NO: 73);
CᵢNLQA[NMeAla]CᵢᵢMLTGKVCᵢᵢᵢ (SEQ ID NO: 74);
CᵢNLQAPCᵢᵢLLTGKVCᵢᵢᵢ (SEQ ID NO: 75);
CᵢNLQAPCᵢᵢ[Nle]LTGKVCᵢᵢᵢ (SEQ ID NO: 76);
CᵢNLQAPCᵢᵢ[Cba]LTGKVCᵢᵢᵢ (SEQ ID NO: 77);
CᵢNLQAPCᵢᵢMETGKVCᵢᵢᵢ (SEQ ID NO: 78);
CᵢNLQAPCᵢᵢMFTGKVCᵢᵢᵢ (SEQ ID NO: 79);
CᵢNLQAPCᵢᵢMYTGKVCᵢᵢᵢ (SEQ ID NO: 80);
CᵢNLQAPCᵢᵢM[Cba]TGKVCᵢᵢᵢ (SEQ ID NO: 81);
CᵢNLQAPCᵢᵢM[tBuAla]TGKVCᵢᵢᵢ (SEQ ID NO: 82);
CᵢNLQAPCᵢᵢM[Cha]TGKVCᵢᵢᵢ (SEQ ID NO: 83);
CᵢNLQAPCᵢᵢMLT[dA]KVCᵢᵢᵢ (SEQ ID NO: 84);
CᵢNLQAPCᵢᵢMLTGRVCᵢᵢᵢ (SEQ ID NO: 85);
CᵢNLQAPCᵢᵢMLTG[Agb]VCᵢᵢᵢ (SEQ ID NO: 86);
CᵢNLQAPCᵢᵢMLTGK[tBuGly]Cᵢᵢᵢ (SEQ ID NO: 87);
CᵢNLQAPCᵢᵢMLTGK[Chg]Cᵢᵢᵢ (SEQ ID NO: 88);
CᵢNLQAPCᵢᵢMLTGK[Cbg]Cᵢᵢᵢ (SEQ ID NO: 89);
CᵢNLQAPCᵢᵢMLTG[HArg]VCᵢᵢᵢ (SEQ ID NO: 90);
CᵢNLQA[Aze]CᵢᵢMLTGKVCᵢᵢᵢ (SEQ ID NO: 91);
CᵢNLQA[Pip]CᵢᵢMLTGKVCᵢᵢᵢ (SEQ ID NO: 92);
CᵢNLQAPCᵢᵢMVTGKVCᵢᵢᵢ (SEQ ID NO: 93);
CᵢNLQAPCᵢᵢM[Nle]TGKVCᵢᵢᵢ (SEQ ID NO: 94);
CᵢNLQAPCᵢᵢM[Nva]TGKVCᵢᵢᵢ (SEQ ID NO: 95);
CᵢNLQAPCᵢᵢMLTGK[Allolle]Cᵢᵢᵢ (SEQ ID NO: 96);
CᵢNLQAPCᵢᵢMLTGK[EPA]Cᵢᵢᵢ (SEQ ID NO: 97);
CᵢNLQQPCᵢᵢMLTGKVCᵢᵢᵢ (SEQ ID NO: 98);
CᵢNLQA[Nva]CᵢᵢMLTGKVCᵢᵢᵢ (SEQ ID NO: 99);
CᵢNLQAPCᵢᵢMHTGKVCᵢᵢᵢ (SEQ ID NO: 100);
CᵢNLQAPCᵢᵢLHTGKVCᵢᵢᵢ (SEQ ID NO: 101);
CᵢNLQQACᵢᵢMQTGKVCᵢᵢᵢ (SEQ ID NO: 102);
CᵢLLHDHCᵢᵢPNTHPKMCᵢᵢᵢ (SEQ ID NO: 103);
CᵢDLTTHNCᵢᵢIWGVCᵢᵢᵢ (SEQ ID NO: 104);
CᵢNLQLHNCᵢᵢIWGVCᵢᵢᵢ (SEQ ID NO: 105);
CᵢNLQLHNCᵢᵢQWGICᵢᵢᵢ (SEQ ID NO: 106);
Cᵢ[3tBuTyr]LPDYLCᵢᵢGDEYCᵢᵢᵢ (SEQ ID NO: 107);
CᵢYLPD[3tBuTyr]LCᵢᵢGDEYCᵢᵢᵢ (SEQ ID NO: 108);
CᵢYLPDYLCᵢᵢGDE[3tBuTyr]Cᵢᵢᵢ (SEQ ID NO: 109);
Cᵢ[K(PYA)]LPDYLCᵢᵢGDEYCᵢᵢᵢ (SEQ ID NO: 110);
CᵢYLP[K(PYA)]YLCᵢᵢGDEYCᵢᵢᵢ (SEQ ID NO: 111);
CᵢYLPDYLCᵢᵢ[K(PYA)]DEYCᵢᵢᵢ (SEQ ID NO: 112);
CᵢYLPOYLCᵢᵢGD[K(PYA)]YCᵢᵢᵢ (SEQ ID NO: 113);
CᵢYLPDYLCᵢᵢGDE[K(PYA)]Cᵢᵢᵢ (SEQ ID NO: 114);
CᵢYLPOYLCᵢᵢ[dK(PYA)]DEYCᵢᵢᵢ (SEQ ID NO: 115);
CᵢALTTHNCᵢᵢQWGICᵢᵢᵢ (SEQ ID NO: 116);
CᵢDLATHNCᵢᵢQWGICᵢᵢᵢ (SEQ ID NO: 117);
CᵢDLTAHNCᵢᵢQWGICᵢᵢᵢ (SEQ ID NO: 118);
CᵢDLTTHNCᵢᵢAWGICᵢᵢᵢ (SEQ ID NO: 119);
CᵢDLTTHNCᵢᵢQWGACᵢᵢᵢ (SEQ ID NO: 120);
CᵢNLTTHNCᵢᵢQWGICᵢᵢᵢ (SEQ ID NO: 121);
CᵢDLQTHNCᵢᵢQWGICᵢᵢᵢ (SEQ ID NO: 122);
CᵢDLTLHNCᵢᵢQWGICᵢᵢᵢ (SEQ ID NO: 123);
CᵢDLTT[His3Me]NCᵢᵢQWGICᵢᵢᵢ (SEQ ID NO: 124);
CᵢDLTT[2Pal]NCᵢᵢQWGICᵢᵢᵢ (SEQ ID NO: 125);
CᵢDLTT[His1Me]NCᵢᵢQWGICᵢᵢᵢ (SEQ ID NO: 126);
CᵢDLTT[4ThiAz]NCᵢᵢQWGICᵢᵢᵢ (SEQ ID NO: 127);
CᵢDLTTHNCᵢᵢVWGICᵢᵢᵢ (SEQ ID NO: 128);
CᵢDLTTHNCᵢᵢ[Cba]WGICᵢᵢᵢ (SEQ ID NO: 129);
CᵢDLTTHNCᵢᵢQ[1Nal]GICᵢᵢᵢ (SEQ ID NO: 130);
CᵢDLTTHNCᵢᵢQ[Trp(Me)]GICᵢᵢᵢ (SEQ ID NO: 131);
CᵢDLTTHNCᵢᵢQWG[tBuGly]Cᵢᵢᵢ (SEQ ID NO: 132);
CᵢDLTTHNCᵢᵢQWG[tBuAla]Cᵢᵢᵢ (SEQ ID NO: 133);
CᵢDLTTHNCᵢᵢQWG[Chg]Cᵢᵢᵢ (SEQ ID NO: 134);
CᵢDLTTHNCᵢᵢQW[dA]ICᵢᵢᵢ (SEQ ID NO: 135);
CᵢS[Cba]PDYLCᵢᵢ[dA]DEYCᵢᵢᵢ (SEQ ID NO: 136)
Cᵢ[Hser][Cba]PDYLCᵢᵢ[dA]DEYCᵢᵢᵢ (SEQ ID NO: 137);
CᵢN[Cba]PDYLCᵢᵢ[dA]DEYCᵢᵢᵢ (SEQ ID NO: 138);
Cᵢ[4Pal][Cba]PDYLCᵢᵢ[dA]DEYCᵢᵢᵢ (SEQ ID NO: 139);
CᵢY[Cba][Cis-HyP]DYLCᵢᵢ[dA]DEYCᵢᵢᵢ (SEQ ID NO: 140);
CᵢY[Cba][NmeAla]DYLCᵢᵢ[dA]DEYCᵢᵢᵢ (SEQ ID NO: 141);
CᵢY[Cba]PNYLCᵢᵢ[dA]DEYCᵢᵢᵢ (SEQ ID NO: 142);
CᵢY[Cba]PQYLCᵢᵢ[dA]DEYCᵢᵢᵢ (SEQ ID NO: 143);
CᵢY[Cba]PDYLCᵢᵢ[dV]DEYCᵢᵢᵢ (SEQ ID NO: 144);
CᵢY[Cba]PDYLCᵢᵢ[dNva]DEYCᵢᵢᵢ (SEQ ID NO: 145);
CᵢY[Cba]PDYLCᵢᵢ[dF]DEYCᵢᵢᵢ (SEQ ID NO: 146);
CᵢY[Cba]PDYLCᵢᵢ[dCha]DEYCᵢᵢᵢ (SEQ ID NO: 147);
CᵢY[Cba]PDYLCᵢᵢ[dK(PYA)DEYCᵢᵢᵢ (SEQ ID NO: 148);
CᵢY[Cba]PDYLCᵢᵢ[dNle]DEYCᵢᵢᵢ (SEQ ID NO: 149);
CᵢY[Cba]PD[DOPA]LCᵢᵢ[dA]DEYCᵢᵢᵢ (SEQ ID NO: 150);
CᵢY[Cba]PO[2FTyr]LCᵢᵢ[dA]DEYCᵢᵢᵢ (SEQ ID NO: 151);
CᵢY[Cba]PDY[Nle]Cᵢᵢ[dA]DEYCᵢᵢᵢ (SEQ ID NO: 152);
[Pen]ᵢY[Cba]PDYLCᵢᵢ[dA]DEYCᵢᵢᵢ (SEQ ID NO: 153);
CᵢY[Cba]PDYL[Pen]ᵢᵢ[dA]DEYCᵢᵢᵢ (SEQ ID NO: 154);
CᵢY[Cba]PDYLCᵢᵢ[dA]DEY[Pen]ᵢᵢᵢ (SEQ ID NO: 155);
CᵢY[Cba]PDYLCᵢᵢ[dA]DEY[dCᵢᵢᵢ] (SEQ ID NO: 156);
[dCᵢ]Y[Cba]PDYLCᵢᵢ[dA]DEYCᵢᵢᵢ (SEQ ID NO: 157);
CᵢDLTTHNCᵢᵢQ[AzaTrp]GICᵢᵢᵢ (SEQ ID NO: 158);
CᵢDLTTHNCᵢᵢQ[2MeTrp]GICᵢᵢᵢ (SEQ ID NO: 159);
CᵢDLTTHNCᵢᵢQ[6MeTrp]GICᵢᵢᵢ (SEQ ID NO: 160);
CᵢDLTTHNCᵢᵢQ[7MeTrp]GICᵢᵢᵢ (SEQ ID NO: 161);
CᵢDLTTHNCᵢᵢQ[6FTrp]GICᵢᵢᵢ (SEQ ID NO: 162);
CᵢDLTTHNCᵢᵢQ[5FTrp]GICᵢᵢᵢ (SEQ ID NO: 163);
CᵢDLTTHNCᵢᵢQ[Dht]GICᵢᵢᵢ (SEQ ID NO: 164);
CᵢDLTT[2FuAla]NCᵢᵢQWGICᵢᵢᵢ (SEQ ID NO: 165);
CᵢDLTTHNCᵢᵢQWG[EPA]Cᵢᵢᵢ (SEQ ID NO: 166);
CᵢDLTTHNCᵢᵢQWG[Nle]Cᵢᵢᵢ (SEQ ID NO: 167); und
CᵢDLTTHNCᵢᵢQWG[Allolle]Cᵢᵢᵢ (SEQ ID NO: 168).

3. Peptidligand nach Anspruch 2, wobei das molekulare Gerüst TATA ist und der bicyclische Peptidligand zusätzlich N- und/oder C-terminale Additionen umfasst und eine Aminosäuresequenz umfasst, die ausgewählt ist aus:
A-(SEQ ID NO: 1)-A-Sar₆-KFI (hierin bezeichnet als BCY14654);
A-(SEQ ID NO: 1)-A (hierin bezeichnet als BCY14456);
A-(SEQ ID NO: 2)-A-Sar₆-KFI (hierin bezeichnet als BCY15125);
A-(SEQ ID NO: 2)-A (hierin bezeichnet als BCY15102);
A-(SEQ ID NO: 2)-A-[K(PYA)] (hierin bezeichnet als BCY18004);
A-(SEQ ID NO: 3)-A-[K(PYA)] (hierin bezeichnet als BCY15687);
A-(SEQ ID NO: 3)-A (hierin bezeichnet als BCY15098);
A-(SEQ ID NO: 4)-A-Sar₆-KFI (hierin bezeichnet als BCY15115);
A-(SEQ ID NO: 4)-A (hierin bezeichnet als BCY15099);
Ac-(SEQ ID NO: 4) (hierin bezeichnet als BCY18293);
Ac-A-(SEQ ID NO: 4)-A (hierin bezeichnet als BCY18294);
(SEQ ID NO: 4)-A (hierin bezeichnet als BCY19694);
K-(SEQ ID NO: 4)-A (hierin bezeichnet als BCY19695);
dK-(SEQ ID NO: 4)-A (hierin bezeichnet als BCY19696);
Dap-(SEQ ID NO: 4)-A (hierin bezeichnet als BCY19697);
Dab-(SEQ ID NO: 4)-A (hierin bezeichnet als BCY19698);
GABA-(SEQ ID NO: 4)-A (hierin bezeichnet als BCY19699);
A-(SEQ ID NO: 5)-A (hierin bezeichnet als BCY15103);
A-(SEQ ID NO: 5)-A-[K(PYA)] (hierin bezeichnet als BCY18005);
A-(SEQ ID NO: 5)-A-Sar₆-KFI (hierin bezeichnet als BCY15118);
A-(SEQ ID NO: 6)-A (hierin bezeichnet als BCY15104);
A-(SEQ ID NO: 6)-A-Sar₆-KFI (hierin bezeichnet als BCY15119);
A-(SEQ ID NO: 7)-A (hierin bezeichnet als BCY15105);
A-(SEQ ID NO: 7)-A-Sar₆-KFI (hierin bezeichnet als BCY15120);
A-(SEQ ID NO: 8)-A (hierin bezeichnet als BCY15106);
A-(SEQ ID NO: 8)-A-Sar₆-KFI (hierin bezeichnet als BCY15121);
A-(SEQ ID NO: 9)-A-[dK(PYA)] (hierin bezeichnet als BCY15013);
A-(SEQ ID NO: 10)-A (hierin bezeichnet als BCY18470);
A-(SEQ ID NO: 10)-A-[dK(PYA)] (hierin bezeichnet als BCY15452);
A-(SEQ ID NO: 11)-A (hierin bezeichnet als BCY15107);
Ac-(SEQ ID NO: 11) (hierin bezeichnet als BCY18260);
Ac-A-(SEQ ID NO: 11)-A (hierin bezeichnet als BCY18261);
A-(SEQ ID NO: 11)- [Aib] (hierin bezeichnet als BCY19717);
(SEQ ID NO: 11)-A (hierin bezeichnet als BCY19718);
A-(SEQ ID NO: 11)-A-[dK(PYA)] (hierin bezeichnet als BCY19719);
A-(SEQ ID NO: 11)-A-[K(PYA)] (hierin bezeichnet als BCY15686);
A-(SEQ ID NO: 12)-A (hierin bezeichnet als BCY15100);
Ac-(SEQ ID NO: 12) (hierin bezeichnet als BCY15634);
Ac-A-(SEQ ID NO: 12)-A (hierin bezeichnet als BCY15635);
dA-(SEQ ID NO: 12) (hierin bezeichnet als BCY16134;
A-(SEQ ID NO: 12)-A-Sar₆-KFI (hierin bezeichnet als BCY15116);
A-(SEQ ID NO: 13)-A (hierin bezeichnet als BCY14455);
(SEQ ID NO: 13)-Sar₆-KFI (hierin bezeichnet als BCY15023);
Ac-(SEQ ID NO: 13)-Sar₆-KFI (hierin bezeichnet als BCY15024);
A-(SEQ ID NO: 13)-A-Sar₆-KFI (hierin bezeichnet als BCY14652);
A-(SEQ ID NO: 14)-A (hierin bezeichnet als BCY15636);
A-(SEQ ID NO: 15)-A (hierin bezeichnet als BCY15638);
A-(SEQ ID NO: 16)-A (hierin bezeichnet als BCY15639);
A-(SEQ ID NO: 17)-A (hierin bezeichnet als BCY15640);
A-(SEQ ID NO: 18)-A (hierin bezeichnet als BCY15641);
A-(SEQ ID NO: 19)-A (hierin bezeichnet als BCY15642);
A-(SEQ ID NO: 20)-A (hierin bezeichnet als BCY15643);
A-(SEQ ID NO: 21)-A (hierin bezeichnet als BCY15644);
A-(SEQ ID NO: 22)-A (hierin bezeichnet als BCY15645);
A-(SEQ ID NO: 23)-A (hierin bezeichnet als BCY15646);
A-(SEQ ID NO: 24)-A (hierin bezeichnet als BCY15648);
A-(SEQ ID NO: 25)-A (hierin bezeichnet als BCY15649);
A-(SEQ ID NO: 26)-A (hierin bezeichnet als BCY15650);
A-(SEQ ID NO: 27)-A (hierin bezeichnet als BCY15651);
A-(SEQ ID NO: 28)-A (hierin bezeichnet als BCY15653);
A-(SEQ ID NO: 29)-A (hierin bezeichnet als BCY15654);
A-(SEQ ID NO: 30)-A (hierin bezeichnet als BCY15655);
A-(SEQ ID NO: 31)-A (hierin bezeichnet als BCY15656);
A-(SEQ ID NO: 32)-A (hierin bezeichnet als BCY15657);
A-(SEQ ID NO: 33)-A (hierin bezeichnet als BCY15658);
A-(SEQ ID NO: 34)-A (hierin bezeichnet als BCY15659);
A-(SEQ ID NO: 35)-A (hierin bezeichnet als BCY15660);
A-(SEQ ID NO: 36)-A (hierin bezeichnet als BCY15661);
A-(SEQ ID NO: 37)-A (hierin bezeichnet als BCY15662);
A-(SEQ ID NO: 38)-A (hierin bezeichnet als BCY16130);
A-(SEQ ID NO: 39)-A (hierin bezeichnet als BCY16131);
A-(SEQ ID NO: 40)-A (hierin bezeichnet als BCY16132);
Ac-(SEQ ID NO: 40) (hierin bezeichnet als BCY16133);
Ac-(SEQ ID NO: 40)-[K(PYA)] (hierin bezeichnet als BCY17224);
A-(SEQ ID NO: 41)-A (hierin bezeichnet als BCY16135);
A-(SEQ ID NO: 42)-A (hierin bezeichnet als BCY16136);
A-(SEQ ID NO: 43)-A (hierin bezeichnet als BCY16137);
A-(SEQ ID NO: 44)-A (hierin bezeichnet als BCY16138);
A-(SEQ ID NO: 45)-A (hierin bezeichnet als BCY16139);
A-(SEQ ID NO: 46)-A (hierin bezeichnet als BCY16140);
A-(SEQ ID NO: 47)-A (hierin bezeichnet als BCY16141);
A-(SEQ ID NO: 48)-A (hierin bezeichnet als BCY16142);
A-(SEQ ID NO: 49)-A (hierin bezeichnet als BCY16143);
A-(SEQ ID NO: 50)-A (hierin bezeichnet als BCY16144);
A-(SEQ ID NO: 51)-A (hierin bezeichnet als BCY16145);
A-(SEQ ID NO: 52)-A-Sar₆-KFI (hierin bezeichnet als BCY15025);
A-(SEQ ID NO: 53)-A-Sar₆-KFI (hierin bezeichnet als BCY15028);
A-(SEQ ID NO: 54)-A-Sar₆-KFI (hierin bezeichnet als BCY15030);
A-(SEQ ID NO: 55)-A-Sar₆-KFI (hierin bezeichnet als BCY15035);
A-(SEQ ID NO: 56)-A-Sar₆-KFI (hierin bezeichnet als BCY15117);
A-(SEQ ID NO: 57)-A-Sar₆-KFI (hierin bezeichnet als BCY15122);
A-(SEQ ID NO: 58)-A (hierin bezeichnet als BCY15108);
A-(SEQ ID NO: 58)-A-Sar₆-KFI (hierin bezeichnet als BCY15123);
A-(SEQ ID NO: 59)-A-Sar₆-KFI (hierin bezeichnet als BCY15124);
A-(SEQ ID NO: 63)-A (hierin bezeichnet als BCY14454);
A-(SEQ ID NO: 64)-A (hierin bezeichnet als BCY18299);
A-(SEQ ID NO: 65)-A (hierin bezeichnet als BCY18301);
A-(SEQ ID NO: 66)-A (hierin bezeichnet als BCY18302);
A-(SEQ ID NO: 67)-A (hierin bezeichnet als BCY18303);
A-(SEQ ID NO: 68)-A (hierin bezeichnet als BCY18304);
A-(SEQ ID NO: 69)-A (hierin bezeichnet als BCY18305);
A-(SEQ ID NO: 70)-A (hierin bezeichnet als BCY18307);
A-(SEQ ID NO: 71)-A (hierin bezeichnet als BCY18309);
A-(SEQ ID NO: 72)-A (hierin bezeichnet als BCY18310);
A-(SEQ ID NO: 73)-A (hierin bezeichnet als BCY18311);
A-(SEQ ID NO: 74)-A (hierin bezeichnet als BCY18312);
A-(SEQ ID NO: 75)-A (hierin bezeichnet als BCY18313);
A-(SEQ ID NO: 76)-A (hierin bezeichnet als BCY18314);
A-(SEQ ID NO: 77)-A (hierin bezeichnet als BCY18315);
A-(SEQ ID NO: 78)-A (hierin bezeichnet als BCY18316);
A-(SEQ ID NO: 79)-A (hierin bezeichnet als BCY18317);
A-(SEQ ID NO: 80)-A (hierin bezeichnet als BCY18318);
A-(SEQ ID NO: 81)-A (hierin bezeichnet als BCY18319);
A-(SEQ ID NO: 82)-A (hierin bezeichnet als BCY18320);
A-(SEQ ID NO: 83)-A (hierin bezeichnet als BCY18321);
A-(SEQ ID NO: 84)-A (hierin bezeichnet als BCY18322);
A-(SEQ ID NO: 85)-A (hierin bezeichnet als BCY18323);
A-(SEQ ID NO: 86)-A (hierin bezeichnet als BCY18324);
A-(SEQ ID NO: 87)-A (hierin bezeichnet als BCY18325);
A-(SEQ ID NO: 88)-A (hierin bezeichnet als BCY18326);
A-(SEQ ID NO: 89)-A (hierin bezeichnet als BCY18327);
A-(SEQ ID NO: 90)-A (hierin bezeichnet als BCY18328);
A-(SEQ ID NO: 91)-A (hierin bezeichnet als BCY19700);
A-(SEQ ID NO: 92)-A (hierin bezeichnet als BCY19701);
A-(SEQ ID NO: 93)-A (hierin bezeichnet als BCY19702);
A-(SEQ ID NO: 94)-A (hierin bezeichnet als BCY19703);
A-(SEQ ID NO: 95)-A (hierin bezeichnet als BCY19704);
A-(SEQ ID NO: 96)-A (hierin bezeichnet als BCY19706);
A-(SEQ ID NO: 97)-A (hierin bezeichnet als BCY19707);
A-(SEQ ID NO: 98)-A (hierin bezeichnet als BCY19708);
A-(SEQ ID NO: 99)-A (hierin bezeichnet als BCY19710);
A-(SEQ ID NO: 100)-A (hierin bezeichnet als BCY19711);
A-(SEQ ID NO: 101)-A (hierin bezeichnet als BCY19712);
A-(SEQ ID NO: 102)-A (hierin bezeichnet als BCY19713);
A-(SEQ ID NO: 103)-A (hierin bezeichnet als BCY14457);
A-(SEQ ID NO: 104)-A (hierin bezeichnet als BCY14458);
A-(SEQ ID NO: 105)-A (hierin bezeichnet als BCY15109);
A-(SEQ ID NO: 106)-A (hierin bezeichnet als BCY15568);
A-(SEQ ID NO: 107)-A (hierin bezeichnet als BCY15637);
A-(SEQ ID NO: 108)-A (hierin bezeichnet als BCY15647);
A-(SEQ ID NO: 109)-A (hierin bezeichnet als BCY15652);
A-(SEQ ID NO: 110)-A (hierin bezeichnet als BCY16834);
A-(SEQ ID NO: 111)-A (hierin bezeichnet als BCY16837);
A-(SEQ ID NO: 112)-A (hierin bezeichnet als BCY16840);
A-(SEQ ID NO: 113)-A (hierin bezeichnet als BCY16842);
A-(SEQ ID NO: 114)-A (hierin bezeichnet als BCY16843);
A-(SEQ ID NO: 115)-A (hierin bezeichnet als BCY17662);
A-(SEQ ID NO: 116)-A (hierin bezeichnet als BCY18263);
A-(SEQ ID NO: 117)-A (hierin bezeichnet als BCY18265);
A-(SEQ ID NO: 118)-A (hierin bezeichnet als BCY18266);
A-(SEQ ID NO: 119)-A (hierin bezeichnet als BCY18269);
A-(SEQ ID NO: 120)-A (hierin bezeichnet als BCY18272);
A-(SEQ ID NO: 121)-A (hierin bezeichnet als BCY18273);
A-(SEQ ID NO: 122)-A (hierin bezeichnet als BCY18277);
A-(SEQ ID NO: 123)-A (hierin bezeichnet als BCY18278);
A-(SEQ ID NO: 124)-A (hierin bezeichnet als BCY18279);
A-(SEQ ID NO: 125)-A (hierin bezeichnet als BCY18280);
A-(SEQ ID NO: 126)-A (hierin bezeichnet als BCY18281);
A-(SEQ ID NO: 127)-A (hierin bezeichnet als BCY18282);
A-(SEQ ID NO: 128)-A (hierin bezeichnet als BCY18285);
A-(SEQ ID NO: 129)-A (hierin bezeichnet als BCY18286);
A-(SEQ ID NO: 130)-A (hierin bezeichnet als BCY18287);
A-(SEQ ID NO: 131)-A (hierin bezeichnet als BCY18288);
A-(SEQ ID NO: 132)-A (hierin bezeichnet als BCY18289);
A-(SEQ ID NO: 133)-A (hierin bezeichnet als BCY18290);
A-(SEQ ID NO: 134)-A (hierin bezeichnet als BCY18291);
A-(SEQ ID NO: 135)-A (hierin bezeichnet als BCY18292);
Ac-(SEQ ID NO: 136) (hierin bezeichnet als BCY18433);
Ac-(SEQ ID NO: 137) (hierin bezeichnet als BCY18434);
Ac-(SEQ ID NO: 138) (hierin bezeichnet als BCY18435);
Ac-(SEQ ID NO: 139) (hierin bezeichnet als BCY18437);
Ac-(SEQ ID NO: 140) (hierin bezeichnet als BCY18439);
Ac-(SEQ ID NO: 141) (hierin bezeichnet als BCY18440);
Ac-(SEQ ID NO: 142) (hierin bezeichnet als BCY18441);
Ac-(SEQ ID NO: 143) (hierin bezeichnet als BCY18442);
Ac-(SEQ ID NO: 144) (hierin bezeichnet als BCY18443);
Ac-(SEQ ID NO: 145) (hierin bezeichnet als BCY18444);
Ac-(SEQ ID NO: 146) (hierin bezeichnet als BCY19682);
Ac-(SEQ ID NO: 147) (hierin bezeichnet als BCY19683);
Ac-(SEQ ID NO: 147) (hierin bezeichnet als BCY19683);
Ac-(SEQ ID NO: 148) (hierin bezeichnet als BCY19684);
Ac-(SEQ ID NO: 149) (hierin bezeichnet als BCY19685);
Ac-(SEQ ID NO: 150) (hierin bezeichnet als BCY19686);
Ac-(SEQ ID NO: 151) (hierin bezeichnet als BCY19687);
Ac-(SEQ ID NO: 152) (hierin bezeichnet als BCY19688);
Ac-(SEQ ID NO: 153) (hierin bezeichnet als BCY19689);
Ac-(SEQ ID NO: 154) (hierin bezeichnet als BCY19690);
Ac-(SEQ ID NO: 155) (hierin bezeichnet als BCY19691);
Ac-(SEQ ID NO: 156) (hierin bezeichnet als BCY19692);
Ac-(SEQ ID NO: 157) (hierin bezeichnet als BCY19693);
A-(SEQ ID NO: 158)-A (hierin bezeichnet als BCY19720);
A-(SEQ ID NO: 159)-A (hierin bezeichnet als BCY19721);
A-(SEQ ID NO: 160)-A (hierin bezeichnet als BCY19722);
A-(SEQ ID NO: 161)-A (hierin bezeichnet als BCY19723);
A-(SEQ ID NO: 162)-A (hierin bezeichnet als BCY19724);
A-(SEQ ID NO: 163)-A (hierin bezeichnet als BCY19725);
A-(SEQ ID NO: 164)-A (hierin bezeichnet als BCY19730);
A-(SEQ ID NO: 165)-A (hierin bezeichnet als BCY19732);
A-(SEQ ID NO: 166)-A (hierin bezeichnet als BCY19733);
A-(SEQ ID NO: 167)-A (hierin bezeichnet als BCY19734); und
A-(SEQ ID NO: 168)-A (hierin bezeichnet als BCY19735);
wobei PYA Pentinsäure darstellt, Sar Sarcosin darstellt und Fl Fluorescein darstellt, Aib Aminoisobuttersäure darstellt, dK D-Lysin darstellt, Dap L-2,3-Diaminopropionsäure darstellt, Dab L-2,4-Diaminobuttersäure darstellt und GABA γ-Aminobuttersäure darstellt.

4. Peptidligand nach Anspruch 1, wobei das bicyclische Peptid spezifisch für FcyRIIIA (auch bekannt als CD16a) ist und wobei der bicyclische Peptidligand eine Aminosäuresequenz umfasst, die Folgendes ist:
CᵢPPTVYCᵢᵢHPLDCᵢᵢᵢ (SEQ ID NO: 60);
CᵢRWSVEDPCᵢᵢGAWCᵢᵢᵢ (SEQ ID NO: 61);
CᵢVGLEELGPCᵢᵢSDLCᵢᵢᵢ (SEQ ID NO: 62; hierin bezeichnet als BCY16494, wenn komplexiert mit TBMT);
CᵢPPAVYCᵢᵢHPLDCᵢᵢᵢ (SEQ ID NO: 169);
CᵢPPEVFCᵢᵢHPLDCᵢᵢᵢ (SEQ ID NO: 170);
CᵢPPEVYCᵢᵢHPLDCᵢᵢᵢ (SEQ ID NO: 171);
CᵢPPEVWCᵢᵢHPLDCᵢᵢᵢ (SEQ ID NO: 172);
CᵢPPQVYCᵢᵢHPLDCᵢᵢᵢ (SEQ ID NO: 173);
CᵢPPEVWCᵢᵢHPLDCᵢᵢᵢ (SEQ ID NO: 174);
CᵢRWS[tBuGly]EDPCᵢᵢGAWCᵢᵢᵢ (SEQ ID NO: 175);
CᵢRWSVED[HyP]CᵢᵢGAWCᵢᵢᵢ (SEQ ID NO: 176);
CᵢRWSVED[Aze]CᵢᵢGAWCᵢᵢᵢ (SEQ ID NO: 177);
CᵢRWSVEDPCᵢᵢ[dA]AWCᵢᵢᵢ (SEQ ID NO: 178);
CᵢRWSVE[Gla]PCᵢᵢGAWCᵢᵢᵢ (SEQ ID NO: 179);
CᵢRWSVED[Aze]CᵢᵢGAWCᵢᵢᵢ (SEQ ID NO: 180);
CᵢMWIRSDPCᵢᵢGAWCᵢᵢᵢ (SEQ ID NO: 181);
CᵢRWYPDDPCᵢᵢGAWGᵢᵢᵢ (SEQ ID NO: 182);
CᵢEWHISEPCᵢᵢGAWCᵢᵢᵢ (SEQ ID NO: 183);
CᵢNWHVYEPCᵢᵢGAWCᵢᵢᵢ (SEQ ID NO: 184);
CᵢRWHFSEPCᵢᵢGAWCᵢᵢᵢ (SEQ ID NO: 185);
CᵢRWNLDDPCᵢᵢGAWCᵢᵢᵢ (SEQ ID NO: 186);
CᵢMWIRSDPCᵢᵢGAWCᵢᵢᵢ (SEQ ID NO: 187);
CᵢRWYPDDPCᵢᵢGAWCᵢᵢᵢ (SEQ ID NO: 188);
CᵢEWHISEPCᵢᵢGAWCᵢᵢᵢ (SEQ ID NO: 189);
CᵢNWHVYEPCᵢᵢGAWCᵢᵢᵢ (SEQ ID NO: 190);
CᵢRWHFSEPCᵢᵢGAWCᵢᵢᵢ (SEQ ID NO: 191);
CᵢRWNLDDPCᵢᵢGAWCᵢᵢᵢ (SEQ ID NO: 192);
CᵢR[2MeTrp]SVEDPCᵢᵢGAWCᵢᵢᵢ (SEQ ID NO: 193);
CᵢR[6MeTrp]SVEDPCᵢᵢGAWCᵢᵢᵢ (SEQ ID NO: 194);
CᵢR[6FTrp]SVEDPCᵢᵢGAWCᵢᵢᵢ (SEQ ID NO: 195);
CᵢR[5FTrp]SVEDPCᵢᵢGAWCᵢᵢᵢ (SEQ ID NO: 196);
CᵢR[6ClTrp]SVEDPCᵢᵢGAWCᵢᵢᵢ (SEQ ID NO: 197);
CᵢR[Trp(S)]SVEDPCᵢᵢGAWCᵢᵢᵢ (SEQ ID NO: 198);
CᵢR[AzaTrp]SVEDPCᵢᵢGAWCᵢᵢᵢ (SEQ ID NO: 199);
CᵢRWSVEDPCᵢᵢGA[2MeTrp]Cᵢᵢᵢ (SEQ ID NO: 200);
CᵢRWSVEDPCᵢᵢGA[6FTrp]Cᵢᵢᵢ (SEQ ID NO: 201);
CᵢRWSVEDPCᵢᵢGA[5FTrp]Cᵢᵢᵢ (SEQ ID NO: 202);
CᵢRWSVEDPCᵢᵢGA[4MeoTrp]Cᵢᵢᵢ (SEQ ID NO: 203);
CᵢRWSVEDPCᵢᵢGA[5MeoTrp]Cᵢᵢᵢ (SEQ ID NO: 204);
CᵢRWSVEDPCᵢᵢGA[Trp(S)]Cᵢᵢᵢ (SEQ ID NO: 205);
CᵢRWSVEDPCᵢᵢGA[DOPA]Cᵢᵢᵢ (SEQ ID NO: 206);
CᵢRWYPDDPCᵢᵢGAWCᵢᵢᵢ (SEQ ID NO: 207);
CᵢRWHFSEPCᵢᵢGAWCᵢᵢᵢ (SEQ ID NO: 208);
CᵢR[2MeTrp]HFSEPCᵢᵢGAWCᵢᵢᵢ (SEQ ID NO: 209);
CᵢRWYFSEPCᵢᵢGAWCᵢᵢᵢ (SEQ ID NO: 210);
CᵢRWSFSEPCᵢᵢGAWCᵢᵢᵢ (SEQ ID NO: 211);
CᵢRWHPSEPCᵢᵢGAWCᵢᵢᵢ (SEQ ID NO: 212);
CᵢRWYPSEPCᵢᵢGAWCᵢᵢᵢ (SEQ ID NO: 213);
CᵢRWHPDEPCᵢᵢGAWCᵢᵢᵢ (SEQ ID NO: 214);
CᵢRWHPEEPCᵢᵢGAWCᵢᵢᵢ (SEQ ID NO: 215);
CᵢRWHFDEPCᵢᵢGAWCᵢᵢᵢ (SEQ ID NO: 216);
CᵢRWHFDDPCᵢᵢGAWCᵢᵢᵢ (SEQ ID NO: 217);
CᵢRWYFDEPCᵢᵢGAWCᵢᵢᵢ (SEQ ID NO: 218);
CᵢRWHis1Me)FSEPCᵢᵢGAWCᵢᵢᵢ (SEQ ID NO: 219);
CᵢRW[His3Me]FSEPCᵢᵢGAWCᵢᵢᵢ (SEQ ID NO: 220);
CᵢRW[4MeoPhe]FSEPCᵢᵢGAWCᵢᵢᵢ (SEQ ID NO: 221);
CᵢRW[DOPA]FSEPCᵢᵢGAWCᵢᵢᵢ (SEQ ID NO: 222);
CᵢRW[26DiMeTyr]FSEPCᵢᵢGAWCᵢᵢᵢ (SEQ ID NO: 223);
CᵢRW[3tBuTyr]FSEPCᵢᵢGAWCᵢᵢᵢ (SEQ ID NO: 224);
CᵢRW[4FPhe]FSEPCᵢᵢGAWCᵢᵢᵢ (SEQ ID NO: 225);
CᵢRWH[4MePhe]SEPCᵢᵢGAWCᵢᵢᵢ (SEQ ID NO: 226);
CᵢRWH[Aze]SEPCᵢᵢGAWCᵢᵢᵢ (SEQ ID NO: 227);
CᵢRWH[HyP]SEPCᵢᵢGAWCᵢᵢᵢ (SEQ ID NO: 228);
CᵢRWH[4tBuPhe]SEPCᵢᵢGAWCᵢᵢᵢ (SEQ ID NO: 229);
CᵢRWH[HPhe]SEPCᵢᵢGAWCᵢᵢᵢ (SEQ ID NO: 230, hierin bezeichnet als BCY21693, wenn komplexiert mit TBMT);
CᵢRWH[4PhePro]SEPCᵢᵢGAWCᵢᵢᵢ (SEQ ID NO: 231);
CᵢRWHF[HSer]EPCᵢᵢGAWCᵢᵢᵢ (SEQ ID NO: 232);
CᵢRWHFS[Gla]PCᵢᵢGAWCᵢᵢᵢ (SEQ ID NO: 233);
CᵢRWHFS[HGlu]PCᵢᵢGAWCᵢᵢᵢ (SEQ ID NO: 234);
CᵢRWHFSEPCᵢᵢ[dA]AWCᵢᵢᵢ (SEQ ID NO: 235);
CᵢRWYFSE[Aze]Cᵢᵢ[dA]AWCᵢᵢᵢ (SEQ ID NO: 236);
CᵢRWHFDE[Aze]Cᵢᵢ[dA]AWCᵢᵢᵢ (SEQ ID NO: 237);
CᵢRWYPSE[Aze]Cᵢᵢ[dA]AWCᵢᵢᵢ (SEQ ID NO: 238);
Cᵢ[HArg]WHFSEPCᵢᵢGAWCᵢᵢᵢ (SEQ ID NO: 239);
CᵢRWH[Cis-HyP]SEPCᵢᵢGAWCᵢᵢᵢ (SEQ ID NO: 240);
CᵢRWHFSEPCᵢᵢGAWCᵢᵢᵢ (SEQ ID NO: 241);
CᵢRWHFSEPCᵢᵢGAWCᵢᵢᵢ (SEQ ID NO: 242);
CᵢRWHFSEPCᵢᵢGAWCᵢᵢᵢ (SEQ ID NO: 243);
CᵢVGLLELGPCᵢᵢSDLCᵢᵢᵢ (SEQ ID NO: 244);
CᵢPGLEELGPCᵢᵢSDLCᵢᵢᵢ (SEQ ID NO: 245);
CᵢV[dA]LEELGPCᵢᵢSDLCᵢᵢᵢ (SEQ ID NO: 246);
CᵢVG[tBuAla]EELGPCᵢᵢSDLCᵢᵢᵢ (SEQ ID NO: 247);
CᵢVG[Cba]EELGPCᵢᵢSDLCᵢᵢᵢ (SEQ ID NO: 248);
CᵢVGFEELGPCᵢᵢSDLCᵢᵢᵢ (SEQ ID NO: 249);
CᵢVG[1Nal]EELGPCᵢᵢSDLCᵢᵢᵢ (SEQ ID NO: 250);
CᵢVG[2Nal]EELGPCᵢᵢSDLCᵢᵢᵢ (SEQ ID NO: 251);
CᵢVGLPELGPCᵢᵢSDLCᵢᵢᵢ (SEQ ID NO: 252);
CᵢVGL[tBuAla]ELGPCᵢᵢSDLCᵢᵢᵢ (SEQ ID NO: 253);
CᵢVGL[Cba]ELGPCᵢᵢSDLCᵢᵢᵢ (SEQ ID NO: 254);
CᵢVGL[Cha]ELGPCᵢᵢSDLCᵢᵢᵢ (SEQ ID NO: 255);
CᵢVGL[Nle]ELGPCᵢᵢSDLCᵢᵢᵢ (SEQ ID NO: 256);
CᵢVGLE[Gla]LGPCᵢᵢSDLCᵢᵢᵢ (SEQ ID NO: 257);
CᵢVGLEELG[Aze]CᵢᵢSDLCᵢᵢᵢ (SEQ ID NO: 258);
CᵢVGLEELGPCᵢᵢSD[tBuAla]Cᵢᵢᵢ (SEQ ID NO: 259);
CᵢVGLEELGPCᵢᵢSD[Cba]Cᵢᵢᵢ (SEQ ID NO: 260);
CᵢVGLEELGPCᵢᵢSD[Cha]Cᵢᵢᵢ (SEQ ID NO: 261);
CᵢVG[HLeu]EELGPCᵢᵢSDLCᵢᵢᵢ (SEQ ID NO: 262);
CᵢVGL[HLeu]ELGPCᵢᵢSDLCᵢᵢᵢ (SEQ ID NO: 263);
CᵢVGLE[HGlu]LGPCᵢᵢSDLCᵢᵢᵢ (SEQ ID NO: 264);
CᵢTWPYCᵢᵢKSWGDVQDCᵢᵢᵢ (SEQ ID NO: 265);
CᵢDWWDPGCᵢᵢTYFCᵢᵢᵢ (SEQ ID NO: 266);
CᵢRWH[4BnPro]SEPCᵢᵢGAWCᵢᵢᵢ (SEQ ID NO: 267); und
CᵢRWH[HPhe]SE[Aze]CᵢᵢGAWCᵢᵢᵢ (SEQ ID NO: 268).

5. Peptidligand nach Anspruch 4, wobei das molekulare Gerüst TATB ist und der bicyclische Peptidligand zusätzlich N- und/oder C-terminale Additionen umfasst und eine Aminosäuresequenz umfasst, die ausgewählt ist aus:
A-(SEQ ID NO: 265)-A (herein referred to as BCY15044);
A-(SEQ ID NO: 265)-AGAAAE (herein referred to as BCY19416).
A-(SEQ ID NO: 266)-A (herein referred to as BCY15045); und
A-(SEQ ID NO: 266)-AGAAAE (herein referred to as BCY19417).

6. Peptidligand nach Anspruch 4, wobei das molekulare Gerüst TATA ist und der bicyclische Peptidligand zusätzlich N- und/oder C-terminale Additionen umfasst und eine Aminosäuresequenz umfasst, die ausgewählt ist aus:
A-(SEQ ID NO: 60)-A (hierin bezeichnet als BCY11808);
A-(SEQ ID NO: 60)-A-[K(PYA)] (hierin bezeichnet als BCY15685);
A-(SEQ ID NO: 60)-A-[Sar6]-[KBiot] (hierin bezeichnet als BCY12621);
A-(SEQ ID NO: 169)-A (hierin bezeichnet als BCY11673);
A-(SEQ ID NO: 170)-A (hierin bezeichnet als BCY11809);
A-(SEQ ID NO: 171)-A (hierin bezeichnet als BCY11810);
A-(SEQ ID NO: 172)-A (hierin bezeichnet als BCY11811)
A-(SEQ ID NO: 173)-A (hierin bezeichnet als BCY11812); und
A-(SEQ ID NO: 174)-A-[Sar6]-[KBiot] (hierin bezeichnet als BCY12622);
wobei Sar Sarcosin und Biot für Biotin darstellt.

7. Peptidligand nach Anspruch 4, wobei das molekulare Gerüst TBMT ist und der bicyclische Peptidligand zusätzlich N- und/oder C-terminale Additionen umfasst und eine Aminosäuresequenz umfasst, die ausgewählt ist aus:
A-(SEQ ID NO: 61)-A (hierin bezeichnet als BCY14950);
Ac-A-(SEQ ID NO: 61)-A (hierin bezeichnet als BCY16599);
A-(SEQ ID NO: 61)-AGAAAE (hierin bezeichnet als BCY19040);
A-(SEQ ID NO: 61)-A-[K(PYA)] (hierin bezeichnet als BCY13883);
Ac-A-(SEQ ID NO: 61)-A-[K(PYA)] (hierin bezeichnet als BCY19596);
A-(SEQ ID NO: 62)-A (hierin bezeichnet als BCY16493);
Ac-(SEQ ID NO: 62) (hierin bezeichnet als BCY16495);
A-(SEQ ID NO: 62)-A-[K(PYA)] (hierin bezeichnet als BCY13886);
A-(SEQ ID NO: 175)-A (hierin bezeichnet als BCY14953);
A-(SEQ ID NO: 176)-A (hierin bezeichnet als BCY14954);
[PYA]-A-(SEQ ID NO: 177) (hierin bezeichnet als BCY14955);
A-(SEQ ID NO: 178)-A (hierin bezeichnet als BCY14956);
A-(SEQ ID NO: 179)-A (hierin bezeichnet als BCY16608);
A-(SEQ ID NO: 180)-A (hierin bezeichnet als BCY16610);
A-(SEQ ID NO: 181)-A (hierin bezeichnet als BCY19032);
A-(SEQ ID NO: 182)-A (hierin bezeichnet als BCY19033);
A-(SEQ ID NO: 183)-A (hierin bezeichnet als BCY19034);
A-(SEQ ID NO: 184)-A (hierin bezeichnet als BCY19035);
A-(SEQ ID NO: 185)-A (hierin bezeichnet als BCY19036);
A-(SEQ ID NO: 186)-A (hierin bezeichnet als BCY19037);
A-(SEQ ID NO: 187)-AGAAAE (hierin bezeichnet als BCY19038);
A-(SEQ ID NO: 188)-AGAAAE (hierin bezeichnet als BCY19039);
A-(SEQ ID NO: 189)-AGAAAE (hierin bezeichnet als BCY19041);
A-(SEQ ID NO: 190)-AGAAAE (hierin bezeichnet als BCY19042);
A-(SEQ ID NO: 191)-AGAAAE (hierin bezeichnet als BCY19043);
A-(SEQ ID NO: 192)-AGAAAE (hierin bezeichnet als BCY19044);
A-(SEQ ID NO: 193)-A (hierin bezeichnet als BCY19045);
A-(SEQ ID NO: 194)-A (hierin bezeichnet als BCY19046);
A-(SEQ ID NO: 195)-A (hierin bezeichnet als BCY19048);
A-(SEQ ID NO: 196)-A (hierin bezeichnet als BCY19049);
A-(SEQ ID NO: 197)-A (hierin bezeichnet als BCY19050);
A-(SEQ ID NO: 198)-A (hierin bezeichnet als BCY19053);
A-(SEQ ID NO: 199)-A (hierin bezeichnet als BCY19055);
A-(SEQ ID NO: 200)-A (hierin bezeichnet als BCY19057);
A-(SEQ ID NO: 201)-A (hierin bezeichnet als BCY19060);
A-(SEQ ID NO: 202)-A (hierin bezeichnet als BCY19061);
A-(SEQ ID NO: 203)-A (hierin bezeichnet als BCY19063);
A-(SEQ ID NO: 204)-A (hierin bezeichnet als BCY19064);
A-(SEQ ID NO: 205)-A (hierin bezeichnet als BCY19065);
A-(SEQ ID NO: 206)-A (hierin bezeichnet als BCY19068);
A-(SEQ ID NO: 207)-A-[K(PYA)] (hierin bezeichnet als BCY20360);
A-(SEQ ID NO: 208)-A-[K(PYA)] (hierin bezeichnet als BCY20361);
A-(SEQ ID NO: 209)-A (hierin bezeichnet als BCY20363);
A-(SEQ ID NO: 210)-A (hierin bezeichnet als BCY20364);
A-(SEQ ID NO: 211)-A (hierin bezeichnet als BCY20365);
A-(SEQ ID NO: 212)-A (hierin bezeichnet als BCY20366);
A-(SEQ ID NO: 213)-A (hierin bezeichnet als BCY20367);
A-(SEQ ID NO: 214)-A (hierin bezeichnet als BCY20368);
A-(SEQ ID NO: 215)-A (hierin bezeichnet als BCY20369);
A-(SEQ ID NO: 216)-A (hierin bezeichnet als BCY20370);
A-(SEQ ID NO: 217)-A (hierin bezeichnet als BCY20371);
A-(SEQ ID NO: 218)-A (hierin bezeichnet als BCY20372);
A-(SEQ ID NO: 219)-A (hierin bezeichnet als BCY20373);
A-(SEQ ID NO: 220)-A (hierin bezeichnet als BCY20374);
A-(SEQ ID NO: 221)-A (hierin bezeichnet als BCY20375);
A-(SEQ ID NO: 222)-A (hierin bezeichnet als BCY20376);
A-(SEQ ID NO: 223)-A (hierin bezeichnet als BCY20377);
A-(SEQ ID NO: 224)-A (hierin bezeichnet als BCY20378);
A-(SEQ ID NO: 225)-A (hierin bezeichnet als BCY20379);
A-(SEQ ID NO: 226)-A (hierin bezeichnet als BCY20380);
A-(SEQ ID NO: 227)-A (hierin bezeichnet als BCY20382);
A-(SEQ ID NO: 228)-A (hierin bezeichnet als BCY20383);
A-(SEQ ID NO: 229)-A (hierin bezeichnet als BCY20384);
A-(SEQ ID NO: 230)-A (hierin bezeichnet als BCY20385);
(SEQ ID NO: 230)-A (hierin bezeichnet als BCY21690);
Ac-(SEQ ID NO: 230)-A (hierin bezeichnet als BCY21691);
A-(SEQ ID NO: 230) (hierin bezeichnet als BCY21692);
Ac-(SEQ ID NO: 230) (hierin bezeichnet als BCY21694);
[Nle]-(SEQ ID NO: 230)-A (hierin bezeichnet als BCY21695);
Ac-[Nle]-(SEQ ID NO: 230)-A (hierin bezeichnet als BCY21696);
Ac-A-(SEQ ID NO: 230)-A (hierin bezeichnet als BCY21697);
A-(SEQ ID NO: 231)-A (hierin bezeichnet als BCY20386);
A-(SEQ ID NO: 232)-A (hierin bezeichnet als BCY20388);
A-(SEQ ID NO: 233)-A (hierin bezeichnet als BCY20389);
A-(SEQ ID NO: 234)-A (hierin bezeichnet als BCY20390);
A-(SEQ ID NO: 235)-A (hierin bezeichnet als BCY20392);
A-(SEQ ID NO: 236)-A (hierin bezeichnet als BCY20394);
A-(SEQ ID NO: 237)-A (hierin bezeichnet als BCY20395);
A-(SEQ ID NO: 238)-A (hierin bezeichnet als BCY20396);
A-(SEQ ID NO: 239)-A (hierin bezeichnet als BCY20397);
A-(SEQ ID NO: 240)-A (hierin bezeichnet als BCY20526);
Ac-A-(SEQ ID NO: 241)-A (hierin bezeichnet als BCY20527);
[Aib]-(SEQ ID NO: 242)-A (hierin bezeichnet als BCY20528);
A-(SEQ ID NO: 243)-[Aib] (hierin bezeichnet als BCY20529);
A-(SEQ ID NO: 244)-A-[K(PYA)] (hierin bezeichnet als BCY16165);
A-(SEQ ID NO: 245)-A (hierin bezeichnet als BCY16498);
A-(SEQ ID NO: 246)-A (hierin bezeichnet als BCY16499);
A-(SEQ ID NO: 247)-A (hierin bezeichnet als BCY16501);
A-(SEQ ID NO: 248)-A (hierin bezeichnet als BCY16502);
A-(SEQ ID NO: 249)-A (hierin bezeichnet als BCY16504);
A-(SEQ ID NO: 250)-A (hierin bezeichnet als BCY16505);
A-(SEQ ID NO: 251)-A (hierin bezeichnet als BCY16506);
A-(SEQ ID NO: 252)-A (hierin bezeichnet als BCY16507);
A-(SEQ ID NO: 253)-A (hierin bezeichnet als BCY16508);
A-(SEQ ID NO: 254)-A (hierin bezeichnet als BCY16509);
A-(SEQ ID NO: 255)-A (hierin bezeichnet als BCY16510);
A-(SEQ ID NO: 256)-A (hierin bezeichnet als BCY16511);
A-(SEQ ID NO: 257)-A (hierin bezeichnet als BCY16512);
A-(SEQ ID NO: 258)-A (hierin bezeichnet als BCY16520);
A-(SEQ ID NO: 259)-A (hierin bezeichnet als BCY16522);
A-(SEQ ID NO: 260)-A (hierin bezeichnet als BCY16523);
A-(SEQ ID NO: 261)-A (hierin bezeichnet als BCY16524);
A-(SEQ ID NO: 262)-A (hierin bezeichnet als BCY16527);
A-(SEQ ID NO: 263)-A (hierin bezeichnet als BCY16528);
A-(SEQ ID NO: 264)-A (hierin bezeichnet als BCY16529),
A-(SEQ ID NO: 267)-A (hierin bezeichnet als BCY20387); und
A-(SEQ ID NO: 268)-A (hierin bezeichnet als BCY21689),
wobei PYA Pentinsäure darstellt und Aib Aminoisobuttersäure darstellt.

8. Peptidligand nach einem der Ansprüche 1 bis 7, wobei das pharmazeutisch annehmbare Salz aus der freien Säure oder dem Natrium-, Kalium-, Calcium- oder Ammoniumsalz ausgewählt ist.

9. Multimerer Bindungskomplex, der mindestens zwei bicyclische Peptidliganden nach einem der Ansprüche 1 bis 8 umfasst, wobei die Peptidliganden gleich oder unterschiedlich sein können.

10. Multimerer Bindungskomplex nach Anspruch 9, der ein NKp46-Homodimer umfasst, das aus BCY15663, BCY15921, BCY15922, BCY15455 und BCY18043 ausgewählt ist.

11. Multimerer Bindungskomplex nach Anspruch 9, der ein CD 1 6a-Homodimer umfasst, das aus BCY15914, BCY15915 und BCY15916 ausgewählt ist.

12. Pharmazeutische Zusammensetzung, die den Peptidliganden nach einem der Ansprüche 1 bis 8 oder den multimeren Bindungskomplex nach einem der Ansprüche 9 bis 11 in Kombination mit einem oder mehreren pharmazeutisch annehmbaren Hilfsstoffen umfasst.

13. Peptidligand nach einem der Ansprüche 1 bis 8 oder multimerer Bindungskomplex nach einem der Ansprüche 9 bis 11 oder pharmazeutische Zusammensetzung aus Anspruch 12 zur Verwendung in der Therapie.

14. Peptidligand nach einem der Ansprüche 1 bis 8 oder multimerer Bindungskomplex nach einem der Ansprüche 9 bis 11 oder pharmazeutische Zusammensetzung aus Anspruch 12 zur Verwendung bei der Vorbeugung, Unterdrückung oder Behandlung einer Krankheit oder Störung, die durch natürliche Killerzellen (NK-Zellen) vermittelt wird, wie beispielsweise entzündliche Erkrankungen, Autoimmunerkrankungen und Krebs.

## Revendications

1. Ligand de peptide spécifique pour des cellules tueuses naturelles (NK), ou sel pharmaceutiquement acceptable de celui-ci, comprenant un polypeptide comprenant au moins trois groupes réactifs, séparés par au moins deux séquences de boucles, et un échafaudage moléculaire qui forme des liaisons covalentes avec les groupes réactifs du polypeptide de sorte qu'au moins deux boucles de polypeptide sont formées sur l'échafaudage moléculaire, dans lequel le ligand de peptide bicyclique comprend une séquence d'acides aminés qui est sélectionnée parmi :
CᵢNLQKPCᵢᵢMKYPCᵢᵢᵢ (SEQ ID N° : 1) ;
CᵢNLQNPCᵢᵢMKFPCᵢᵢᵢ (SEQ ID N° : 2) ;
CᵢNLQAPCᵢᵢMQTGKVCᵢᵢᵢ (SEQ ID N° : 3) ;
CᵢNLQAPCᵢᵢMLTGKVCᵢᵢᵢ (SEQ ID N° : 4 ; ici dénommée BCY18295 lorsqu'elle est complexée avec TATA) ;
CᵢLLHDHCᵢᵢPNTHPKLCᵢᵢᵢ (SEQ ID N° : 5) ;
CᵢLLHEHCᵢᵢPNTHPKMCᵢᵢᵢ (SEQ ID N° : 6) ;
CᵢLLHDHCᵢᵢPNSHPEICᵢᵢᵢ (SEQ ID N° : 7) ;
CᵢLLHDHCᵢᵢPNTHPIICᵢᵢᵢ (SEQ ID N° : 8) ;
CᵢYLPDWLCᵢᵢGDEYCᵢᵢᵢ (SEQ ID N° : 9) ;
CᵢYLPDYLCᵢᵢGDEYCᵢᵢᵢ (SEQ ID N° : 10) ;
CᵢDLTTHNCᵢᵢQWGICᵢᵢᵢ (SEQ ID N° : 11 ; ici dénommée BCY18262 lorsqu'elle est complexée avec TATA) ;
CᵢYLPDYLCᵢᵢGDEYCᵢᵢᵢ (SEQ ID N° : 12 ; ici dénommée BCY15633 lorsqu'elle est complexée avec TATA) ;
CᵢYLPDWLCᵢᵢGDEYCᵢᵢᵢ (SEQ ID N° : 13) ;
Cᵢ[2,6DiMeTyr]LPDYLCᵢᵢGDEYCᵢᵢᵢ (SEQ ID N° : 14) ;
CᵢY[tBuAla]PDYLCᵢᵢGDEYCᵢᵢᵢ (SEQ ID N° : 15) ;
CᵢY[Cba]PDYLCᵢᵢGDEYCᵢᵢᵢ (SEQ ID N° : 16) ;
CᵢYL[HyP]DYLCᵢᵢGDEYCᵢᵢᵢ (SEQ ID N° : 17) ;
CᵢYL[Aze]DYLCᵢᵢGDEYCᵢᵢᵢ (SEQ ID N° : 18) ;
CᵢYL[Aib]DYLCᵢᵢGDEYCᵢᵢᵢ (SEQ ID N° : 19) ;
CᵢYLPD[1Nal]LCᵢᵢGDEYCᵢᵢᵢ (SEQ ID N° : 20) ;
CᵢYLPD[2Nal]LCᵢᵢGDEYCᵢᵢᵢ (SEQ ID N° : 21) ;
CᵢYLPD[3Pal]LCᵢᵢGDEYCᵢᵢᵢ (SEQ ID N° : 22) ;
CᵢYLPD[2,6DiMeTyr]LCᵢᵢDEYCᵢᵢᵢ (SEQ ID N° : 23) ;
CᵢYLPDW[tBuAla]CᵢᵢGDEYCᵢᵢᵢ (SEQ ID N° : 24) ;
CᵢYLPDW[Cba]CᵢᵢGDEYCᵢᵢᵢ (SEQ ID N° : 25) ;
CᵢYLPDYLCᵢᵢ[dA]DEYCᵢᵢᵢ (SEQ ID N° : 26) ;
CᵢYLPDYLCᵢᵢGDE[2,6DiMeTyr]Cᵢᵢᵢ (SEQ ID N° : 27) ;
CᵢALPDYLCᵢᵢGDEYCᵢᵢᵢ (SEQ ID N° : 28) ;
CᵢYAPDYLCᵢᵢGDEYCᵢᵢᵢ (SEQ ID N° : 29) ;
CᵢYLADYLCᵢᵢGDEYCᵢᵢᵢ (SEQ ID N° : 30) ;
CᵢYLPAYLCᵢᵢGDEYCᵢᵢᵢ (SEQ ID N° : 31) ;
CᵢYLPDALCᵢᵢGDEYCᵢᵢᵢ (SEQ ID N° : 32) ;
CᵢYLPDYACᵢᵢGDEYCᵢᵢᵢ (SEQ ID N° : 33) ;
CᵢYLPDYLCᵢᵢADEYCᵢᵢᵢ (SEQ ID N° : 34) ;
CᵢYLPDYLCᵢᵢGAEYCᵢᵢᵢ (SEQ ID N° : 35) ;
CᵢYLPDYLCᵢᵢGDAYCᵢᵢᵢ (SEQ ID N° : 36) ;
CᵢYLPDYLCᵢᵢGDEACᵢᵢᵢ (SEQ ID N° : 37) ;
CᵢY[C5A]PDYLCᵢᵢGDEYCᵢᵢᵢ (SEQ ID N° : 38) ;
CᵢY[Cha]PDYLCᵢᵢGDEYCᵢᵢᵢ (SEQ ID N° : 39) ;
CᵢY[Cba]PDYLC_{ii[}dA]DEYCᵢᵢᵢ (SEQ ID N° : 40) ;
CᵢYLPD[4FPhe]LCᵢᵢGDEYCᵢᵢᵢ (SEQ ID N° : 41) ;
CᵢYLPD[4MeOPhe]LCᵢᵢGDEYCᵢᵢᵢ (SEQ ID N° : 42) ;
CᵢYLPD[NO2Phe]LCᵢᵢGDEYCᵢᵢᵢ (SEQ ID N° : 43) ;
CᵢYLPD[4MePhe]LCᵢᵢGDEYCᵢᵢᵢ (SEQ ID N° : 44) ;
CᵢYLPD[pCoPhe]LCᵢᵢGDEYCᵢᵢᵢ (SEQ ID N° : 45) ;
CᵢYLPD[pCaPhe]LCᵢᵢGDEYCᵢᵢᵢ (SEQ ID N° : 46) ;
CᵢYLPD[4tBuPhe]LCᵢᵢGDEYCᵢᵢᵢ (SEQ ID N° : 47) ;
CᵢYLPD[CF3Phe]LCᵢᵢGDEYCᵢᵢᵢ (SEQ ID N° : 48) ;
CᵢYLPD[4CNPhe]LCᵢᵢGDEYCᵢᵢᵢ (SEQ ID N° : 49) ;
CᵢYLPD[4CIPhe]LCᵢᵢGDEYCᵢᵢᵢ (SEQ ID N° : 50) ;
CᵢYLPD[HPhe]LCᵢᵢGDEYCᵢᵢᵢ (SEQ ID N° : 51) :
Cᵢ[2,6DiMeTyr]LPDWLCᵢᵢGDEYCᵢᵢᵢ (SEQ ID N° : 52) ;
CᵢY[Cba]PDWLCᵢᵢGDEYCᵢᵢᵢ (SEQ ID N° : 53) ;
CᵢYL[Aze]DWLCᵢᵢGDEYCᵢᵢᵢ (SEQ ID N° : 54) ;
CᵢYLPDWLCᵢᵢ[dA]DEYCᵢᵢᵢ (SEQ ID N° : 55) ;
CᵢSLPDYLCᵢᵢGDEYCᵢᵢᵢ (SEQ ID N° : 56) ;
CᵢDLTTHNCᵢᵢQWGICᵢᵢᵢ (SEQ ID N° : 57) ;
CᵢDLTTHNCᵢᵢLWGVCᵢᵢᵢ (SEQ ID N° : 58) ;
CᵢNLQLHNCᵢᵢIWGVCᵢᵢᵢ (SEQ ID N° : 59) ;
CᵢPPTVYCᵢᵢHPLDCᵢᵢᵢ (SEQ ID N° : 60) ;
CᵢRWSVEDPCᵢᵢGAWCᵢᵢᵢ (SEQ ID N° : 61) ;
CᵢVGLEELGPCᵢᵢSDLCᵢᵢᵢ (SEQ ID N° : 62 ; ici dénommée BCY16494 lorsqu'elle est complexée avec TBMT) ;
CᵢNLQQACᵢᵢLHTGKVCᵢᵢᵢ (SEQ ID N° : 63) ;
CᵢNLQAACᵢᵢMLTGKVCᵢᵢᵢ (SEQ ID N° : 64) ;
CᵢNLQAPCᵢᵢMATGKVCᵢᵢᵢ (SEQ ID N° : 65) ;
CᵢNLQAPCᵢᵢMLAGKVCᵢᵢᵢ (SEQ ID N° : 66) :
CᵢNLQAPCᵢᵢMLTAKVCᵢᵢᵢ (SEQ ID N° : 67) ;
CᵢNLQAPCᵢᵢMLTGAVCᵢᵢᵢ (SEQ ID N° : 68) ;
CᵢNLQAPCᵢᵢMLTGKACᵢᵢᵢ (SEQ ID N° : 69) ;
CᵢN[tBuAla]QAPCᵢᵢMLTGKVCᵢᵢᵢ (SEQ ID N° : 70) ;
CᵢNLQA[Sar]CᵢᵢMLTGKVCᵢᵢᵢ (SEQ ID N° : 71) ;
CᵢNLQA[Cis-HyP]CᵢᵢMLTGKVCᵢᵢᵢ (SEQ ID N° : 72) ;
CᵢNLQA[HyP]CᵢᵢMLTGKVCᵢᵢᵢ (SEQ ID N° : 73) ;
CᵢNLQA[NMeAla]CᵢᵢMLTGKVCᵢᵢᵢ (SEQ ID N° : 74) ;
CᵢNLQAPCᵢᵢLLTGKVCᵢᵢᵢ (SEQ ID N° : 75) ;
CᵢNLQAPCᵢᵢ [NIe]LTGKVCᵢᵢᵢ (SEQ ID N° : 76) ;
CᵢNLQAPCᵢᵢ[Cba]LTGKVCᵢᵢᵢ (SEQ ID N° : 77) ;
CᵢNLQAPCᵢᵢMETGKVCᵢᵢᵢ (SEQ ID N° : 78) ;
CᵢNLQAPCᵢᵢMFTGKVCᵢᵢᵢ (SEQ ID N° : 79) ;
CᵢNLQAPCᵢᵢMYTGKVCᵢᵢᵢ (SEQ ID N° : 80) ;
CᵢNLQAPCᵢᵢM[Cba]TGKVCᵢᵢᵢ (SEQ ID N° : 81) ;
CᵢNLQAPCᵢᵢM[tBuAla]TGKVCᵢᵢᵢ (SEQ ID N° : 82) ;
CᵢNLQAPCᵢᵢM[Cha]TGKVCᵢᵢᵢ (SEQ ID N° : 83) ;
CᵢNLQAPCᵢᵢMLT[dA]KVCᵢᵢᵢ (SEQ ID N° : 84) ;
CᵢNLQAPCᵢᵢMLTGRVCᵢᵢᵢ (SEQ ID N° : 85) ;
CᵢNLQAPCᵢᵢMLTG[Agb]VCᵢᵢᵢ (SEQ ID N° : 86) ;
CᵢNLQAPCᵢᵢMLTGK[tBuGly]Cᵢᵢᵢ (SEQ ID N° : 87) ;
CᵢNLQAPCᵢᵢMLTGK[Chg]Cᵢᵢᵢ (SEQ ID N° : 88) ;
CᵢNLQAPCᵢᵢMLTGK[Cbg]Cᵢᵢᵢ (SEQ ID N° : 89) ;
CᵢNLQAPCᵢᵢMLTG[HArg]VCᵢᵢᵢ (SEQ ID N° : 90) ;
CᵢNLQA[Aze]CᵢᵢMLTGKVCᵢᵢᵢ (SEQ ID N° : 91) ;
CᵢNLQA[Pip]CᵢᵢMLTGKVCᵢᵢᵢ (SEQ ID N° : 92) ;
CᵢNLQAPCᵢᵢMVTGKVCᵢᵢᵢ (SEQ ID N° : 93) ;
CᵢNLQAPCᵢᵢM[NIe]TGKVCᵢᵢᵢ (SEQ ID N° : 94) ;
CᵢNLQAPCᵢᵢM[Nva]TGKVCᵢᵢᵢ (SEQ ID N° : 95) ;
CᵢNLQAPCᵢᵢMLTGK[AllolIe]Cᵢᵢᵢ (SEQ ID N° : 96) ;
CᵢNLQAPCᵢᵢMLTGK[EPA]Cᵢᵢᵢ (SEQ ID N° : 97) ;
CᵢNLQQPCᵢᵢMLTGKVCᵢᵢᵢ (SEQ ID N° : 98) ;
CᵢNLQA[Nva]CᵢᵢMLTGKVCᵢᵢᵢ (SEQ ID N° : 99) ;
CᵢNLQAPCᵢᵢMHTGKVCᵢᵢᵢ (SEQ ID N° : 100) ;
CᵢNLQAPCᵢᵢLHTGKVCᵢᵢᵢ (SEQ ID N° : 101) ;
CᵢNLQQACᵢᵢMQTGKVCᵢᵢᵢ (SEQ ID N° : 102) ;
CᵢLLHDHCᵢᵢPNTHPKMCᵢᵢᵢ (SEQ ID N° : 103) ;
CᵢDLTTHNCᵢᵢIWGVCᵢᵢᵢ (SEQ ID N° : 104) ;
CᵢNLQLHNCᵢᵢIWGVCᵢᵢᵢ (SEQ ID N° : 105) ;
CᵢNLQLHNCᵢᵢQWGICᵢᵢᵢ (SEQ ID N° : 106) ;
Cᵢ[3tBuTyr]LPDYLCᵢᵢGDEYCᵢᵢᵢ (SEQ ID N° : 107) ;
CᵢYLPD[3tBuTyr]LCᵢᵢGDEYCᵢᵢᵢ (SEQ ID N° : 108) ;
CᵢYLPDYLCᵢᵢGDE[3tBuTyr]Cᵢᵢᵢ (SEQ ID N° : 109) ;
Cᵢ[K(PYA)]LPDYLCᵢᵢGDEYCᵢᵢᵢ (SEQ ID N° : 110) ;
CᵢYLP[K(PYA)]YLCᵢᵢGDEYCᵢᵢᵢ (SEQ ID N° : 111) ;
CᵢYLPDYLCᵢᵢ[K(PYA)]DEYCᵢᵢᵢ (SEQ ID N° : 112) ;
CᵢYLPDYLCᵢᵢGD[K(PYA)]YCᵢᵢᵢ (SEQ ID N° : 113) ;
CᵢYLPDYLCᵢᵢGDE[K(PYA)]Cᵢᵢᵢ (SEQ ID N° : 114) ;
CᵢYLPDYLCᵢᵢ[dK(PYA)]DEYCᵢᵢᵢ (SEQ ID N° : 115) ;
CᵢALTTHNCᵢᵢQWGICᵢᵢᵢ (SEQ ID N° : 116) ;
CᵢDLATHNCᵢᵢQWGICᵢᵢᵢ (SEQ ID N° : 117) ;
CᵢDLTAHNCᵢᵢQWGICᵢᵢᵢ (SEQ ID N° : 118) ;
CᵢDLTTHNCᵢᵢAWGICᵢᵢᵢ (SEQ ID N° : 119) ;
CᵢDLTTHNCᵢᵢQWGACᵢᵢᵢ (SEQ ID N° : 120) ;
CᵢNLTTHNCᵢᵢQWGICᵢᵢᵢ (SEQ ID N° : 121) ;
CᵢDLQTHNCᵢᵢQWGICᵢᵢᵢ (SEQ ID N° : 122) ;
CᵢDLTLHNCᵢᵢQWGICᵢᵢᵢ (SEQ ID N° : 123) ;
CᵢDLTT[His3Me]NCᵢᵢQWGICᵢᵢᵢ (SEQ ID N° : 124) ;
CᵢDLTT[2Pal]NCᵢᵢQWGICᵢᵢᵢ (SEQ ID N° : 125) ;
CᵢDLTT[His1Me]NCᵢᵢQWGICᵢᵢᵢ (SEQ ID N° : 126) ;
CᵢDLTT[4ThiAz]NCᵢᵢQWGICᵢᵢᵢ (SEQ ID N° : 127) ;
CᵢDLTTHNCᵢᵢVWGICᵢᵢᵢ (SEQ ID N° : 128) ;
CᵢDLTTHNCᵢᵢ[Cba]WGICᵢᵢᵢ (SEQ ID N° : 129) ;
CᵢDLTTHNCᵢᵢQ[lNal]GICᵢᵢᵢ (SEQ ID N° : 130) ;
CᵢDLTTHNCᵢᵢQ[Trp(Me)]GICᵢᵢᵢ (SEQ ID N° : 131) ;
CᵢDLTTHNCᵢᵢQWG[tBuGly]Cᵢᵢᵢ (SEQ ID N° : 132) ;
CᵢDLTTHNCᵢᵢQWG[tBuAla]Cᵢᵢᵢ (SEQ ID N° : 133) ;
CᵢDLTTHNCᵢᵢQWG[Chg]Cᵢᵢᵢ (SEQ ID N° : 134) ;
CiDLTTHNCᵢᵢQW[dA]ICᵢᵢᵢ (SEQ ID N° : 135) ;
CᵢS[Cba]PDYLCᵢᵢ[dA]DEYCᵢᵢᵢ (SEQ ID N° : 136) ;
Cᵢ[HSer][Cba]PDYLCᵢᵢ[dA]DEYCᵢᵢᵢ (SEQ ID N° : 137) ;
CᵢN[Cba]PDYLCᵢᵢ[dA]DEYCᵢᵢᵢ (SEQ ID N° : 138) ;
Cᵢ[4Pal][Cba]PDYLCᵢᵢ[dA]DEYCᵢᵢᵢ (SEQ ID N° : 139) ;
CᵢY[Cba][Cis-HyP]DYLCᵢᵢ[dA]DEYCᵢᵢᵢ (SEQ ID N° : 140) ;
CᵢY[Cba][NMeAla]DYLCᵢᵢ[dA]DEYCᵢᵢᵢ (SEQ ID N° : 141) ;
CᵢY[Cba]PNYLCᵢᵢ[dA]DEYCᵢᵢᵢ (SEQ ID N° : 142) ;
CᵢY[Cba]PQYLCᵢᵢ[dA]DEYCᵢᵢᵢ (SEQ ID N° : 143) ;
CᵢY[Cba]PDYLCᵢᵢ[dV]DEYCᵢᵢᵢ (SEQ ID N° : 144) ;
CᵢY[Cba]PDYLCᵢᵢ[dNva]DEYCᵢᵢᵢ (SEQ ID N° : 145) ;
CᵢY[Cba]PDYLCᵢᵢ[dF]DEYCᵢᵢᵢ (SEQ ID N° : 146) ;
CᵢY[Cba]PDYLCᵢᵢ[dCha]DEYCᵢᵢᵢ (SEQ ID N° : 147) ;
CᵢY[Cba]PDYLCᵢᵢ[dK(PYA)]DEYCᵢᵢᵢ (SEQ ID N° : 148) ;
CᵢY[Cba]PDYLCᵢᵢ[dNIe]DEYCᵢᵢᵢ (SEQ ID N° : 149) ;
CᵢY[Cba]PD[DOPAILCᵢᵢ[dA]DEYCᵢᵢᵢ (SEQ ID N° : 150) ;
CᵢY[Cba]PD[2FTyr]LCᵢᵢ[dA]DEYCᵢᵢᵢ (SEQ ID N° :151) ;
CᵢY[Cba]PDY[Nle]Cᵢᵢ[dA]DEYCᵢᵢᵢ (SEQ ID N° : 152) ;
[Pen]ᵢY[Cba]PDYLCᵢᵢ[dA]DEYCᵢᵢᵢ (SEQ ID N° : 153) ;
CᵢY[Cba]PDYL[Pen]ᵢᵢ[dA]DEYCᵢᵢᵢ (SEQ ID N° : 154) ;
CᵢY[Cba]PDYLCᵢᵢ[dA]DEY[Pen]ᵢᵢᵢ (SEQ ID N° : 155) ;
CᵢY[Cba]PDYLCᵢᵢ[dA]DEY[dCᵢᵢᵢ] (SEQ ID N° : 156) ;
[dCᵢ]Y[Cba]PDYLCᵢᵢ[dA]DEYCᵢᵢᵢ (SEQ ID N° : 157) ;
CᵢDLTTHNCᵢᵢQ[AzaTrp]GICᵢᵢᵢ (SEQ ID N° : 158) ;
CᵢDLTTHNCᵢᵢQtZMeTrplGICᵢᵢᵢ (SEQ ID N° : 159) ;
CᵢDLTTHNCᵢᵢQteMeTrplGICᵢᵢᵢ (SEQ ID N° : 160) ;
CᵢDLTTHNCᵢᵢQ[7MeTrp]GICᵢᵢᵢ (SEQ ID N° : 161) ;
CᵢDLTTHNCᵢᵢQ[6FTrp]GICᵢᵢᵢ (SEQ ID N° : 162) ;
CᵢDLTTHNCᵢᵢQ[5FTrp]GICᵢᵢᵢ (SEQ ID N° : 163) ;
CᵢDLTTHNCᵢᵢQ[Dht]GICᵢᵢᵢ (SEQ ID N° : 164) ;
CᵢDLTT[2FuAla]NCᵢᵢQWGICᵢᵢᵢ (SEQ ID N° : 165) ;
CᵢDLTTHNCᵢᵢQWG[EPA]Cᵢᵢᵢ (SEQ ID N° : 166) ;
CᵢDLTTHNCᵢᵢQWG[Nle]Cᵢᵢᵢ (SEQ ID N° : 167) ;
CᵢDLTTHNCᵢᵢQWG[Allolle]Cᵢᵢᵢ (SEQ ID N° : 168) ;
CᵢPPAVYCᵢᵢHPLDCᵢᵢᵢ (SEQ ID N° : 169) ;
CᵢPPEVFCᵢᵢHPLDCᵢᵢᵢ (SEQ ID N° : 170) ;
CᵢPPEVYCᵢᵢHPLDCᵢᵢᵢ (SEQ ID N° : 171) ;
CᵢPPEVWCᵢᵢHPLDCᵢᵢᵢ (SEQ ID N° : 172) ;
CᵢPPQVYCᵢᵢHPLDCᵢᵢᵢ (SEQ ID N° : 173) ;
CᵢPPEVWCᵢᵢHPLDCᵢᵢᵢ (SEQ ID N° : 174) ;
CᵢRWS[tBuGly]EDPCᵢᵢGAWCᵢᵢᵢ (SEQ ID N° : 175) ;
CᵢRWSVED[HyP]CᵢᵢGAWCᵢᵢᵢ (SEQ ID N° : 176) ;
CᵢRWSVED[Aze]CᵢᵢGAWCᵢᵢᵢ (SEQ ID N° : 177) ;
CᵢRWSVEDPCᵢᵢ[dA]AWCᵢᵢᵢ (SEQ ID N° : 178) ;
CᵢRWSVE[Gla]PCᵢᵢGAWCᵢᵢᵢ (SEQ ID N° : 179) ;
CᵢRWSVED[Aze]CᵢᵢGAWCᵢᵢᵢ (SEQ ID N° : 180) ;
CᵢMWIRSDPCᵢᵢGAWCᵢᵢᵢ (SEQ ID N° : 181) ;
CᵢRWYPDDPCᵢᵢGAWCᵢᵢᵢ (SEQ ID N° : 182) ;
CᵢEWHISEPCᵢᵢGAWCᵢᵢᵢ (SEQ ID N° : 183) ;
CᵢNWHVYEPCᵢᵢGAWCᵢᵢᵢ (SEQ ID N° : 184) ;
CᵢRWHFSEPCᵢᵢGAWCᵢᵢᵢ (SEQ ID N° : 185) ;
CᵢRWNLDDPCᵢᵢGAWCᵢᵢᵢ (SEQ ID N° : 186) ;
CᵢMWIRSDPCᵢᵢGAWCᵢᵢᵢ (SEQ ID N° : 187) ;
CᵢRWYPDDPCᵢᵢGAWCᵢᵢᵢ (SEQ ID N° : 188) ;
CᵢEWHISEPCᵢᵢGAWCᵢᵢᵢ (SEQ ID N° : 189) ;
CᵢNWHVYEPCᵢᵢGAWCᵢᵢᵢ (SEQ ID N° : 190) ;
CᵢRWHFSEPCᵢᵢGAWCᵢᵢᵢ (SEQ ID N° : 191) ;
CᵢRWNLDDPCᵢᵢGAWCᵢᵢᵢ (SEQ ID N° : 192) ;
CᵢR[2MeTrp]SVEDPCᵢᵢGAWCᵢᵢᵢ (SEQ ID N° : 193) ;
CᵢR[6MeTrp]SVEDPCᵢᵢGAWCᵢᵢᵢ (SEQ ID N° : 194) ;
CᵢR[6FTrp]SVEDPCᵢᵢGAWCᵢᵢᵢ (SEQ ID N°: 195) ;
CᵢR[5FTrp]SVEDPCᵢᵢGAWCᵢᵢᵢ (SEQ ID N°: 196) ;
CᵢR[6CITrp]SVEDPCᵢᵢGAWCᵢᵢᵢ (SEQ ID N° : 197) ;
CᵢR[Trp(S)]SVEDPCᵢᵢGAWCᵢᵢᵢ (SEQ ID N° : 198) ;
CᵢR[AzaTrp]SVEDPCᵢᵢGAWCᵢᵢᵢ (SEQ ID N° : 199) ;
CᵢRWSVEDPCᵢᵢGA[2MeTrp]Cᵢᵢᵢ (SEQ ID N° : 200) ;
CᵢRWSVEDPCᵢᵢA[6FTrp]Cᵢᵢᵢ (SEQ ID N° : 201) ;
CᵢRWSVEDPCᵢᵢGA[5FTrp]Cᵢᵢᵢ (SEQ ID N° : 202) ;
CᵢRWSVEDPCᵢᵢGA[4MeoTrp]Cᵢᵢᵢ (SEQ ID N° : 203) ;
CᵢRWSVEDPCᵢᵢGA[5MeoTrp]Cᵢᵢᵢ (SEQ ID N° : 204) ;
CᵢRWSVEDPCᵢᵢGA[Trp(S)]Cᵢᵢᵢ (SEQ ID N° : 205) ;
CᵢRWSVEDPCᵢᵢGA[DOPA]Cᵢᵢᵢ (SEQ ID N° : 206) ;
CᵢRWYPDDPCᵢᵢGAWCᵢᵢᵢ (SEQ ID N° : 207) ;
CᵢRWHFSEPCᵢᵢGAWCᵢᵢᵢ (SEQ ID N° : 208) ;
CᵢR[2MeTrp]HFSEPCᵢᵢGAWCᵢᵢᵢ (SEQ ID N° : 209) ;
CᵢRWYFSEPCᵢᵢGAWCᵢᵢᵢ (SEQ ID N° : 210) ;
CᵢRWSFSEPCᵢᵢGAWCᵢᵢᵢ (SEQ ID N° : 211) ;
CᵢRWHPSEPCᵢᵢGAWCᵢᵢᵢ (SEQ ID N° : 212) ;
CᵢRWYPSEPCᵢᵢGAWCᵢᵢᵢ (SEQ ID N° : 213) ;
CᵢRWHPDEPCᵢᵢGAWCᵢᵢᵢ (SEQ ID N° : 214) ;
CᵢRWHPEEPCᵢᵢGAWCᵢᵢᵢ (SEQ ID N° : 215) ;
CᵢRWHFDEPCᵢᵢGAWCᵢᵢᵢ (SEQ ID N° : 216) ;
CᵢRWHFDDPCᵢᵢGAWCᵢᵢᵢ (SEQ ID N° : 217) ;
CᵢRWYFDEPCᵢᵢGAWCᵢᵢᵢ (SEQ ID N° : 218) ;
CᵢRW[His1Me]FSEPCᵢᵢGAWCᵢᵢᵢ (SEQ ID N° : 219) ;
CᵢRW[His3Me]FSEPCᵢᵢGAWCᵢᵢᵢ (SEQ ID N° : 220) ;
CᵢRW[4MeoPhe]FSEPCᵢᵢGAWCᵢᵢᵢ (SEQ ID N° : 221) ;
CᵢRW[DOPA]FSEPCᵢᵢGAWCᵢᵢᵢ (SEQ ID N° : 222) ;
CᵢRW[26DiMeTyr]FSEPCᵢᵢGAWCᵢᵢᵢ (SEQ ID N° : 223) ;
CᵢRW[3tBuTyr]FSEPCᵢᵢGAWCᵢᵢᵢ (SEQ ID N° : 224) ;
CᵢRW[4FPhe]FSEPCᵢᵢGAWCᵢᵢᵢ (SEQ ID N° : 225) ;
CᵢRWH[4MePhe]SEPCᵢᵢGAWCᵢᵢᵢ (SEQ ID N° : 226) ;
CᵢRWH[Aze]SEPCᵢᵢGAWCᵢᵢᵢ (SEQ ID N° : 227) ;
CᵢRWH[HyP]SEPCᵢᵢGAWCᵢᵢᵢ (SEQ ID N° : 228) ;
CᵢRWH[4tBuPhe]SEPCᵢᵢGAWCᵢᵢᵢ (SEQ ID N° : 229) ;
CᵢRWH[HPhe]SEPCᵢᵢGAWCᵢᵢᵢ (SEQ ID N° : 230 ; ici dénommée BCY21693 lorsqu'elle est complexée avec TBMT) ;
CᵢRWH[4PhePro]SEPCᵢᵢGAWCᵢᵢᵢ (SEQ ID N° : 231) ;
CᵢRWHF[HSer]EPCᵢᵢGAWCᵢᵢᵢ (SEQ ID N° : 232) ;
CᵢRWHFS[Gla]PCᵢᵢGAWCᵢᵢᵢ (SEQ ID N° : 233) ;
CᵢRWHFS[HGlu]PCᵢᵢGAWCᵢᵢᵢ (SEQ ID N° : 234) ;
CᵢRWHFSEPC_{ii[}dA]AWCᵢᵢᵢ (SEQ ID N° : 235) ;
CᵢRWYFSE[Aze]C_{ii[}dA]AWCᵢᵢᵢ (SEQ ID N° : 236) ;
CᵢRWHFDE[Aze]Cᵢᵢ[dA]AWCᵢᵢᵢ (SEQ ID N° : 237) ;
CᵢRWYPSE[Aze]Cᵢᵢ[dA]AWCᵢᵢᵢ (SEQ ID N° : 238) ;
Cᵢ[HArg]WHFSEPCᵢᵢGAWCᵢᵢᵢ (SEQ ID N° : 239) ;
CᵢRWH[Cis-HyP]SEPCᵢᵢGAWCᵢᵢᵢ (SEQ ID N° : 240) ;
CᵢRWHFSEPCᵢᵢGAWCᵢᵢᵢ (SEQ ID N° : 241) ;
CᵢRWHFSEPCᵢᵢGAWCᵢᵢᵢ (SEQ ID N° : 242) ;
CᵢRWHFSEPCᵢᵢGAWCᵢᵢᵢ (SEQ ID N° : 243) ;
CᵢVGLLELGPCᵢᵢSDLCᵢᵢᵢ (SEQ ID N° : 244) ;
CᵢPGLEELGPCᵢᵢSDLCᵢᵢᵢ (SEQ ID N° : 245) ;
CᵢV[dA]LEELGPCᵢᵢSDLCᵢᵢᵢ (SEQ ID N° : 246) ;
CᵢVG[tBuAla]EELGPCᵢᵢSDLCᵢᵢᵢ (SEQ ID N° : 247) ;
CᵢVG[Cba]EELGPCᵢᵢSDLCᵢᵢᵢ (SEQ ID N° : 248) ;
CᵢVGFEELGPCᵢᵢSDLCᵢᵢᵢ (SEQ ID N° : 249) ;
CᵢVG[1Nal]EELGPCᵢᵢSDLCᵢᵢᵢ (SEQ ID N° : 250) ;
CᵢVG[2Nal]EELGPCᵢᵢSDLCᵢᵢᵢ (SEQ ID N° : 251) ;
CᵢVGLPELGPCᵢᵢSDLCᵢᵢᵢ (SEQ ID N° : 252) ;
CᵢVGL[tBuAla]ELGPCᵢᵢSDLCᵢᵢᵢ (SEQ ID N° : 253) ;
CᵢVGL[Cba]ELGPCᵢᵢSDLCᵢᵢᵢ (SEQ ID N° : 254) ;
CᵢVGL[Cha]ELGPCᵢᵢSDLCᵢᵢᵢ (SEQ ID N° : 255) ;
CᵢVGL[Nle]ELGPCᵢᵢSDLCᵢᵢᵢ (SEQ ID N° : 256) ;
CᵢVGLE[Gla]LGPCᵢᵢSDLCᵢᵢᵢ (SEQ ID N° : 257) ;
CᵢVGLEELG[Aze]CᵢᵢSDLCᵢᵢᵢ (SEQ ID N° : 258) ;
CᵢVGLEELGPCᵢᵢSD[tBuAla]Cᵢᵢᵢ (SEQ ID N° : 259) ;
CᵢVGLEELGPCᵢᵢSD[Cba]Cᵢᵢᵢ (SEQ ID N° : 260) ;
CᵢVGLEELGPCᵢᵢSD[Cha]Cᵢᵢᵢ (SEQ ID N° : 261) ;
CᵢVG[HLeu]EELGPCᵢᵢSDLCᵢᵢᵢ (SEQ ID N° : 262) ;
CᵢVGL[HLeu]ELGPCᵢᵢSDLCᵢᵢᵢ (SEQ ID N° : 263) ;
CᵢVGLE[HGlu]LGPCᵢᵢSDLCᵢᵢᵢ (SEQ ID N° : 264) ;
CᵢTWPYCᵢᵢKSWGDVQDCᵢᵢᵢ (SEQ ID N° : 265) ;
CᵢDWWDPGCᵢᵢTYFCᵢᵢᵢ (SEQ ID N° : 266) ;
CᵢRWH[4BnPro]SEPCᵢᵢGAWCᵢᵢᵢ (SEQ ID N° : 267) ; et
CᵢRWH[HPhe]SE[Aze]CᵢᵢGAWCᵢᵢᵢ (SEQ ID N° : 268) ;
où Cᵢ, Penᵢ, Cᵢᵢ, Penᵢᵢ, Cᵢᵢᵢ et Penᵢᵢᵢ représentent respectivement des premier, deuxième et troisième groupes réactifs, et où 2,6DiMeTyr représente la 2,6-diméthyl-tyrosine, tBuAla représente la β-t-Butyl-L-alanine, Cba représente la β-cyclobutylalanine, HyP représente l'hydroxyproline, Aze représente l'acide azétidine-2-carboxylique, Aib représente l'acide aminoisobutyrique, 1Nal représente la 1-naphtylalanine, 2Nal représente la 2-naphtylalanine, 3Pal représente la 3-pyridylalanine, C5A représente la bêta-cyclopentyl-L-alanine, Cha représente la 3-cyclohexyl-L-alanine, 4FPhe représente la 4-fluoro-L-phénylalanine, 4MeOPhe représente la 4-méthoxy-phénylalanine, NO2Phe représente la 4-nitro-phénylalanine, 4MePhe représente la 4-méthyl-phénylalanine, pCoPhe représente la para-carboxy-phénylalanine, pCaPhe représente la p-carboxamido-phénylalanine, 4tBuPhe représente la 4-tert-butyl-phénylalanine, CF3Phe représente la 4-trifluorométhyl-phénylalanine, 4CNPhe représente la 4-cyano-phénylalanine, 4CIPhe représente la 4-chloro-phénylalanine, HPhe représente l'homophénylalanine, 3tBuTyr représente la 3-tert-butyltyrosine, His3Me représente la 3-méthylhistidine, 2Pal représente la 2-pyridylalanine, HislMe représente la 1-méthylhistidine, 4ThiAz représente la bêta-4-thiazol-L-ylalanine, Trp(Me) représente le 1-méthyltryptophane, tBuGly représente la tert-butylglycine, Chg représente la cyclohexylglycine, HSer représente l'homosérine, NMeAla représente la N-méthylalanine, 4Pal représente la 4-pyridylalanine, Cis-HyP représente la L-4-cis-hydroxyproline, dA représente la D-alanine, dV représente la D-valine, Nva représente la norvaline, dNva représente la D-norvaline, Nie représente la norleucine, dNle représente la D-norleucine, dF représente la D-phénylalanine, dCha représente la 3-cyclohexyl-D-alanine, DOPA représente la 3,4-Dihydroxyphénylalanine, 2FTyr représente la 2-fluorotyrosine, dC représente la D-cystéine, AzaTrp représente l'azatryptophane, 2MeTrp représente le 2-méthyltryptophane, 6MeTrp représente le 6-méthyltryptophane, 6FTrp représente le 6-fluorotryptophane, 5FTrp représente le 5-fluorotryptophane, Dht représente le 2,3-Dihydrotryptophane, 2FuAla représente la 2-furylalanine, Allolle représente la L-allo-isoleucine, Sar représente la sarcosine, Agb représente la norarginine, Cbg représente la cyclobutylglycine, HArg représente l'homoarginine, Pep représente l'acide pipécolique, EPA représente la diéthyl-L-alanine, Gla représente l'acide y-carboxyglutamique, 6CITrp représente le 6-Chloro-L-tryptophane, 4MeoTrp représente le 4-Méthoxy-L-tryptophane, SMeoTrp représente le 5-Méthoxy-L-tryptophane, Trp(S) représente la 3-(3-Benzothiényl)-L-alanine, 26DiMeTyr représente la 2,6-Diméthyl-L-tyrosine, 4PhePro représente la (2S,4S)-4-PhenyIProline, HGlu représente l'acide L-2-aminoadipique, 4BnPro représente l'acide (2S,4R)-4-benzyl-pyrrolidine-2-carboxylique et Hleu représente l'homoleucine.

2. Ligand de peptide tel que défini dans la revendication 1, dans lequel ledit peptide bicyclique est spécifique pour NKp46 et dans lequel le ligand de peptide bicyclique comprend une séquence d'acides aminés qui est sélectionnée parmi :
CᵢNLQKPCᵢᵢMKYPCᵢᵢᵢ (SEQ ID N° : 1) ; et
CᵢNLQNPCᵢᵢMKFPCᵢᵢᵢ (SEQ ID N° : 2) ;
CᵢNLQAPCᵢᵢMQTGKVCᵢᵢᵢ (SEQ ID N° : 3) ;
CᵢNLQAPCᵢᵢMLTGKVCᵢᵢᵢ (SEQ ID N° : 4 ; ici dénommée BCY18295 lorsqu'elle est complexée avec TATA) ;
CᵢLLHDHCᵢᵢPNTHPKLCᵢᵢᵢ (SEQ ID N° : 5) ;
CᵢLLHEHCᵢᵢPNTHPKMCᵢᵢᵢ (SEQ ID N° : 6) ;
CᵢLLHDHCᵢᵢPNSHPEICᵢᵢᵢ (SEQ ID N° : 7) ;
CᵢLLHDHCᵢᵢPNTHPIICᵢᵢᵢ (SEQ ID N° : 8) ;
CᵢYLPDWLCᵢᵢGDEYCᵢᵢᵢ (SEQ ID N° : 9) ;
CᵢYLPDYLCᵢᵢGDEYCᵢᵢᵢ (SEQ ID N° : 10) ;
CᵢDLTTHNCᵢᵢQWGICᵢᵢᵢ (SEQ ID N° : 11 ; ici dénommée BCY18262 lorsqu'elle est complexée avec TATA) ;
CᵢYLPDYLCᵢᵢGDEYCᵢᵢᵢ (SEQ ID N° : 12 ; ici dénommée BCY15633 lorsqu'elle est complexée avec TATA) ;
CᵢYLPDWLCᵢᵢGDEYCᵢᵢᵢ (SEQ ID N° : 13) ;
C_{i[}2,6DiMeTyr]LPDYLCᵢᵢGDEYCᵢᵢᵢ (SEQ ID N° : 14) ;
CᵢY[tBuAla]PDYLCᵢᵢGDEYCᵢᵢᵢ (SEQ ID N° : 15) ;
CᵢY[Cba]PDYLCᵢᵢGDEYCᵢᵢᵢ (SEQ ID N° : 16) ;
CᵢYL[HyP]DYLCᵢᵢGDEYCᵢᵢᵢ (SEQ ID N° : 17) ;
CᵢYLAze]DYLCᵢᵢGDEYCᵢᵢᵢ (SEQ ID N° : 18) ;
CᵢYL[Aib]DYLCᵢᵢGDEYCᵢᵢᵢ (SEQ ID N° : 19) ;
CᵢYLPD[1Nal]LCᵢᵢGDEYCᵢᵢᵢ (SEQ ID N° : 20) ;
CᵢYLPD[2Nal]LCᵢᵢGDEYCᵢᵢᵢ (SEQ ID N° : 21) ;
CᵢYLPD[3Pal]LCᵢᵢGDEYCᵢᵢᵢ (SEQ ID N° : 22) ;
CᵢYLPD[2₇6DiMeTyr]LCᵢᵢGDEYCᵢᵢᵢ (SEQ ID N° : 23) ;
CᵢYLPDW[tBuAla]CᵢᵢGDEYCᵢᵢᵢ (SEQ ID N° : 24) ;
CᵢYLPDW[Cba]CᵢᵢGDEYCᵢᵢᵢ (SEQ ID N° : 25) ;
CᵢYLPDYLCᵢᵢ[dA]DEYCᵢᵢᵢ (SEQ ID N° : 26) ;
CᵢYLPDYLCᵢᵢGDE[2,6DiMeTyr]Cᵢᵢᵢ (SEQ ID N° : 27) ;
CᵢALPDYLCᵢᵢGDEYCᵢᵢᵢ (SEQ ID N° : 28) ;
CᵢYAPDYLCᵢᵢGDEYCᵢᵢᵢ (SEQ ID N° : 29) ;
CᵢYLADYLCᵢᵢGDEYCᵢᵢᵢ (SEQ ID N° : 30) ;
CᵢYLPAYLCᵢᵢGDEYCᵢᵢᵢ (SEQ ID N° : 31) ;
CᵢYLPDALCᵢᵢGDEYCᵢᵢᵢ (SEQ ID N° : 32) ;
CᵢYLPDYACᵢᵢGDEYCᵢᵢᵢ (SEQ ID N° : 33) ;
CᵢYLPDYLCᵢᵢADEYCᵢᵢᵢ (SEQ ID N° : 34) ;
CᵢYLPDYLCᵢᵢGAEYCᵢᵢᵢ (SEQ ID N° : 35) ;
CᵢYLPDYLCᵢᵢGDAYCᵢᵢᵢ (SEQ ID N° : 36) ;
CᵢYLPDYLCᵢᵢGDEACᵢᵢᵢ (SEQ ID N° : 37) ;
CᵢY[C5A]PDYLCᵢᵢGDEYCᵢᵢᵢ (SEQ ID N° : 38) ;
CᵢY[Cha]PDYLCᵢᵢGDEYCᵢᵢᵢ (SEQ ID N° : 39) ;
CᵢY[Cba]PDYLCᵢᵢ[dA]DEYCᵢᵢᵢ (SEQ ID N° : 40) ;
CᵢYLPD[4FPhe]LCᵢᵢGDEYCᵢᵢᵢ (SEQ ID N° : 41) ;
CᵢYLPD[4MeOPhe]LCᵢᵢGDEYCᵢᵢᵢ (SEQ ID N° : 42) ;
CᵢYLPD[NO2Phe]LCᵢᵢGDEYCᵢᵢᵢ (SEQ ID N° : 43) ;
CᵢYLPD[4MePhe]LCᵢᵢGDEYCᵢᵢᵢ (SEQ ID N° : 44) ;
CᵢYLPD[pCoPhe]LCᵢiGDEYCᵢᵢᵢ (SEQ ID N° : 45) ;
CᵢYLPD[pCaPhe]LCᵢᵢGDEYCᵢᵢᵢ (SEQ ID N° : 46) ;
CᵢYLPD[4tBuPhe]LCᵢᵢGDEYCᵢᵢᵢ (SEQ ID N° : 47) ;
CᵢYLPD[CF3Phe]LCᵢᵢGDEYCᵢᵢᵢ (SEQ ID N° : 48) ;
CᵢYLPD[4CNPhe]LCᵢᵢDEYCᵢᵢᵢ (SEQ ID N° : 49) ;
CᵢYLPD[4ClPhe]LCᵢᵢGDEYCᵢᵢᵢ (SEQ ID N° : 50) ;
CᵢYLPD[HPhe]LCᵢᵢGDEYCᵢᵢᵢ (SEQ ID N° : 51) ;
Cᵢ[2,6DiMeTyr]LPDWLCᵢᵢGDEYCᵢᵢᵢ (SEQ ID N° : 52) ;
CᵢY[Cba]PDWLCᵢᵢGDEYCᵢᵢᵢ (SEQ ID N° : 53) ;
CᵢYL[Aze]DWLCᵢᵢGDEYCᵢᵢᵢ (SEQ ID N° : 54) ;
CᵢYLPDWLCᵢᵢ[dA]DEYCᵢᵢᵢ (SEQ ID N° : 55) ;
CᵢSLPDYLCᵢᵢGDEYCᵢᵢᵢ (SEQ ID N° : 56) ;
CᵢDLTTHNCᵢᵢQWGICᵢᵢᵢ (SEQ ID N° : 57) ;
CᵢDLTTHNCᵢᵢLWGVCᵢᵢᵢ (SEQ ID N° : 58) ;
CᵢNLQLHNCᵢᵢIWGVCᵢᵢᵢ (SEQ ID N° : 59) ;
CᵢNLQQACᵢᵢLHTGKVCᵢᵢᵢ (SEQ ID N° : 63) ;
CᵢNLQAACᵢᵢMLTGKVCᵢᵢᵢ (SEQ ID N° : 64) ;
CᵢNLQAPCᵢᵢMATGKVCᵢᵢᵢ (SEQ ID N° : 65) ;
CᵢNLQAPCᵢᵢMLAGKVCᵢᵢᵢ (SEQ ID N° : 66) ;
CᵢNLQAPCᵢᵢMLTAKVCᵢᵢᵢ (SEQ ID N° : 67) ;
CᵢNLQAPCᵢᵢMLTGAVCᵢᵢᵢ (SEQ ID N° : 68) ;
CᵢNLQAPCᵢᵢMLTGKACᵢᵢᵢ (SEQ ID N° : 69) ;
CᵢN[tBuAla]QAPCᵢᵢMLTGKVCᵢᵢᵢ (SEQ ID N° : 70) ;
CᵢNLQA[Sar]CᵢᵢMLTGKVCᵢᵢᵢ (SEQ ID N° : 71) ;
CᵢNLQA[Cis-HyP]CᵢᵢMLTGKVCᵢᵢᵢ (SEQ ID N° : 72) ;
CᵢNLQA[HyP]CᵢᵢMLTGKVCᵢᵢᵢ (SEQ ID N° : 73) ;
CᵢNLQA[NMeAla]CᵢᵢMLTGKVCᵢᵢᵢ (SEQ ID N° : 74) ;
CᵢNLQAPCᵢᵢLLTGKVCᵢᵢᵢ (SEQ ID N° : 75) ;
CᵢNLQAPCᵢᵢ[Nle]LTGKVCᵢᵢᵢ (SEQ ID N° : 76) ;
CᵢNLQAPCᵢᵢ[Cba]LTGKVCᵢᵢᵢ (SEQ ID N° : 77) ;
CᵢNLQAPCᵢᵢMETGKVCᵢᵢᵢ (SEQ ID N° : 78) ;
CᵢNLQAPCᵢᵢMFTGKVCᵢᵢᵢ (SEQ ID N° : 79) ;
CᵢNLQAPCᵢᵢMYTGKVCᵢᵢᵢ (SEQ ID N° : 80) ;
CᵢNLQAPCᵢᵢM[Cba]TGKVCᵢᵢᵢ (SEQ ID N° : 81) ;
CᵢNLQAPCᵢᵢM[tBuAla]TGKVCᵢᵢᵢ (SEQ ID N° : 82) ;
CᵢNLQAPCᵢᵢM[Cha]TGKVCᵢᵢᵢ (SEQ ID N° : 83) ;
CᵢNLQAPCᵢᵢMLT[dA]KVCᵢᵢᵢ (SEQ ID N° : 84) ;
CᵢNLQAPCᵢᵢMLTGRVCᵢᵢᵢ (SEQ ID N° : 85) ;
CᵢNLQAPCᵢᵢMLTG[Agb]VCᵢᵢᵢ (SEQ ID N° : 86) ;
CᵢNLQAPCᵢᵢMLTGK[tBuGly]Cᵢᵢᵢ (SEQ ID N° : 87) ;
CᵢNLQAPCᵢᵢMLTGK[Chg]Cᵢᵢᵢ (SEQ ID N° : 88) ;
CᵢNLQAPCᵢᵢMLTGK[Cbg]Cᵢᵢᵢ (SEQ ID N° : 89) ;
CᵢNLQAPCᵢᵢMLTG[HArg]VCᵢᵢᵢ (SEQ ID N° : 90) ;
CᵢNLQA[Aze]CᵢᵢMLTGKVCᵢᵢᵢ (SEQ ID N° : 91) ;
CᵢNLQA[Pip]CᵢᵢMLTGKVCᵢᵢᵢ (SEQ ID N° : 92) ;
CᵢNLQAPCᵢᵢMVTGKVCᵢᵢᵢ (SEQ ID N° : 93) ;
CᵢNLQAPCᵢᵢM[Nle]TGKVCᵢᵢᵢ (SEQ ID N° : 94) ;
CᵢNLQAPCᵢᵢM[Nva]TGKVCᵢᵢᵢ (SEQ ID N° : 95) ;
CᵢNLQAPCᵢᵢMLTGK[AlloIle]Cᵢᵢᵢ (SEQ ID N° : 96) ;
CᵢNLQAPCᵢᵢMLTGK[EPA]Cᵢᵢᵢ (SEQ ID N° :97) ;
CᵢNLQQPCᵢᵢMLTGKVCᵢᵢᵢ (SEQ ID N° : 98) ;
CᵢNLQA[Nva]CᵢᵢMLTGKVCᵢᵢᵢ (SEQ ID N° : 99) ;
CᵢNLQAPCᵢᵢMHTGKVCᵢᵢᵢ (SEQ ID N° : 100) ;
CᵢNLQAPCᵢᵢLHTGKVCᵢᵢᵢ (SEQ ID N° : 101) ;
CᵢNLQQACᵢᵢMQTGKVCᵢᵢᵢ (SEQ ID N° : 102) ;
CᵢLLHDHCᵢᵢPNTHPKMCᵢᵢᵢ (SEQ ID N° : 103) ;
CᵢDLTTHNCᵢᵢlWGVCᵢᵢᵢ (SEQ ID N° : 104) ;
CᵢNLQLHNCᵢᵢIWGVCᵢᵢᵢ (SEQ ID N° : 105) ;
CᵢNLQLHNCᵢᵢQWGICᵢᵢᵢ (SEQ ID N° : 106) ;
Cᵢ[3tBuTyr]LPDYLCᵢᵢGDEYCᵢᵢᵢ (SEQ ID N° : 107) ;
CᵢYLPD[3tBuTyr]LCᵢᵢGDEYCᵢᵢᵢ (SEQ ID N° : 108) ;
CᵢYLPDYLCᵢᵢGDE[3tBuTyr]Cᵢᵢᵢ (SEQ ID N° : 109) ;
Cᵢ[K(PYA)]LPDYLCᵢᵢGDEYCᵢᵢᵢ (SEQ ID N° : 110) ;
CᵢYLP[K(PYA)]YLCᵢᵢGDEYCᵢᵢᵢ (SEQ ID N° : 111) ;
CᵢYLPDYLCᵢᵢ[K(PYA)]DEYCᵢᵢᵢ (SEQ ID N° : 112) ;
CᵢYLPDYLCᵢᵢGD[K(PYA)]YCᵢᵢᵢ (SEQ ID N° : 113) ;
CᵢYLPDYLCᵢᵢGDE[K(PYA)]Cᵢᵢᵢ (SEQ ID N° : 114) ;
CᵢYLPDYLCᵢᵢ[dK(PYA)]DEYCᵢᵢᵢ (SEQ ID N° : 115) ;
CᵢALTTHNCᵢᵢQWGICᵢᵢᵢ (SEQ ID N° : 116) ;
CᵢDLATHNCᵢᵢQWGICᵢᵢᵢ (SEQ ID N° : 117) ;
CᵢDLTAHNCᵢᵢQWGICᵢᵢᵢ (SEQ ID N° : 118) ;
CᵢDLTTHNCᵢᵢAWGICᵢᵢᵢ (SEQ ID N° : 119) ;
CᵢDLTTHNCᵢᵢQWGACᵢᵢᵢ (SEQ ID N° : 120) ;
CᵢNLTTHNCᵢᵢQWGICᵢᵢᵢ (SEQ ID N° : 121) ;
CᵢDLQTHNCᵢᵢQWGICᵢᵢᵢ (SEQ ID N° : 122) ;
CᵢDLTLHNCᵢᵢQWGICᵢᵢᵢ (SEQ ID N° : 123) ;
CᵢDLTT[His3Me]NCᵢᵢQWGICᵢᵢᵢ (SEQ ID N° : 124) ;
CᵢDLTT[2Pal]NCᵢᵢQWGICᵢᵢᵢ (SEQ ID N° : 125) ;
CᵢDLTT[His1Me]NCᵢᵢQWGICᵢᵢᵢ (SEQ ID N° : 126) ;
CᵢDLTT[4ThiAz]NCᵢᵢQWGICᵢᵢᵢ (SEQ ID N° : 127) ;
CᵢDLTTHNCᵢᵢVWGICᵢᵢᵢ (SEQ ID N° : 128) ;
CᵢDLTTHNCᵢᵢ[Cba]WGICᵢᵢᵢ (SEQ ID N° : 129) ;
CᵢDLTTHNCᵢᵢQ[lNal]GICᵢᵢᵢ (SEQ ID N° : 130) :
CᵢDLTTHNCᵢᵢQ[Trp(Me)]GICᵢᵢᵢ (SEQ ID N° : 131) ;
CᵢDLTTHNCᵢᵢQWG[tBuGly]Cᵢᵢᵢ (SEQ ID N° : 132) ;
CᵢDLTTHNCᵢᵢQWG[tBuAla]Cᵢᵢᵢ (SEQ ID N° : 133) ;
CᵢDLTTHNCᵢᵢQWG[Chg]Cᵢᵢᵢ (SEQ ID N° : 134) ;
CᵢDLTTHNCᵢᵢQW[dA]ICᵢᵢᵢ (SEQ ID N° : 135) ;
CᵢS[Cba]PDYLCᵢᵢ[dA]DEYCᵢᵢᵢ (SEQ ID N° : 136) ;
Cᵢ[Hser][Cba]PDYLCᵢᵢ[dA]DEYCᵢᵢᵢ (SEQ ID N° : 137) ;
CᵢN[Cba]PDYLCᵢᵢ[dA]DEYCᵢᵢᵢ (SEQ ID N° : 138) ;
C_{i[}4Pal][Cba]PDYLCᵢᵢ[dA]DEYCᵢᵢᵢ (SEQ ID N° : 139) ;
CᵢY[Cba][Cis-HyP]DYLCᵢᵢ[dA]DEYCᵢᵢᵢ (SEQ ID N° : 140) ;
CᵢY[Cba][NmeAla]DYLCᵢᵢ[dA]DEYCᵢᵢᵢ (SEQ ID N° : 141) ;
CᵢY[Cba]PNYLCᵢᵢ[dA]DEYCᵢᵢᵢ (SEQ ID N° : 142) ;
CᵢY[Cba]PQYLCᵢᵢ[dA]DEYCᵢᵢᵢ (SEQ ID N° : 143) ;
CᵢY[Cba]PDYLCᵢᵢ[dV]DEYCᵢᵢᵢ (SEQ ID N° : 144) ;
CᵢY[Cba]PDYLCᵢᵢ[dNva]DEYCᵢᵢᵢ (SEQ ID N° : 145) ;
CᵢY[Cba]PDYLCᵢᵢ[dF]DEYCᵢᵢᵢ (SEQ ID N° : 146) ;
CᵢY[Cba]PDYLCᵢᵢ[dCha]DEYCᵢᵢᵢ (SEQ ID N° : 147) ;
CᵢY[Cba]PDYLCᵢᵢ[dK(PYA)]DEYCᵢᵢᵢ (SEQ ID N° : 148) ;
CᵢY[Cba]PDYLCᵢᵢ[dNle]DEYCᵢᵢᵢ (SEQ ID N° : 149) ;
CᵢY[Cba]PD[DOPA]LCᵢᵢ[dA]DEYCᵢᵢᵢ (SEQ ID N° : 150) ;
CᵢY[Cba]PD[2FTyr]LCᵢᵢ[dA]DEYCᵢᵢᵢ (SEQ ID N° : 151) ;
CᵢY[Cba]PDY[Nle]Cᵢᵢ[dA]DEYCᵢᵢᵢ (SEQ ID N° : 152) ;
[Pen]ᵢY[Cba]PDYLCᵢᵢ[dA]DEYCᵢᵢᵢ (SEQ ID N° : 153) ;
CᵢY[Cba]PDYL[Pen]ᵢᵢ[dA]DEYCᵢᵢᵢ (SEQ ID N° : 154) ;
CᵢY[Cba]PDYLCᵢᵢ[dA]DEY[Pen]ᵢᵢᵢ (SEQ ID N° : 155) ;
CᵢY[Cba]PDYLCᵢᵢ[dA]DEY[dCᵢᵢᵢ] (SEQ ID N° : 156) ;
[dCᵢ]Y[Cba]PDYLCᵢᵢ[dA]DEYCᵢᵢᵢ (SEQ ID N° : 157) ;
CᵢDLTTHNCᵢᵢQ[AzaTrp]GICᵢᵢᵢ (SEQ ID N° : 158) ;
CᵢDLTTHNCᵢᵢQ[2MeTrp]GICᵢᵢᵢ (SEQ ID N° : 159) ;
CᵢDLTTHNCᵢᵢQ[6MeTrp]GICᵢᵢᵢ (SEQ ID N° : 160) ;
CᵢDLTTHNCᵢᵢQ[7MeTrp]GICᵢᵢᵢ (SEQ ID N° : 161) ;
CᵢDLTTHNCᵢᵢQ[6FTrp]GICᵢᵢᵢ (SEQ ID N° : 162) ;
CᵢDLTTHNCᵢᵢQ[5FTrp]GICᵢᵢᵢ (SEQ ID N° : 163) ;
CᵢDLTTHNCᵢᵢQ[Dht]GICᵢᵢᵢ (SEQ ID N° : 164) ;
CᵢDLTT[2FuAla]NCᵢᵢQWGICᵢᵢᵢ (SEQ ID N° : 165) ;
CᵢDLTTHNCᵢᵢQWG[EPA]Cᵢᵢᵢ (SEQ ID N° : 166) ;
CᵢDLTTHNCᵢᵢQWG[Nle]Cᵢᵢᵢ (SEQ ID N° : 167) ; et
CᵢDLTTHNCᵢᵢQWG[Allolle]Cᵢᵢᵢ (SEQ ID N° : 168).

3. Ligand de peptide tel que défini dans la revendication 2, dans lequel l'échafaudage moléculaire est TATA et le ligand de peptide bicyclique comprend en outre des ajouts N et/ou C-terminaux et comprend une séquence d'acides aminés qui est sélectionnée parmi :
A-(SEQ ID N° : 1)-A-Sar₆-KFI (ici dénommée BCY14654) ;
A-(SEQ ID N° : 1)-A (ici dénommée BCY14456) ;
A-(SEQ ID N° : 2)-A-Sar₆-KFI (ici dénommée BCY15125) ;
A-(SEQ ID N° : 2)-A (ici dénommée BCY15102) ;
A-(SEQ ID N° : 2)-A-[K(PYA)] (ici dénommée BCY18004) ;
A-(SEQ ID N° : 3)-A-[K(PYA)] (ici dénommée BCY15687) ;
A-(SEQ ID N° : 3)-A (ici dénommée BCY15098) ;
A-(SEQ ID N° : 4)-A-Sar₆-KFI (ici dénommée BCY15115) ;
A-(SEQ ID N° : 4)-A (ici dénommée BCY15099) ;
Ac-(SEQ ID N° : 4) (ici dénommée BCY18293) ;
Ac-A-(SEQ ID N° : 4)-A (ici dénommée BCY18294) ;
(SEQ ID N° : 4)-A (ici dénommée BCY19694) ;
K-(SEQ ID N° : 4)-A (ici dénommée BCY19695) ;
dK-(SEQ ID N° : 4)-A (ici dénommée BCY19696) ;
Dap-(SEQ ID N° : 4)-A (ici dénommée BCY19697) ;
Dab-(SEQ ID N° : 4)-A (ici dénommée BCY19698) ;
GABA-(SEQ ID N° : 4)-A (ici dénommée BCY19699) ;
A-(SEQ ID N° : 5)-A (ici dénommée BCY15103) ;
A-(SEQ ID N° :5)-A-[K(PYA)] (ici dénommée BCY18005) ;
A-(SEQ ID N° : 5)-A-Sar₆-KFI (ici dénommée BCY15118) ;
A-(SEQ ID N° : 6)-A (ici dénommée BCY15104) ;
A-(SEQ ID N° : 6)-A-Sar₆-KFI (ici dénommée BCY15119) ;
A-(SEQ ID N° : 7)-A (ici dénommée BCY15105) ;
A-(SEQ ID N° : 7)-A-Sar₆-KFI (ici dénommée BCY15120) ;
A-(SEQ ID N° : 8)-A (ici dénommée BCY15106) ;
A-(SEQ ID N° : 8)-A-Sar₆-KFI (ici dénommée BCY15121) ;
A-(SEQ ID N° : 9)-A-[dK(PYA)] (ici dénommée BCY15013) ;
A-(SEQ ID N° : 10)-A (ici dénommée BCY18470) ;
A-(SEQ ID N° :10)-A-[dK(PYA)] (ici dénommée BCY15452) ;
A-(SEQ ID N° : 11)-A (ici dénommée BCY15107) ;
Ac-(SEQ ID N° : 11) (ici dénommée BCY18260) ;
Ac-A-(SEQ ID N° : 11)-A (ici dénommée BCY18261) ;
A-(SEQ ID N° : 11)-[Aib] (ici dénommée BCY19717) ;
(SEQ ID N° : 11)-A (ici dénommée BCY19718) ;
A-(SEQ ID N° : 11)-A-[dK(PYA)] (ici dénommée BCY19719) ;
A-(SEQ ID N° : 11)-A-[K(PYA)] (ici dénommée BCY15686) ;
A-(SEQ ID N° : 12)-A (ici dénommée BCY15100) ;
Ac-(SEQ ID N° : 12) (ici dénommée BCY15634) ;
Ac-A-(SEQ ID N° : 12)-A (ici dénommée BCY15635) ;
dA-(SEQ ID N° :12) (ici dénommée BCY16134) ;
A-(SEQ ID N° : 12)-A-Sar₆-KFI (ici dénommée BCY15116) ;
A-(SEQ ID N° : 13)-A (ici dénommée BCY14455) ;
(SEQ ID N° : 13)-Sar₆-KFI (ici dénommée BCY15023) ;
Ac-(SEQ ID N° : 13)-Sar₆-KFI (ici dénommée BCY15024) ;
A-(SEQ ID N° : 13)-A-Sar₆-KFI (ici dénommée BCY14652) ;
A-(SEQ ID N° : 14)-A (ici dénommée BCY15636) ;
A-(SEQ ID N° : 15)-A (ici dénommée BCY15638) ;
A-(SEQ ID N° : 16)-A (ici dénommée BCY15639) ;
A-(SEQ ID N° : 17)-A (ici dénommée BCY15640) ;
A-(SEQ ID N° : 18)-A (ici dénommée BCY15641) ;
A-(SEQ ID N° : 19)-A (ici dénommée BCY15642) ;
A-(SEQ ID N° : 20)-A (ici dénommée BCY15643) ;
A-(SEQ ID N° : 21)-A (ici dénommée BCY15644) ;
A-(SEQ ID N° : 22)-A (ici dénommée BCY15645) ;
A-(SEQ ID N° : 23)-A (ici dénommée BCY15646) ;
A-(SEQ ID N° : 24)-A (ici dénommée BCY15648) ;
A-(SEQ ID N° : 25)-A (ici dénommée BCY15649) ;
A-(SEQ ID N° : 26)-A (ici dénommée BCY15650) ;
A-(SEQ ID N° : 27)-A (ici dénommée BCY15651) ;
A-(SEQ ID N° : 28)-A (ici dénommée BCY15653) ;
A-(SEQ ID N° : 29)-A (ici dénommée BCY15654) ;
A-(SEQ ID N° : 30)-A (ici dénommée BCY15655) ;
A-(SEQ ID N° : 31)-A (ici dénommée BCY15656) ;
A-(SEQ ID N° : 32)-A (ici dénommée BCY15657) ;
A-(SEQ ID N° : 33)-A (ici dénommée BCY15658) ;
A-(SEQ ID N° : 34)-A (ici dénommée BCY15659) ;
A-(SEQ ID N° : 35)-A (ici dénommée BCY15660) ;
A-(SEQ ID N° : 36)-A (ici dénommée BCY15661) ;
A-(SEQ ID N° : 37)-A (ici dénommée BCY15662) ;
A-(SEQ ID N° : 38)-A (ici dénommée BCY16130) ;
A-(SEQ ID N° : 39)-A (ici dénommée BCY16131) ;
A-(SEQ ID N° : 40)-A (ici dénommée BCY16132) ;
Ac-(SEQ ID N° : 40) (ici dénommée BCY16133) ;
Ac-(SEQ ID N° : 40)-[K(PYA)] (ici dénommée BCY17224) ;
A-(SEQ ID N° : 41)-A (ici dénommée BCY16135) ;
A-(SEQ ID N° : 42)-A (ici dénommée BCY16136) ;
A-(SEQ ID N° : 43)-A (ici dénommée BCY16137) ;
A-(SEQ ID N° : 44)-A (ici dénommée BCY16138) ;
A-(SEQ ID N° : 45)-A (ici dénommée BCY16139) ;
A-(SEQ ID N° : 46)-A (ici dénommée BCY16140) ;
A-(SEQ ID N° : 47)-A (ici dénommée BCY16141) ;
A-(SEQ ID N° : 48)-A (ici dénommée BCY16142) ;
A-(SEQ ID N° : 49)-A (ici dénommée BCY16143) ;
A-(SEQ ID N° : 50)-A (ici dénommée BCY16144) ;
A-(SEQ ID N° : 51)-A (ici dénommée BCY16145) ;
A-(SEQ ID N° : 52)-A-Sar₆-KFI (ici dénommée BCY15025) ;
A-(SEQ ID N° : 53)-A-Sar₆-KFI (ici dénommée BCY15028) ;
A-(SEQ ID N° : 54)-A-Sar₆-KFI (ici dénommée BCY15030) ;
A-(SEQ ID N° : 55)-A-Sar₆-KFI (ici dénommée BCY15035) ;
A-(SEQ ID N° : 56)-A-Sar₆-KFI (ici dénommée BCY15117) ;
A-(SEQ ID N° : 57)-A-Sar₆-KFI (ici dénommée BCY15122) ;
A-(SEQ ID N° : 58)-A (ici dénommée BCY15108) ;
A-(SEQ ID N° : 58)-A-Sar₆-KFI (ici dénommée BCY15123) ;
A-(SEQ ID N° : 59)-A-Sar₆-KFI (ici dénommée BCY15124) ;
A-(SEQ ID N° : 63)-A (ici dénommée BCY14454) ;
A-(SEQ ID N° : 64)-A (ici dénommée BCY18299) ;
A-(SEQ ID N° : 65)-A (ici dénommée BCY18301) ;
A-(SEQ ID N° : 66)-A (ici dénommée BCY18302) ;
A-(SEQ ID N° : 67)-A (ici dénommée BCY18303) ;
A-(SEQ ID N° : 68)-A (ici dénommée BCY18304) ;
A-(SEQ ID N° : 69)-A (ici dénommée BCY18305) ;
A-(SEQ ID N° : 70)-A (ici dénommée BCY18307) ;
A-(SEQ ID N° : 71)-A (ici dénommée BCY18309) ;
A-(SEQ ID N° : 72)-A (ici dénommée BCY18310) ;
A-(SEQ ID N° : 73)-A (ici dénommée BCY18311) ;
A-(SEQ ID N° : 74)-A (ici dénommée BCY18312) ;
A-(SEQ ID N° : 75)-A (ici dénommée BCY18313) ;
A-(SEQ ID N° : 76)-A (ici dénommée BCY18314) ;
A-(SEQ ID N° : 77)-A (ici dénommée BCY18315) ;
A-(SEQ ID N° : 78)-A (ici dénommée BCY18316) ;
A-(SEQ ID N° : 79)-A (ici dénommée BCY18317) ;
A-(SEQ ID N° : 80)-A (ici dénommée BCY18318) ;
A-(SEQ ID N° : 81)-A (ici dénommée BCY18319) ;
A-(SEQ ID N° : 82)-A (ici dénommée BCY18320) ;
A-(SEQ ID N° : 83)-A (ici dénommée BCY18321) ;
A-(SEQ ID N° : 84)-A (ici dénommée BCY18322) ;
A-(SEQ ID N° : 85)-A (ici dénommée BCY18323) ;
A-(SEQ ID N° : 86)-A (ici dénommée BCY18324) ;
A-(SEQ ID N° : 87)-A (ici dénommée BCY18325) ;
A-(SEQ ID N° : 88)-A (ici dénommée BCY18326) ;
A-(SEQ ID N° : 89)-A (ici dénommée BCY18327) ;
A-(SEQ ID N° : 90)-A (ici dénommée BCY18328) ;
A-(SEQ ID N° : 91)-A (ici dénommée BCY19700) ;
A-(SEQ ID N° : 92)-A (ici dénommée BCY19701) ;
A-(SEQ ID N° : 93)-A (ici dénommée BCY19702) ;
A-(SEQ ID N° : 94)-A (ici dénommée BCY19703) ;
A-(SEQ ID N° : 95)-A (ici dénommée BCY19704) ;
A-(SEQ ID N° : 96)-A (ici dénommée BCY19706) ;
A-(SEQ ID N° : 97)-A (ici dénommée BCY19707) ;
A-(SEQ ID N° : 98)-A (ici dénommée BCY19708) ;
A-(SEQ ID N° : 99)-A (ici dénommée BCY19710) ;
A-(SEQ ID N° : 100)-A (ici dénommée BCY19711) ;
A-(SEQ ID N° : 101)-A (ici dénommée BCY19712) ;
A-(SEQ ID N° : 102)-A (ici dénommée BCY19713) ;
A-(SEQ ID N° : 103)-A (ici dénommée BCY14457) ;
A-(SEQ ID N° : 104)-A (ici dénommée BCY14458) ;
A-(SEQ ID N° : 105)-A (ici dénommée BCY15109) ;
A-(SEQ ID N° : 106)-A (ici dénommée BCY15568) ;
A-(SEQ ID N° : 107)-A (ici dénommée BCY15637) ;
A-(SEQ ID N° : 108)-A (ici dénommée BCY15647) ;
A-(SEQ ID N° : 109)-A (ici dénommée BCY15652) ;
A-(SEQ ID N° : 110)-A (ici dénommée BCY16834) ;
A-(SEQ ID N° : 111)-A (ici dénommée BCY16837) ;
A-(SEQ ID N° : 112)-A (ici dénommée BCY16840) ;
A-(SEQ ID N° : 113)-A (ici dénommée BCY16842) ;
A-(SEQ ID N° : 114)-A (ici dénommée BCY16843) ;
A-(SEQ ID N° : 115)-A (ici dénommée BCY17662) ;
A-(SEQ ID N° : 116)-A (ici dénommée BCY18263) ;
A-(SEQ ID N° : 117)-A (ici dénommée BCY18265) ;
A-(SEQ ID N° : 118)-A (ici dénommée BCY18266) ;
A-(SEQ ID N° : 119)-A (ici dénommée BCY18269) ;
A-(SEQ ID N° : 120)-A (ici dénommée BCY18272) ;
A-(SEQ ID N° : 121)-A (ici dénommée BCY18273) ;
A-(SEQ ID N° : 122)-A (ici dénommée BCY18277) ;
A-(SEQ ID N° : 123)-A (ici dénommée BCY18278) ;
A-(SEQ ID N° : 124)-A (ici dénommée BCY18279) ;
A-(SEQ ID N° : 125)-A (ici dénommée BCY18280) ;
A-(SEQ ID N° : 126)-A (ici dénommée BCY18281) ;
A-(SEQ ID N° : 127)-A (ici dénommée BCY18282) ;
A-(SEQ ID N° : 128)-A (ici dénommée BCY18285) ;
A-(SEQ ID N° : 129)-A (ici dénommée BCY18286) ;
A-(SEQ ID N° : 130)-A (ici dénommée BCY18287) ;
A-(SEQ ID N° : 131)-A (ici dénommée BCY18288) ;
A-(SEQ ID N° : 132)-A (ici dénommée BCY18289) ;
A-(SEQ ID N° : 133)-A (ici dénommée BCY18290) ;
A-(SEQ ID N° : 134)-A (ici dénommée BCY18291) ;
A-(SEQ ID N° : 135)-A (ici dénommée BCY18292) ;
Ac-(SEQ ID N° : 136) (ici dénommée BCY18433) ;
Ac-(SEQ ID N° : 137) (ici dénommée BCY18434) ;
Ac-(SEQ ID N° : 138) (ici dénommée BCY18435) ;
Ac-(SEQ ID N° : 139) (ici dénommée BCY18437) ;
Ac-(SEQ ID N° : 140) (ici dénommée BCY18439) ;
Ac-(SEQ ID N° : 141) (ici dénommée BCY18440) ;
Ac-(SEQ ID N° : 142) (ici dénommée BCY18441) ;
Ac-(SEQ ID N° : 143) (ici dénommée BCY18442) ;
Ac-(SEQ ID N° : 144) (ici dénommée BCY18443) ;
Ac-(SEQ ID N° : 145) (ici dénommée BCY18444) ;
Ac-(SEQ ID N° : 146) (ici dénommée BCY19682) ;
Ac-(SEQ ID N° : 147) (ici dénommée BCY19683) ;
Ac-(SEQ ID N° : 148) (ici dénommée BCY19684) ;
Ac-(SEQ ID N° : 149) (ici dénommée BCY19685) ;
Ac-(SEQ ID N° : 150) (ici dénommée BCY19686) ;
Ac-(SEQ ID N° : 151) (ici dénommée BCY19687) ;
Ac-(SEQ ID N° : 152) (ici dénommée BCY19688) ;
Ac-(SEQ ID N° : 153) (ici dénommée BCY19689) ;
Ac-(SEQ ID N° : 154) (ici dénommée BCY19690) ;
Ac-(SEQ ID N° : 155) (ici dénommée BCY19691) ;
Ac-(SEQ ID N° : 156) (ici dénommée BCY19692) ;
Ac-(SEQ ID N° : 157) (ici dénommée BCY19693) ;
A-(SEQ ID N° : 158)-A (ici dénommée BCY19720) ;
A-(SEQ ID N° : 159)-A (ici dénommée BCY19721) ;
A-(SEQ ID N° : 160)-A (ici dénommée BCY19722) ;
A-(SEQ ID N° : 161)-A (ici dénommée BCY19723) ;
A-(SEQ ID N° : 162)-A (ici dénommée BCY19724) ;
A-(SEQ ID N° : 163)-A (ici dénommée BCY19725) ;
A-(SEQ ID N° : 164)-A (ici dénommée BCY19730) ;
A-(SEQ ID N° : 165)-A (ici dénommée BCY19732) ;
A-(SEQ ID N° : 166)-A (ici dénommée BCY19733) ;
A-(SEQ ID N° : 167)-A (ici dénommée BCY19734) ; et
A-(SEQ ID N° : 168)-A (ici dénommée BCY19735) ;
où PYA représente l'acide pentynoïque, Sar représente la sarcosine et FI représente la fluorescéine, Aib représente l'acide aminoisobutyrique, dK représente la D-lysine, Dap représente l'acide L-2,3-diaminopropionique, Dab représente l'acide L-2,4-diaminobutyrique, et GABA représente l'acide γ-aminobutyrique.

4. Ligand de peptide tel que défini dans la revendication 1, dans lequel ledit peptide bicyclique est spécifique pour FcyRlllA (également connu sous le nom de CD16a) et dans lequel le ligand de peptide bicyclique comprend une séquence d'acides aminés qui est :
CᵢPPTVYCᵢᵢHPLDCᵢᵢᵢ (SEQ ID N° : 60) ;
CᵢRWSVEDPCᵢᵢGAWCᵢᵢᵢ (SEQ ID N° : 61) ;
CᵢNGLEELGPCᵢᵢSDLCᵢᵢᵢ (SEQ ID N° : 62 ; ici dénommée BCY16494 lorsqu'elle est complexée avec TBMT) ;
CᵢPPAVYCᵢᵢHPLDCᵢᵢᵢ (SEQ ID N° : 169) ;
CᵢPPEVFCᵢᵢHPLDCᵢᵢᵢ (SEQ ID N° : 170) ;
CᵢPPEVYCᵢᵢHPLDCᵢᵢᵢ (SEQ ID N° : 171) ;
CᵢPPEVWCᵢᵢHPLDCᵢᵢᵢ (SEQ ID N° : 172) ;
CᵢPPQVYCᵢᵢHPLDCᵢᵢᵢ (SEQ ID N° : 173) ;
CᵢPPEVWCᵢᵢHPLDCᵢᵢᵢ (SEQ ID N° : 174) ;
CᵢRWS[tBuGly]EDPCᵢiGAWCᵢᵢᵢ (SEQ ID N° : 175) ;
CᵢRWSVED[HyP]CᵢᵢGAWCᵢᵢᵢ (SEQ ID N° : 176) ;
CᵢRWSVED[Aze]CᵢᵢGAWCᵢᵢᵢ (SEQ ID N° : 177) ;
CᵢRWSVEDPCᵢᵢ[dA]AWCᵢᵢᵢ (SEQ ID N° : 178) ;
CᵢRWSVE[Gla]PCᵢᵢGAWCᵢᵢᵢ (SEQ ID N° : 179) ;
CᵢRWSVED[Aze]CᵢᵢGAWCᵢᵢᵢ (SEQ ID N° : 180) ;
CᵢMWIRSDPCᵢᵢGAWCᵢᵢᵢ (SEQ ID N° : 181) ;
CᵢRWYPDDPCᵢᵢGAWCᵢᵢᵢ (SEQ ID N° : 182) ;
CᵢEWHISEPCᵢᵢGAWCᵢᵢᵢ (SEQ ID N° : 183) ;
CᵢNWHVYEPCᵢᵢGAWCᵢᵢᵢ (SEQ ID N° : 184) ;
CᵢRWHFSEPCᵢᵢGAWCᵢᵢᵢ (SEQ ID N° : 185) ;
CᵢRWNLDDPCᵢᵢGAWCᵢᵢᵢ (SEQ ID N° : 186) ;
CᵢMWIRSDPCᵢᵢGAWCᵢᵢᵢ (SEQ ID N° : 187) ;
CᵢRWYPDDPCᵢᵢGAWCᵢᵢᵢ (SEQ ID N° : 188) ;
CᵢEWHISEPCᵢᵢGAWCᵢᵢᵢ (SEQ ID N° : 189) ;
CᵢNWHVYEPCᵢᵢGAWCᵢᵢᵢ (SEQ ID N° : 190) ;
CᵢRWHFSEPCᵢᵢGAWCᵢᵢᵢ (SEQ ID N° : 191) ;
CᵢRWNLDDPCᵢᵢGAWCᵢᵢᵢ (SEQ ID N° : 192) ;
CᵢR[2MeTrp]SVEDPCᵢᵢGAWCᵢᵢᵢ (SEQ ID N° : 193) ;
CᵢR[6MeTrp]SVEDPCᵢᵢGAWCᵢᵢᵢ (SEQ ID N° : 194) ;
CᵢR[6FTrp]SVEDPCᵢᵢGAWCᵢᵢᵢ (SEQ ID N° : 195) ;
CᵢR[5FTrp]SVEDPCᵢᵢGAWCᵢᵢᵢ (SEQ ID N° : 196) ;
CᵢR[6CITrp]SVEDPCᵢᵢGAWCᵢᵢᵢ (SEQ ID N° : 197) ;
CᵢR[Trp(S)]SVEDPCᵢᵢGAWCᵢᵢᵢ (SEQ ID N° : 198) ;
CᵢR[AzaTrp]SVEDPCᵢᵢGAWCᵢᵢᵢ (SEQ ID N° : 199) ;
CᵢRWSVEDPCᵢᵢGA[2MeTrp]Cᵢᵢᵢ (SEQ ID N° : 200) ;
CᵢRWSVEDPCᵢᵢGA[6FTrp]Cᵢᵢᵢ (SEQ ID N° : 201) ;
CᵢRWSVEDPCᵢᵢGA[5FTrp]Cᵢᵢᵢ (SEQ ID N° : 202) ;
CᵢRWSVEDPCᵢᵢGA[4MeoTrp]Cᵢᵢᵢ (SEQ ID N° : 203) ;
CᵢRWSVEDPCᵢᵢGA[5MeoTrp]Cᵢᵢᵢ (SEQ ID N° : 204) ;
CᵢRWSVEDPCᵢᵢGA[Trp(S)]Cᵢᵢᵢ (SEQ ID N° : 205) ;
CᵢRWSVEDPCᵢᵢA[DOPA]Cᵢᵢᵢ (SEQ ID N° : 206) ;
CᵢRWYPDDPCᵢᵢGAWCᵢᵢᵢ (SEQ ID N° : 207) ;
CᵢRWHFSEPCᵢᵢGAWCᵢᵢᵢ (SEQ ID N° : 208) ;
CᵢR[2MeTrpHFSEPCᵢᵢGAWCᵢᵢᵢ (SEQ ID N°: 209) ;
CᵢRWYFSEPCᵢᵢGAWCᵢᵢᵢ (SEQ ID N° : 210) ;
CᵢRWSFSEPCᵢᵢGAWCᵢᵢᵢ (SEQ ID N° : 211) ;
CᵢRWHPSEPCᵢᵢGAWCᵢᵢᵢ (SEQ ID N° : 212) ;
CᵢRWYPSEPCᵢᵢGAWCᵢᵢᵢ (SEQ ID N° : 213) ;
CᵢRWHPDEPCᵢᵢGAWCᵢᵢᵢ (SEQ ID N° : 214) ;
CᵢRWHPEEPCᵢᵢGAWCᵢᵢᵢ (SEQ ID N° : 215) ;
CᵢRWHFDEPCᵢᵢGAWCᵢᵢᵢ (SEQ ID N° : 216) ;
CᵢRWHFDDPCᵢᵢGAWCᵢᵢᵢ (SEQ ID N° : 217) ;
CᵢRWYFDEPCᵢᵢGAWCᵢᵢᵢ (SEQ ID N° : 218) ;
CᵢRW[HislMe]FSEPCᵢᵢGAWCᵢᵢᵢ (SEQ ID N° : 219) ;
CᵢRW[His3Me]FSEPCᵢᵢGAWCᵢᵢᵢ (SEQ ID N° : 220) ;
CᵢRW[4MeoPhe]FSEPCᵢᵢGAWCᵢᵢᵢ (SEQ ID N° : 221) ;
CᵢRW[DOPA]FSEPCᵢᵢGAWCᵢᵢᵢ (SEQ ID N° : 222) ;
CᵢRW[26DiMeTyr]FSEPCᵢᵢGAWCᵢᵢᵢ (SEQ ID N° : 223) ;
CᵢRW[3tBuTyr] FSEPCᵢᵢGAWCᵢᵢᵢ (SEQ ID N° : 224) ;
CᵢRW[4FPhe]FSEPCᵢᵢGAWCᵢᵢᵢ (SEQ ID N° : 225) ;
CᵢRWH[4MePhe]SEPCᵢᵢGAWCᵢᵢᵢ (SEQ ID N° : 226) ;
CᵢRWH[Aze]SEPCᵢᵢGAWCᵢᵢᵢ (SEQ ID N° : 227) ;
CᵢRWH[HyP]SEPCᵢᵢGAWCᵢᵢᵢ (SEQ ID N° : 228) ;
CᵢRWH[4tBuPhe]SEPCᵢᵢGAWCᵢᵢᵢ (SEQ ID N° : 229) ;
CᵢRWH[HPhe]SEPCᵢᵢGAWCᵢᵢᵢ (SEQ ID N° : 230, ici dénommée BCY21693 lorsqu'elle est complexée avec TBMT) ;
CᵢRWH[4PhePro]SEPCᵢᵢGAWCᵢᵢᵢ (SEQ ID N° : 231) ;
CᵢRWHF[HSer]EPCᵢᵢGAWCᵢᵢᵢ (SEQ ID N° : 232) ;
CᵢRWHFS[Gla]PCᵢᵢGAWCᵢᵢᵢ (SEQ ID N° : 233) ;
CᵢRWHFS[HGlu]PCᵢᵢGAWCᵢᵢᵢ (SEQ ID N° : 234) ;
CᵢRWHFSEPCᵢᵢ[dA]AWCᵢᵢᵢ (SEQ ID N° : 235) ;
CᵢRWYFSE[Aze]Cᵢi[dA]AWCᵢᵢᵢ (SEQ ID N° : 236) ;
CᵢRWHFDE[Aze]Cᵢᵢ[dA]AWCᵢᵢᵢ (SEQ ID N° : 237) ;
CᵢRWYPSE[Aze]Cᵢᵢ[dA]AWCᵢᵢᵢ (SEQ ID N° : 238) ;
Cᵢ[HArg]WHFSEPCᵢᵢGAWCᵢᵢᵢ (SEQ ID N° : 239) ;
CᵢRWH[Cis-HyP]SEPCᵢᵢGAWCᵢᵢᵢ (SEQ ID N° : 240) ;
CᵢRWHFSEPCᵢᵢGAWCᵢᵢᵢ (SEQ ID N° : 241) ;
CᵢRWHFSEPCᵢᵢGAWCᵢᵢᵢ (SEQ ID N° : 242) ;
CᵢRWHFSEPCᵢᵢGAWCᵢᵢᵢ (SEQ ID N° : 243) ;
CᵢVGLLELGPCᵢᵢSDLCᵢᵢᵢ (SEQ ID N° : 244) ;
CᵢPGLEELGPCᵢᵢSDLCᵢᵢᵢ (SEQ ID N° : 245) ;
CᵢV[dA]LEELGPCᵢᵢSDLCᵢᵢᵢ (SEQ ID N° : 246) ;
CᵢVG[tBuAla]EELGPCᵢᵢSDLCᵢᵢᵢ (SEQ ID N° : 247) ;
CᵢVG[Cba]EELGPCᵢᵢSDLCᵢᵢᵢ (SEQ ID N° : 248) ;
CᵢGFEELGPCᵢᵢSDLCᵢᵢᵢ (SEQ ID N° : 249) ;
CᵢVG[lNal]EELGPCᵢᵢSDLCᵢᵢᵢ (SEQ ID N° : 250) ;
CᵢVG[2Nal]EELGPCᵢᵢSDLCᵢᵢᵢ (SEQ ID N° : 251) ;
CᵢVGLPELGPCᵢᵢSDLCᵢᵢᵢ (SEQ ID N° : 252) ;
CᵢVGL[tBuAla]ELGPCᵢᵢSDLCᵢᵢᵢ (SEQ ID N° : 253) ;
CᵢVGL[Cba]ELGPCᵢᵢSDLCᵢᵢᵢ (SEQ ID N° : 254) ;
CᵢVGL[Cha]ELGPCᵢᵢSDLCᵢᵢᵢ (SEQ ID N° : 255) ;
CᵢVGL[Nle]ELGPCᵢᵢSDLCᵢᵢᵢ (SEQ ID N° : 256) ;
CᵢVGLE[Gla]LGPCᵢᵢSDLCᵢᵢᵢ (SEQ ID N° : 257) ;
CᵢVGLEELG[Aze]CᵢᵢSDLCᵢᵢᵢ (SEQ ID N° : 258) ;
CᵢVGLEELGPCᵢᵢSD[BuAla]Cᵢᵢᵢ (SEQ ID N° : 259) ;
CᵢVGLEELGPCᵢᵢSD[Cba]Cᵢᵢᵢ (SEQ ID N° : 260) ;
CᵢVGLEELGPCᵢᵢSD[Cha]Cᵢᵢᵢ (SEQ ID N° : 261) ;
CᵢVG[HLeu]EELGPCᵢᵢSDLCᵢᵢᵢ (SEQ ID N° : 262) ;
CᵢVGL[HLeu]ELGPCᵢᵢSDLCᵢᵢᵢ (SEQ ID N° : 263) ;
CᵢVGLE[HGlu]LGPCᵢᵢSDLCᵢᵢᵢ (SEQ ID N° : 264) ;
CᵢTWPYCᵢᵢKSWGDVQDCᵢᵢᵢ (SEQ ID N° : 265) ;
CᵢDWWDPGCᵢᵢTYFCᵢᵢᵢ (SEQ ID N° : 266) ;
CᵢRWH[4BnPro]SEPCᵢᵢGAWCᵢᵢᵢ (SEQ ID N° : 267) ; et
CᵢRWH[HPhe]SE[Aze]CᵢᵢGAWCᵢᵢᵢ (SEQ ID N° : 268).

5. Ligand de peptide tel que défini dans la revendication 4, dans lequel l'échafaudage moléculaire est TATB et le ligand de peptide bicyclique comprend en outre des ajouts N et/ou C-terminaux et comprend une séquence d'acides aminés qui est sélectionnée parmi :
A-(SEQ ID N° : 265)-A (ici dénommée BCY15044) ;
A-(SEQ ID N° : 265)-AGAAAE (ici dénommée BCY19416) ;
A-(SEQ ID N° : 266)-A (ici dénommée BCY15045) ; et
A-(SEQ ID N° : 266)-AGAAAE (ici dénommée BCY19417).

6. Ligand de peptide tel que défini dans la revendication 4, dans lequel l'échafaudage moléculaire est TATA et le ligand de peptide bicyclique comprend en outre des ajouts N et/ou C-terminaux et comprend une séquence d'acides aminés qui est sélectionnée parmi :
A-(SEQ ID N° : 60)-A (ici dénommée BCY11808) ;
A-(SEQ ID N° : 60)-A-[K(PYA)] (ici dénommée BCY15685) ;
A-(SEQ ID N° : 60)-A-[Sar6]-[KBiot] (ici dénommée BCY12621) ;
A-(SEQ ID N° : 169)-A (ici dénommée BCY11673) ;
A-(SEQ ID N° : 170)-A (ici dénommée BCY11809) ;
A-(SEQ ID N° : 171)-A (ici dénommée BCY11810) ;
A-(SEQ ID N° : 172)-A (ici dénommée BCY11811) ;
A-(SEQ ID N° : 173)-A (ici dénommée BCY11812) ; et
A-(SEQ ID N° : 174)-A-[Sar6]-[KBiot] (ici dénommée BCY12622) ;
où Sar représente la sarcosine et Biot représente la biotine.

7. Ligand de peptide tel que défini dans la revendication 4, dans lequel l'échafaudage moléculaire est TBMT et le ligand de peptide bicyclique comprend en outre des ajouts N et/ou C-terminaux et comprend une séquence d'acides aminés qui est sélectionnée parmi :
A-(SEQ ID N° : 61)-A (ici dénommée BCY14950) ;
Ac-A-(SEQ ID N° : 61)-A (ici dénommée BCY16599) ;
A-(SEQ ID N° : 61)-AGAAAE (ici dénommée BCY19040) ;
A-(SEQ ID N° : 61)-A-[K(PYA)] (ici dénommée BCY13883) ;
Ac-A-(SEQ ID N° : 61)-A-[K(PYA)] (ici dénommée BCY19596) ;
A-(SEQ ID N° : 62)-A (ici dénommée BCY16493) ;
Ac-(SEQ ID N° : 62) (ici dénommée BCY16495) ;
A-(SEQ ID N° : 62)-A-[K(PYA)] (ici dénommée BCY13886) ;
A-(SEQ ID N° : 175)-A (ici dénommée BCY14953) ;
A-(SEQ ID N° : 176)-A (ici dénommée BCY14954) ;
[PYA]-A-(SEQ ID N° : 177) (ici dénommée BCY14955) ;
A-(SEQ ID N° : 178)-A (ici dénommée BCY14956) ;
A-(SEQ ID N° : 179)-A (ici dénommée BCY16608) ;
A-(SEQ ID N° : 180)-A (ici dénommée BCY16610) ;
A-(SEQ ID N° : 181)-A (ici dénommée BCY19032) ;
A-(SEQ ID N° : 182)-A (ici dénommée BCY19033) ;
A-(SEQ ID N° : 183)-A (ici dénommée BCY19034) ;
A-(SEQ ID N° : 184)-A (ici dénommée BCY19035) ;
A-(SEQ ID N° : 185)-A (ici dénommée BCY19036) ;
A-(SEQ ID N° : 186)-A (ici dénommée BCY19037) ;
A-(SEQ ID N° : 187)-AGAAAE (ici dénommée BCY19038) ;
A-(SEQ ID N° : 188)-AGAAAE (ici dénommée BCY19039) ;
A-(SEQ ID N° : 189)-AGAAAE (ici dénommée BCY19041) ;
A-(SEQ ID N° : 190)-AGAAAE (ici dénommée BCY19042) ;
A-(SEQ ID N° : 191)-AGAAAE (ici dénommée BCY19043) ;
A-(SEQ ID N° : 192)-AGAAAE (ici dénommée BCY19044) ;
A-(SEQ ID N° : 193)-A (ici dénommée BCY19045) ;
A-(SEQ ID N° : 194)-A (ici dénommée BCY19046) ;
A-(SEQ ID N° : 195)-A (ici dénommée BCY19048) ;
A-(SEQ ID N° : 196)-A (ici dénommée BCY19049) ;
A-(SEQ ID N° : 197)-A (ici dénommée BCY19050) ;
A-(SEQ ID N° : 198)-A (ici dénommée BCY19053) ;
A-(SEQ ID N° : 199)-A (ici dénommée BCY19055) ;
A-(SEQ ID N° : 200)-A (ici dénommée BCY19057) ;
A-(SEQ ID N° : 201)-A (ici dénommée BCY19060) ;
A-(SEQ ID N° : 202)-A (ici dénommée BCY19061) ;
A-(SEQ ID N° : 203)-A (ici dénommée BCY19063) ;
A-(SEQ ID N° : 204)-A (ici dénommée BCY19064) ;
A-(SEQ ID N° : 205)-A (ici dénommée BCY19065) ;
A-(SEQ ID N° : 206)-A (ici dénommée BCY19068) ;
A-(SEQ ID N° : 207)-A-[K(PYA)] (ici dénommée BCY20360) ;
A-(SEQ ID N° : 208)-A-[K(PYA)] (ici dénommée BCY20361) ;
A-(SEQ ID N° : 209)-A (ici dénommée BCY20363) ;
A-(SEQ ID N° : 210)-A (ici dénommée BCY20364) ;
A-(SEQ ID N° : 211)-A (ici dénommée BCY20365) ;
A-(SEQ ID N° : 212)-A (ici dénommée BCY20366) ;
A-(SEQ ID N° : 213)-A (ici dénommée BCY20367) ;
A-(SEQ ID N° : 214)-A (ici dénommée BCY20368) ;
A-(SEQ ID N° : 215)-A (ici dénommée BCY20369) ;
A-(SEQ ID N° : 216)-A (ici dénommée BCY20370) ;
A-(SEQ ID N° : 217)-A (ici dénommée BCY20371) ;
A-(SEQ ID N° : 218)-A (ici dénommée BCY20372) ;
A-(SEQ ID N° : 219)-A (ici dénommée BCY20373) ;
A-(SEQ ID N° : 220)-A (ici dénommée BCY20374) ;
A-(SEQ ID N° : 221)-A (ici dénommée BCY20375) ;
A-(SEQ ID N° : 222)-A (ici dénommée BCY20376) ;
A-(SEQ ID N° : 223)-A (ici dénommée BCY20377) ;
A-(SEQ ID N° : 224)-A (ici dénommée BCY20378) ;
A-(SEQ ID N° : 225)-A (ici dénommée BCY20379) ;
A-(SEQ ID N° : 226)-A (ici dénommée BCY20380) ;
A-(SEQ ID N° : 227)-A (ici dénommée BCY20382) ;
A-(SEQ ID N° : 228)-A (ici dénommée BCY20383) ;
A-(SEQ ID N° : 229)-A (ici dénommée BCY20384) ;
A-(SEQ ID N° : 230)-A (ici dénommée BCY20385) ;
(SEQ ID N° : 230)-A (ici dénommée BCY21690) ;
Ac-(SEQ ID N° : 230)-A (ici dénommée BCY21691) ;
A-(SEQ ID N° : 230) (ici dénommée BCY21692) ;
Ac-(SEQ ID N° : 230) (ici dénommée BCY21694) ;
[Nle]-(SEQ ID N° : 230)-A (ici dénommée BCY21695) ;
Ac-[Nle]-(SEQ ID N° : 230)-A (ici dénommée BCY21696) ;
Ac-A-(SEQ ID N° : 230)-A (ici dénommée BCY21697) ;
A-(SEQ ID N° : 231)-A (ici dénommée BCY20386) ;
A-(SEQ ID N° : 232)-A (ici dénommée BCY20388) ;
A-(SEQ ID N° : 233)-A (ici dénommée BCY20389) ;
A-(SEQ ID N° : 234)-A (ici dénommée BCY20390) ;
A-(SEQ ID N° : 235)-A (ici dénommée BCY20392) ;
A-(SEQ ID N° : 236)-A (ici dénommée BCY20394) ;
A-(SEQ ID N° : 237)-A (ici dénommée BCY20395) ;
A-(SEQ ID N° : 238)-A (ici dénommée BCY20396) ;
A-(SEQ ID N° : 239)-A (ici dénommée BCY20397) ;
A-(SEQ ID N° : 240)-A (ici dénommée BCY20526) ;
Ac-A-(SEQ ID N° : 241)-A (ici dénommée BCY20527) ;
[Aib]-(SEQ ID N° : 242)-A (ici dénommée BCY20528) ;
A-(SEQ ID N° : 243)-[Aib] (ici dénommée BCY20529) ;
A-(SEQ ID N° : 244)-A-[K(PYA)] (ici dénommée BCY16165) ;
A-(SEQ ID N° : 245)-A (ici dénommée BCY16498) ;
A-(SEQ ID N° : 246)-A (ici dénommée BCY16499) ;
A-(SEQ ID N° : 247)-A (ici dénommée BCY16501) ;
A-(SEQ ID N° : 248)-A (ici dénommée BCY16502) ;
A-(SEQ ID N° : 249)-A (ici dénommée BCY16504) ;
A-(SEQ ID N° : 250)-A (ici dénommée BCY16505) ;
A-(SEQ ID N° : 251)-A (ici dénommée BCY16506) ;
A-(SEQ ID N° : 252)-A (ici dénommée BCY16507) ;
A-(SEQ ID N° : 253)-A (ici dénommée BCY16508) ;
A-(SEQ ID N° : 254)-A (ici dénommée BCY16509) ;
A-(SEQ ID N° : 255)-A (ici dénommée BCY16510) ;
A-(SEQ ID N° : 256)-A (ici dénommée BCY16511) ;
A-(SEQ ID N° : 257)-A (ici dénommée BCY16512) ;
A-(SEQ ID N° : 258)-A (ici dénommée BCY16520) ;
A-(SEQ ID N° : 259)-A (ici dénommée BCY16522) ;
A-(SEQ ID N° : 260)-A (ici dénommée BCY16523) ;
A-(SEQ ID N° : 261)-A (ici dénommée BCY16524) ;
A-(SEQ ID N° : 262)-A (ici dénommée BCY16527) ;
A-(SEQ ID N° : 263)-A (ici dénommée BCY16528) ;
A-(SEQ ID N° : 264)-A (ici dénommée BCY16529) ;
A-(SEQ ID N° : 267)-A (ici dénommée BCY20387) ; et
A-(SEQ ID N° : 268)-A (ici dénommée BCY21689),
où PYA représente l'acide pentynoïque et Aib représente l'acide aminoisobutryque.

8. Ligand de peptide tel que défini dans l'une quelconque des revendications 1 à 7, dans lequel le sel pharmaceutiquement acceptable est sélectionné parmi l'acide libre ou le sel de sodium, de potassium, de calcium ou d'ammonium.

9. Complexe de liaison multimérique qui comprend au moins deux ligands de peptide bicyclique tels que définis dans l'une quelconque des revendications 1 à 8, dans lequel lesdits ligands de peptide peuvent être identiques ou différents.

10. Complexe de liaison multimérique tel que défini dans la revendication 9, qui comprend un homodimère NKp46 sélectionné parmi BCY15663, BCY15921, BCY15922, BCY15455 et BCY18043.

11. Complexe de liaison multimérique tel que défini dans la revendication 9, qui comprend un homodimère CD16a sélectionné parmi BCY15914, BCY15915, et BCY15916.

12. Composition pharmaceutique qui comprend le ligand de peptide tel que défini dans l'une quelconque des revendications 1 à 8 ou le complexe de liaison multimérique tel que défini dans l'une quelconque des revendications 9 à 11, en combinaison avec un ou plusieurs excipients pharmaceutiquement acceptables.

13. Ligand de peptide tel que défini dans l'une quelconque des revendications 1 à 8, ou complexe de liaison multimérique tel que défini dans l'une quelconque des revendications 9 à 11, ou composition pharmaceutique de la revendication 12, destiné à être utilisé dans une thérapie.

14. Ligand de peptide tel que défini dans l'une quelconque des revendications 1 à 8, ou complexe de liaison multimérique tel que défini dans l'une quelconque des revendications 9 à 11, ou composition pharmaceutique de la revendication 12, destiné à être utilisé dans la prévention, la suppression ou le traitement d'une maladie ou d'un trouble médié par les cellules tueuses naturelles (NK), tel que des troubles inflammatoires, une maladie auto-immune et le cancer.
